(19) Europäisches Patentamt
European Patent Office
Office européen des brevets

(11) EP 3 446 700 A1

# (12) EUROPEAN PATENT APPLICATION

(43) Date of publication:
**27.02.2019 Bulletin 2019/09**

(51) Int Cl.:
***A61K 38/37*** *(2006.01)*

(21) Application number: **18174446.7**

(22) Date of filing: **30.10.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**30.10.2012 US 201261720356 P**
**01.02.2013 US 201361759880 P**
**15.03.2013 US 201361800293 P**
**29.04.2013 US 201361817085 P**
**31.05.2013 US 201361829884 P**
**26.06.2013 US 201361839477 P**
**08.08.2013 US 201361863860 P**
**12.09.2013 US 201361876927 P**
**19.09.2013 US 201361879955 P**

(62) Document number(s) of the earlier application(s) in accordance with Art. 76 EPC:
**13852306.3 / 2 914 293**

(71) Applicant: **Bioverativ Therapeutics Inc.**
**Waltham MA 02451 (US)**

(72) Inventors:
- **JIANG, Haiyan**
  **Belmont, MA Massachusetts 02478 (US)**
- **NEELAKANTAN, Srividya**
  **East Walpole, MA Massachusetts 02032 (US)**
- **NESTOROV, Ivan**
  **Acton, MA Massachusetts 01720 (US)**
- **LI, Shuanglian**
  **Lexington, MA Massachusetts 02420 (US)**

(74) Representative: **Miller, David James et al**
**Mathys & Squire LLP**
**The Shard**
**32 London Bridge Street**
**London SE1 9SG (GB)**

Remarks:
•This application was filed on 25-05-2018 as a divisional application to the application mentioned under INID code 62.
•Claims filed after the date of receipt of the divisional application (Rule 68(4) EPC).

## (54) METHODS OF USING FVIII POLYPEPTIDE

(57) The present invention provides methods of reducing or decreasing the annualized bleeding rate of a human subject having hemophilia by administering a long acting Factor VIII polypeptide. The long-acting FVIII polypeptide can be used for individual prophylaxis, weekly prophylaxis, episodic (on-demand) treatment, or perioperative management of hemophilia.

EP 3 446 700 A1

SEQUENCE LISTING

```
<110>  BIOGEN IDEC MA INC.
       Jiang, Haiyan
       Neelakantan, Srividya
       Nestorov, Ivan
       Li, Shuanglian

<120>  METHODS OF USING FVIII POLYPEPTIDE

<130>  2159.406PC09/EKS/C-K/CMC

<140>  To be assigned
<141>  2013-10-30

<150>  61/720,356
<151>  2012-10-30

<150>  61/759,880
<151>  2013-02-01

<150>  61/800,293
<151>  2013-03-15

<150>  61/817,085
<151>  2013-04-29

<150>  61/829,884
<151>  2013-05-31

<150>  61/839,477
<151>  2013-06-26

<150>  61/863,860
<151>  2013-08-08

<150>  61/876,927
<151>  2013-09-12

<150>  61/879,955
<151>  2013-09-19

<160>  18

<170>  PatentIn version 3.5

<210>  1
<211>  5052
<212>  DNA
<213>  Artificial Sequence

<220>
<223>  B-Domain Deleted FVIIIFc

<400>  1
atgcaaatag agctctccac ctgcttcttt ctgtgccttt tgcgattctg ctttagtgcc      60

accagaagat actacctggg tgcagtggaa ctgtcatggg actatatgca aagtgatctc     120

ggtgagctgc ctgtggacgc aagatttcct cctagagtgc caaaatcttt tccattcaac     180

acctcagtcg tgtacaaaaa gactctgttt gtagaattca cggatcacct tttcaacatc     240
```

gctaagccaa ggccaccctg gatgggtctg ctaggtccta ccatccaggc tgaggtttat 300
gatacagtgg tcattacact taagaacatg gcttcccatc ctgtcagtct tcatgctgtt 360
ggtgtatcct actggaaagc ttctgaggga gctgaatatg atgatcagac cagtcaaagg 420
gagaaagaag atgataaagt cttccctggt ggaagccata catatgtctg gcaggtcctg 480
aaagagaatg gtccaatggc ctctgaccca ctgtgcctta cctactcata tctttctcat 540
gtggacctgg taaaagactt gaattcaggc ctcattggag ccctactagt atgtagagaa 600
gggagtctgg ccaaggaaaa gacacagacc ttgcacaaat ttatactact tttgctgta 660
tttgatgaag ggaaaagttg gcactcagaa acaaagaact ccttgatgca ggatagggat 720
gctgcatctg ctcgggcctg gcctaaaatg cacacagtca atggttatgt aaacaggtct 780
ctgccaggtc tgattggatg ccacaggaaa tcagtctatt ggcatgtgat tggaatgggc 840
accactcctg aagtgcactc aatattcctc gaaggtcaca catttcttgt gaggaaccat 900
cgccaggcgt ccttggaaat ctcgccaata actttcctta ctgctcaaac actcttgatg 960
gaccttggac agtttctact gttttgtcat atctcttccc accaacatga tggcatggaa 1020
gcttatgtca aagtagacag ctgtccagag gaaccccaac tacgaatgaa aaataatgaa 1080
gaagcggaag actatgatga tgatcttact gattctgaaa tggatgtggt caggtttgat 1140
gatgacaact ctccttcctt tatccaaatt cgctcagttg ccaagaagca tcctaaaact 1200
tgggtacatt acattgctgc tgaagaggag gactgggact atgctccctt agtcctcgcc 1260
cccgatgaca gaagttataa aagtcaatat ttgaacaatg gccctcagcg gattggtagg 1320
aagtacaaaa aagtccgatt tatggcatac acagatgaaa cctttaagac tcgtgaagct 1380
attcagcatg aatcaggaat cttgggacct ttactttatg gggaagttgg agacacactg 1440
ttgattatat ttaagaatca agcaagcaga ccatataaca tctaccctca cggaatcact 1500
gatgtccgtc ctttgtattc aaggagatta ccaaaaggtg taaaacattt gaaggatttt 1560
ccaattctgc caggagaaat attcaaatat aaatggacag tgactgtaga agatgggcca 1620
actaaatcag atcctcggtg cctgacccgc tattactcta gtttcgttaa tatggagaga 1680
gatctagctt caggactcat tggccctctc ctcatctgct acaaagaatc tgtagatcaa 1740
agaggaaacc agataatgtc agacaagagg aatgtcatcc tgttttctgt atttgatgag 1800
aaccgaagct ggtacctcac agagaatata caacgctttc tccccaatcc agctggagtg 1860
cagcttgagg atccagagtt ccaagcctcc aacatcatgc acagcatcaa tggctatgtt 1920
tttgatagtt tgcagttgtc agtttgtttg catgaggtgg catactggta cattctaagc 1980
attggagcac agactgactt cctttctgtc ttcttctctg gatatacctt caaacacaaa 2040
atggtctatg aagacacact caccctattc ccattctcag gagaaactgt cttcatgtcg 2100
atggaaaacc caggtctatg gattctgggg tgccacaact cagactttcg gaacagaggc 2160

atgaccgcct tactgaaggt ttctagttgt gacaagaaca ctggtgatta ttacgaggac 2220

agttatgaag atatttcagc atacttgctg agtaaaaaca atgccattga accaagaagc 2280

ttctctcaaa acccaccagt cttgaaacgc catcaacggg aaataactcg tactactctt 2340

cagtcagatc aagaggaaat tgactatgat gataccatat cagttgaaat gaagaaggaa 2400

gattttgaca tttatgatga ggatgaaaat cagagccccc gcagctttca aaagaaaaca 2460

cgacactatt ttattgctgc agtggagagg ctctgggatt atgggatgag tagctcccca 2520

catgttctaa gaaacagggc tcagagtggc agtgtccctc agttcaagaa agttgttttc 2580

caggaattta ctgatggctc ctttactcag cccttatacc gtggagaact aaatgaacat 2640

ttgggactcc tggggccata tataagagca gaagttgaag ataatatcat ggtaactttc 2700

agaaatcagg cctctcgtcc ctattccttc tattctagcc ttatttctta tgaggaagat 2760

cagaggcaag gagcagaacc tagaaaaaac tttgtcaagc ctaatgaaac caaaacttac 2820

ttttggaaag tgcaacatca tatggcaccc actaaagatg agtttgactg caaagcctgg 2880

gcttatttct ctgatgttga cctggaaaaa gatgtgcact caggcctgat tggacccctt 2940

ctggtctgcc acactaacac actgaaccct gctcatggga gacaagtgac agtacaggaa 3000

tttgctctgt ttttcaccat ctttgatgag accaaaagct ggtacttcac tgaaaatatg 3060

gaaagaaact gcagggctcc ctgcaatatc cagatggaag atcccacttt taaagagaat 3120

tatcgcttcc atgcaatcaa tggctacata atggatacac tacctggctt agtaatggct 3180

caggatcaaa ggattcgatg gtatctgctc agcatgggca gcaatgaaaa catccattct 3240

attcatttca gtggacatgt gttcactgta cgaaaaaaag aggagtataa aatggcactg 3300

tacaatctct atccaggtgt ttttgagaca gtggaaatgt taccatccaa agctggaatt 3360

tggcgggtgg aatgccttat tggcgagcat ctacatgctg ggatgagcac actttttctg 3420

gtgtacagca ataagtgtca gactcccctg ggaatggctt ctggacacat tagagatttt 3480

cagattacag cttcaggaca atatggacag tgggccccaa agctggccag acttcattat 3540

tccggatcaa tcaatgcctg gagcaccaag gagccctttt cttggatcaa ggtggatctg 3600

ttggcaccaa tgattattca cggcatcaag acccagggtg cccgtcagaa gttctccagc 3660

ctctacatct ctcagtttat catcatgtat agtcttgatg ggaagaagtg gcagacttat 3720

cgaggaaatt ccactggaac cttaatggtc ttctttggca atgtggattc atctgggata 3780

aaacacaata tttttaaccc tccaattatt gctcgataca tccgtttgca cccaactcat 3840

tatagcattc gcagcactct tcgcatggag ttgatgggct gtgatttaaa tagttgcagc 3900

atgccattgg gaatggagag taaagcaata tcagatgcac agattactgc ttcatcctac 3960

tttaccaata tgtttgccac ctggtctcct tcaaaagctc gacttcacct ccaagggagg 4020

agtaatgcct ggagacctca ggtgaataat ccaaaagagt ggctgcaagt ggacttccag 4080

aagacaatga aagtcacagg agtaactact cagggagtaa aatctctgct taccagcatg 4140

tatgtgaagg agttcctcat ctccagcagt caagatggcc atcagtggac tctctttttt 4200

cagaatggca aagtaaaggt ttttcaggga aatcaagact ccttcacacc tgtggtgaac 4260

tctctagacc caccgttact gactcgctac cttcgaattc acccccagag ttgggtgcac 4320

cagattgccc tgaggatgga ggttctgggc tgcgaggcac aggacctcta cgacaaaact 4380

cacacatgcc caccgtgccc agctccagaa ctcctgggcg gaccgtcagt cttcctcttc 4440

cccccaaaac ccaaggacac cctcatgatc tcccggaccc ctgaggtcac atgcgtggtg 4500

gtggacgtga gccacgaaga ccctgaggtc aagttcaact ggtacgtgga cggcgtggag 4560

gtgcataatg ccaagacaaa gccgcgggag gagcagtaca acagcacgta ccgtgtggtc 4620

agcgtcctca ccgtcctgca ccaggactgg ctgaatggca aggagtacaa gtgcaaggtc 4680

tccaacaaag ccctcccagc ccccatcgag aaaaccatct ccaaagccaa agggcagccc 4740

cgagaaccac aggtgtacac cctgccccca tcccgggatg agctgaccaa gaaccaggtc 4800

agcctgacct gcctggtcaa aggcttctat cccagcgaca tcgccgtgga gtgggagagc 4860

aatgggcagc cggagaacaa ctacaagacc acgcctcccg tgttggactc cgacggctcc 4920

ttcttcctct acagcaagct caccgtggac aagagcaggt ggcagcaggg gaacgtcttc 4980

tcatgctccg tgatgcatga ggctctgcac aaccactaca cgcagaagag cctctccctg 5040

tctccgggta aa 5052

<210> 2
<211> 1684
<212> PRT
<213> Artificial Sequence

<220>
<223> B-Domain Deleted FVIIIFc

<400> 2

Met Gln Ile Glu Leu Ser Thr Cys Phe Phe Leu Cys Leu Leu Arg Phe
1               5                   10                  15

Cys Phe Ser Ala Thr Arg Arg Tyr Tyr Leu Gly Ala Val Glu Leu Ser
            20                  25                  30

Trp Asp Tyr Met Gln Ser Asp Leu Gly Glu Leu Pro Val Asp Ala Arg
        35                  40                  45

Phe Pro Pro Arg Val Pro Lys Ser Phe Pro Phe Asn Thr Ser Val Val
    50                  55                  60

```
Tyr Lys Lys Thr Leu Phe Val Glu Phe Thr Asp His Leu Phe Asn Ile
65                  70                  75                  80

Ala Lys Pro Arg Pro Pro Trp Met Gly Leu Leu Gly Pro Thr Ile Gln
                85                  90                  95

Ala Glu Val Tyr Asp Thr Val Val Ile Thr Leu Lys Asn Met Ala Ser
            100                 105                 110

His Pro Val Ser Leu His Ala Val Gly Val Ser Tyr Trp Lys Ala Ser
        115                 120                 125

Glu Gly Ala Glu Tyr Asp Asp Gln Thr Ser Gln Arg Glu Lys Glu Asp
    130                 135                 140

Asp Lys Val Phe Pro Gly Gly Ser His Thr Tyr Val Trp Gln Val Leu
145                 150                 155                 160

Lys Glu Asn Gly Pro Met Ala Ser Asp Pro Leu Cys Leu Thr Tyr Ser
                165                 170                 175

Tyr Leu Ser His Val Asp Leu Val Lys Asp Leu Asn Ser Gly Leu Ile
            180                 185                 190

Gly Ala Leu Leu Val Cys Arg Glu Gly Ser Leu Ala Lys Glu Lys Thr
        195                 200                 205

Gln Thr Leu His Lys Phe Ile Leu Leu Phe Ala Val Phe Asp Glu Gly
    210                 215                 220

Lys Ser Trp His Ser Glu Thr Lys Asn Ser Leu Met Gln Asp Arg Asp
225                 230                 235                 240

Ala Ala Ser Ala Arg Ala Trp Pro Lys Met His Thr Val Asn Gly Tyr
                245                 250                 255

Val Asn Arg Ser Leu Pro Gly Leu Ile Gly Cys His Arg Lys Ser Val
            260                 265                 270

Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300

Leu Glu Ile Ser Pro Ile Thr Phe Leu Thr Ala Gln Thr Leu Leu Met
305                 310                 315                 320
```

Asp Leu Gly Gln Phe Leu Leu Phe Cys His Ile Ser Ser His Gln His
325 330 335

Asp Gly Met Glu Ala Tyr Val Lys Val Asp Ser Cys Pro Glu Glu Pro
340 345 350

Gln Leu Arg Met Lys Asn Asn Glu Glu Ala Glu Asp Tyr Asp Asp Asp
355 360 365

Leu Thr Asp Ser Glu Met Asp Val Val Arg Phe Asp Asp Asp Asn Ser
370 375 380

Pro Ser Phe Ile Gln Ile Arg Ser Val Ala Lys Lys His Pro Lys Thr
385 390 395 400

Trp Val His Tyr Ile Ala Ala Glu Glu Glu Asp Trp Asp Tyr Ala Pro
405 410 415

Leu Val Leu Ala Pro Asp Asp Arg Ser Tyr Lys Ser Gln Tyr Leu Asn
420 425 430

Asn Gly Pro Gln Arg Ile Gly Arg Lys Tyr Lys Lys Val Arg Phe Met
435 440 445

Ala Tyr Thr Asp Glu Thr Phe Lys Thr Arg Glu Ala Ile Gln His Glu
450 455 460

Ser Gly Ile Leu Gly Pro Leu Leu Tyr Gly Glu Val Gly Asp Thr Leu
465 470 475 480

Leu Ile Ile Phe Lys Asn Gln Ala Ser Arg Pro Tyr Asn Ile Tyr Pro
485 490 495

His Gly Ile Thr Asp Val Arg Pro Leu Tyr Ser Arg Arg Leu Pro Lys
500 505 510

Gly Val Lys His Leu Lys Asp Phe Pro Ile Leu Pro Gly Glu Ile Phe
515 520 525

Lys Tyr Lys Trp Thr Val Thr Val Glu Asp Gly Pro Thr Lys Ser Asp
530 535 540

Pro Arg Cys Leu Thr Arg Tyr Tyr Ser Ser Phe Val Asn Met Glu Arg
545 550 555 560

Asp Leu Ala Ser Gly Leu Ile Gly Pro Leu Leu Ile Cys Tyr Lys Glu
565 570 575

```
Ser Val Asp Gln Arg Gly Asn Gln Ile Met Ser Asp Lys Arg Asn Val
            580                 585                 590

Ile Leu Phe Ser Val Phe Asp Glu Asn Arg Ser Trp Tyr Leu Thr Glu
        595                 600                 605

Asn Ile Gln Arg Phe Leu Pro Asn Pro Ala Gly Val Gln Leu Glu Asp
    610                 615                 620

Pro Glu Phe Gln Ala Ser Asn Ile Met His Ser Ile Asn Gly Tyr Val
625                 630                 635                 640

Phe Asp Ser Leu Gln Leu Ser Val Cys Leu His Glu Val Ala Tyr Trp
                645                 650                 655

Tyr Ile Leu Ser Ile Gly Ala Gln Thr Asp Phe Leu Ser Val Phe Phe
            660                 665                 670

Ser Gly Tyr Thr Phe Lys His Lys Met Val Tyr Glu Asp Thr Leu Thr
        675                 680                 685

Leu Phe Pro Phe Ser Gly Glu Thr Val Phe Met Ser Met Glu Asn Pro
    690                 695                 700

Gly Leu Trp Ile Leu Gly Cys His Asn Ser Asp Phe Arg Asn Arg Gly
705                 710                 715                 720

Met Thr Ala Leu Leu Lys Val Ser Ser Cys Asp Lys Asn Thr Gly Asp
                725                 730                 735

Tyr Tyr Glu Asp Ser Tyr Glu Asp Ile Ser Ala Tyr Leu Leu Ser Lys
            740                 745                 750

Asn Asn Ala Ile Glu Pro Arg Ser Phe Ser Gln Asn Pro Pro Val Leu
        755                 760                 765

Lys Arg His Gln Arg Glu Ile Thr Arg Thr Thr Leu Gln Ser Asp Gln
    770                 775                 780

Glu Glu Ile Asp Tyr Asp Asp Thr Ile Ser Val Glu Met Lys Lys Glu
785                 790                 795                 800

Asp Phe Asp Ile Tyr Asp Glu Asp Glu Asn Gln Ser Pro Arg Ser Phe
                805                 810                 815

Gln Lys Lys Thr Arg His Tyr Phe Ile Ala Ala Val Glu Arg Leu Trp
```

820 825 830

Asp Tyr Gly Met Ser Ser Ser Pro His Val Leu Arg Asn Arg Ala Gln
835 840 845

Ser Gly Ser Val Pro Gln Phe Lys Lys Val Val Phe Gln Glu Phe Thr
850 855 860

Asp Gly Ser Phe Thr Gln Pro Leu Tyr Arg Gly Glu Leu Asn Glu His
865 870 875 880

Leu Gly Leu Leu Gly Pro Tyr Ile Arg Ala Glu Val Glu Asp Asn Ile
885 890 895

Met Val Thr Phe Arg Asn Gln Ala Ser Arg Pro Tyr Ser Phe Tyr Ser
900 905 910

Ser Leu Ile Ser Tyr Glu Glu Asp Gln Arg Gln Gly Ala Glu Pro Arg
915 920 925

Lys Asn Phe Val Lys Pro Asn Glu Thr Lys Thr Tyr Phe Trp Lys Val
930 935 940

Gln His His Met Ala Pro Thr Lys Asp Glu Phe Asp Cys Lys Ala Trp
945 950 955 960

Ala Tyr Phe Ser Asp Val Asp Leu Glu Lys Asp Val His Ser Gly Leu
965 970 975

Ile Gly Pro Leu Leu Val Cys His Thr Asn Thr Leu Asn Pro Ala His
980 985 990

Gly Arg Gln Val Thr Val Gln Glu Phe Ala Leu Phe Phe Thr Ile Phe
995 1000 1005

Asp Glu Thr Lys Ser Trp Tyr Phe Thr Glu Asn Met Glu Arg Asn
1010 1015 1020

Cys Arg Ala Pro Cys Asn Ile Gln Met Glu Asp Pro Thr Phe Lys
1025 1030 1035

Glu Asn Tyr Arg Phe His Ala Ile Asn Gly Tyr Ile Met Asp Thr
1040 1045 1050

Leu Pro Gly Leu Val Met Ala Gln Asp Gln Arg Ile Arg Trp Tyr
1055 1060 1065

Leu Leu Ser Met Gly Ser Asn Glu Asn Ile His Ser Ile His Phe
1070 1075 1080

Ser Gly His Val Phe Thr Val Arg Lys Lys Glu Glu Tyr Lys Met
1085 1090 1095

Ala Leu Tyr Asn Leu Tyr Pro Gly Val Phe Glu Thr Val Glu Met
1100 1105 1110

Leu Pro Ser Lys Ala Gly Ile Trp Arg Val Glu Cys Leu Ile Gly
1115 1120 1125

Glu His Leu His Ala Gly Met Ser Thr Leu Phe Leu Val Tyr Ser
1130 1135 1140

Asn Lys Cys Gln Thr Pro Leu Gly Met Ala Ser Gly His Ile Arg
1145 1150 1155

Asp Phe Gln Ile Thr Ala Ser Gly Gln Tyr Gly Gln Trp Ala Pro
1160 1165 1170

Lys Leu Ala Arg Leu His Tyr Ser Gly Ser Ile Asn Ala Trp Ser
1175 1180 1185

Thr Lys Glu Pro Phe Ser Trp Ile Lys Val Asp Leu Leu Ala Pro
1190 1195 1200

Met Ile Ile His Gly Ile Lys Thr Gln Gly Ala Arg Gln Lys Phe
1205 1210 1215

Ser Ser Leu Tyr Ile Ser Gln Phe Ile Ile Met Tyr Ser Leu Asp
1220 1225 1230

Gly Lys Lys Trp Gln Thr Tyr Arg Gly Asn Ser Thr Gly Thr Leu
1235 1240 1245

Met Val Phe Phe Gly Asn Val Asp Ser Ser Gly Ile Lys His Asn
1250 1255 1260

Ile Phe Asn Pro Pro Ile Ile Ala Arg Tyr Ile Arg Leu His Pro
1265 1270 1275

Thr His Tyr Ser Ile Arg Ser Thr Leu Arg Met Glu Leu Met Gly
1280 1285 1290

Cys Asp Leu Asn Ser Cys Ser Met Pro Leu Gly Met Glu Ser Lys
1295 1300 1305

Ala Ile Ser Asp Ala Gln Ile Thr Ala Ser Ser Tyr Phe Thr Asn
1310 1315 1320

Met Phe Ala Thr Trp Ser Pro Ser Lys Ala Arg Leu His Leu Gln
1325 1330 1335

Gly Arg Ser Asn Ala Trp Arg Pro Gln Val Asn Asn Pro Lys Glu
1340 1345 1350

Trp Leu Gln Val Asp Phe Gln Lys Thr Met Lys Val Thr Gly Val
1355 1360 1365

Thr Thr Gln Gly Val Lys Ser Leu Leu Thr Ser Met Tyr Val Lys
1370 1375 1380

Glu Phe Leu Ile Ser Ser Ser Gln Asp Gly His Gln Trp Thr Leu
1385 1390 1395

Phe Phe Gln Asn Gly Lys Val Lys Val Phe Gln Gly Asn Gln Asp
1400 1405 1410

Ser Phe Thr Pro Val Val Asn Ser Leu Asp Pro Pro Leu Leu Thr
1415 1420 1425

Arg Tyr Leu Arg Ile His Pro Gln Ser Trp Val His Gln Ile Ala
1430 1435 1440

Leu Arg Met Glu Val Leu Gly Cys Glu Ala Gln Asp Leu Tyr Asp
1445 1450 1455

Lys Thr His Thr Cys Pro Pro Cys Pro Ala Pro Glu Leu Leu Gly
1460 1465 1470

Gly Pro Ser Val Phe Leu Phe Pro Pro Lys Pro Lys Asp Thr Leu
1475 1480 1485

Met Ile Ser Arg Thr Pro Glu Val Thr Cys Val Val Val Asp Val
1490 1495 1500

Ser His Glu Asp Pro Glu Val Lys Phe Asn Trp Tyr Val Asp Gly
1505 1510 1515

Val Glu Val His Asn Ala Lys Thr Lys Pro Arg Glu Glu Gln Tyr
1520 1525 1530

Asn Ser Thr Tyr Arg Val Val Ser Val Leu Thr Val Leu His Gln
1535 1540 1545

```
Asp Trp Leu Asn Gly Lys Glu Tyr Lys Cys Lys Val Ser Asn Lys
    1550                1555                1560

Ala Leu Pro Ala Pro Ile Glu Lys Thr Ile Ser Lys Ala Lys Gly
    1565                1570                1575

Gln Pro Arg Glu Pro Gln Val Tyr Thr Leu Pro Pro Ser Arg Asp
    1580                1585                1590

Glu Leu Thr Lys Asn Gln Val Ser Leu Thr Cys Leu Val Lys Gly
    1595                1600                1605

Phe Tyr Pro Ser Asp Ile Ala Val Glu Trp Glu Ser Asn Gly Gln
    1610                1615                1620

Pro Glu Asn Asn Tyr Lys Thr Thr Pro Pro Val Leu Asp Ser Asp
    1625                1630                1635

Gly Ser Phe Phe Leu Tyr Ser Lys Leu Thr Val Asp Lys Ser Arg
    1640                1645                1650

Trp Gln Gln Gly Asn Val Phe Ser Cys Ser Val Met His Glu Ala
    1655                1660                1665

Leu His Asn His Tyr Thr Gln Lys Ser Leu Ser Leu Ser Pro Gly
    1670                1675                1680

Lys


<210>  3
<211>  741
<212>  DNA
<213>  Artificial Sequence

<220>
<223>  Fc Portion

<400>  3
atggagacag acacactcct gctatgggta ctgctgctct gggttccagg ttccactggt      60

gacaaaactc acacatgccc accgtgccca gcacctgaac tcctgggagg accgtcagtc     120

ttcctcttcc ccccaaaacc caaggacacc ctcatgatct cccggacccc tgaggtcaca     180

tgcgtggtgg tggacgtgag ccacgaagac cctgaggtca agttcaactg gtacgtggac     240

ggcgtggagg tgcataatgc caagacaaag ccgcgggagg agcagtacaa cagcacgtac     300

cgtgtggtca gcgtcctcac cgtcctgcac caggactggc tgaatggcaa ggagtacaag     360
```

tgcaaggtct ccaacaaagc cctcccagcc cccatcgaga aaaccatctc caaagccaaa 420

gggcagcccc gagaaccaca ggtgtacacc ctgcccccat cccgcgatga gctgaccaag 480

aaccaggtca gcctgacctg cctggtcaaa ggcttctatc ccagcgacat cgccgtggag 540

tgggagagca atgggcagcc ggagaacaac tacaagacca cgcctcccgt gttggactcc 600

gacggctcct tcttcctcta cagcaagctc accgtggaca agagcaggtg gcagcagggg 660

aacgtcttct catgctccgt gatgcatgag gctctgcaca accactacac gcagaagagc 720

ctctccctgt ctccgggtaa a 741

<210> 4
<211> 247
<212> PRT
<213> Artificial Sequence

<220>
<223> Fc Portion

<400> 4

```
Met Glu Thr Asp Thr Leu Leu Leu Trp Val Leu Leu Leu Trp Val Pro
1               5                   10                  15

Gly Ser Thr Gly Asp Lys Thr His Thr Cys Pro Pro Cys Pro Ala Pro
            20                  25                  30

Glu Leu Leu Gly Gly Pro Ser Val Phe Leu Phe Pro Pro Lys Pro Lys
        35                  40                  45

Asp Thr Leu Met Ile Ser Arg Thr Pro Glu Val Thr Cys Val Val Val
    50                  55                  60

Asp Val Ser His Glu Asp Pro Glu Val Lys Phe Asn Trp Tyr Val Asp
65                  70                  75                  80

Gly Val Glu Val His Asn Ala Lys Thr Lys Pro Arg Glu Glu Gln Tyr
                85                  90                  95

Asn Ser Thr Tyr Arg Val Val Ser Val Leu Thr Val Leu His Gln Asp
            100                 105                 110

Trp Leu Asn Gly Lys Glu Tyr Lys Cys Lys Val Ser Asn Lys Ala Leu
        115                 120                 125

Pro Ala Pro Ile Glu Lys Thr Ile Ser Lys Ala Lys Gly Gln Pro Arg
    130                 135                 140

Glu Pro Gln Val Tyr Thr Leu Pro Pro Ser Arg Asp Glu Leu Thr Lys
145                 150                 155                 160
```

Asn Gln Val Ser Leu Thr Cys Leu Val Lys Gly Phe Tyr Pro Ser Asp
165 170 175

Ile Ala Val Glu Trp Glu Ser Asn Gly Gln Pro Glu Asn Asn Tyr Lys
180 185 190

Thr Thr Pro Pro Val Leu Asp Ser Asp Gly Ser Phe Phe Leu Tyr Ser
195 200 205

Lys Leu Thr Val Asp Lys Ser Arg Trp Gln Gln Gly Asn Val Phe Ser
210 215 220

Cys Ser Val Met His Glu Ala Leu His Asn His Tyr Thr Gln Lys Ser
225 230 235 240

Leu Ser Leu Ser Pro Gly Lys
245

<210> 5
<211> 7734
<212> DNA
<213> Artificial Sequence

<220>
<223> Full Length FVIIIFc

<400> 5
atgcaaatag agctctccac ctgcttcttt ctgtgccttt tgcgattctg ctttagtgcc 60

accagaagat actacctggg tgcagtggaa ctgtcatggg actatatgca aagtgatctc 120

ggtgagctgc ctgtggacgc aagatttcct cctagagtgc caaaatcttt tccattcaac 180

acctcagtcg tgtacaaaaa gactctgttt gtagaattca cggatcacct tttcaacatc 240

gctaagccaa ggccaccctg gatgggtctg ctaggtccta ccatccaggc tgaggtttat 300

gatacagtgg tcattacact taagaacatg gcttcccatc ctgtcagtct tcatgctgtt 360

ggtgtatcct actggaaagc ttctgaggga gctgaatatg atgatcagac cagtcaaagg 420

gagaaagaag atgataaagt cttccctggt ggaagccata catatgtctg gcaggtcctg 480

aaagagaatg gtccaatggc ctctgaccca ctgtgcctta cctactcata tctttctcat 540

gtggacctgg taaaagactt gaattcaggc ctcattggag ccctactagt atgtagagaa 600

gggagtctgg ccaaggaaaa gacacagacc ttgcacaaat ttatactact tttgctgta 660

tttgatgaag ggaaaagttg gcactcagaa acaaagaact ccttgatgca ggatagggat 720

gctgcatctg ctcgggcctg gcctaaaatg cacacagtca atggttatgt aaacaggtct 780

ctgccaggtc tgattggatg ccacaggaaa tcagtctatt ggcatgtgat tggaatgggc 840

accactcctg aagtgcactc aatattcctc gaaggtcaca catttcttgt gaggaaccat 900
cgccaggcgt ccttggaaat ctcgccaata actttcctta ctgctcaaac actcttgatg 960
gaccttggac agtttctact gttttgtcat atctcttccc accaacatga tggcatggaa 1020
gcttatgtca aagtagacag ctgtccagag gaacccaac tacgaatgaa aaataatgaa 1080
gaagcggaag actatgatga tgatcttact gattctgaaa tggatgtggt caggtttgat 1140
gatgacaact ctccttcctt tatccaaatt cgctcagttg ccaagaagca tcctaaaact 1200
tgggtacatt acattgctgc tgaagaggag gactgggact atgctccctt agtcctcgcc 1260
cccgatgaca gaagttataa aagtcaatat ttgaacaatg gccctcagcg gattggtagg 1320
aagtacaaaa aagtccgatt tatggcatac acagatgaaa cctttaagac tcgtgaagct 1380
attcagcatg aatcaggaat cttgggacct ttactttatg gggaagttgg agacacactg 1440
ttgattatat ttaagaatca agcaagcaga ccatataaca tctaccctca cggaatcact 1500
gatgtccgtc ctttgtattc aaggagatta ccaaaaggtg taaaacattt gaaggatttt 1560
ccaattctgc caggagaaat attcaaatat aaatggacag tgactgtaga agatgggcca 1620
actaaatcag atcctcggtg cctgacccgc tattactcta gtttcgttaa tatggagaga 1680
gatctagctt caggactcat tggccctctc ctcatctgct acaaagaatc tgtagatcaa 1740
agaggaaacc agataatgtc agacaagagg aatgtcatcc tgttttctgt atttgatgag 1800
aaccgaagct ggtacctcac agagaatata caacgctttc tccccaatcc agctggagtg 1860
cagcttgagg atccagagtt ccaagcctcc aacatcatgc acagcatcaa tggctatgtt 1920
tttgatagtt tgcagttgtc agtttgtttg catgaggtgg catactggta cattctaagc 1980
attggagcac agactgactt cctttctgtc ttcttctctg gatatacctt caaacacaaa 2040
atggtctatg aagacacact caccctattc ccattctcag gagaaactgt cttcatgtcg 2100
atggaaaacc caggtctatg gattctgggg tgccacaact cagactttcg gaacagaggc 2160
atgaccgcct tactgaaggt ttctagttgt gacaagaaca ctggtgatta ttacgaggac 2220
agttatgaag atatttcagc atacttgctg agtaaaaaca atgccattga accaagaagc 2280
ttctcccaga attcaagaca ccctagcact aggcaaaagc aatttaatgc caccacaatt 2340
ccagaaaatg acatagagaa gactgaccct tggtttgcac acagaacacc tatgcctaaa 2400
atacaaaatg tctcctctag tgatttgttg atgctcttgc gacagagtcc tactccacat 2460
gggctatcct tatctgatct ccaagaagcc aaatatgaga ctttttctga tgatccatca 2520
cctggagcaa tagacagtaa taacagcctg tctgaaatga cacacttcag gccacagctc 2580
catcacagtg gggacatggt atttaccccct gagtcaggcc tccaattaag attaaatgag 2640
aaactgggga caactgcagc aacagagttg aagaaacttg atttcaaagt ttctagtaca 2700
tcaaataatc tgatttcaac aattccatca gacaatttgg cagcaggtac tgataataca 2760

agttccttag gacccccaag tatgccagtt cattatgata gtcaattaga taccactcta 2820
tttggcaaaa agtcatctcc ccttactgag tctggtggac ctctgagctt gagtgaagaa 2880
aataatgatt caaagttgtt agaatcaggt ttaatgaata gccaagaaag ttcatgggga 2940
aaaaatgtat cgtcaacaga gagtggtagg ttatttaaag ggaaaagagc tcatggacct 3000
gctttgttga ctaaagataa tgccttattc aaagttagca tctctttgtt aaagacaaac 3060
aaaacttcca ataattcagc aactaataga aagactcaca ttgatggccc atcattatta 3120
attgagaata gtccatcagt ctggcaaaat atattagaaa gtgacactga gtttaaaaaa 3180
gtgacacctt tgattcatga cagaatgctt atggacaaaa atgctacagc tttgaggcta 3240
aatcatatgt caaataaaac tacttcatca aaaaacatgg aaatggtcca acagaaaaaa 3300
gagggcccca ttccaccaga tgcacaaaat ccagatatgt cgttctttaa gatgctattc 3360
ttgccagaat cagcaaggtg gatacaaagg actcatggaa agaactctct gaactctggg 3420
caaggcccca gtccaaagca attagtatcc ttaggaccag aaaaatctgt ggaaggtcag 3480
aatttcttgt ctgagaaaaa caaagtggta gtaggaaagg gtgaatttac aaaggacgta 3540
ggactcaaag agatggtttt tccaagcagc agaaacctat ttcttactaa cttggataat 3600
ttacatgaaa ataatacaca caatcaagaa aaaaaaattc aggaagaaat agaaaagaag 3660
gaaacattaa tccaagagaa tgtagttttg cctcagatac atacagtgac tggcactaag 3720
aatttcatga agaacctttt cttactgagc actaggcaaa atgtagaagg ttcatatgac 3780
ggggcatatg ctccagtact tcaagatttt aggtcattaa atgattcaac aaatagaaca 3840
aagaaacaca cagctcattt ctcaaaaaaa ggggaggaag aaaacttgga aggcttggga 3900
aatcaaacca agcaaattgt agagaaatat gcatgcacca caaggatatc tcctaataca 3960
agccagcaga attttgtcac gcaacgtagt aagagagctt tgaaacaatt cagactccca 4020
ctagaagaaa cagaacttga aaaaaggata attgtggatg acacctcaac ccagtggtcc 4080
aaaaacatga aacatttgac cccgagcacc ctcacacaga tagactacaa tgagaaggag 4140
aaaggggcca ttactcagtc tcccttatca gattgcctta cgaggagtca tagcatccct 4200
caagcaaata gatctccatt acccattgca aaggtatcat catttccatc tattagacct 4260
atatatctga ccagggtcct attccaagac aactcttctc atcttccagc agcatcttat 4320
agaaagaaag attctggggt ccaagaaagc agtcatttct tacaaggagc caaaaaaaat 4380
aacctttctt tagccattct aaccttggag atgactggtg atcaaagaga ggttggctcc 4440
ctggggacaa gtgccacaaa ttcagtcaca tacaagaaag ttgagaacac tgttctcccg 4500
aaaccagact tgcccaaaac atctggcaaa gttgaattgc ttccaaaagt tcacatttat 4560
cagaaggacc tattccctac ggaaactagc aatgggtctc ctggccatct ggatctcgtg 4620

gaagggagcc ttcttcaggg aacagaggga gcgattaagt ggaatgaagc aaacagacct 4680

ggaaaagttc cctttctgag agtagcaaca gaaagctctg caaagactcc ctccaagcta 4740

ttggatcctc ttgcttggga taaccactat ggtactcaga taccaaaaga agagtggaaa 4800

tcccaagaga agtcaccaga aaaaacagct tttaagaaaa aggataccat tttgtccctg 4860

aacgcttgtg aaagcaatca tgcaatagca gcaataaatg agggacaaaa taagcccgaa 4920

atagaagtca cctgggcaaa gcaaggtagg actgaaaggc tgtgctctca aaacccacca 4980

gtcttgaaac gccatcaacg ggaaataact cgtactactc ttcagtcaga tcaagaggaa 5040

attgactatg atgataccat atcagttgaa atgaagaagg aagattttga catttatgat 5100

gaggatgaaa atcagagccc ccgcagcttt caaaagaaaa cacgacacta ttttattgct 5160

gcagtggaga ggctctggga ttatgggatg agtagctccc cacatgttct aagaaacagg 5220

gctcagagtg gcagtgtccc tcagttcaag aaagttgttt tccaggaatt tactgatggc 5280

tcctttactc agcccttata ccgtggagaa ctaaatgaac atttgggact cctggggcca 5340

tatataagag cagaagttga agataatatc atggtaactt tcagaaatca ggcctctcgt 5400

ccctattcct tctattctag ccttatttct tatgaggaag atcagaggca aggagcagaa 5460

cctagaaaaa actttgtcaa gcctaatgaa accaaaactt acttttggaa agtgcaacat 5520

catatggcac ccactaaaga tgagtttgac tgcaaagcct gggcttattt ctctgatgtt 5580

gacctggaaa aagatgtgca ctcaggcctg attggacccc ttctggtctg ccacactaac 5640

acactgaacc ctgctcatgg gagacaagtg acagtacagg aatttgctct gtttttcacc 5700

atctttgatg agaccaaaag ctggtacttc actgaaaata tggaaagaaa ctgcagggct 5760

ccctgcaata tccagatgga agatcccact tttaaagaga attatcgctt ccatgcaatc 5820

aatggctaca taatggatac actacctggc ttagtaatgg ctcaggatca aaggattcga 5880

tggtatctgc tcagcatggg cagcaatgaa aacatccatt ctattcattt cagtggacat 5940

gtgttcactg tacgaaaaaa agaggagtat aaaatggcac tgtacaatct ctatccaggt 6000

gtttttgaga cagtggaaat gttaccatcc aaagctggaa tttggcgggt ggaatgcctt 6060

attggcgagc atctacatgc tgggatgagc acactttttc tggtgtacag caataagtgt 6120

cagactcccc tgggaatggc ttctggacac attagagatt ttcagattac agcttcagga 6180

caatatggac agtgggcccc aaagctggcc agacttcatt attccggatc aatcaatgcc 6240

tggagcacca aggagccctt ttcttggatc aaggtggatc tgttggcacc aatgattatt 6300

cacggcatca agacccaggg tgcccgtcag aagttctcca gcctctacat ctctcagttt 6360

atcatcatgt atagtcttga tgggaagaag tggcagactt atcgaggaaa ttccactgga 6420

accttaatgg tcttctttgg caatgtggat tcatctggga taaaacacaa tatttttaac 6480

cctccaatta ttgctcgata catccgtttg cacccaactc attatagcat tcgcagcact 6540

cttcgcatgg agttgatggg ctgtgattta aatagttgca gcatgccatt gggaatggag 6600

agtaaagcaa tatcagatgc acagattact gcttcatcct actttaccaa tatgtttgcc 6660

acctggtctc cttcaaaagc tcgacttcac ctccaaggga ggagtaatgc ctggagacct 6720

caggtgaata atccaaaaga gtggctgcaa gtggacttcc agaagacaat gaaagtcaca 6780

ggagtaacta ctcagggagt aaaatctctg cttaccagca tgtatgtgaa ggagttcctc 6840

atctccagca gtcaagatgg ccatcagtgg actctctttt ttcagaatgg caaagtaaag 6900

gtttttcagg gaaatcaaga ctccttcaca cctgtggtga actctctaga cccaccgtta 6960

ctgactcgct accttcgaat tcacccccag agttgggtgc accagattgc cctgaggatg 7020

gaggttctgg gctgcgaggc acaggacctc tacgacaaaa ctcacacatg cccaccgtgc 7080

ccagctccag aactcctggg cggaccgtca gtcttcctct tcccccaaa acccaaggac 7140

accctcatga tctcccggac ccctgaggtc acatgcgtgg tggtggacgt gagccacgaa 7200

gaccctgagg tcaagttcaa ctggtacgtg gacggcgtgg aggtgcataa tgccaagaca 7260

aagccgcggg aggagcagta caacagcacg taccgtgtgg tcagcgtcct caccgtcctg 7320

caccaggact ggctgaatgg caaggagtac aagtgcaagg tctccaacaa agccctccca 7380

gcccccatcg agaaaaccat ctccaaagcc aaagggcagc cccgagaacc acaggtgtac 7440

accctgcccc catcccggga tgagctgacc aagaaccagg tcagcctgac ctgcctggtc 7500

aaaggcttct atcccagcga catcgccgtg gagtgggaga gcaatgggca gccggagaac 7560

aactacaaga ccacgcctcc cgtgttggac tccgacggct ccttcttcct ctacagcaag 7620

ctcaccgtgg acaagagcag gtggcagcag gggaacgtct tctcatgctc cgtgatgcat 7680

gaggctctgc acaaccacta cacgcagaag agcctctccc tgtctccggg taaa 7734

<210> 6
<211> 2578
<212> PRT
<213> Artificial Sequence

<220>
<223> Full Length FVIIIFc

<400> 6

Met Gln Ile Glu Leu Ser Thr Cys Phe Phe Leu Cys Leu Leu Arg Phe
1 5 10 15

Cys Phe Ser Ala Thr Arg Arg Tyr Tyr Leu Gly Ala Val Glu Leu Ser
20 25 30

Trp Asp Tyr Met Gln Ser Asp Leu Gly Glu Leu Pro Val Asp Ala Arg
35 40 45

```
Phe Pro Pro Arg Val Pro Lys Ser Phe Pro Phe Asn Thr Ser Val Val
    50                  55                  60

Tyr Lys Lys Thr Leu Phe Val Glu Phe Thr Asp His Leu Phe Asn Ile
65                  70                  75                  80

Ala Lys Pro Arg Pro Pro Trp Met Gly Leu Leu Gly Pro Thr Ile Gln
                85                  90                  95

Ala Glu Val Tyr Asp Thr Val Val Ile Thr Leu Lys Asn Met Ala Ser
            100                 105                 110

His Pro Val Ser Leu His Ala Val Gly Val Ser Tyr Trp Lys Ala Ser
        115                 120                 125

Glu Gly Ala Glu Tyr Asp Asp Gln Thr Ser Gln Arg Glu Lys Glu Asp
    130                 135                 140

Asp Lys Val Phe Pro Gly Gly Ser His Thr Tyr Val Trp Gln Val Leu
145                 150                 155                 160

Lys Glu Asn Gly Pro Met Ala Ser Asp Pro Leu Cys Leu Thr Tyr Ser
                165                 170                 175

Tyr Leu Ser His Val Asp Leu Val Lys Asp Leu Asn Ser Gly Leu Ile
            180                 185                 190

Gly Ala Leu Leu Val Cys Arg Glu Gly Ser Leu Ala Lys Glu Lys Thr
        195                 200                 205

Gln Thr Leu His Lys Phe Ile Leu Leu Phe Ala Val Phe Asp Glu Gly
    210                 215                 220

Lys Ser Trp His Ser Glu Thr Lys Asn Ser Leu Met Gln Asp Arg Asp
225                 230                 235                 240

Ala Ala Ser Ala Arg Ala Trp Pro Lys Met His Thr Val Asn Gly Tyr
                245                 250                 255

Val Asn Arg Ser Leu Pro Gly Leu Ile Gly Cys His Arg Lys Ser Val
            260                 265                 270

Tyr Trp His Val Ile Gly Met Gly Thr Thr Pro Glu Val His Ser Ile
        275                 280                 285

Phe Leu Glu Gly His Thr Phe Leu Val Arg Asn His Arg Gln Ala Ser
    290                 295                 300
```

```
Leu Glu Ile Ser Pro Ile Thr Phe Leu Thr Ala Gln Thr Leu Leu Met
305                 310                 315                 320

Asp Leu Gly Gln Phe Leu Leu Phe Cys His Ile Ser Ser His Gln His
                325                 330                 335

Asp Gly Met Glu Ala Tyr Val Lys Val Asp Ser Cys Pro Glu Glu Pro
            340                 345                 350

Gln Leu Arg Met Lys Asn Asn Glu Glu Ala Glu Asp Tyr Asp Asp Asp
        355                 360                 365

Leu Thr Asp Ser Glu Met Asp Val Val Arg Phe Asp Asp Asp Asn Ser
    370                 375                 380

Pro Ser Phe Ile Gln Ile Arg Ser Val Ala Lys Lys His Pro Lys Thr
385                 390                 395                 400

Trp Val His Tyr Ile Ala Ala Glu Glu Glu Asp Trp Asp Tyr Ala Pro
                405                 410                 415

Leu Val Leu Ala Pro Asp Asp Arg Ser Tyr Lys Ser Gln Tyr Leu Asn
            420                 425                 430

Asn Gly Pro Gln Arg Ile Gly Arg Lys Tyr Lys Lys Val Arg Phe Met
        435                 440                 445

Ala Tyr Thr Asp Glu Thr Phe Lys Thr Arg Glu Ala Ile Gln His Glu
    450                 455                 460

Ser Gly Ile Leu Gly Pro Leu Leu Tyr Gly Glu Val Gly Asp Thr Leu
465                 470                 475                 480

Leu Ile Ile Phe Lys Asn Gln Ala Ser Arg Pro Tyr Asn Ile Tyr Pro
                485                 490                 495

His Gly Ile Thr Asp Val Arg Pro Leu Tyr Ser Arg Arg Leu Pro Lys
            500                 505                 510

Gly Val Lys His Leu Lys Asp Phe Pro Ile Leu Pro Gly Glu Ile Phe
        515                 520                 525

Lys Tyr Lys Trp Thr Val Thr Val Glu Asp Gly Pro Thr Lys Ser Asp
    530                 535                 540

Pro Arg Cys Leu Thr Arg Tyr Tyr Ser Ser Phe Val Asn Met Glu Arg
```

545 550 555 560

Asp Leu Ala Ser Gly Leu Ile Gly Pro Leu Leu Ile Cys Tyr Lys Glu
565 570 575

Ser Val Asp Gln Arg Gly Asn Gln Ile Met Ser Asp Lys Arg Asn Val
580 585 590

Ile Leu Phe Ser Val Phe Asp Glu Asn Arg Ser Trp Tyr Leu Thr Glu
595 600 605

Asn Ile Gln Arg Phe Leu Pro Asn Pro Ala Gly Val Gln Leu Glu Asp
610 615 620

Pro Glu Phe Gln Ala Ser Asn Ile Met His Ser Ile Asn Gly Tyr Val
625 630 635 640

Phe Asp Ser Leu Gln Leu Ser Val Cys Leu His Glu Val Ala Tyr Trp
645 650 655

Tyr Ile Leu Ser Ile Gly Ala Gln Thr Asp Phe Leu Ser Val Phe Phe
660 665 670

Ser Gly Tyr Thr Phe Lys His Lys Met Val Tyr Glu Asp Thr Leu Thr
675 680 685

Leu Phe Pro Phe Ser Gly Glu Thr Val Phe Met Ser Met Glu Asn Pro
690 695 700

Gly Leu Trp Ile Leu Gly Cys His Asn Ser Asp Phe Arg Asn Arg Gly
705 710 715 720

Met Thr Ala Leu Leu Lys Val Ser Ser Cys Asp Lys Asn Thr Gly Asp
725 730 735

Tyr Tyr Glu Asp Ser Tyr Glu Asp Ile Ser Ala Tyr Leu Leu Ser Lys
740 745 750

Asn Asn Ala Ile Glu Pro Arg Ser Phe Ser Gln Asn Ser Arg His Pro
755 760 765

Ser Thr Arg Gln Lys Gln Phe Asn Ala Thr Thr Ile Pro Glu Asn Asp
770 775 780

Ile Glu Lys Thr Asp Pro Trp Phe Ala His Arg Thr Pro Met Pro Lys
785 790 795 800

Ile Gln Asn Val Ser Ser Ser Asp Leu Leu Met Leu Leu Arg Gln Ser
805 810 815

Pro Thr Pro His Gly Leu Ser Leu Ser Asp Leu Gln Glu Ala Lys Tyr
820 825 830

Glu Thr Phe Ser Asp Asp Pro Ser Pro Gly Ala Ile Asp Ser Asn Asn
835 840 845

Ser Leu Ser Glu Met Thr His Phe Arg Pro Gln Leu His His Ser Gly
850 855 860

Asp Met Val Phe Thr Pro Glu Ser Gly Leu Gln Leu Arg Leu Asn Glu
865 870 875 880

Lys Leu Gly Thr Thr Ala Ala Thr Glu Leu Lys Lys Leu Asp Phe Lys
885 890 895

Val Ser Ser Thr Ser Asn Asn Leu Ile Ser Thr Ile Pro Ser Asp Asn
900 905 910

Leu Ala Ala Gly Thr Asp Asn Thr Ser Ser Leu Gly Pro Pro Ser Met
915 920 925

Pro Val His Tyr Asp Ser Gln Leu Asp Thr Thr Leu Phe Gly Lys Lys
930 935 940

Ser Ser Pro Leu Thr Glu Ser Gly Gly Pro Leu Ser Leu Ser Glu Glu
945 950 955 960

Asn Asn Asp Ser Lys Leu Leu Glu Ser Gly Leu Met Asn Ser Gln Glu
965 970 975

Ser Ser Trp Gly Lys Asn Val Ser Ser Thr Glu Ser Gly Arg Leu Phe
980 985 990

Lys Gly Lys Arg Ala His Gly Pro Ala Leu Leu Thr Lys Asp Asn Ala
995 1000 1005

Leu Phe Lys Val Ser Ile Ser Leu Leu Lys Thr Asn Lys Thr Ser
1010 1015 1020

Asn Asn Ser Ala Thr Asn Arg Lys Thr His Ile Asp Gly Pro Ser
1025 1030 1035

Leu Leu Ile Glu Asn Ser Pro Ser Val Trp Gln Asn Ile Leu Glu
1040 1045 1050

Ser Asp Thr Glu Phe Lys Lys Val Thr Pro Leu Ile His Asp Arg
1055 1060 1065

Met Leu Met Asp Lys Asn Ala Thr Ala Leu Arg Leu Asn His Met
1070 1075 1080

Ser Asn Lys Thr Thr Ser Ser Lys Asn Met Glu Met Val Gln Gln
1085 1090 1095

Lys Lys Glu Gly Pro Ile Pro Pro Asp Ala Gln Asn Pro Asp Met
1100 1105 1110

Ser Phe Phe Lys Met Leu Phe Leu Pro Glu Ser Ala Arg Trp Ile
1115 1120 1125

Gln Arg Thr His Gly Lys Asn Ser Leu Asn Ser Gly Gln Gly Pro
1130 1135 1140

Ser Pro Lys Gln Leu Val Ser Leu Gly Pro Glu Lys Ser Val Glu
1145 1150 1155

Gly Gln Asn Phe Leu Ser Glu Lys Asn Lys Val Val Val Gly Lys
1160 1165 1170

Gly Glu Phe Thr Lys Asp Val Gly Leu Lys Glu Met Val Phe Pro
1175 1180 1185

Ser Ser Arg Asn Leu Phe Leu Thr Asn Leu Asp Asn Leu His Glu
1190 1195 1200

Asn Asn Thr His Asn Gln Glu Lys Lys Ile Gln Glu Glu Ile Glu
1205 1210 1215

Lys Lys Glu Thr Leu Ile Gln Glu Asn Val Val Leu Pro Gln Ile
1220 1225 1230

His Thr Val Thr Gly Thr Lys Asn Phe Met Lys Asn Leu Phe Leu
1235 1240 1245

Leu Ser Thr Arg Gln Asn Val Glu Gly Ser Tyr Asp Gly Ala Tyr
1250 1255 1260

Ala Pro Val Leu Gln Asp Phe Arg Ser Leu Asn Asp Ser Thr Asn
1265 1270 1275

Arg Thr Lys Lys His Thr Ala His Phe Ser Lys Lys Gly Glu Glu
1280 1285 1290

Glu Asn Leu Glu Gly Leu Gly Asn Gln Thr Lys Gln Ile Val Glu
1295 1300 1305

Lys Tyr Ala Cys Thr Thr Arg Ile Ser Pro Asn Thr Ser Gln Gln
1310 1315 1320

Asn Phe Val Thr Gln Arg Ser Lys Arg Ala Leu Lys Gln Phe Arg
1325 1330 1335

Leu Pro Leu Glu Glu Thr Glu Leu Glu Lys Arg Ile Ile Val Asp
1340 1345 1350

Asp Thr Ser Thr Gln Trp Ser Lys Asn Met Lys His Leu Thr Pro
1355 1360 1365

Ser Thr Leu Thr Gln Ile Asp Tyr Asn Glu Lys Glu Lys Gly Ala
1370 1375 1380

Ile Thr Gln Ser Pro Leu Ser Asp Cys Leu Thr Arg Ser His Ser
1385 1390 1395

Ile Pro Gln Ala Asn Arg Ser Pro Leu Pro Ile Ala Lys Val Ser
1400 1405 1410

Ser Phe Pro Ser Ile Arg Pro Ile Tyr Leu Thr Arg Val Leu Phe
1415 1420 1425

Gln Asp Asn Ser Ser His Leu Pro Ala Ala Ser Tyr Arg Lys Lys
1430 1435 1440

Asp Ser Gly Val Gln Glu Ser Ser His Phe Leu Gln Gly Ala Lys
1445 1450 1455

Lys Asn Asn Leu Ser Leu Ala Ile Leu Thr Leu Glu Met Thr Gly
1460 1465 1470

Asp Gln Arg Glu Val Gly Ser Leu Gly Thr Ser Ala Thr Asn Ser
1475 1480 1485

Val Thr Tyr Lys Lys Val Glu Asn Thr Val Leu Pro Lys Pro Asp
1490 1495 1500

Leu Pro Lys Thr Ser Gly Lys Val Glu Leu Leu Pro Lys Val His
1505 1510 1515

Ile Tyr Gln Lys Asp Leu Phe Pro Thr Glu Thr Ser Asn Gly Ser

1520 1525 1530

Pro Gly His Leu Asp Leu Val Glu Gly Ser Leu Leu Gln Gly Thr
1535 1540 1545

Glu Gly Ala Ile Lys Trp Asn Glu Ala Asn Arg Pro Gly Lys Val
1550 1555 1560

Pro Phe Leu Arg Val Ala Thr Glu Ser Ser Ala Lys Thr Pro Ser
1565 1570 1575

Lys Leu Leu Asp Pro Leu Ala Trp Asp Asn His Tyr Gly Thr Gln
1580 1585 1590

Ile Pro Lys Glu Glu Trp Lys Ser Gln Glu Lys Ser Pro Glu Lys
1595 1600 1605

Thr Ala Phe Lys Lys Lys Asp Thr Ile Leu Ser Leu Asn Ala Cys
1610 1615 1620

Glu Ser Asn His Ala Ile Ala Ala Ile Asn Glu Gly Gln Asn Lys
1625 1630 1635

Pro Glu Ile Glu Val Thr Trp Ala Lys Gln Gly Arg Thr Glu Arg
1640 1645 1650

Leu Cys Ser Gln Asn Pro Pro Val Leu Lys Arg His Gln Arg Glu
1655 1660 1665

Ile Thr Arg Thr Thr Leu Gln Ser Asp Gln Glu Glu Ile Asp Tyr
1670 1675 1680

Asp Asp Thr Ile Ser Val Glu Met Lys Lys Glu Asp Phe Asp Ile
1685 1690 1695

Tyr Asp Glu Asp Glu Asn Gln Ser Pro Arg Ser Phe Gln Lys Lys
1700 1705 1710

Thr Arg His Tyr Phe Ile Ala Ala Val Glu Arg Leu Trp Asp Tyr
1715 1720 1725

Gly Met Ser Ser Ser Pro His Val Leu Arg Asn Arg Ala Gln Ser
1730 1735 1740

Gly Ser Val Pro Gln Phe Lys Lys Val Val Phe Gln Glu Phe Thr
1745 1750 1755

Asp Gly Ser Phe Thr Gln Pro Leu Tyr Arg Gly Glu Leu Asn Glu
1760 1765 1770

His Leu Gly Leu Leu Gly Pro Tyr Ile Arg Ala Glu Val Glu Asp
1775 1780 1785

Asn Ile Met Val Thr Phe Arg Asn Gln Ala Ser Arg Pro Tyr Ser
1790 1795 1800

Phe Tyr Ser Ser Leu Ile Ser Tyr Glu Glu Asp Gln Arg Gln Gly
1805 1810 1815

Ala Glu Pro Arg Lys Asn Phe Val Lys Pro Asn Glu Thr Lys Thr
1820 1825 1830

Tyr Phe Trp Lys Val Gln His His Met Ala Pro Thr Lys Asp Glu
1835 1840 1845

Phe Asp Cys Lys Ala Trp Ala Tyr Phe Ser Asp Val Asp Leu Glu
1850 1855 1860

Lys Asp Val His Ser Gly Leu Ile Gly Pro Leu Leu Val Cys His
1865 1870 1875

Thr Asn Thr Leu Asn Pro Ala His Gly Arg Gln Val Thr Val Gln
1880 1885 1890

Glu Phe Ala Leu Phe Phe Thr Ile Phe Asp Glu Thr Lys Ser Trp
1895 1900 1905

Tyr Phe Thr Glu Asn Met Glu Arg Asn Cys Arg Ala Pro Cys Asn
1910 1915 1920

Ile Gln Met Glu Asp Pro Thr Phe Lys Glu Asn Tyr Arg Phe His
1925 1930 1935

Ala Ile Asn Gly Tyr Ile Met Asp Thr Leu Pro Gly Leu Val Met
1940 1945 1950

Ala Gln Asp Gln Arg Ile Arg Trp Tyr Leu Leu Ser Met Gly Ser
1955 1960 1965

Asn Glu Asn Ile His Ser Ile His Phe Ser Gly His Val Phe Thr
1970 1975 1980

Val Arg Lys Lys Glu Glu Tyr Lys Met Ala Leu Tyr Asn Leu Tyr
1985 1990 1995

```
Pro Gly  Val Phe Glu Thr Val  Glu Met Leu Pro Ser  Lys Ala Gly
    2000                 2005                 2010

Ile Trp  Arg Val Glu Cys Leu  Ile Gly Glu His Leu  His Ala Gly
    2015                 2020                 2025

Met Ser  Thr Leu Phe Leu Val  Tyr Ser Asn Lys Cys  Gln Thr Pro
    2030                 2035                 2040

Leu Gly  Met Ala Ser Gly His  Ile Arg Asp Phe Gln  Ile Thr Ala
    2045                 2050                 2055

Ser Gly  Gln Tyr Gly Gln Trp  Ala Pro Lys Leu Ala  Arg Leu His
    2060                 2065                 2070

Tyr Ser  Gly Ser Ile Asn Ala  Trp Ser Thr Lys Glu  Pro Phe Ser
    2075                 2080                 2085

Trp Ile  Lys Val Asp Leu Leu  Ala Pro Met Ile Ile  His Gly Ile
    2090                 2095                 2100

Lys Thr  Gln Gly Ala Arg Gln  Lys Phe Ser Ser Leu  Tyr Ile Ser
    2105                 2110                 2115

Gln Phe  Ile Ile Met Tyr Ser  Leu Asp Gly Lys Lys  Trp Gln Thr
    2120                 2125                 2130

Tyr Arg  Gly Asn Ser Thr Gly  Thr Leu Met Val Phe  Phe Gly Asn
    2135                 2140                 2145

Val Asp  Ser Ser Gly Ile Lys  His Asn Ile Phe Asn  Pro Pro Ile
    2150                 2155                 2160

Ile Ala  Arg Tyr Ile Arg Leu  His Pro Thr His Tyr  Ser Ile Arg
    2165                 2170                 2175

Ser Thr  Leu Arg Met Glu Leu  Met Gly Cys Asp Leu  Asn Ser Cys
    2180                 2185                 2190

Ser Met  Pro Leu Gly Met Glu  Ser Lys Ala Ile Ser  Asp Ala Gln
    2195                 2200                 2205

Ile Thr  Ala Ser Ser Tyr Phe  Thr Asn Met Phe Ala  Thr Trp Ser
    2210                 2215                 2220

Pro Ser  Lys Ala Arg Leu His  Leu Gln Gly Arg Ser  Asn Ala Trp
    2225                 2230                 2235
```

Arg Pro Gln Val Asn Asn Pro Lys Glu Trp Leu Gln Val Asp Phe
2240 2245 2250

Gln Lys Thr Met Lys Val Thr Gly Val Thr Thr Gln Gly Val Lys
2255 2260 2265

Ser Leu Leu Thr Ser Met Tyr Val Lys Glu Phe Leu Ile Ser Ser
2270 2275 2280

Ser Gln Asp Gly His Gln Trp Thr Leu Phe Phe Gln Asn Gly Lys
2285 2290 2295

Val Lys Val Phe Gln Gly Asn Gln Asp Ser Phe Thr Pro Val Val
2300 2305 2310

Asn Ser Leu Asp Pro Pro Leu Leu Thr Arg Tyr Leu Arg Ile His
2315 2320 2325

Pro Gln Ser Trp Val His Gln Ile Ala Leu Arg Met Glu Val Leu
2330 2335 2340

Gly Cys Glu Ala Gln Asp Leu Tyr Asp Lys Thr His Thr Cys Pro
2345 2350 2355

Pro Cys Pro Ala Pro Glu Leu Leu Gly Gly Pro Ser Val Phe Leu
2360 2365 2370

Phe Pro Pro Lys Pro Lys Asp Thr Leu Met Ile Ser Arg Thr Pro
2375 2380 2385

Glu Val Thr Cys Val Val Val Asp Val Ser His Glu Asp Pro Glu
2390 2395 2400

Val Lys Phe Asn Trp Tyr Val Asp Gly Val Glu Val His Asn Ala
2405 2410 2415

Lys Thr Lys Pro Arg Glu Glu Gln Tyr Asn Ser Thr Tyr Arg Val
2420 2425 2430

Val Ser Val Leu Thr Val Leu His Gln Asp Trp Leu Asn Gly Lys
2435 2440 2445

Glu Tyr Lys Cys Lys Val Ser Asn Lys Ala Leu Pro Ala Pro Ile
2450 2455 2460

Glu Lys Thr Ile Ser Lys Ala Lys Gly Gln Pro Arg Glu Pro Gln

2465 2470 2475

Val Tyr Thr Leu Pro Pro Ser Arg Asp Glu Leu Thr Lys Asn Gln
2480 2485 2490

Val Ser Leu Thr Cys Leu Val Lys Gly Phe Tyr Pro Ser Asp Ile
2495 2500 2505

Ala Val Glu Trp Glu Ser Asn Gly Gln Pro Glu Asn Asn Tyr Lys
2510 2515 2520

Thr Thr Pro Pro Val Leu Asp Ser Asp Gly Ser Phe Phe Leu Tyr
2525 2530 2535

Ser Lys Leu Thr Val Asp Lys Ser Arg Trp Gln Gln Gly Asn Val
2540 2545 2550

Phe Ser Cys Ser Val Met His Glu Ala Leu His Asn His Tyr Thr
2555 2560 2565

Gln Lys Ser Leu Ser Leu Ser Pro Gly Lys
2570 2575

<210> 7
<211> 11
<212> PRT
<213> Artificial Sequence

<220>
<223> Albumin-binding Peptide Core

<400> 7

Asp Ile Cys Leu Pro Arg Trp Gly Cys Leu Trp
1 5 10

<210> 8
<211> 32
<212> PRT
<213> Artificial Sequence

<220>
<223> CTP peptide

<400> 8

Asp Pro Arg Phe Gln Asp Ser Ser Ser Ser Lys Ala Pro Pro Pro Ser
1 5 10 15

Leu Pro Ser Pro Ser Arg Leu Pro Gly Pro Ser Asp Thr Pro Ile Leu
20 25 30

<210> 9
<211> 28
<212> PRT
<213> Artificial Sequence

<220>
<223> CTP peptide

<400> 9

Ser Ser Ser Ser Lys Ala Pro Pro Pro Ser Leu Pro Ser Pro Ser Arg
1 5 10 15

Leu Pro Gly Pro Ser Asp Thr Pro Ile Leu Pro Gln
20 25

<210> 10
<211> 20
<212> PRT
<213> Artificial Sequence

<220>
<223> PAS peptide

<400> 10

Ala Ser Pro Ala Ala Pro Ala Pro Ala Ser Pro Ala Ala Pro Ala Pro
1 5 10 15

Ser Ala Pro Ala
20

<210> 11
<211> 20
<212> PRT
<213> Artificial Sequence

<220>
<223> PAS peptide

<400> 11

Ala Ala Pro Ala Ser Pro Ala Pro Ala Ala Pro Ser Ala Pro Ala Pro
1 5 10 15

Ala Ala Pro Ser
20

<210> 12
<211> 20
<212> PRT
<213> Artificial Sequence

<220>
<223> PAS peptide

<400> 12

```
Ala Pro Ser Ser Pro Ser Pro Ser Ala Pro Ser Ser Pro Ser Pro Ala
1               5                   10                  15


Ser Pro Ser Ser
            20


<210>  13
<211>  19
<212>  PRT
<213>  Artificial Sequence

<220>
<223>  PAS peptide

<400>  13

Ala Pro Ser Ser Pro Ser Pro Ser Ala Pro Ser Ser Pro Ser Pro Ala
1               5                   10                  15


Ser Pro Ser



<210>  14
<211>  20
<212>  PRT
<213>  Artificial Sequence

<220>
<223>  PAS peptide

<400>  14

Ser Ser Pro Ser Ala Pro Ser Pro Ser Ser Pro Ala Ser Pro Ser Pro
1               5                   10                  15


Ser Ser Pro Ala
            20


<210>  15
<211>  24
<212>  PRT
<213>  Artificial Sequence

<220>
<223>  PAS peptide

<400>  15

Ala Ala Ser Pro Ala Ala Pro Ser Ala Pro Pro Ala Ala Ala Ser Pro
1               5                   10                  15


Ala Ala Pro Ser Ala Pro Pro Ala
            20
```

<210> 16
<211> 20
<212> PRT
<213> Artificial Sequence

<220>
<223> PAS peptide

<400> 16

Ala Ser Ala Ala Ala Pro Ala Ala Ala Ser Ala Ala Ala Ser Ala Pro
1 5 10 15

Ser Ala Ala Ala
20

<210> 17
<211> 21
<212> PRT
<213> Artificial Sequence

<220>
<223> fragment of Factor VIII B domain

<400> 17

Ser Phe Ser Gln Asn Ser Arg His Pro Ser Gln Asn Pro Pro Val Leu
1 5 10 15

Lys Arg His Gln Arg
20

<210> 18
<211> 6
<212> PRT
<213> Artificial Sequence

<220>
<223> His tag

<400> 18

His His His His His His
1 5

## Description

BACKGROUND OF THE INVENTION

### Field of the Invention

The present invention relates generally to the field of therapeutics for hemostatic disorders.

### Background Art

Hemophilia A is an X-linked bleeding disorder caused by mutations and/or deletions in the factor VIII (FVIII) gene resulting in a deficiency of FVIII activity (Peyvandi, F. et al. Haemophilia 12:82-89 (2006)). The disease is characterized by spontaneous hemorrhage and excessive bleeding after trauma. Over time, the repeated bleeding into muscles and joints, which often begins in early childhood, results in hemophilic arthropathy and irreversible joint damage. This damage is progressive and can lead to severely limited mobility of joints, muscle atrophy and chronic pain (Rodriguez-Merchan, E.C., Semin. Thromb. Hemost. 29:87-96 (2003), which is herein incorporated by reference in its entirety).

The A2 domain is necessary for the procoagulant activity of the factor VIII molecule. Studies show that porcine factor VIII has six-fold greater procoagulant activity than human factor VIII (Lollar, P., and E. T. Parker, J. Biol. Chem. 266:12481-12486 (1991)), and that the difference in coagulant activity between human and porcine factor VIII appears to be based on a difference in amino acid sequence between one or more residues in the human and porcine A2 domains (Lollar, P., et al., J. Biol. Chem. 267:23652-23657 (1992)), incorporated herein by reference in its entirety.

Treatment of hemophilia A is by replacement therapy targeting restoration of FVIII activity to 1 to 5 % of normal levels to prevent spontaneous bleeding (Mannucci, P.M., et al., N. Engl. J. Med. 344:1773-1779 (2001), which is herein incorporated by reference in its entirety). There are plasma-derived and recombinant FVIII products available to treat bleeding episodes on-demand or to prevent bleeding episodes from occurring by treating prophylactically. Based on the short half-life of these products, however, e.g., 8-12 hours, treatment regimens require administration of frequent intravenous injections (3-4 times/week). Such frequent administration is painful and inconvenient.

Reduced mortality, prevention of joint damage, and improved quality of life have been important achievements due to the development of plasma-derived and recombinant FVIII. Prolonged protection from bleeding would represent another key advancement in the treatment of hemophilia A patients. However, to date, no products that allow for prolonged hemostatic protection have been developed. Therefore, there remains a need for improved methods of treating hemophilia due to factor VIII deficiency that are more tolerable, longer lasting, and more effective than current therapies.

BRIEF SUMMARY OF THE INVENTION

The present invention is directed to a method of reducing the annualized bleeding rate in a subject having hemophilia comprising administering to the subject an effective dose of a long-acting FVIII polypeptide at a dosing interval of about three days or longer. In one embodiment, the administration is prophylactic and individualized for the subject resulting in an annualized bleeding rate less than about 5.0, less than about 4.9, less than about 4.8, less than about 4.7, less than about 4.6, or less than about 4.5. For example, the median annualized bleeding rate for the individualized prophylaxis regimen is about 1.6. In another embodiment, the administration is prophylactic and weekly resulting in an annualized bleeding rate less than about 9.0, less than about 8.9, less than about 8.8, less than about 8.7, less than about 8.6, less than about 8.5, or less than about 8.4. For example, the median annualized bleeding rate for the weekly prophylaxis regimen is about 3.6. In other embodiments, the administration is on-demand or episodic resulting in an annualized bleeding rate less than about 55, less than about 54, less than about 53, less than about 52, less than about 51, less than about 50, less than about 49, less than about 48, or less than about 47. For example, the median annualized bleeding rate for the on-demand (episodic) treatment is about 33.6.

In certain embodiments, the effective dose is between about 20 IU/kg to about 90 IU/kg, *e.g.,* 20-30 IU/kg, 30-40 IU/kg, 40-50 IU/kg, 50-60 IU/kg, 60-70 IU/kg, 70-80 IU/kg, or 80-90 IU/kg, *e.g.,* 20 IU/kg, 25 IU/kg, 30 IU/kg, 35 IU/kg, 40 IU/kg, 45 IU/kg, 50 IU/kg, 55 IU/kg, 60 IU/kg, 65 IU/kg, 70 IU/kg, 75 IU/kg, 80 IU/kg, 85 IU/kg, or 90 IU/kg. In one aspect, the administration is prophylactic and individualized at an effective dose of about 25 IU/kg to about 65 IU/kg twice weekly or every three days or about 50 IU/kg to about 65 IU/kg every four or five days. An example of the individualized prophylaxis regimen includes a first dose of the long-acting FVIII polypeptide at about 25 IU/kg and a second dose of the long-acting FVIII polypeptide at about 50 IU/kg. In another aspect, the administration is prophylactic and at an effective dose of 65 IU/kg weekly. In other aspects, the administration is on-demand or episodic at an effective dose of 10 IU/kg to 75 IU/kg every 12 to 24 hours. In still other aspects, the effective dose is a fixed dose, which is standard across all body weights. For example, the fixed dose is about 2000 IU, about 2,500 IU, about 3,000 IU, about 3,500 IU, or about 4,000 IU per dose. In yet other aspects, the effective dose is a stratified dose.

In some embodiments, a trough level of the long-acting FVIII polypeptide is above 1%, above 2%, or above 3% of normal. In other embodiments, the long-acting FVIII polypeptide has a $T_{1/2beta}$ (activity) of about 7 hours to about 48 hours, about 6 hours to about 49 hours, or about 5 hours to about 50 hours. In yet other embodiments, the long-acting FVIII polypeptide has a $T_{1/2beta}$ (activity) mean of at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, or at least about 25 hours. In still other embodiments, the $T_{1/2beta}$ (activity) mean is about 19 hours. In certain embodiments, the $T_{1/2beta}$ (activity) mean is at least about 1.5 fold higher than a polypeptide consisting of amino acids 20 to 1457 of SEQ ID NO: 2 (SQ BDD FVIII, REFACTO®) or 20 to 2351 of SEQ ID NO: 6 (full-length mature FVIII, ADVATE®). In certain embodiments, a plasma trough level of the long-acting FVIII polypeptide is between about 1% and about 5%, between about 1% and about 6%, between about 1% and about 7%, between about 1% and about 8%, between about 1% and about 9%, between about 1% and about 10%, between about 1% and about 11%, between about 1% and about 12%, between about 1% and about 13%, between about 1% and about 14%, or between about 1% and about 15% above the baseline in the subject.

In one aspect, the long-acting FVIII polypeptide is a long-acting FVIII polypeptide comprising a FVIII polypeptide and a heterologous moiety. In another aspect, the heterologous moiety is an neonatal Fc receptor (FcRn) binding partner, *e.g.*, an Fc region. In other aspects, the long-acting FVIII polypeptide further comprises a second FcRn binding partner, *e.g.*, a second Fc region. In some aspects, the first FcRn binding partner and the second FcRn binding partner are associated by a covalent bond, *e.g.,* a disulfide bond. In a specific embodiment, the long-acting FVIII polypeptide is FVIII monomer dimer hybrid.

The invention also includes a method of estimating rFVIIIFc dosing information individualized for a patient, the method comprising: (a) receiving, by a computer-based system containing the rFVIIIFc population pharmacokinetic (popPK) model of Example 4 and a Bayesian estimation program, at least one of patient information and desired treatment outcome information, (b) calculating, by the computer-based system, individualized rFVIIIFc dosing information using the popPK model, the Bayesian estimation program, and the received information, and (c) outputting, by the computer-based system, the individualized dosing information.

In some embodiments, the method also comprises selecting a dosing regimen based on the output individualized dosing information of (c) and administering rFVIIIFc to the patient according to the selected dosing regimen.

In some embodiments, the desired treatment outcome information is desired rise in plasma FVIII activity level following dosing and the output information is dose for acute treatment.

In some embodiments, the desired treatment outcome information is desired dosing interval and the output information is dose for prophylaxis.

In some embodiments, the desired treatment outcome information is desired dose and the output information is interval for prophylaxis.

The invention also includes a method of estimating a rFVIIIFc dosing regimen based on median popPK, the method comprising: (a) receiving, by a computer-based system containing the rFVIIIFc popPK model of Example 4 and a Bayesian estimation program, at least one of patient information and desired treatment outcome information, (b) calculating, by the computer-based system, median rFVIIIFc PK information using the popPK model, the Bayesian estimation program, and the received information, and (c) outputting, by the computer-based system, the median PK information.

In some embodiments, the method also comprises selecting a dosing regimen based on the output median PK information of (c), and administering rFVIIIFc to a patient according to the selected dosing regimen.

The invention also includes a method of estimating individual patient rFVIIIFc PK, the method comprising: (a) receiving, by a computer-based system containing the rFVIIIFc population pharmacokinetic (popPK) model of Example 4 and a Bayesian estimation program, individual rFVIIIFc PK information, (b) estimating, by the computer-based system, individualized patient rFVIIIFc PK information using the popPK model, the Bayesian estimation program, and the received information, and (c) outputting, by the computer-based system, the individualized patient PK information.

In some embodiments, the method also comprises selecting a dosing regimen based on the output individualized patient PK information of (c), and administering rFVIIIFc to the patient according to the selected regimen.

In some embodiments (a) further comprises receiving, by the computer-based system, patient information.

In some embodiments the patient information is age, Von Willebrand Factor level, body weight, and hematocrit.

Additional embodiments include a computer readable storage medium having instructions stored thereon that, when executed by a processor, cause the processor to perform any of the above methods.

Additional embodiments include a system comprising a processor and a memory, the memory having instructions stored thereon that, when executed by the processor, cause the processor to perform any of the above methods.

BRIEF DESCRIPTION OF THE DRAWINGS

Figure 1 shows details on the design of the sequential PK subgroup (Arm 1) dosing and PK sampling.

Figure 2 shows that rFVIIIFc demonstrated an approximate 50% longer elimination half-life and mean residence time compared with octocog alfa (P<0.001).

Figure 3 shows the study design for the Kids-A-LONG study.

Figure 4 shows patient disposition enrolled in the A-LONG study (N=165).

Figure 5 shows subgroup analyses of annualized bleeding rates (ABR). The X-axis shows median annualized bleeding rates (25% - 75%), and the Y-axis shows subgroups: overall (Arm 3 (n=23), Arm 2 (n=23), and Arm 1 (n=117)); subgroups of Arm 1 by pre-study regimen (prophylaxis (n=86; and episodic (n=31)); by bleeds in prior 12 months (<12 (n=55); 12-20 (n=25); 21-50 (n=29); and >50 (n=7)); by age category (years) (12-17 (n=11); 18-65 (n=106)); by number of target joints (none present (n=45); ≤median (n=44); and >median (n=28)); and by prophylaxis interval compliance (<80% (n=4) and ≥80% (n=113).

Figure 6 shows the number of bleeding episodes in the prior 12 months compared with the on-study ABR by prior FVIII regimen. X-axis shows median annualized bleed rate (25% - 75%). Diamond shows baseline median, and square shows on-study median. Y axis shows Arm 1 individualized prophylaxis (prior prophylaxis (n=85) and prior episodic (n=31); Arm 2 weekly prophylaxis (prior episodic (n=23); and Arm 3 episodic treatment (prior episodic (n=23)).

Figure 7 shows dose modification in Arm 1 (individualized prophylaxis).

Figure 8 is a plot of the individual time to 1% estimates for ADVATE® and rFVIIIFc. Each point represents one subject who received both ADVATE® and rFVIIIFc. Doses were 25, 50, or 65 IU/kg. The diagonal line is the identity line.

Figures 9A-D show goodness of fit diagnostic plots for the two-compartment model of pharmacokinetics of long-acting rFVIIIFc. Figure 9A shows observed (DV) v. predicted (PRED). Figure 9B shows observed (DV) v. individual predicted (IPRE). Figure 9C shows conditional weighted residuals (CWRES) v. Predicted (PRED). Figure 9D shows conditional weighted residuals (CWRES) v. Time (TIME).

Figures 10A to 10C show the predicted steady-state activity profiles of selected rFVIIIFc prophylaxis dosing regimens. The line in the middle represents the median; the dark shaded area covers the 25th to 75th percentiles; the light shaded area covers the 5th to 95th percentiles. Figure 10A shows a dosing regimen of 50IU/kg at every 3 days. Figure 10B shows a dosing regimen of 50IU/kg at every 4 days. Figure 10C shows a dosing regimen of 50IU/kg at every 5 days.

Figure 11 shows the predicted FVIII activity for the hypothetical perioperative dosing regimen in Table 20. The middle line shows the 50 percentile. The light shaded areas represent the $5^{th}$ to $25^{th}$ percentile and $75^{th}$ to $95^{th}$ percentile. The dark shaded areas represent the $25^{th}$ to $50^{th}$ percentile and the $50^{th}$ to $75^{th}$ percentile.

Figure 12 shows the predicted FVIII activity for the hypothetical perioperative dosing regimen in Table 21.

Figure 13 is a representative plot comparing the simulated and observed FVIII activities within the first 21 days after the first rFVIIIFc surgical dose (n=13; 9 major surgeries, 4 minor surgeries). The upper line represents the line of identity (unity line); the lower line (linear regression line) is the nonparametric fit of the data. There was good correlation between the observed FVIII activity data and that predicted by the PK model (relative prediction error 95% CI).

Figure 14 shows a proposed input and output for individual PK assessment.

Figure 15 shows a proposed input and output for individualized dosing regimen selection for episodic treatment.

Figure 16 shows a proposed input and output for dosing regimen selections without individualized PK assessment.

Figure 17 shows another proposed output for dosing regimen selections without individualized PK assessment.

Figure 18 shows an example computer system that can be used in embodiments.

Figure 19A-B shows the goodness of fit diagnostics of the final model for rFVIIIFc. Figure 19A shows Observed (DV) vs Predicted (PRED), and Figure 19B shows Observed (DV) vs Individual Predicted (IPRE).

Figure 19C-D shows shows the goodness of fit diagnostics of the final model for rFVIIIFc. Figure 19C shows Conditional Weighted Residuals (CWRES) vs Predicted (PRED), and Figure 19D shows Conditional Weighted Residuals (CWRES) vs Time (TIME).

Figure 20 shows the visual predictive check (VPC) results (Baseline PK profiles) of the final model for rFVIIIFc.

Figure 21 shows the external validation of the rFVIIIFc model. Observed (DV) vs Individual Predicted (IPRE) values from the validation set only.

Figure 22A-B shows the goodness of fit diagnostics of the base model for ADVATE®. Figure 22A shows Observed (DV) vs Individual Predicted (IPRE); Figure 22B shows Conditional Weighted Residuals (CWRES) vs Predicted (PRED).

Figure 23 shows an ADVATE® model comparison. The lower line in the graph is the current model; the upper line is the model reported by Bjorkman *et al.* Superimposed are the ADVATE® activity data from the Phase 3 study dataset.

Figure 24A-B shows steady state activity profiles of selected rFVIII prophylaxis dosing regimens. Figure 24A shows 50 IU/dL E3D; Figure 24B shows 50 IU/dL E4D.

Figure 24C-D shows steady state activity profiles of selected rFVIII prophylaxis dosing regimens. Figure 24C shows 50 IU/dL E5D; Figure 24D shows 65 IU/dL QW.

Figure 25A shows a comparison of individual Bayesian parameter estimates for ADVATE® and rFVIIIFc. Clearance - Each point represents one individual. Both agents were administered at a nominal dose of 50 IU/kg.

Figure 25B shows a comparison of individual Bayesian parameter estimates for ADVATE® and rFVIIIFc. Central Volume - each point represents one individual. The center line is the identity line. Both agents were administered at a nominal dose of 50 IU/kg.

Figure 25C shows a comparison of individual Bayesian parameter estimates for ADVATE® and rFVIIIFc. Time to 1 IU/dL (Time 1%) - each point represents one individual. The center line is the identity line. Both agents were administered at a nominal dose of 50 IU/kg.

## DETAILED DESCRIPTION OF THE INVENTION

The present invention provides methods of using Factor VIII for reducing the annualized bleeding rate in subjects having Hemophilia A. The present invention also provides improved Factor VIII chimeric polypeptides and methods of production.

### *I. Definitions*

It must be noted that, as used in this specification and the appended claims, the singular forms "a", "an" and "the" include plural referents unless the context clearly dictates otherwise. The terms "a" (or "an"), as well as the terms "one or more," and "at least one" can be used interchangeably herein.

The term "about" is used herein to mean approximately, roughly, around, or in the regions of. When the term "about" is used in conjunction with a numerical range, it modifies that range by extending the boundaries above and below the numerical values set forth. In general, the term "about" is used herein to modify a numerical value above and below the stated value by a variance of 10 percent, up or down (higher or lower).

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

It is understood that wherever embodiments are described herein with the language "comprising," otherwise analogous embodiments described in terms of "consisting of' and/or "consisting essentially of' are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. Unless otherwise indicated, amino acid sequences are written left to right in amino to carboxy orientation. The headings provided herein are not limitations of the various aspects or embodiments of the disclosure, which can be had by reference to the specification as a whole. Accordingly, the terms defined immediately below are more fully defined by reference to the specification in its entirety. Amino acids are referred to herein by either their commonly known three letter symbols or by the one-letter symbols recommended by the IUPAC-IUB Biochemical Nomenclature Commission. Nucleotides, likewise, are referred to by their commonly accepted single-letter codes.

The term "polypeptide," "peptide" and "protein" are used interchangeably and refer to a polymeric compound comprised of covalently linked amino acid residues.

The term "polynucleotide" and "nucleic acid" are used interchangeably and refer to a polymeric compound comprised of covalently linked nucleotide residues. Polynucleotides can be DNA, cDNA, RNA, single stranded, or double stranded, vectors, plasmids, phage, or viruses. Polynucleotides include those in Table 15, which encode the polypeptides of Table 16 (see Table 15). Polynucleotides also include fragments of the polynucleotides of Table 15, *e.g.*, those that encode fragments of the polypeptides of Table 16, such as the Factor VIII, Fc, signal sequence, propeptide, 6His (SEQ ID NO: 18) and other fragments of the polypeptides of Table 16.

"Administering," as used herein, means to proscribe or give a pharmaceutically acceptable Factor VIII polypeptide of the invention to a subject via a pharmaceutically acceptable route. Examples of routes of administration include, but are not limited to, intravenous, *e.g.*, intravenous injection and intravenous infusion, *e.g.*, via central venous access. Additional routes of administration include subcutaneous, intramuscular, oral, nasal, and pulmonary administration. A long-acting FVIII polypeptides (a FVIII chimeric or hybrid proteins) can be administered as part of a pharmaceutical composition comprising at least one excipient. Advantages of the present invention include: improved regimen compliance; reduced break through bleeds; increased protection of joints from bleeds; prevention of joint damage; reduced morbidity; reduced mortality; prolonged protection from bleeding; decreased thrombotic events; and improved quality of life.

The terms "long-acting" and "long-lasting" are used interchangeably herein. In one embodiment, the term "long-acting" or "long-lasting" indicates that a FVIII activity as a result of administration of the "long-acting" FVIII polypeptide is longer than the FVIII activity of a wild-type FVIII (*e.g.*, ADVATE® or plasma-derived FVIII ("pdFVIII")). The "longer" FVIII activity can be measured by any known methods in the art, *e.g.*, aPTT assay, chromogenic assay, ROTEM®, TGA, etc. In one embodiment, the "longer" FVIII activity can be shown by the $T_{1/2beta}$ (activity). In another embodiment, the "longer" FVIII activity can be shown by the level of FVIII antigen present in plasma, *e.g.*, by the $T_{1/2beta}$ (antigen). In other embodiments, the long-acting or long-lasting FVIII polypeptide works longer in a coagulation cascade, *e.g.*, is active for a longer period, compared to a wild-type FVIII polypeptide, *i.e.*, a polypeptide consisting of amino acids 20 to 1457 of SEQ ID NO: 2, *i.e.*, SQ BDD FVIII (REFACTO®) or a polypeptide consisting of amino acids 20 to 2351 of SEQ ID NO: 6 (ADVATE®).

The term "chimeric polypeptide," as used herein, means a polypeptide that includes within it at least two polypeptides (or portions thereof such as subsequences or peptides) from different sources. Chimeric polypeptides can include two, three, four, five, six, seven, or more polypeptides or portions thereof from different sources, such as different genes, different cDNAs, or different animal or other species. Chimeric polypeptides can include one or more linkers joining the different polypeptides or portions thereof. Thus, the polypeptides or portions thereof can be joined directly or they can be joined indirectly, via linkers, or both, within a single chimeric polypeptide. Chimeric polypeptides can include additional peptides such as signal sequences and sequences such as 6His (SEQ ID NO: 18) and FLAG that aid in protein purification or detection. In addition, chimeric polypeptides can have amino acid or peptide additions to the N- and/or C-termini. Exemplary chimeric polypeptides of the invention are Factor VIII-FcRn BP chimeric polypeptides, *e.g.*, Factor VIII-Fc polypeptides such as the FVIIIFc, SEQ ID NO:2 or 6 (Table 16) with or without its signal sequence and propeptide.

"Hybrid" polypeptides and proteins, as used herein, means a combination of a chimeric polypeptide with a second polypeptide. The chimeric polypeptide and the second polypeptide in a hybrid can be associated with each other via protein-protein interactions, such as charge-charge or hydrophobic interactions. The chimeric polypeptide and the second polypeptide in a hybrid can be associated with each other via disulfide or other covalent bond(s). Hybrids are described in WO 2004/101740 and WO 2006/074199, each of which is incorporated herein by reference in its entirety. See also U.S. Patent Nos. 7,404,956 and 7,348,004, each of which is incorporated herein by reference in its entirety. The second polypeptide can be a second copy of the same chimeric polypeptide or it can be a non-identical chimeric polypeptide. See, *e.g.*, Table 16. In one embodiment, the second polypeptide is a polypeptide comprising an FcRn binding partner. FcRn binding partners binds to FcRn and protects the FcRn binding partner containing molecule from catabolism, thus extending the plasma half-life. In another embodiment, the chimeric polypeptide is a chimeric Factor VIII-Fc polypeptide and the second polypeptide consists essentially of Fc, *e.g.*, a rFVIIIFc recombinant fusion protein consisting of a single molecule of recombinant B-domain deleted human FVIII (BDD-rFVIII) fused to the dimeric Fc domain of the human IgG1, with no intervening linker sequence. This hybrid polypeptide is referred to herein as FVIIIFc monomeric Fc fusion protein, FVIIIFc monomer hybrid, monomeric FVIIIFc hybrid, and FVIIIFc monomer-dimer. See Table 16A. The Examples provide preclinical and clinical data for this hybrid polypeptide.

The second polypeptide in a hybrid can comprise or consist essentially of a sequence at least 90% or 95% identical to the amino acid sequence shown in Table 16A(ii) without a signal sequence (amino acids 21 to 247 of SEQ ID NO:4) or at least 90% or 95% identical to the amino acid sequence shown in Table 16A(ii) with a signal sequence (amino acids 1 to 247 of SEQ ID NO:4). The second polypeptide can comprise or consist essentially of a sequence identical to the amino acid sequence shown in Table 16A(ii) without a signal sequence (amino acids 21 to 247 of SEQ ID NO:4) or identical to the amino acid sequence shown in Table 16A(ii) with a signal sequence (amino acids 1 to 247 of SEQ ID NO:4).

Factor VIII coagulant activity is expresses as International Unit(s) (IU). One IU of Factor VIII activity corresponds approximately to the quantity of Factor VIII in one milliliter of normal human plasma. Several assays are available for measuring Factor VIII activity, including the one stage clotting assay (activated partial thromboplastin time; aPTT), thrombin generation time (TGA) and rotational thromboelastometry (ROTEM®).

"Dosing interval," as used herein, means the amount of time that elapses between multiple doses being administered to a subject. Dosing interval can thus be indicated as ranges. The dosing interval in the methods of the invention using a chimeric FVIII-FcRn BP, *e.g.*, a chimeric FVIII -Fc, can be at least about one and one-half to eight times longer than the dosing interval required for an equivalent amount (in IU/kg) of the Factor VIII without the FcRn BP, *e.g.*, Fc portion (*i.e.*, a polypeptide consisting of said FVIII). The dosing interval when administering, *e.g.*, a Factor VIII-Fc chimeric polypeptide (or a hybrid) of the invention can be at least about one and one-half times longer than the dosing interval required for an equivalent amount of the Factor VIII without the FcRn BP, *e.g.*, Fc portion (*i.e.*, a polypeptide consisting of the Factor VIII). The dosing interval can be at least about one and one-half to eight times longer than the dosing interval required for an equivalent amount of the Factor VIII without, *e.g.*, the Fc portion (or a polypeptide consisting of the Factor VIII).

The term "dosing frequency" as used herein refers to the frequency of administering doses of a long-acting FVIII polypeptide in a given time. Dosing frequency can be indicated as the number of doses per a given time, *e.g.*, once a week or once in two weeks.

The term "prophylaxis of one or more bleeding episode" or "prophylactic treatment" as used herein means administering a FVIII polypeptide in multiple doses to a subject over a course of time to increase the level of FVIII activity in a subject's plasma. In one embodiment, "prophylaxis of one or more bleeding episode" indicates use of a long-acting FVIII polypeptide to prevent or inhibit occurrence of one or more spontaneous or uncontrollable bleeding or bleeding episodes or to reduce the frequency of one or more spontaneous or uncontrollable bleeding or bleeding episodes. In another embodiment, the increased FVIII activity level is sufficient to decrease the incidence of spontaneous bleeding or to prevent bleeding in the event of an unforeseen injury. Prophylactic treatment decreases or prevents bleeding episodes, for example, those described under on-demand treatment. Prophylactic treatment can be individualized, as discussed under "dosing interval", *e.g.*, to compensate for inter-patient variability.

The term "about twice weekly" as used herein means approximate number, and "about twice weekly" can include twice in one week, *e.g.*, a first dose in three days and a second dose in three days, a first dose in three days and a second dose in four days, a first dose in four days and a second dose in three days, a first dose in four days and a second dose in four days. The term "about twice weekly" can also include every three days, every four days, or every five days.

The term "about once a week" as used herein means approximate number, and "about once a week" can include every seven days $\pm$ two days, *i.e.*, every five days to every nine days. The dosing frequency of "once a week" thus can be every five days, every six days, every seven days, every eight days, or every nine days.

The term "individualized prophylaxis" or "prophylactic and individualized" as used herein means use of a long-acting FVIII polypeptide for an individualized dosing and/or dosing interval or frequency to prevent or inhibit occurrence of one or more spontaneous and/or uncontrollable bleeding or bleeding episodes or to reduce the frequency of one or more spontaneous and/or uncontrollable bleeding or bleeding episodes. "Individualized" within the context of prophylaxis is used synonymous with "tailored" throughout this application. For example, "individualized prophylaxis" also means "tailored prophylaxis" and "prophylactic and individualized" also means "prophylactic and tailored." In one embodiment, the "individualized interval" includes every 3 days $\pm$2 days, i.e. every day to every five days. The dosing frequency of the "individualized interval prophylaxis" thus can be every day, every two days, every three days, every four days, or every five days.

The term "on-demand treatment," as used herein, means treatment that is intended to take place over a short course of time and is in response to an existing condition, such as a bleeding episode, or a perceived short term need such as planned surgery. The "on-demand treatment" is used interchangeably with "episodic" treatment. Conditions that can require on-demand treatment include a bleeding episode, hemarthrosis, muscle bleed, oral bleed, hemorrhage, hemorrhage into muscles, oral hemorrhage, trauma, trauma capitis, gastrointestinal bleeding, intracranial hemorrhage, intra-abdominal hemorrhage, intrathoracic hemorrhage, bone fracture, central nervous system bleeding, bleeding in the retropharyngeal space, bleeding in the retroperitoneal space, or bleeding in the illiopsoas sheath. Bleeding episodes other than these are also included. The subject can be in need of surgical prophylaxis, peri-operative management, or treatment for surgery. Such surgeries include minor surgery, major surgery, tooth extraction, tonsillectomy, other dental/thoraco-facial surgeries, inguinal herniotomy, synovectomy, total knee replacement, other joint replacement, craniotomy, osteosynthesis, trauma surgery, intracranial surgery, intra-abdominal surgery, or intrathoracic surgery. Surgeries other than these are also included.

Additional conditions that can require on-demand treatment include minor hemorrhage, hemarthroses, superficial muscle hemorrhage, soft tissue hemorrhage, moderate hemorrhage, intramuscle or soft tissue hemorrhage with dissection, mucous membrane hemorrhage, hematuria, major hemorrhage, hemorrhage of the pharynx, hemorrhage of the retropharynx, hemorrhage of the retroperitonium, hemorrhage of the central nervous system, bruises, cuts, scrapes, joint hemorrhage, nose bleed, mouth bleed, gum bleed, intracranial bleeding, intraperitoneal bleeding, minor spontaneous hemorrhage, bleeding after major trauma, moderate skin bruising, or spontaneous hemorrhage into joints, muscles, internal organs or the brain. Additional reasons for on-demand treatment include the need for peri-operative management for surgery or dental extraction, major surgery, extensive oral surgery, urologic surgery, hernia surgery, orthopedic surgery such as replacement of knee, hip, or other major joint.

The term "treatment" or "treating" as used herein means amelioration or reduction of one or more symptoms of bleeding diseases or disorders including, but not limited to, hemophilia B. In one embodiment, "treatment of' or "treating" a bleeding disease or disorder includes prevention of one or more symptoms of a bleeding disease or disorder. In a bleeding disease or disorder caused by a FVIII deficiency (*e.g.*, a low baseline FVIII activity), the term "treatment" or "treating" means a FVIII replacement therapy. By administering a long-acting FVIII polypeptide to a subject, the subject can achieve and/or maintain a plasma trough level of a FVIII activity at about 1 IU/dl or above 1 IU/dl. In other embodiments, "treatment" or "treating" means reduction of the frequency of one or more symptoms of bleeding diseases or disorders, *e.g.*, spontaneous or uncontrollable bleeding episodes. "Treatment," however, need not be a cure.

The term "perioperative management" as used herein means use of a long-acting FVIII polypeptide before, concurrently with, or after an operative procedure, *e.g.,* a surgical operation. The use for "perioperative management" of one or more bleeding episode includes surgical prophylaxis before (*i.e.*, preoperative), during (*i.e.*, intraoperative), or after (*i.e.*, postoperative) a surgery to prevent one or more bleeding or bleeding episode or reducing or inhibiting spontaneous and/or uncontrollable bleeding episodes before, during, and after a surgery.

"Baseline," as used herein, is the lowest measured plasma Factor VIII level in a subject prior to administering a dose. The FVIII plasma levels can be measured at two time points prior to dosing: at a screening visit and immediately prior to dosing. Alternatively, (a) the baseline in patients whose pretreatment FVIII activity is <1%, who have no detectable FVIII antigen, and have nonsense genotypes can be defined as 0%, (b) the baseline for patients with pretreatment FVIII activity <1% and who have detectable FVIII antigen can be set at 0.5%, (c) the baseline for patients whose pretreatment FVIII activity is between 1 - 2% is Cmin (the lowest activity throughout the PK study), and (d) the baseline for patients whose pretreatment FVIII activity is ≥2% can be set at 2%. Activity above the baseline pre-dosing can be considered residue drug from prior treatment, and can be decayed to baseline and subtracted from the PK data following rFVIIIFc dosing.

"$T_{1/2\beta}$," or "$T_{1/2\ beta}$" or "Beta HL," as used herein, is half-life associated with elimination phase, t1/2β=(ln2)/elimination rate constant associated with the terminal phase. The $T_{1/2beta}$ can be measured by FVIII activity or by FVIII antigen level in plasma. The $T_{1/2\ beta}$ based on activity is shown as $T_{1/2beta}$ (activity), and the $T_{1/2beta}$ based on the FVIII antigen level can be shown as $T_{1/2beta}$ (antigen). Both $T_{1/2beta}$ (activity) and $T_{1/2beta}$ (antigen) can be shown as ranges or a geometric mean.

"Trough," as used herein, is the lowest plasma FVIII activity level reached after administering a dose of chimeric polypeptide of the invention or another FVIII molecule and before the next dose is administered, if any. Trough is used interchangeably herein with "threshhold." Baseline FVIII levels are subtracted from measured FVIII levels to calculate the trough level.

The term "annualized bleeding rate" ("ABR") as used herein refers to the number of bleeding episodes (including spontaneous and traumatic bleeds) experienced by a subject during a defined time period, extrapolated to 1 year. For example two bleeds in six months would indicate an ABR of four. The median ABR provides the middle value among all observed ABRs, indicating that half of the subjects had individual ABRs less than or equal to the median and half had ABRs greater than or equal to the median.

The term "inter quartile range" ("IQR") as used herein refers to a measure of statistical dispersion, being equal to the difference between the upper and lower quartiles. Unlike (total) range, the interquartile range is a robust statistic, having a breakdown point of 25%, and is thus often preferred to the total range. For a symmetric distribution (where the median equals the midline, the average of the first and third quartiles), half the IQR equals the median absolute deviation (MAD). The median is the corresponding measure of central tendency.

"Subject," as used herein means a human. Subject as used herein includes an individual who is known to have at least one incidence of uncontrolled bleeding episodes, who has been diagnosed with a disease or disorder associated with uncontrolled bleeding episodes, *e.g.*, a bleeding disease or disorder, *e.g.*, hemophilia A, who is susceptible to uncontrolled bleeding episodes, *e.g.*, hemophilia, or any combinations thereof. Subjects can also include an individual who is in danger of one or more uncontrollable bleeding episodes prior to a certain activity, *e.g.*, a surgery, a sport activity, or any strenuous activities. The subject can have a baseline FVIII activity less than 1%, less than 0.5%, less than 2%, less than 2.5%, less than 3%, or less than 4%. Subjects also include pediatric humans. Pediatric human subjects are birth to 20 years, preferably birth to 18 years, birth to 16 years, birth to 15 years, birth to 12 years, birth to 11 years, birth to 6 years, birth to 5 years, birth to 2 years, or 2 to 11 years of age.

"Therapeutic dose," "dose," "effective dose," or "dosing amount" as used herein, means a dose that achieves a plasma trough level of a FVIII activity at least about 1 IU/dl or above 1 IU/dl in the subject administered with the long-acting FVIII polypeptide. For the purpose of this invention, in one embodiment, the "dose" refers to the amount of the doses that a plasma trough level of a FVIII activity is maintained at least about 1 IU/dl or above 1 IU/dl, at least about 2 IU/dl or above 2 IU/dl, at least about 3 IU/dl or above 3 IU/dl, at least about 4 IU/dl or above 4 IU/dl, or at least about 5 IU/dl or above 5 IU/dl throughout the administration of the long-acting FVIII polypeptide. In another embodiment, the "dose" reduces or decreases frequency of bleeding or bleeding disorder. In other embodiments, the "dose" stops ongoing, uncontrollable bleeding or bleeding episodes. In still other embodiments, the "dose" prevents spontaneous bleeding or bleeding episodes in a subject susceptible to such spontaneous bleeding or bleeding episodes. The "dose" or "therapeutic dose" need not cure hemophilia.

The term "target joint" is defined as a major joint (*e.g.*, hip, elbow, wrist, shoulder, knee, and ankle) into which repeated bleeding occurs (frequency of ≥3 bleeding episodes into the same joint in a consecutive 6-month period).

The term "bleeding episode" as used herein adopts a standardized definition of a bleeding episode. A bleeding episode started from the first sign of bleeding, and ended 72 hours after the last treatment for the bleeding, within which any symptoms of bleeding at the same location, or injections less than or equal to 72 hours apart, were considered the same bleeding episode. Any injection to treat the bleeding episode, taken more than 72 hours after the preceding one, was considered the first injection to treat a new bleeding episode at the same location. Any bleeding at a different location was considered a separate bleeding episode regardless of time from last injection. This definition has been proposed by the Subcommittee on Standards and Criteria, FVIII/FIX subcommittee of the International Society of Thrombosis and Hemostasis and has been used by the PedNet multicenter study in hemophilia.

The term "annualized bleeding rates (ABRs) as used herein refers to the number of bleeding episodes that are annualized for each patient using the following formula:

$$ABR = \frac{\text{Number of bleeding episodes during efficacy period}}{\text{Total number of days during the efficacy period}} \times 365.25$$

"Variant," as used herein, refers to a polynucleotide or polypeptide differing from the original polynucleotide or polypeptide, but retaining essential properties thereof, *e.g.*, Factor VIII coagulant activity or Fc (FcRn binding) activity. Generally, variants are overall closely similar, and, in many regions, identical to the original polynucleotide or polypeptide. Variants include polypeptide and polynucleotide fragments, deletions, insertions, and modified versions of original polypeptides.

### *II. Method of Administering*

Treatment of hemophilia A is a replacement therapy targeting restoration of FVIII activity to 1 to 5 % of normal levels to prevent spontaneous bleeding (Mannucci, P.M. et al., N. Engl. J. Med. 344:1773-9 (2001), herein incorporated by reference in its entirety).

The present invention provides a method of reducing or decreasing an annualized bleeding rate (ABR) of a subject having hemophilia comprising administering to the subject an effective dose of a long-acting FVIII polypeptide. In one embodiment, the long-acting FVIII polypeptide is administered at a dosing interval of every three days or longer. In another embodiment, the effective dose is between about 20IU/kg and about 90IU/kg. In other embodiments, the effective dose is 20-30 IU/kg, 30-40 IU/kg, 40-50 IU/kg, 50-60 IU/kg, 60-70 IU/kg, 70-80 IU/kg, or 80-90 IU/kg. In yet other embodiments, the effective dose is 20 IU/kg, 25 IU/kg, 30 IU/kg, 35 IU/kg, 40 IU/kg, 45 IU/kg, 50 IU/kg, 55 IU/kg, 60 IU/kg, 65 IU/kg, 70 IU/kg, 75 IU/kg, 80 IU/kg, 85 IU/kg, or 90 IU/kg.

In certain embodiments, administration of a long-acting FVIII polypeptide is for individualized (tailored) prophylaxis and results in an ABR of less than about 5.5, less than about 5.4, less than about 5.3, less than about 5.2, less than about 5.1, less than about 5.0, less than about 4.9, less than about 4.8, less than about 4.7, less than about 4.6, or less than about 4.5. In other embodiments, the administration results in an ABR of about 4.7 to 0. In some embodiments, the median of the ABR is about 1.6. In yet other embodiments, the mean of the ABR is about 2.9. In one aspect of the individualized prophylaxis regimen, the effective dose is about 25IU/kg to about 65IU/kg given every three to five days. For example, the effective dose is about 25IU/kg to about 65IU/kg given every three days. In another aspect, the effective dose is about 50IU/kg to about 65IU/kg every four days or every five days. In other aspects, the effective dose is up to 65IU/kg every three days. In still other embodiments, the effective dose of the long-acting FVIII polypeptide is modified based on the patient's pharmacokinetic profile. In one example, the patent is administered with two doses initially, a first dose of 25IU/kg on day 1 and a second dose of 50IU/kg on day 4. If the pharmacokinetic data show less than 1% trough level of normal FVIII activity after the initial two doses, the patient is then administered with about 25IU/kg to about 65IU/kg every three days. In another example, if the pharmacokinetic data show higher than 1% trough level of normal FVIII activity for five days from the dosing, the patient is then administered with about 50IU/kg to about 65IU/kg every five days. If the patient experiences more than two spontaneous bleeds over eight weeks period after administration of about 50IU/kg to about 65IU/kg every five days, the patient is administered one of the followings: (1) up to about 65 IU/kg every three days (for the target trough level of up to 5% of normal), (2) about 50IU/kg to about 65IU/kg every four days (for the target trough level up to 5% of normal), or (3) about 25IU/kg to about 65IU/kg every three days (for the target trough level of 1% to 3% of normal). If the patient receiving about 50IU/kg to about 65IU/kg every four days still experiences more than two spontaneous bleeds, the maximum dose can go up to about 65IU/kg every three days. In some examples, the Interquartile Range (IQR) of the ABR for the individualized prophylaxis regimen is 0 to 4.7.

In some embodiments, administration of a long-acting FVIII polypeptide is for weekly prophylaxis and results in an ABR of less than about 9.0, less than about 8.9, less than about 8.8, less than about 8.7, less than about 8.6, less than about 8.5, or less than about 8.4. In one example, the administration results in an ABR between about 8.4 and 0. In another example, the median of the ABR for weekly prophylaxis is about 3.6. In other examples, the mean of the ABR for weekly prophylaxis is about 8.8. In other embodiments, an effective dose for weekly prophylaxis is about 65IU/kg once every week, *e.g.*, every five days, every six days, every seven days, every eight days, or every nine days. In some examples, the IQR of the ABR for the weekly prophylaxis regimen is 1.9 to 8.4.

In other embodiments, administration of a long-acting FVIII polypeptide is for episodic or on-demand treatment and results in an ABR of less than about 55, less than about 54, less than about 53, less than about 52, less than about 51, less than about 50, less than about 49, less than about 48, or less than about 47. In one example, the administration results in an ABR between about 49 and 0, *e.g.*, between 48.7 and 0. In another example, the median of the ABR for episodic (on-demand) treatment is about 33.5. In other examples, the mean of the ABR for episodic (on-demand) treatment is about 37.23. In other embodiments, an effective dose for on-demand treatment is about 10IU/kg to 75IU/kg every 12 to 24 hours. In some examples, the IQR of the ABR for the on-demand treatment is 21.1 to 48.7.

In some embodiments, the effective dose for individualized prophylaxis, weekly prophylaxis, or episodic treatment is a fixed dose or a stratified dose. In one aspect, the fixed dose is about 2,000IU per dose, about 2,500IU per dose, about 3,000IU per dose, about 3,500IU per dose, or about 4,000IU per dose.

The present invention provides a method of administering Factor VIII to a human subject in need thereof (*e.g.*, human patient), comprising administering to the subject a therapeutic dose of a long-acting Factor VIII polypeptide, *e.g.*, a Factor VIII-Fc polypeptide, or a hybrid of such a polypeptide at a dosing interval at least about one and one-half times longer than the dosing interval required for an equivalent dose of the Factor VIII without the non-Factor VIII portion (a polypeptide consisting of the Factor VIII portion), *e.g.*, without the Fc portion. The present invention is also directed to a method of reducing an annualized bleeding rate in a human subject in need thereof comprising administering the long-acting Factor VIII polypeptide.

The dosing interval can be at least about one and one-half to six times longer, one and one-half to five times longer, one and one-half to four times longer, one and one-half to three times longer, or one and one-half to two times longer, than the dosing interval required for an equivalent dose of the Factor VIII without the non-Factor VIII portion (a polypeptide consisting of the Factor VIII portion), *e.g.*, without the Fc portion. The dosing interval can be at least about one and one-half, two, two and one-half, three, three and one-half, four, four and one-half, five, five and one-half or six times longer than the dosing interval required for an equivalent dose of the Factor VIII without the non-Factor VIII portion (a polypeptide consisting of the Factor VIII portion), *e.g.*, without the Fc portion. The dosing interval can be about every three, four, five, six, seven, eight, nine, ten, eleven, twelve, thirteen, or fourteen days or longer.

The dosing interval can be at least about one and one-half to 5, one and one-half, 2, 3, 4, or 5 days or longer.

The present invention also provides a method of administering Factor VIII to a subject in need thereof, comprising administering to the subject a therapeutic dose of a polypeptide comprising a Factor VIII and an Fc or a hybrid of such a polypeptide at a dosing interval of about every three, four, five, six, seven, eight, nine, ten, eleven, twelve, thirteen, or fourteen days or longer.

The methods of the invention can be practiced on a subject in need of prophylactic treatment or on-demand treatment.

A "B domain" of Factor VIII, as used herein, is the same as the B domain known in the art that is defined by internal amino acid sequence identity and sites of proteolytic cleavage by thrombin, *e.g.*, residues Ser741-Arg1648 of full length mature human factor VIII. The other human factor VIII domains are defined by the following amino acid residues: A1, residues Ala1-Arg372; A2, residues Ser373-Arg740; A3, residues Ser1690-Ile2032; C1, residues Arg2033-Asn2172; C2, residues Ser2173-Tyr2332. The A3-C1-C2 sequence includes residues Ser1690-Tyr2332. The remaining sequence, residues Glu1649-Arg1689, is usually referred to as the factor VIII light chain activation peptide. The locations of the boundaries for all of the domains, including the B domains, for porcine, mouse and canine factor VIII are also known in the art. In one embodiment, the B domain of Factor VIII is deleted ("B domain deleted factor VIII" or "BDD FVIII"). An example of a BDD FVIII is REFACTO® (recombinant BDD FVIII), which has the same sequence as the Factor VIII portion of the sequence in Table 16A(i) (amino acids 1 to 1457 or 20 to 1457 of SEQ ID NO:2). In another embodiment, the B domain deleted Factor VIII contains an intact intracellular processing site, which corresponds to Arginine at residue 754 of B domain deleted Factor VIII, which corresponds to Arginine residue 773 of SEQ ID NO: 2, or residue 1648 of full-length Factor VIII, which corresponds to Arginine residue 1667 of SEQ ID NO: 6. The sequence residue numbers used herein without referring to any SEQ ID Numbers correspond to the Factor VIII sequence without the signal peptide sequence (19 amino acids) unless otherwise indicated. For example, S743/Q1638 of full-length Factor VIII corresponds to S762/Q1657 of SEQ ID NO: 6 due to the 19 amino acid signal peptide sequence. In other embodiments, the B domain deleted FVIII comprises a substitution or mutation at an amino acid position corresponding to Arginine 1645, a substitution or mutation at an amino acid position corresponding to Arginine 1648, or a substitution or mutation at amino acid positions corresponding to Arginine 1645 and Arginine 1648 in full-length Factor VIII. In some embodiments, the amino acid substituted at the amino acid position corresponding to Arginine 1645 is a different amino acid from the amino acid substituted at the amino acid position corresponding to Arginine 1648. In certain embodiments, the substitution or mutation is an amino acid other than arginine, *e.g.*, alanine.

A "B domain deleted factor VIII" can have the full or partial deletions disclosed in U.S. Patent Nos. 6,316,226, 6,346,513, 7,041,635, 5,789,203, 6,060,447, 5,595,886, 6,228,620, 5,972,885, 6,048,720, 5,543,502, 5,610,278, 5,171,844, 5,112,950, 4,868,112, and 6,458,563, each of which is incorporated herein by reference in its entirety. In some embodiments, a B domain deleted factor VIII sequence of the present invention comprises any one of the deletions disclosed at col. 4, line 4 to col. 5, line 28 and examples 1-5 of U.S. Patent No. 6,316,226 (also in US 6,346,513). In some embodiments, a B domain deleted factor VIII of the present invention has a deletion disclosed at col. 2, lines 26-51 and examples 5-8 of U.S. Patent No. 5,789,203 (also US 6,060,447, US 5,595,886, and US 6,228,620). In some embodiments, a B domain deleted factor VIII has a deletion described in col. 1, lines 25 to col. 2, line 40 of US Patent No. 5,972,885; col. 6, lines 1-22 and example 1 of U.S. Patent no. 6,048,720; col. 2, lines 17-46 of U.S. Patent No. 5,543,502; col. 4, line 22 to col. 5, line 36 of U.S. Patent no. 5,171,844; col. 2, lines 55-68, figure 2, and example 1 of U.S. Patent No. 5,112,950; col. 2, line 2 to col. 19, line 21 and Table 2 of U.S. Patent No. 4,868,112; col. 2, line 1 to col. 3, line 19, col. 3, line 40 to col. 4, line 67, col. 7, line 43 to col. 8, line 26, and col. 11, line 5 to col. 13, line 39 of U.S. Patent no. 7,041,635; or col. 4, lines 25-53, of U.S. Patent No. 6,458,563. In some embodiments, a B domain deleted factor VIII has a deletion of most of the B domain, but still contains amino-terminal sequences of the B domain that are essential for *in vivo* proteolytic processing of the primary translation product into two polypeptide chain (*i.e.*, intracellular processing site), as disclosed in WO 91/09122, which is incorporated herein by reference in its entirety. In some embodiments, a B domain deleted factor VIII is constructed with a deletion of amino acids 747-1638, *i.e.*, virtually a complete deletion of the B domain. Hoeben R.C., et al. J. Biol. Chem. 265 (13): 7318-7323 (1990), incorporated herein by reference in its entirety. A B domain deleted factor VIII can also contain a deletion of amino acids 771-1666 or amino acids 868-1562 of factor VIII. Meulien P., et al. Protein Eng. 2(4): 301-6 (1988), incorporated herein by reference in its entirety. Additional B domain deletions that are part of the invention include, *e.g.*,: deletion of amino acids 982 through 1562 or 760 through 1639 (Toole et al., Proc. Natl. Acad. Sci. U.S.A. 83:5939-5942 (1986)), 797 through 1562 (Eaton et al., Biochemistry 25:8343-8347 (1986)), 741 through 1646 (Kaufman (PCT published application No. WO 87/04187)), 747-1560 (Sarver et al., DNA 6:553-564 (1987)), 741 through 1648 (Pasek (PCT application No.88/00831)), 816 through 1598 or 741 through 1689 (Lagner (Behring Inst. Mitt. (1988) No 82:16-25, EP 295597)), each of which is incorporated herein by reference in its entirety. In some embodiments, B domain deleted FVIII comprises a partial deletion in B domain, *i.e.*, having 21 amino acids from B domain (i.e., SFSQNSRHPSQNPPVLKRHQR, which is SEQ ID NO: 17) disclosed in US Publication No. 20100286067 and US Publication No. US 20120093840, both of which are incorporated herein by reference in their entireties. Each of the foregoing deletions can be made in any Factor VIII sequence. Each of the foregoing deletions can be made in any Factor VIII sequence.

In one embodiment, the B domain deleted Factor VIII portion in the long-acting polypeptide is processed into two chains connected (or associated) by a metal bond, the first chain comprising a heavy chain (A1-A2-partial B) and a second chain comprising a light chain (A3-C1-C2) . In another embodiment, the B domain deleted Factor VIII portion is a single chain Factor VIII. The single chain Factor VIII can comprise an intracellular processing site, which corresponds to Arginine at residue 754 of B domain deleted Factor VIII (residue 773 of SEQ ID NO: 2) or at residue 1648 of full-length Factor VIII (residue 1657 of SEQ ID NO: 6).

The metal bond between the heavy chain and the light chain can be any metal known in the art. For example, the metals useful for the invention can be a divalent metal ion. The metals that can be used to associate the heavy chain and light chain include, but not limited to, $Ca^{2+}$, $Mn^{2+}$, or $Cu^{2+}$. Fatouros et al., Intern. J. Pharm. 155(1): 121-131 (1997); Wakabayashi et al., JBC. 279(13): 12677-12684 (2004).

In some embodiments, the long-acting FVIII polypeptide comprises a Factor VIII portion and a non-Factor VIII portion, e.g., a heterologous moiety. In one embodiment, the heterologous moiety is capable of extending *in vivo* or *in vitro* half-life of the FVIII polypeptide. Exemplary non-Factor VIII portions include, *e.g.*, Fc, albumin, a PAS sequence, transferrin, CTP (28 amino acid C-terminal peptide (CTP) of human chorionic gonadotropin (hCG) with its 4 O-glycans), polyethylene glycol (PEG), hydroxyethyl starch (HES), albumin binding polypeptide, albumin-binding small molecules, or any combination thereof. Exemplary long-acting polypeptides of the invention include, *e.g.*, Factor VIII-Fc polypeptides, Factor VIII-albumin polypeptides, Factor VIII-PAS polypeptides, Factor VIII-transferrin polypeptides, Factor VIII-CTP polypeptides, Factor VIII-PEG polypeptides, Factor VIII-HES polypeptides, Factor VIII-albumin binding polypeptide polypeptides, or Factor VIII-albumin-binding small molecule polypeptides.

Exemplary long-acting Factor VIII-Fc polypeptides include, *e.g.*, SEQ ID NO:2 or 6 (Table 16), with or without their signal sequences and the Fc polypeptide of SEQ ID NO:4 (Table 16).

The long-acting polypeptide can comprise a sequence at least 60%, 70%, 80%, 90%, 95%, 96%, 97%, 98%, 99%, or 100% identical to the Factor VIII and Fc amino acid sequence shown in Table 16A(i) without a signal sequence (amino acids 20 to 1684 of SEQ ID NO:2) or at least 60%, 70%, 80%, 90%, 95%, 96%, 97%, 98%, 99%, or 100% identical to the Factor VIII and Fc amino acid sequence shown in Table 16A(i) with a signal sequence (amino acids 1 to 1684 of SEQ ID NO:2), wherein the sequence has Factor VIII activity. The Factor VIII activity can be measured by activated Partial Thromboplastin Time (aPPT) assay, chromogenic assay, or other known methods. The chimeric polypeptide can comprise a sequence identical to the Factor VIII and Fc amino acid sequence shown in Table 16A(i) without a signal sequence (amino acids 20 to 1684 of SEQ ID NO:2) or identical to the Factor VIII and Fc amino acid sequence shown in Table 16A(i) with a signal sequence (amino acids 1 to 1684 of SEQ ID NO:2).

As discussed above, exemplary long-acting polypeptides also include Factor VIII fused to one or more albumin polypeptides, albumin binding polypeptides, or albumin-binding small molecules. In one embodiment, the albumin is human albumin. The albumin or albumin binding protein can be fused to either the N-terminal end of FVIII or to the C-terminal end of FVIII or inserted between two amino acids in FVIII. Examples of albumin, e.g., fragments thereof, that can be used in the present invention are known. *e.g.*, U.S. Patent No. 7,592,010; U.S. Patent No. 6,686,179; and Schulte, Thrombosis Res. 124 Suppl. 2:S6-S8 (2009), each of which is incorporated herein by reference in its entirety.

The albumin binding polypeptides can compromise, without limitation, bacterial albumin-binding domains, albumin-binding peptides, or albumin-binding antibody fragments that can bind to albumin. Domain 3 from streptococcal protein G, as disclosed by Kraulis et al, FEBS Lett. 378:190-194 (1996) and Linhult et al., Protein Sci. 11:206-213 (2002) is an example of a bacterial albumin-binding domain. Examples of albumin-binding peptides include a series of peptides having the core sequence DICLPRWGCLW (SEQ ID NO: 7). See, *e.g.*, Dennis et al., J. Biol. Chem. 2002, 277: 35035-35043 (2002). Examples of albumin-binding antibody fragments are disclosed in Muller and Kontermann, Curr. Opin. Mol. Ther. 9:319-326 (2007); Rooverset et al., Cancer Immunol. Immunother. 56:303-317 (2007), and Holt et al., Prot. Eng. Design Sci., 21:283-288 (2008), which are incorporated herein by reference in their entireties.

In certain aspects, a recombinant FVIII polypeptide of the invention comprises at least one attachment site for a non-polypeptide small molecule, variant, or derivative that can bind to albumin thereof. An example of such albumin binding moieties is 2-(3-maleimidopropanamido)-6-(4-(4-iodophenyl)butanamido)hexanoate ("Albu" tag) as disclosed by Trusselet et al., Bioconjugate Chem. 20:2286-2292 (2009).

As discussed above, exemplary long-acting polypeptides also include Factor VIII fused to at least one C-terminal peptide (CTP) of the β subunit of human chorionic gonadotropin or fragment, variant, or derivative thereof. The CTP can be fused to Factor VIII either the N-terminal end of FVIII or to the C-terminal end of FVIII or inserted between two amino acids in FVIII. One or more CTP peptides fused to or inserted into a recombinant protein is known to increase the *in vivo* half-life of that protein. *See, e.g.*, U.S. Patent No. 5,712,122, incorporated by reference herein in its entirety. Exemplary CTP peptides include DPRFQDSSSSKAPPPSLPSPSRLPGPSDTPIL (SEQ ID NO: 8) or SSSSKAPPPSLPSPSRLPGPSDTPILPQ. (SEQ ID NO: 9). *See*, *e.g.*, U.S. Patent Application Publication No. US 2009/0087411 A1, incorporated by reference.

As discussed above, exemplary long-acting polypeptides also include Factor VIII fused to at least one PAS sequence or fragment, variant, or derivative thereof. The PAS sequence can be fused to either the N-terminal end of FVIII or to the C-terminal end of FVIII or inserted between two amino acids in FVIII. A PAS peptide or PAS sequence, as used herein, means an amino acid sequence comprising mainly alanine and serine residues or comprising mainly alanine, serine, and proline residues, the amino acid sequence forming random coil conformation under physiological conditions. Accordingly, the PAS sequence is a building block, an amino acid polymer, or a sequence cassette comprising, consisting essentially of, or consisting of alanine, serine, and proline which can be used as a part of the heterologous moiety in the chimeric protein. An amino acid polymer also can form random coil conformation when residues other than alanine, serine, and proline are added as a minor constituent in the PAS sequence. By "minor constituent" is meant that that amino acids other than alanine, serine, and proline can be added in the PAS sequence to a certain degree, *e.g.*, up to about 12%, *i.e.,* about 12 of 100 amino acids of the PAS sequence, up to about 10%, up to about 9%, up to about 8%, about 6%, about 5%, about 4%, about 3%, *i.e.* about 2%, or about 1%, of the amino acids. The amino acids different from alanine, serine and proline can be selected from the group consisting of Arg, Asn, Asp, Cys, Gln, Glu, Gly, His, Ile, Leu, Lys, Met, Phe, Thr, Trp, Tyr, and Val. Under physiological conditions, a PAS peptide forms a random coil conformation and thereby can mediate an increased *in vivo* and/or *in vitro* stability to a recombinant protein of the invention, and has procoagulant activity.

Non-limiting examples of the PAS peptides include ASPAAPAPASPAAPAPSAPA (SEQ ID NO: 10), AAPASPAPAAPSAPAPAAPS (SEQ ID NO: 11), APSSPSPSAPSSPSPASPSS (SEQ ID NO: 12), APSSPSPSAPSSPSPASPS (SEQ ID NO: 13), SSPSAPSPSSPASPSPSSPA (SEQ ID NO: 14), AASPAAPSAPPAAASPAAPSAPPA (SEQ ID NO: 15), ASAAAPAAASAAASAPSAAA (SEQ ID NO: 16) or any variants, derivatives, fragments, or combinations thereof. Additional examples of PAS sequences are known from, *e.g.*, US Pat. Publ. No. 2010/0292130 A1 and PCT Appl. Publ. No. WO 2008/155134 A1. European issued patent EP2173890.

As discussed above, exemplary long-acting polypeptides also include Factor VIII fused to at least one transferrin peptide or fragment, variant, or derivative thereof. At least one transferrin peptide can be fused to either the N-terminal end of FVIII or to the C-terminal end of FVIII or inserted between two amino acids in FVIII. Any transferrin can be fused to or inserted into a recombinant FVIII protein of the invention. As an example, wild-type human Tf (Tf) is a 679 amino acid protein, of approximately 75 KDa (not accounting for glycosylation), with two main domains, N (about 330 amino acids) and C (about 340 amino acids), which appear to originate from a gene duplication. *See* GenBank accession numbers NM001063, XM002793, M12530, XM039845, XM 039847 and S95936 (www.ncbi.nlm.nih.gov), all of which are herein incorporated by reference in their entirety.

Transferrin transports iron through transferrin receptor (TfR)-mediated endocytosis. After the iron is released into an endosomal compartment and Tf-TfR complex is recycled to cell surface, the Tf is released back extracellular space for next cycle of iron transporting. Tf possesses a long half-life that is in excess of 14-17 days (Li et al., Trends Pharmacol. Sci. 23:206-209 (2002)).Transferrin fusion proteins have been studied for half-life extension, targeted deliver for cancer therapies, oral delivery and sustained activation of proinsulin (Brandsma et al., Biotechnol. Adv., 29: 230-238 (2011); Bai et al., Proc. Natl. Acad. Sci. USA 102:7292-7296 (2005); Kim et al., J. Pharmacol. Exp. Ther., 334:682-692 (2010); Wang et al., J. Controlled Release 155:386-392 (2011)).

As discussed above, exemplary long-acting polypeptides also include Factor VIII fused to at least one polyethylene glycol (PEG) moieties.

PEGylated FVIII can refer to a conjugate formed between FVIII and at least one polyethylene glycol (PEG) molecule. PEG is commercially available in a large variety of molecular weights and average molecular weight ranges. Typical examples of PEG average molecular weight ranges include, but are not limited to, about 200, about 300, about 400, about 600, about 1000, about 1300-1600, about 1450, about 2000, about 3000, about 3000-3750, about 3350, about 3000-7000, about 3500-4500, about 5000-7000, about 7000-9000, about 8000, about 10000, about 8500-11500, about 16000-24000, about 35000, about 40000, about 60000, and about 80000 daltons. These average molecular weights are provided merely as examples and are not meant to be limiting in any way.

A recombinant FVIII protein of the invention can be PEGylated to include mono- or poly-(e.g., 2-4) PEG moieties. PEGylation can be carried out by any of the PEGylation reactions known in the art. Methods for preparing a PEGylated protein product will generally include (i) reacting a polypeptide with polyethylene glycol (such as a reactive ester or aldehyde derivative of PEG) under conditions whereby the peptide of the invention becomes attached to one or more PEG groups; and (ii) obtaining the reaction product(s). In general, the optimal reaction conditions for the reactions will be determined case by case based on known parameters and the desired result.

There are a number of PEG attachment methods available to those skilled in the art, for example Malik F et al., Exp. Hematol. 20:1028-35 (1992); Francis, Focus on Growth Factors 3(2):4-10 (1992); European Pat. Pub. Nos. EP0401384, EP0154316, and EP0401384; and International Pat. Appl. Pub. Nos. WO92/16221 and WO95/34326. As a non-limiting example, FVIII variants can contain cysteine substitutions in one or more insertion sites in FVIII, and the cysteines can be further conjugated to PEG polymer. See Mei et al., Blood 116:270-279 (2010) and U.S. Patent No. 7,632,921, which are incorporated herein by reference in their entireties.

As discussed above, exemplary long-acting polypeptides also include Factor VIII fused to at least one hydroxyethyl starch (HES) polymer. HES is a derivative of naturally occurring amylopectin and is degraded by alpha-amylase in the body. HES exhibits advantageous biological properties and is used as a blood volume replacement agent and in hemodilution therapy in the clinics. See, *e.g.*, Sommermeyer et al., Krankenhauspharmazie 8:271-278 (1987); and Weidler et al., Arzneim.-Forschung/Drug Res. 41: 494-498 (1991).

HES is mainly characterized by the molecular weight distribution and the degree of substitution. HES has a mean molecular weight (weight mean) of from 1 to 300 kD, from 2 to 200kD, from 3 to 100 kD, or from 4 to 70kD. Hydroxyethyl starch can further exhibit a molar degree of substitution of from 0.1 to 3, from 0.1 to 2,from 0.1 to 0.9, or from 0.1 to 0.8, and a ratio between C2:C6 substitution in the range of from 2 to 20 with respect to the hydroxyethyl groups. HES with a mean molecular weight of about 130 kD is VOLUVEN® from Fresenius. VOLUVEN® is an artificial colloid, employed, *e.g.*, for volume replacement used in the therapeutic indication for therapy and prophylaxis of hypovolaemia. There are a number of HES attachment methods available to those skilled in the art, *e.g.*, the same PEG attachment methods described above.

In some embodiments, a long-acting polypeptide comprising a Factor VIII portion has an increased half-life ($t_{1/2}$) over a polypeptide consisting of the same Factor VIII portion without the non Factor VIII portion. A long-acting Factor VIII polypeptide with an increased $t_{1/2}$ can be referred to herein as a long-acting Factor VIII. Long-acting chimeric Factor VIII polypeptides include, *e.g.,* Factor VIII fused to Fc (including, *e.g.,* chimeric Factor VIII polypeptides in the form of a hybrid such as a FVIIIFc monomer dimer hybrid; see Example, Table 16A; and US Patent Nos. 7,404,956 and 7,348,004), and Factor VIII fused to albumin.

The Factor VIII polypeptide as used herein is functional factor VIII polypeptide in its normal role in coagulation, unless otherwise specified. Thus, the term Factor VIII includes variant polypeptides that are functional. Factor VIII proteins can be the human, porcine, canine, and murine factor VIII proteins. As described in the Background Art section, the full length polypeptide and polynucleotide sequences are known, as are many functional fragments, mutants and modified versions. Examples of human factor VIII sequences are shown as subsequences in SEQ ID NOs:2 or 6 (Table 16). Factor VIII polypeptides include, e.g., full-length factor VIII, full-length factor VIII minus Met at the N-terminus, mature factor VIII (minus the signal sequence), mature factor VIII with an additional Met at the N-terminus, and/or factor VIII with a full or partial deletion of the B domain. Factor VIII variants include B domain deletions, whether partial or full deletions.

A great many functional factor VIII variants are known, as is discussed above and below. In addition, hundreds of nonfunctional mutations in factor VIII have been identified in hemophilia patients, and it has been determined that the effect of these mutations on factor VIII function is due more to where they lie within the 3-dimensional structure of factor VIII than on the nature of the substitution (Cutler et al., Hum. Mutat. 19:274-8 (2002)), incorporated herein by reference in its entirety. In addition, comparisons between factor VIII from humans and other species have identified conserved residues that are likely to be required for function (Cameron et al., Thromb. Haemost. 79:317-22 (1998); US 6,251,632), incorporated herein by reference in its entirety.

The human factor VIII gene was isolated and expressed in mammalian cells (Toole, J. J., et al., Nature 312:342-347 (1984); Gitschier, J., et al., Nature 312:326-330 (1984); Wood, W. I., et al., Nature 312:330-337 (1984); Vehar, G. A., et al., Nature 312:337-342 (1984); WO 87/04187; WO 88/08035; WO 88/03558; U.S. Pat. No. 4,757,006), each of which is incorporated herein by reference in its entirety, and the amino acid sequence was deduced from cDNA. Capon et al., U.S. Pat. No. 4,965,199, incorporated herein by reference in its entirety, discloses a recombinant DNA method for producing factor VIII in mammalian host cells and purification of human factor VIII. Human factor VIII expression in CHO (Chinese hamster ovary) cells and BHK (baby hamster kidney cells) has been reported. Human factor VIII has been modified to delete part or all of the B domain (U.S. Pat. Nos. 4,994,371 and 4,868,112, each of which is incorporated herein by reference in its entirety), and replacement of the human factor VIII B domain with the human factor V B domain has been performed (U.S. Pat. No. 5,004,803, incorporated herein by reference in its entirety). The cDNA sequence encoding human factor VIII and predicted amino acid sequence are shown in SEQ ID NOs:1 and 2, respectively, of US Application Publ. No. 2005/0100990, incorporated herein by reference in its entirety.

U.S. Pat. No. 5,859,204, Lollar, J. S., incorporated herein by reference in its entirety, reports functional mutants of factor VIII having reduced antigenicity and reduced immunoreactivity. U.S. Pat. No. 6,376,463, Lollar, J. S., incorporated herein by reference in its entirety, also reports mutants of factor VIII having reduced immunoreactivity. US Application Publ. No. 2005/0100990, Saenko et al., incorporated herein by reference in its entirety, reports functional mutations in the A2 domain of factor VIII.

A number of functional factor VIII molecules, including B-domain deletions, are disclosed in the following patents US 6,316,226 and US 6,346,513, both assigned to Baxter; US 7,041,635 assigned to In2Gen; US 5,789,203, US 6,060,447, US 5,595,886, and US 6,228,620 assigned to Chiron; US 5,972,885 and US 6,048,720 assigned to Biovitrum, US 5,543,502 and US 5,610,278 assigned to Novo Nordisk; US 5,171,844 assigned to Immuno Ag; US 5,112,950 assigned to Transgene S.A.; US 4,868,112 assigned to Genetics Institute, each of which is incorporated herein by reference in its entirety.

The porcine factor VIII sequence is published, (Toole, J. J., et al., Proc. Natl. Acad. Sci. USA 83:5939-5942 (1986)), incorporated herein by reference in its entirety, and the complete porcine cDNA sequence obtained from PCR amplification of factor VIII sequences from a pig spleen cDNA library has been reported (Healey, J. F. et al., Blood 88:4209-4214 (1996), incorporated herein by reference in its entirety). Hybrid human/porcine factor VIII having substitutions of all domains, all subunits, and specific amino acid sequences were disclosed in U.S. Pat. No. 5,364,771 by Lollar and Runge, and in WO 93/20093, incorporated herein by reference in its entirety. More recently, the nucleotide and corresponding amino acid sequences of the A1 and A2 domains of porcine factor VIII and a chimeric factor VIII with porcine A1 and/or A2 domains substituted for the corresponding human domains were reported in WO 94/11503, incorporated herein by reference in its entirety. U.S. Pat. No. 5,859,204, Lollar, J. S., also discloses the porcine cDNA and deduced amino acid sequences. US Pat. No. 6,458,563, incorporated herein by reference in its entirety assigned to Emory discloses a B-domain deleted porcine Factor VIII.

The Factor VIII (or Factor VIII portion of a chimeric polypeptide) can be at least 60%, 70%, 80%, 90%, 95%, 96%, 97%, 98%, 99%, or 100% identical to a Factor VIII amino acid sequence shown in Table 16 without a signal sequence (amino acids 20 to 1457 of SEQ ID NO:2; and amino acids 20 to 2351 of SEQ ID NO:6), wherein the Factor VIII portion has Factor VIII activity. The Factor VIII (or Factor VIII portion of a chimeric polypeptide) can be identical to a Factor VIII amino acid sequence shown in Table 16 without a signal sequence (amino acids 20 to 1457 of SEQ ID NO:2; and amino acids 20 to 2351 of SEQ ID NO:6).

The Factor VIII (or Factor VIII portion of a chimeric polypeptide) can be at least 60%, 70%, 80%, 90%, 95%, 96%, 97%, 98%, 99%, or 100% identical to a Factor VIII amino acid sequence shown in Table 16 with a signal sequence (amino acids 1 to 1457 of SEQ ID NO:2 and amino acids 1 to 2351 of SEQ ID NO:6), wherein the Factor VIII portion has Factor VIII activity. The Factor VIII (or Factor VIII portion of a chimeric polypeptide) can be identical to a Factor VIII amino acid sequence shown in Table 16 with a signal sequence (amino acids 1 to 1457 of SEQ ID NO:2 and amino acids 1 to 2351 of SEQ ID NO:6).

In one embodiment, an FcRn binding partner can be an Fc region. An FcRn binding partner is any molecule that can be specifically bound by the FcRn receptor with consequent active transport by the FcRn receptor of the FcRn binding partner. Thus, the term Fc includes any variants of IgG Fc that are functional. The region of the Fc portion of IgG that binds to the FcRn receptor has been described based on X-ray crystallography (Burmeister et al. , Nature 372:379 (1994), incorporated herein by reference in its entirety). The major contact area of the Fc with the FcRn is near the junction of the CH2 and CH3 domains. Fc-FcRn contacts are all within a single Ig heavy chain. The FcRn binding partners include, *e.g.*, whole IgG, the Fc fragment of IgG, and other fragments of IgG that include the complete binding region of FcRn. The major contact sites include amino acid residues 248, 250-257, 272, 285, 288, 290-291, 308-311, and 314 of the CH2 domain and amino acid residues 385-387, 428, and 433-436 of the CH3 domain. References made to amino acid numbering of immunoglobulins or immunoglobulin fragments, or regions, are all based on Kabat et al. 1991, Sequences of Proteins of Immunological Interest, U. S. Department of Public Health, Bethesda; MD, incorporated herein by reference in its entirety. (The FcRn receptor has been isolated from several mammalian species including humans. The sequences of the human FcRn, rat FcRn, and mouse FcRn are known (Story et al., J. Exp. Med. 180: 2377 (1994), incorporated herein by reference in its entirety.) An Fc can comprise the CH2 and CH3 domains of an immunoglobulin with or without the hinge region of the immunoglobulin. Exemplary Fc variants are provided in WO 2004/101740 and WO 2006/074199, incorporated herein by reference in its entirety.

Fc (or Fc portion of a chimeric polypeptide) can contain one or more mutations, and combinations of mutations.

Fc (or Fc portion of a chimeric polypeptide) can contain mutations conferring increased half-life such as M252Y, S254T, T256E, and combinations thereof, as disclosed in Oganesyan et al., Mol. Immunol. 46:1750 (2009), which is incorporated herein by reference in its entirety; H433K, N434F, and combinations thereof, as disclosed in Vaccaro et al., Nat. Biotechnol. 23:1283 (2005), which is incorporated herein by reference in its entirety; the mutants disclosed at pages 1-2, paragraph [0012], and Examples 9 and 10 of US 2009/0264627 A1, which is incorporated herein by reference in its entirety; and the mutants disclosed at page 2, paragraphs [0014] to [0021] of US 20090163699 A1, which is incorporated herein by reference in its entirety.

Fc (or Fc portion of a chimeric polypeptide) can also include, *e.g.*, the following mutations: The Fc region of IgG can be modified according to well recognized procedures such as site directed mutagenesis and the like to yield modified IgG or Fc fragments or portions thereof that will be bound by FcRn. Such modifications include, *e.g.*, modifications remote from the FcRn contact sites as well as modifications within the contact sites that preserve or even enhance binding to the FcRn. For example the following single amino acid residues in human IgG1 Fc (Fcγ1) can be substituted without significant loss of Fc binding affinity for FcRn: P238A, S239A, K246A, K248A, D249A, M252A, T256A, E258A, T260A, D265A, S267A, H268A, E269A, D270A, E272A, L274A, N276A, Y278A, D280A, V282A, E283A, H285A, N286A, T289A, K290A, R292A, E293A, E294A, Q295A, Y296F, N297A, S298A, Y300F, R301A, V303A, V305A, T307A, L309A, Q311A, D312A, N315A, K317A, E318A, K320A, K322A, S324A, K326A, A327Q, P329A, A330Q, A330S, P331A, P331S, E333A, K334A, T335A, S337A, K338A, K340A, Q342A, R344A, E345A, Q347A, R355A, E356A, M358A, T359A, K360A, N361A, Q362A, Y373A, S375A D376A, A378Q, E380A, E382A, S383A, N384A, Q386A, E388A, N389A, N390A, Y391F, K392A, L398A, S400A, D401A, D413A, K414A, R416A, Q418A, Q419A, N421A, V422A, S424A, E430A, N434A, T437A, Q438A, K439A, S440A, S444A, and K447A, where for example P238A represents wildtype proline substituted by alanine at position number 238. In addition to alanine other amino acids can be substituted for the wildtype amino acids at the positions specified above. Mutations can be introduced singly into Fc giving rise to more than one hundred FcRn binding partners distinct from native Fc. Additionally, combinations of two, three, or more of these individual mutations can be introduced together, giving rise to hundreds more FcRn binding partners. Certain of these mutations can confer new functionality upon the FcRn binding partner. For example, one embodiment incorporates N297A, removing a highly conserved N-glycosylation site. The effect of this mutation is to reduce immunogenicity, thereby enhancing circulating half-life of the FcRn binding partner, and to render the FcRn binding partner incapable of binding to FcγRI, FcγRIIA, FcγRIIB, and FcγRIIIA, without compromising affinity for FcRn (Routledge et al. 1995, Transplantation 60:847, which is incorporated herein by reference in its entirety; Friend et al. 1999, Transplantation 68:1632, which is incorporated herein by reference in its entirety; Shields et al. 1995, J. Biol. Chem. 276:6591, which is incorporated herein by reference in its entirety). Additionally, at least three human Fc gamma receptors appear to recognize a binding site on IgG within the lower hinge region, generally amino acids 234-237. Therefore, another example of new functionality and potential decreased immunogenicity can arise from mutations of this region, as for example by replacing amino acids 233-236 of human IgG1 "ELLG" to the corresponding sequence from IgG2 "PVA" (with one amino acid deletion). It has been shown that FcγRI, FcγRII, and FcγRIII which mediate various effector functions will not bind to IgG1 when such mutations have been introduced (Ward and Ghetie, Therapeutic Immunology 2:77 (1995), which is incorporated herein by reference in its entirety; and Armour et al., Eur. J. Immunol. 29:2613 (1999), which is incorporated herein by reference in its entirety). As a further example of new functionality arising from mutations described above affinity for FcRn can be increased beyond that of wild type in some instances. This increased affinity can reflect an increased "on" rate, a decreased "off’ rate or both an increased "on" rate and a decreased "off’ rate. Mutations believed to impart an increased affinity for FcRn include, *e.g.*, T256A, T307A, E380A, and N434A (Shields et al., J. Biol. Chem. 276:6591 (2001), which is incorporated herein by reference in its entirety).

The Fc (or Fc portion of a chimeric polypeptide) can be at least 60%, 70%, 80%, 90%, 95%, 96%, 97%, 98%, 99%, or 100% identical to the Fc amino acid sequence shown in Table 16 (amino acids 1458 to 1684 of SEQ ID NO:2 or amino acids 2352 to 2578 of SEQ ID NO:6). The Fc (or Fc portion of a chimeric polypeptide) can be identical to the Fc amino acid sequence shown in Table 16 (amino acids 1458 to 1684 of SEQ ID NO:2 and amino acids 2352 to 2578 of SEQ ID NO:6).

In certain embodiment, the long-acting FVIII polypeptide is a FVIII monomer-dimer hybrid. To obtain a FVIII monomer-dimer hybrid, the coding sequence of human recombinant B-domain deleted FVIII was obtained by reverse transcription-polymerase chain reaction (RT-PCR) from human liver poly A RNA (Clontech) using FVIII-specific primers. The FVIII sequence includes the native signal sequence for FVIII. The B-domain deletion was from serine 743 (S743; 2287 bp) to glutamine 1638 (Q1638; 4969 bp) for a total deletion of 2682 bp. Then, the coding sequence for human recombinant Fc was obtained by RT-PCR from a human leukocyte cDNA library (Clontech) using Fc specific primers. Primers were designed such that the B-domain deleted FVIII sequence was fused directly to the N-terminus of the Fc sequence with no intervening linker. The FVIIIFc DNA sequence was cloned into the mammalian dual expression vector pBUDCE4.1 (Invitrogen) under control of the CMV promoter. A second identical Fc sequence including the mouse Igk signal sequence was obtained by RT-PCR and cloned downstream of the second promoter, EF1$\alpha$, in the expression vector pBUDCE4.1.

The rFVIIIFc expression vector was transfected into human embryonic kidney 293 cells (HEK293H; Invitrogen) using Lipofectamine 2000 transfection reagent (Invitrogen). Stable clonal cell lines were generated by selection with Zeocin (Invitrogen). One clonal cell line, 3C4-22 was used to generate FVIIIFc for characterization in vivo. Recombinant FVIIIFc was produced and purified (McCue et al. 2009) at Biogen Idec (Cambridge, MA). The transfection strategy described above was expected to yield three products, *i.e.*, monomeric rFVIIIFc hybrids, dimeric rFVIIIFc hybrids and dimeric Fc. However, there was essentially no dimeric rFVIIIFc detected in the conditioned medium from these cells. Rather, the conditioned medium contained Fc and monomeric rFVIIIFc. It is possible that the size of dimeric rFVIIIFc was too great and prevented efficient secretion from the cell. This result was beneficial since it rendered the purification of the monomer less complicated than if all three proteins had been present. The material used in these studies had a specific activity of approximately 9000 IU/mg.

The dosing interval when administering a long-acting Factor VIII polypeptide, *e.g.*, a chimeric Factor VIII-Fc polypeptide (a polypeptide comprising a Factor VIII or a hybrid) of the invention can be at least about one and one-half times longer than the dosing interval required for an equivalent dose of the Factor VIII without the non-Factor VIII portion, e.g., without the Fc portion (a polypeptide consisting of the Factor VIII). The dosing interval can be at least about one and one-half to six times longer, one and one-half to five times longer, one and one-half to four times longer, one and one-half to three times longer, or one and one-half to two times longer, than the dosing interval required for an equivalent dose of the Factor VIII without the non-Factor VIII portion, *e.g.*, without the Fc portion (a polypeptide consisting of the Factor VIII). The dosing interval can be at least about one and one-half, two, two and one-half, three, three and one-half, four, four and one-half, five, five and one-half or six times longer than the dosing interval required for an equivalent dose of the Factor VIII without the non-Factor VIII portion, *e.g.*, without the Fc portion (a polypeptide consisting of the Factor VIII). The dosing interval can be about every three, four, five, six, seven, eight, nine, ten, eleven, twelve, thirteen, or fourteen days or longer. The dosing interval can be at least about one and one-half to 5, one and one-half, 2, 3, 4, or 5 days or longer. For on-demand treatment, the dosing interval of said chimeric polypeptide or hybrid is about once every 24-36, 24-48, 24-72, 24-96, 24-120, 24-144, 24-168, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, or 72 hours or longer.

In one embodiment, the effective dose is 25-80 IU/kg (25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 62, 64, 65, 66, 67, 68, 69, 70, 71, 72, 73, 74, 75, 76, 77, 78, 79, or 80 IU/kg) and the dosing interval is once every 3-5, 3-6, 3-7, 3, 4, 5, 6, 7, or 8 or more days, or three times per week, or no more than three times per week. In one embodiment, the effective dose is 80 IU/kg and the dosing interval is once every 3 days. In a further embodiment, the effective dose of 80 IU/kg given at a dosing interval of every 3 days is administered to a pediatric subject. In another embodiment, the effective dose is 65 IU/kg and the dosing interval is once weekly, or once every 6-7 days. The doses can be administered repeatedly as long as they are necessary (*e.g.*, at least 10, 20, 28, 30, 40, 50, 52, or 57 weeks, at least 1, 2, 3, 4, 5, 6, 7, 8, 9, or 10 years). In certain embodiments, the effective dose for on-demand treatment is 20-50IU/Kg (20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 IU/kg). The on-demand treatment can be one time dosing or repeated dosing. For repeated dosing, the dosing interval can be every 12-24 hours, every 24-36 hours, every 24-48 hours, every 36-48 hours, or every 48-72 hours.

In one embodiment, the subject is in need of on-demand (episodic) treatment. In another embodiment, on-demand (episodic) treatment resolves greater than 80% (greater than 80%, greater than 81%, greater than 82%, greater than 83%, greater than 84%, greater than 85%, greater than 86%, greater than 87%, greater than 88%, greater than 89%, greater than 90%, greater than 91%, greater than 92%, greater than 93%, greater than 94%, greater than 95%, greater than 96%, greater than 97%, greater than 98%, greater than 99%, or 100%) or 80-100%, 80-90%, 85-90%, 90-100%, 90-95%, or 95-100% of bleeds (e.g., spontaneous bleeds) in a single dose. In another embodiment, greater than 80% (greater than 81%, greater than 82%, greater than 83%, greater than 84%, greater than 85%, greater than 86%, greater than 87%, greater than 88%, greater than 89%, greater than 90%, greater than 91%, greater than 92%, greater than 93%, greater than 94%, greater than 95%, greater than 96%, greater than 97%, greater than 98%, or 100%) or 80-100%, 80-90%, 85-90%, 90-100%, 90-95%, or 95-100% of bleeding episodes are rated excellent or good by physicians after on-demand (episodic) treatment. In other embodiments, greater than 5%, (greater than 6%, greater than 7%, greater than 8%, greater than 9%, greater than 10%, greater than 11%, greater than 12%, greater than 13%, greater than 14%, greater than 15%, greater than 16%, greater than 17%, greater than 18%, greater than 19%, greater than 20%), or 5-20%, 5-15%, 5-10%, 10-20%, or 10-15% of bleeding episodes are rated as fair by physicians after on-demand treatment.

In other embodiments, the long-acting FVIII polypeptide is used for prophylaxis. Prophylaxis can be demonstrated by better Cmax, better Tmax, and/or greater mean residence time versus short-acting FVIII. In some embodiments, prophylaxis results in no spontaneous bleeding episodes within about 24, 36, 48, 72, or 96 hours (*e.g.*, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 96, 87, 88, 89, 90, 91, 92, 93, 94, 95, or 96 hours), after injection (*e.g.*, the last injection). In certain embodiments, prophylaxis results in greater than 30% (*e.g.*, greater than 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 96, 87, 88, 89, or 90%, for example, greater than 50%), mean reduction in annualized bleeding episodes with once weekly dosing (*e.g.*, at 65 IU/kg).

The therapeutic doses that can be used in the methods of the invention are about 10-100 IU/kg, more specifically, 10-20, 20-30, 30-40, 40-50, 50-60, 60-70, 70-80, 80-90, or 90-100 IU/kg, and more specifically, 10, 15, 20, 25, 30, 35, 40, 45, 50, 55, 60, 65, 70, 75, 80, 85, 90, 95, or 100 IU/kg.

Additional therapeutic doses that can be used in the methods of the invention are about 10 to about 150 IU/kg, more specifically, about 100-110, 110-120, 120-130, 130-140, 140-150 IU/kg, and more specifically, about 110, 115, 120, 125, 130, 135, 140, 145, or 150 IU/kg.

In one embodiment, variant FVIII polynucleotides can comprise, or alternatively consist of, a nucleotide sequence which is at least 85%, 90%, 95%, 96%, 97%, 98% or 99% identical to, for example, the nucleotide coding sequence in SEQ ID NO:1, 3, or 5 (the factor VIII portion, the Fc portion, individually or together) or the complementary strand thereto, the nucleotide coding sequence of known mutant and recombinant factor VIII or Fc such as those disclosed in the publications and patents cited herein or the complementary strand thereto, a nucleotide sequence encoding the polypeptide of SEQ ID NO:2, 4, or 6 (the factor VIII portion, the Fc portion, individually or together), and/or polynucleotide fragments of any of these nucleic acid molecules (*e.g.*, those fragments described herein). Polynucleotides which hybridize to these nucleic acid molecules under stringent hybridization conditions or lower stringency conditions are also included as variants, as are polypeptides encoded by these polynucleotides as long as they are functional.

Variant FVIII polypeptides can comprise, or alternatively consist of, an amino acid sequence which is at least 85%, 90%, 95%, 96%, 97%, 98%, 99% identical to, for example, the polypeptide sequence shown in SEQ ID NOs:2, 4, or 6 (the factor VIII portion, the Fc portion, individually or together), and/or polypeptide fragments of any of these polypeptides (*e.g.*, those fragments described herein).

By a nucleic acid having a nucleotide sequence at least, for example, 95% "identical" to a reference nucleotide sequence, it is intended that the nucleotide sequence of the nucleic acid is identical to the reference sequence except that the nucleotide sequence can include up to five point mutations per each 100 nucleotides of the reference nucleotide sequence. In other words, to obtain a nucleic acid having a nucleotide sequence at least 95% identical to a reference nucleotide sequence, up to 5% of the nucleotides in the reference sequence can be deleted or substituted with another nucleotide, or a number of nucleotides up to 5% of the total nucleotides in the reference sequence can be inserted into the reference sequence. The query sequence can be, for example, the entire sequence shown in SEQ ID NO:1 or 3, the ORF (open reading frame), or any fragment specified as described herein.

As a practical matter, whether any particular nucleic acid molecule or polypeptide is at least 85%, 90%, 95%, 96%, 97%, 98% or 99% identical to a nucleotide sequence or polypeptide of the present invention can be determined conventionally using known computer programs. In one embodiment, a method for determining the best overall match between a query sequence (reference or original sequence) and a subject sequence, also referred to as a global sequence alignment, can be determined using the FASTDB computer program based on the algorithm of Brutlag et al., Comp. App. Biosci. 6:237-245 (1990), which is herein incorporated by reference in its entirety In a sequence alignment the query and subject sequences are both DNA sequences. An RNA sequence can be compared by converting U's to T's. The result of said global sequence alignment is in percent identity. In another embodiment, parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter.

If the subject sequence is shorter than the query sequence because of 5' or 3' deletions, not because of internal deletions, a manual correction must be made to the results. This is because the FASTDB program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignment of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequence are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence can include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence can be inserted, deleted, (indels) or substituted with another amino acid. These alterations of the reference sequence can occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 85%, 90%, 95%, 96%, 97%, 98% or 99% identical to, for instance, the amino acid sequences of SEQ ID NO:2 (the factor VIII portion, the Fc portion, individually or together) or 4, or a known factor VIII or Fc polypeptide sequence, can be determined conventionally using known computer programs. In one embodiment, a method for determining the best overall match between a query sequence (reference or original sequence) and a subject sequence, also referred to as a global sequence alignment, can be determined using the FASTDB computer program based on the algorithm of Brutlag et al., Comp. App. Biosci. 6:237-245(1990), incorporated herein by reference in its entirety. In a sequence alignment the query and subject sequences are either both nucleotide sequences or both amino acid sequences. The result of said global sequence alignment is in percent identity. In another embodiment, parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is because the FASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequence are manually corrected for. No other manual corrections are to made for the purposes of the present invention.

The polynucleotide variants can contain alterations in the coding regions, noncoding regions, or both. In one embodiment, the polynucleotide variants contain alterations which produce silent substitutions, additions, or deletions, but do not alter the properties or activities of the encoded polypeptide. In another embodiment, nucleotide variants are produced by silent substitutions due to the degeneracy of the genetic code. In other embodiments, variants in which 5-10, 1-5, or 1-2 amino acids are substituted, deleted, or added in any combination. Polynucleotide variants can be produced for a variety of reasons, e.g., to optimize codon expression for a particular host (change codons in the human mRNA to others, *e.g.*, a bacterial host such as *E. coli*).

Naturally occurring variants are called "allelic variants," and refer to one of several alternate forms of a gene occupying a given locus on a chromosome of an organism (Genes II, Lewin, B., ed., John Wiley & Sons, New York (1985)). These allelic variants can vary at either the polynucleotide and/or polypeptide level and are included in the present invention. Alternatively, non-naturally occurring variants can be produced by mutagenesis techniques or by direct synthesis.

Using known methods of protein engineering and recombinant DNA technology, variants can be generated to improve or alter the characteristics of the polypeptides. For instance, one or more amino acids can be deleted from the N-terminus or C-terminus of the secreted protein without substantial loss of biological function. Ron et al., J. Biol. Chem. 268: 2984-2988 (1993), incorporated herein by reference in its entirety, reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli et al., J. Biotechnology 7:199-216 (1988), incorporated herein by reference in its entirety.)

Moreover, ample evidence demonstrates that variants often retain a biological activity similar to that of the naturally occurring protein. For example, Gayle and coworkers (J. Biol. Chem 268:22105-22111 (1993), incorporated herein by reference in its entirety) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See Abstract.) In fact, only 23 unique amino acid sequences, out of more than 3,500 nucleotide sequences examined, produced a protein that significantly differed in activity from wild-type.

As stated above, polypeptide variants include, *e.g.*, modified polypeptides. Modifications include, *e.g.*, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation (Mei et al., Blood 116:270-79 (2010), which is incorporated herein by reference in its entirety), proteolytic processing, phosphorylation, prenylation, racemization, selenoylation, sulfation, transfer-RNA mediated addition of amino acids to proteins such as arginylation, and ubiquitination. In some embodiments, Factor VIII is modified, *e.g.*, pegylated, at any convenient location. In some embodiments, Factor VIII is pegylated at a surface exposed amino acid of Factor VIII, *e.g.*, a surface exposed cysteine, which can be an engineered cysteine. *Id.* In some embodiments, modified Factor VIII, *e.g.*, pegylated Factor VIII, is a long-acting Factor VIII.

The long-acting polypeptide used herein can comprise processed Factor VIII or single chain Factor VIII or a combination thereof. "Processed Factor VIII," as used herein means Factor VIII that has been cleaved at Arginine 1648 (for full-length Factor VIII) or Arginine 754 (for B-domain deleted Factor VIII), *i.e.,* intracellular processing site. Due to the cleavage at the intracellular processing site, processed Factor VIII comprises two polypeptide chains, the first chain being a heavy chain and the second chain being a light chain. For example, the processed Factor VIII-Fc fusion protein (*i.e*., Heavy chain and Light chain fused to Fc) run at approximately 90 kDa and 130 kDa on a non-reducing SDS-PAGE, respectively, and 90 kDa and 105 kDa on a reducing SDS-PAGE, respectively. Therefore, in one embodiment, at least about 50%, about 60%, about 70%, about 75%, about 80%, about 85%, about 90%, about 95%, about 96%, about 97%, about 98%, about 99%, or about 100% of the Factor VIII portion in the long-acting polypeptide is processed Factor VIII. In another embodiment, about 50%, about 60%, about 70%, about 75%, about 80%, about 85%, about 90%, about 95%, about 96%, about 97%, about 98%, about 99%, or about 100% of the Factor VIII portion in the long-acting polypeptide is processed Factor VIII. In a particular embodiment, the long-acting polypeptide comprising processed Factor VIII is purified (or isolated) from the chimeric polypeptide comprising single chain Factor VIII, and at least about 75%, about 80%, about 85%, about 90%, about 95%, about 96%, about 97%, about 98%, about 99%, or about 100% of the Factor VIII portion in the long-acting polypeptide is processed Factor VIII. In some embodiments, the long-acting FVIII polypeptide comprises about 15% to 25% of single chain FVIII polypeptide and about 75% to about 85% of processed FVIII polypeptide.

"Single chain Factor VIII," "SC Factor VIII," or "SCFVIII" as used herein means Factor VIII that has not been cleaved at the Arginine site (residue 1648 for full-length Factor VIII (*i.e.*, residue 1667 of SEQ ID NO: 6) or residue 754 for B-domain deleted Factor VIII (*i.e.*, residue 773 of SEQ ID NO: 2). Therefore, single chain Factor VIII in the long-acting polypeptide used herein comprises a single chain. In one embodiment, the single chain Factor VIII contains an intact intracellular processing site. In another embodiment, the single chain Factor VIII of the invention comprises a substitution or mutation at an amino acid position corresponding to Arginine 1645, a substitution or mutation at an amino acid position corresponding to Arginine 1648, or a substitution or mutation at amino acid positions corresponding to Arginine 1645 and Arginine 1648 in full-length Factor VIII. In other embodiments, the amino acid substituted at the amino acid position corresponding to Arginine 1645 is a different amino acid from the amino acid substituted at the amino acid position corresponding to Arginine 1648. In certain embodiments, the substitution or mutation is an amino acid other than arginine, e.g., isoleucine, leucine, lysine, methionine, phenylalanine, threonine, tryptophan, valine, alanine, asparagine, aspartic acid, cysteine, glutamic acid, glutamine, glycine, proline, selenocysteine, serine, tyrosine, histidine, ornithine, pyrrolysine, or taurine. The single chain Factor VIII-Fc fusion protein can run at approximately 220 kDa on a non reducing SDS-PAGE and at approximately 195 kDa on a reducing SDS-PAGE.

The Factor VIII portion in the long-acting polypeptide used herein has Factor VIII activity. Factor VIII activity can be measured by any known methods in the art. For example, one of those methods can be a chromogenic assay. The chromogenic assay mechanism is based on the principles of the blood coagulation cascade, where activated Factor VIII accelerates the conversion of Factor X into Factor Xa in the presence of activated Factor IX, phospholipids and calcium ions. The Factor Xa activity is assessed by hydrolysis of a p-nitroanilide (pNA) substrate specific to Factor Xa. The initial rate of release of p-nitroaniline measured at 405 nM is directly proportional to the Factor Xa activity and thus to the Factor VIII activity in the sample. The chromogenic assay is recommended by the Factor VIII and Factor IX Subcommittee of the Scientific and Standardization Committee (SSC) of the International Society on Thrombosis and Hemostasis (ISTH). Since 1994, the chromogenic assay has also been the reference method of the European Pharmacopoeia for the assignment of FVIII concentrate potency. Thus, in one embodiment, the long-acting polypeptide comprising single chain Factor VIII has Factor VIII activity comparable to a long-acting polypeptide comprising processed Factor VIII (*e.g.*, a chimeric polypeptide consisting essentially of or consisting of two Fc portions and processed Factor VIII, wherein said processed Factor VIII is fused to one of the two Fc portions), when the Factor VIII activity is measured in vitro by a chromogenic assay.

The bleeding condition can be caused by a blood coagulation disorder. A blood coagulation disorder can also be referred to as a coagulopathy. In one example, the blood coagulation disorder, which can be treated with a pharmaceutical composition of the current disclosure, is hemophilia. In another example, the blood coagulation disorder, which can be treated with a pharmaceutical composition of the present disclosure is hemophilia A.

In some embodiments, the type of bleeding associated with the bleeding condition is selected from hemarthrosis, muscle bleed, oral bleed, hemorrhage, hemorrhage into muscles, oral hemorrhage, trauma, trauma capitis, gastrointestinal bleeding, intracranial hemorrhage, intra-abdominal hemorrhage, intrathoracic hemorrhage, bone fracture, central nervous system bleeding, bleeding in the retropharyngeal space, bleeding in the retroperitoneal space, and bleeding in the illiopsoas sheath.

In other embodiments, the subject suffering from bleeding condition is in need of treatment for surgery, including, e.g., surgical prophylaxis or peri-operative management. In one example, the surgery is selected from minor surgery and major surgery. Exemplary surgical procedures include tooth extraction, tonsillectomy, inguinal herniotomy, synovectomy, craniotomy, osteosynthesis, trauma surgery, intracranial surgery, intra-abdominal surgery, intrathoracic surgery, joint replacement surgery (e.g., total knee replacement, hip replacement, and the like), heart surgery, and caesarean section.

In another example, the subject is concomitantly treated with FIX. Because the compounds of the invention are capable of activating FIXa, they could be used to pre-activate the FIXa polypeptide before administration of the FIXa to the subject.

A long-acting FVIII polypeptide can be formulated as a pharmaceutical composition. The pharmaceutical composition can be formulated for administration to humans. The pharmaceutical compositions used in the methods of this invention comprise pharmaceutically acceptable carriers, including, *e.g.*, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Various methods of formulating the invention are well known in the art.

### *III. Method of Making*

A long-acting FVIII polypeptide can be manufactured in a host cell comprising a vector encoding the long-acting FVIII polypeptide. In one embodiment, the host cell is transformed with one or more vectors comprising a first nucleotide sequence encoding a FVIII polypeptide and a first FcRn polypeptide, a second nucleotide sequence encoding a second FcRn polypeptide, and optionally a third nucleotide sequence encoding a protein convertase, *e.g.*, PC5. As used herein, an expression vector refers to any nucleic acid construct which contains the necessary elements for the transcription and translation of an inserted coding sequence, or in the case of an RNA viral vector, the necessary elements for replication and translation, when introduced into an appropriate host cell. Expression vectors can include plasmids, phagemids, viruses, and derivatives thereof.

A gene expression control sequence as used herein is any regulatory nucleotide sequence, such as a promoter sequence or promoter-enhancer combination, which facilitates the efficient transcription and translation of the coding nucleic acid to which it is operably linked. The gene expression control sequence can, for example, be a mammalian or viral promoter, such as a constitutive or inducible promoter. Constitutive mammalian promoters include, but are not limited to, the promoters for the following genes: hypoxanthine phosphoribosyl transferase (HPRT), adenosine deaminase, pyruvate kinase, beta-actin promoter, and other constitutive promoters. Exemplary viral promoters which function constitutively in eukaryotic cells include, for example, promoters from the cytomegalovirus (CMV), simian virus (*e.g.*, SV40), papilloma virus, adenovirus, human immunodeficiency virus (HIV), Rous sarcoma virus, cytomegalovirus, the long terminal repeats (LTR) of Moloney leukemia virus, and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

Examples of vectors include, but are not limited to viral vectors or plasmid vectors. Plasmid vectors have been extensively described in the art and are well-known to those of skill in the art. See, *e.g.*, Sambrook et al., Molecular Cloning: A Laboratory Manual, Second Edition, Cold Spring Harbor Laboratory Press, 1989. In the last few years, plasmid vectors have been found to be particularly advantageous for delivering genes to cells in vivo because of their inability to replicate within and integrate into a host genome. These plasmids, however, having a promoter compatible with the host cell, can express a peptide from a gene operably encoded within the plasmid. Some commonly used plasmids available from commercial suppliers include pBR322, pUC18, pUC19, various pcDNA plasmids, pRC/CMV, various pCMV plasmids, pSV40, and pBlueScript. Additional examples of specific plasmids include pcDNA3.1, catalog number V79020; pcDNA3.1/hygro, catalog number V87020; pcDNA4/myc-His, catalog number V86320; and pBudCE4.1, catalog number V53220, all from Invitrogen (Carlsbad, CA.). Other plasmids are well-known to those of ordinary skill in the art. Additionally, plasmids can be custom designed using standard molecular biology techniques to remove and/or add specific fragments of DNA.

The expression vector or vectors are then transfected or co-transfected into a suitable target cell, which will express the polypeptides. Transfection techniques known in the art include, but are not limited to, calcium phosphate precipitation (Wigler et al. (1978) Cell 14:725), electroporation (Neumann et al. (1982) EMBO J 1:841), and liposome-based reagents. A variety of host-expression vector systems can be utilized to express the proteins described herein including both prokaryotic and eukaryotic cells. These include, but are not limited to, microorganisms such as bacteria (*e.g.*, *E. coli*) transformed with recombinant bacteriophage DNA or plasmid DNA expression vectors containing an appropriate coding sequence; yeast or filamentous fungi transformed with recombinant yeast or fungi expression vectors containing an appropriate coding sequence; insect cell systems infected with recombinant virus expression vectors (*e.g.*, baculovirus) containing an appropriate coding sequence; plant cell systems infected with recombinant virus expression vectors (*e.g.*, cauliflower mosaic virus or tobacco mosaic virus) or transformed with recombinant plasmid expression vectors (*e.g.*, Ti plasmid) containing an appropriate coding sequence; or animal cell systems, including mammalian cells (*e.g.*, HEK 293, CHO, Cos, HeLa, HKB11, and BHK cells).

In one embodiment, the host cell is a eukaryotic cell. As used herein, a eukaryotic cell refers to any animal or plant cell having a definitive nucleus. Eukaryotic cells of animals include cells of vertebrates, *e.g.*, mammals, and cells of invertebrates, *e.g.*, insects. Eukaryotic cells of plants specifically can include, without limitation, yeast cells. A eukaryotic cell is distinct from a prokaryotic cell, *e.g.*, bacteria.

In certain embodiments, the eukaryotic cell is a mammalian cell. A mammalian cell is any cell derived from a mammal. Mammalian cells specifically include, but are not limited to, mammalian cell lines. In one embodiment, the mammalian cell is a human cell. In another embodiment, the mammalian cell is a HEK 293 cell, which is a human embryonic kidney cell line. HEK 293 cells are available as CRL-1533 from American Type Culture Collection, Manassas, VA, and as 293-H cells, Catalog No. 11631-017 or 293-F cells, Catalog No. 11625-019 from Invitrogen (Carlsbad, Calif.). In some embodiments, the mammalian cell is a PER.C6® cell, which is a human cell line derived from retina. PER.C6® cells are available from Crucell (Leiden, The Netherlands). In other embodiments, the mammalian cell is a Chinese hamster ovary (CHO) cell. CHO cells are available from American Type Culture Collection, Manassas, VA. (*e.g.*, CHO-K1; CCL-61). In still other embodiments, the mammalian cell is a baby hamster kidney (BHK) cell. BHK cells are available from American Type Culture Collection, Manassas, Va. (*e.g.*, CRL-1632). In some embodiments, the mammalian cell is a HKB11 cell, which is a hybrid cell line of a HEK293 cell and a human B cell line. Mei et al., Mol. Biotechnol. 34(2): 165-78 (2006).

The method can further comprise purification steps. Various known purifications steps are well known in the art.

***IV. Method, System, and Storage Medium for Estimating Patient Individualized Dosing Information, Patient Individualized PK Information, and Patient Median PK Information***

The invention also includes a method of estimating a rFVIIIFc dosing information individualized for a patient, the method comprising: (a) receiving, by a computer-based system containing the rFVIIIFc population pharmacokinetic (popPK) model of Example 4, *e.g.*, Table 17, and, optionally, a Bayesian estimation program, at least one of patient information and desired treatment outcome information, (b) calculating, by the computer-based system, individualized rFVIIIFc dosing information using the popPK model, the optional Bayesian estimation program, and the received information, and (c) outputting, by the computer-based system, the individualized dosing information.

In some embodiments, the method also comprises selecting a dosing regimen based on the output individualized dosing information of (c) and administering rFVIIIFc to the patient according to the selected dosing regimen.

In some embodiments, the desired treatment outcome information is desired rise in plasma FVIII activity level following dosing and the output information is dose for acute treatment.

In some embodiments, the desired treatment outcome information is desired dosing interval and the output information is dose for prophylaxis.

In some embodiments, the desired treatment outcome information is desired dose and the output information is interval for prophylaxis.

The invention also includes a method of estimating a rFVIIIFc dosing regimen based on median popPK, the method comprising: (a) receiving, by a computer-based system containing the rFVIIIFc popPK model of Example 4, e.g., Table 17, and, optionally, a Bayesian estimation program, at least one of patient information and desired treatment outcome information, (b) calculating, by the computer-based system, median rFVIIIFc PK information using the popPK model, the optional Bayesian estimation program, and the received information, and (c) outputting, by the computer-based system, the median PK information.

In some embodiments, the method also comprises selecting a dosing regimen based on the output median PK information of (c), and administering rFVIIIFc to a patient according to the selected dosing regimen.

The invention also includes a method of estimating individual patient rFVIIIFc PK, the method comprising: (a) receiving, by a computer-based system containing the rFVIIIFc population pharmacokinetic (popPK) model of Example 4, e.g., Table 17, and, optionally, a Bayesian estimation program, individual rFVIIIFc PK information, (b) estimating, by the computer-based system, individualized patient rFVIIIFc PK information using the popPK model, the optional Bayesian estimation program, and the received information, and (c) outputting, by the computer-based system, the individualized patient PK information.

In some embodiments, the method also comprises selecting a dosing regimen based on the output individualized patient PK information of (c), and administering rFVIIIFc to the patient according to the selected regimen.

In some embodiments (a) further comprises receiving, by the computer-based system, patient information.

In some embodiments the patient information is age, e.g., 12 and older, Von Willebrand Factor, hematocrit, or body weight. Additional patient information includes diagnostic (baseline) FVIII level, PK determinations, time of PK sampling, dosing history if PK samples were taken from multiple doses, actual dose, FVIII activity level, etc.

In some embodiments, desired treatment outcome information is, e.g., desired PK or desired regimen outcome, e.g., desired rise in plasma FVIII activity level following dose, desired dosing interval, and desired dose.

In some embodiments, output information is, e.g., PK curve, PK parameter such as incremental recovery (Cmax/dose), mean residence time, terminal t1/2, clearance, Vss, AUC/dose, doses and associated troughs, and intervals and associated troughs.

For example, for assessing individualized patient PK, the system can recommend that the user input 2-3 optimized PK sampling time points. In this case, system output can include PK curve and one or more selected PK parameters, e.g., incremental recovery (Cmax/Dose), mean residence time, terminal t1/2, clearance, Vss, AUC, and time to 1 or X%, etc. E.g., Figure 14.

As additional examples, to select an individualized dosing regimen using the output individual PK parameters discussed in the preceding paragraph, (i) the dose selected for acute treatment can be based on user input of the desired rise in plasma FVIII activity level following the dose, (ii) the dose selected for prophylaxis can be based on user input of the desired dosing interval, or (iii) the selected interval for prophylaxis can be based on user input for the desired dose. In the first case, the system can output the dose (IU) based in the patient's incremental recovery. E.g., Figure 15. In the second case, system output can be a table of doses and associated troughs, e.g., x IU/kg, 1% trough, y IU/kg, 2% trough, etc. E.g., Figure 16, top. In the third case, system output can be a table of intervals and associated troughs, e.g., x days, 1% trough, y IU/kg, 2% trough, etc., E.g., Figure 16, bottom.

The user may wish to use the system without inputting any individualized PK data. In this case, the dosing output would be based on the population median rather than being individualized for the particular patient. E.g., Figure 17. In this way, the user inputs, e.g., body weight and age, and (i) the desired rise in plasma FVIII activity level following the dose, (ii) the desired dose interval for prophylaxis, or (iii) the desired dose for prophylaxis. In the first case, the system can output the dose. In the second case, the system can output the dose and associated trough. E.g., Table 19 In the third case, the system can output the interval and associated trough. E.g., Table 18.

In some embodiments, the system is compliant with patient privacy laws. In some embodiments, the system is encrypted, e.g., with SSL. In some embodiments, input patient information is made anonymous.

In some embodiments, the system includes a user help function.

The method can be carried out by, e.g., a physician, a nurse, or another healthcare practitioner.

Additional embodiments include a computer readable storage medium having instructions stored thereon that, when executed by a processor, cause the processor to perform any of the above methods.

Additional embodiments include a system comprising a processor and a memory, the memory having instructions stored thereon that, when executed by the processor, cause the processor to perform any of the above methods.

The user of the system or computer readable storage medium, can be, e.g., a physician, a nurse, or another healthcare practitioner.

For additional embodiments of these aspects of the invention, see Examples 4 and 6 and the Figures discussed therein.

### *V. Example Computing Environment*

Various modeling techniques, dosage calculations, and estimations described herein can be implemented by software, firmware, hardware, or a combination thereof. Figure 19 illustrates an example computer system 1900 in which the embodiments, or portions thereof, can be implemented as computer-readable code. For example, the modeling of Examples 3 and 4, and/or the patient treatment simulation of Example 6 can be implemented in system 1900.

Computer system 1900 includes one or more processors, such as processor 1904. Processor 1904 is connected to a communication infrastructure 1906 (for example, a bus or network).

Computer system 1900 also includes a main memory 1908, preferably random access memory (RAM), and may also include a secondary memory 1910. In accordance with implementations, user interface data may be stored, for example and without limitation, in main memory 1908. Main memory 1908 may include, for example, cache, and/or static and/or dynamic RAM. Secondary memory 1910 may include, for example, a hard disk drive and/or a removable storage drive. Removable storage drive 1914 may include a floppy disk drive, a magnetic tape drive, an optical disk drive, a flash memory, or the like. The removable storage drive 1914 reads from and/or writes to removable storage unit 1916 in a well-known manner. Removable storage unit 1916 may include a floppy disk, magnetic tape, optical disk, etc. which is read by and written to by removable storage drive 1914. As will be appreciated by persons skilled in the relevant art(s), removable storage unit 1916 includes a computer readable storage medium having stored therein computer software and/or data.

Computer system 1900 may also include a display interface 1902. Display interface 1902 may be adapted to communicate with display unit 1930. Display unit 1930 may include a computer monitor or similar means for displaying graphics, text, and other data received from main memory 1908 via communication infrastructure 1906. In alternative implementations, secondary memory 1910 may include other similar means for allowing computer programs or other instructions to be loaded into computer system 1900. Such means may include, for example, a removable storage unit 1922 and an interface 1920. Examples of such means may include a program cartridge and cartridge interface (such as that found in video game devices), a removable memory chip (such as an EPROM, or PROM) and associated socket, and other removable storage units 1922 and interfaces 1920 which allow software and data to be transferred from the removable storage unit 1922 to computer system 1900.

Computer system 1900 may also include a communications interface 1924. Communications interface 1924 allows software and data to be transferred between computer system 1900 and external devices. Communications interface 1924 may include a modem, a network interface (such as an Ethernet card), a communications port, a PCMCIA slot and card, or the like. Software and data transferred via communications interface 1924 are in the form of signals which may be electronic, electromagnetic, optical, or other signals capable of being received by communications interface 1924. These signals are provided to communications interface 1924 via a communications path 1926. Communications path 1926 carries signals and may be implemented using wire or cable, fiber optics, a phone line, a cellular phone link, an RF link or other communications channels.

In this document, the term "computer readable storage medium" is used to generally refer to non-transitory storage media such as removable storage unit 1916, removable storage unit 1922, and a hard disk installed in hard disk drive 1912. Computer readable storage medium can also refer to one or more memories, such as main memory 1908 and secondary memory 1910, which can be memory semiconductors (e.g. DRAMs, etc.). These computer program products are means for providing software to computer system 1900.

Computer programs (also called computer control logic) are stored in main memory 1908 and/or secondary memory 1910. Computer programs may also be received via communications interface 1924 and stored on main memory 1908 and/or secondary memory 1910. Such computer programs, when executed, enable computer system 1900 to implement embodiments as discussed herein. In particular, the computer programs, when executed, enable processor 1904 to implement processes of the present disclosure, such as certain methods discussed above. Accordingly, such computer programs represent controllers of the computer system 1900. Where embodiments use software, the software may be stored in a computer program product and loaded into computer system 1900 using removable storage drive 1914, interface 1920, or hard drive 1912.

Embodiments may be directed to computer program products comprising software stored on any computer readable medium. Such software, when executed in one or more data processing device, causes a data processing device(s) to operate as described herein. Embodiments may employ any computer useable or readable medium. Examples of computer readable storage media include, but are not limited to, non-transitory primary storage devices (e.g., any type of random access memory), and non-transitory secondary storage devices (e.g., hard drives, floppy disks, CD ROMS, ZIP disks, tapes, magnetic storage devices, and optical storage devices, MEMS, nano-technological storage device, etc.). Other computer readable media include communication mediums (e.g., wired and wireless communications networks, local area networks, wide area networks, intranets, etc.).

## Examples

### Example 1. Dosage and Method of Administration/ Method of Calculating Initial Estimated Dose

rFVIIIFc is long-acting anti-hemophilic factor (recombinant) indicated in adults and children (≥12 years) with hemophilia A (congenital Factor FVIII deficiency) for, *e.g.*, control and prevention of bleeding episodes, routine prophylaxis to prevent or reduce the frequency of bleeding episodes, and perioperative management (surgical prophylaxis).

Dosing of rFVIIIFc can be estimated as described in this example, but can also be determined by standard tests such as FVIII activity assays described elsewhere herein.

1 IU of rFVIIIFc per kg body weight is expected to increase the circulating level of Factor VIII by 2 [IU/dL]. rFVIIIFc has been shown to have a prolonged circulating half-life.

Since patients can vary in their pharmacokinetic (*e.g.*, half-life, *in vivo* recovery) and clinical responses to rFVIIIFc, the expected *in vivo* peak increase in Factor VIII level expressed as IU/dL (or % of normal) or the required dose can be estimated using the following formulas:

$$\text{IU/dL (or \% of normal)} = [\text{Total Dose (IU)}/\text{body weight (kg)}] \times 2\ (\text{IU/dL per IU/kg})$$

OR

$$\text{Dose (IU)} = \text{body weight (kg)} \times \text{Desired Factor VIII Rise (IU/dL or \% of normal)} \times 0.5\ (\text{IU/kg per IU/dL})$$

The following table (Table 1) can be used to guide dosing in bleeding episodes:

**TABLE 1: Guide to rFVIIIFc Dosing for Treatment of Bleeding**

| Severity of Bleed | Desired Factor VIII Level (IU/dL or % of normal) | Dose (IU/kg)/Frequency of Doses (hrs) |
|---|---|---|
| **Minor and Moderate** | **40-60** | **20-30 IU/kg** |

(continued)

| Severity of Bleed | Desired Factor VIII Level (IU/dL or % of normal) | Dose (IU/kg)/Frequency of Doses (hrs) |
|---|---|---|
| For example: joint, superficial muscle/no neurovascular compromise (except iliopsoas), deep laceration and renal, superficial soft tissue, mucous membranes | | Repeat every 24-48 hours until bleeding is resolved |
| **Major** For example: iliopsoas and deep muscle with neurovascular injury, or substantial blood loss, retroperitoneum, CNS, throat and neck, gastrointestinal. | **80-100** | **40-50 IU/kg** Repeat every 12-24 hours until bleeding is resolved |

**Adapted from WFH 2012**

Subsequent dosage and duration of treatment depends on the individual clinical response, the severity of the Factor VIII deficiency, and the location and extent of bleeding (see pharmacokinetics in Examples 2-4 below).

The following table (Table 2) can be used to guide dosing for perioperative management (surgical prophylaxis):

**TABLE 2: Guide to rFVIIIFc Dosing for Perioperative Management (Surgical Prophylaxis)**

| Type of Surgery | Target Factor VIII Level (IU/dL or % of normal) | Dose (IU/kg)/Frequency of Doses (hrs) |
|---|---|---|
| **Minor** Minor operations including uncomplicated dental extraction | **50 to 80** | **25-40 IU/kg** A single infusion can be sufficient. Repeat every 24 hours as needed to control bleeding. |
| **Major** Major operations including intra-abdominal, joint replacement surgery | **80 to 120** | An initial preoperative dose of 40-60 IU/kg followed by a repeat dose of 40-50 IU/kg after 8-24 hours and then every 24 hours to maintain FVIII activity within the target range. rFVIIIFc has a longer half-life than plasma and recombinant FVIII products (see Example 3) |

For routine prophylaxis, the recommended regimen is 50 IU/kg every 3-5 days. The dose can be adjusted based on patient response in the range of 25-65 IU/kg (see Examples 3 and 4.

For weekly prophylaxis, the recommended dose is 65 IU/kg.

rFVIIIFc is contraindicated in patients who have manifested severe hypersensitivity reactions, including anaphylaxis, to the product or its components. Severe hypersensitivity reactions were not observed in clinical trials; however, these have been known to occur with use of other factor VIII replacement factors.

The clinical response to rFVIIIFc can vary. If bleeding is not controlled with the recommended dose, the plasma level of Factor VIII can be determined, and a sufficient dose of rFVIIIFc can be administered to achieve a satisfactory clinical response. If the patient's plasma Factor VIII level fails to increase as expected or if bleeding is not controlled after rFVIIIFc administration, the presence of an inhibitor (neutralizing antibodies) should be suspected, and appropriate testing performed. Patients using rFVIIIFc can be monitored for the development of Factor VIII inhibitors by appropriate clinical observations and laboratory tests known to those of ordinary skill in the art.

Patient's plasma can be monitored for Factor VIII activity levels, *e.g.*, the one-stage clotting assay to confirm adequate Factor VIII levels have been achieved and maintained, when clinically indicated. Patient's plasma can further be monitored for the development of Factor VIII inhibitors.

Example 2. Phase 3 Clinical Trial of Extended Half-Life Recombinant Fc Fusion Factor VIII ("A-LONG" Study)

***Summary of A-LONG study***

In the A-LONG study, 165 male patients aged 12 years of age and older were enrolled. The A-LONG study had three treatment arms: individualized prophylaxis, weekly prophylaxis and episodic (on-demand) treatment (Arms 1, 2 and 3, respectively). In a subgroup of patients across treatment arms, rFVIIIFc was evaluated in the perioperative management of patients who required a major surgical procedure during the study.

Overall, 92.7 percent of patients completed the study. Recombinant FVIIIFc was generally well-tolerated. No inhibitors to rFVIIIFc were detected and no cases of anaphylaxis were reported in any patients, all of whom switched from commercially-available Factor VIII products. No serious adverse events were assessed to be related to drug by the investigator.

The most common adverse events (incidence of ≥5 percent) occurring outside of the perioperative management period were nasopharyngitis, arthralgia, headache, and upper respiratory tract infection.

The median (mean) annualized bleeding rates (ABR), including spontaneous and traumatic bleeds, were 1.6 (2.9) in the individualized prophylaxis arm, 3.6 (8.8) in the weekly prophylaxis arm and 33.6 (37.2) in the episodic treatment arm. In the individualized prophylaxis arm, the median dosing interval was 3.5 days. During the last 3 months on study, 30 percent of patients in the individualized prophylaxis arm achieved a mean dosing interval of at least 5 days.

Control of bleeding was assessed in all patients who experienced a bleeding episode during the study. Overall, 98 percent of bleeding episodes were controlled by one or two injections of rFVIIIFc.

In addition, rFVIIIFc was assessed in the perioperative management of 9 patients undergoing 9 major surgical procedures. The treating physicians rated the hemostatic efficacy of rFVIIIFc as excellent or good in 100 percent of these surgeries.

A-LONG included pharmacokinetic (PK) analysis of rFVIIIFc in all patients in the study. In a protocol-defined subset of patients with extensive PK sampling, the approximate terminal half-life of rFVIIIFc was 19.0 hours compared to 12.4 hours for ADVATE® [Antihemophilic Factor (Recombinant), Plasma/Albumin-Free Method], consistent with the results obtained in the Phase 1/2 study of rFVIIIFc.

***About the A-LONG Study and the rFVIIIFc Program***

A-LONG was a global, open-label, multi-center Phase 3 study that evaluated the efficacy, safety and pharmacokinetics of intravenously-injected rFVIIIFc. The study was designed to evaluate rFVIIIFc in the control and prevention of bleeding, routine prophylaxis and perioperative management in patients with hemophilia A. A-LONG involved 60 hemophilia treatment centers in 19 countries on 6 continents.

The A-LONG study had three treatment arms. In Arm 1 (individualized prophylaxis; n=117), patients were treated with 25-65 IU/kg of rFVIIIFc, at an interval of every three to five days, which was individualized to maintain factor trough levels sufficient to prevent bleeding. In Arm 2 (weekly prophylaxis; n=24), patients were treated with a weekly dose of 65 IU/kg. In Arm 3 (episodic treatment; n=23), patients received rFVIIIFc episodic treatment as needed for bleeding. In a subgroup of patients across treatment arms, rFVIIIFc was evaluated in the surgical setting.

The primary efficacy measures were the per-patient annualized bleeding rate in Arm 1 vs. Arm 3, and pharmacokinetics of rFVIIIFc vs. rFVIII (one-stage clotting [activated partial thromboplastin time] assay and the chromogenic assays calibrated to normal human references plasma with potency traceable to Word Health Organization standards). The safety endpoints included the incidence of adverse events and inhibitor development (Nijmegen-modified Bethesda assay) in patients studied for up to 54 weeks. Secondary efficacy endpoints included ABR in Arm 2 vs. Arm 3, annualized number of spontaneous and joint bleeding episodes per patient, number of injections and dose per injections to resolve a bleed, and investigators' assessments of patients' response to surgery with rFVIIIFc using a bleeding response scale (surgery subgroup only), response to treatment of bleeding episodes and the pharmacokinetics of rFVIIIFc versus ADVATE®. Non-neutralizing antibodies (NNAs) were assessed with an electrochemiluminescence-based anti-rFVIIIFc binding-antibody assay.

Ongoing clinical studies of rFVIIIFc include the Kids A-LONG and ASPIRE studies. Kids A-LONG is a Phase 3, open-label study in previously-treated children with hemophilia A under age 12, which is actively recruiting patients. ASPIRE is a long-term open-label study for patients who completed the A-LONG study or who complete the Kids A-LONG study.

***A-LONG Study Design***

Design: Global, open-label, multicenter, Phase 3 study. The study protocol was approved by local Institutional Review Boards for each participating institution, and the study was conducted in accordance with the International Conference on Harmonisation guidelines for Good Clinical Practice.

Objectives: To evaluate the efficacy and safety of intravenously-injected recombinant factor VIII Fc fusion protein (rFVIIIFc) in the control and prevention of bleeding episodes, routine prophylaxis, and perioperative management in individuals with severe hemophilia A.

**Key inclusion criteria:**

a. Male
b. ≥12 years of age and at least 40 kg
c. Diagnosis of severe hemophilia A defined as <1% (<1 IU/dL) endogenous factor VIII (FVIII) activity
d. Previously Treated Patients (PTPs): history of ≥150 prior documented exposure days (EDs) with any currently marketed FVIII product, been on prophylaxis ≥ 2 times per week with a FVIII product (for Arm 1 only), or experienced ≥ 12 bleeds in the past 12 months on an episodic regimen.
e. No current/prior FVIII inhibitors of measurable activity.
f. No history of inhibitors, or history of hypersensitivity or anaphylaxis associated with any FVIII or intravenous immunoglobulin administration
g. All subjects (or their guardians in the case of minors) gave informed written consent prior to study participation.

**Treatment arms:** Details of A-LONG study design is described below. Arm 1 (individualized prophylaxis) was administered 25-65 IU/kg every 3-5 days (maximum 5-day dosing interval). Subjects were treated with an initial dose of 25 IU/kg on Day 1 and 50 IU/kg on Day 4, which was subsequently adjusted to maintain trough factor levels sufficient to prevent bleeding. Further dose adjustments to target trough levels of 3 to 5 IU/dL permitted after week 7 if subject experienced ≥2 moderate/severe bleeds over a rolling 8-week period; 10 to 20 IU/kg rFVIIIFc (target 20-40 IU/dL FVIII) for minor bleeding episodes; 15 to 30 IU/kg rFVIIIFc (target 30-60 IU/dL FVIII) for moderate to major bleeding episodes; 40 to 50 IU/kg rFVIIIFc (target 80-100 IU/dL FVIII) for major to life-threatening bleeding episodes.
Arm 2 (weekly prophylaxis) was administered 65 IU/kg dose. Subjects were treated with 65 IU/kg once weekly with no dose or interval adjustment.
Arm 3 (episodic [on-demand] treatment) was administered 10-50 IU/kg. Subjects received rFVIIIFc episodic treatment as needed for bleeding. A Perioperative Management Subgroup was established. In this subgroup, rFVIIIFc was administered prior to and following surgery in the subset of patients requiring a major surgical procedure during the study. Subjects in any treatment arm could be enrolled in the surgery subgroup. Eligibility: required major surgery; ≥12 exposure days to rFVIIIFc with negative inhibitor titre following this period and within 4 weeks prior to surgery; and, completed, at minimum, abbreviated PK sampling.
All subjects (excluding sequential PK subgroup) underwent PK sampling from pre-injection with rFVIIIFc up to 96-hours post-injection, according to schedule: pre-injection, 30 (±3) minutes, 3 hours (± 15 minutes), 72 (±2) hours (Day 3), and 96 (±2) hours (Day 4) from start of injection.
Pharmacokinetic (PK) Assessment was performed. All subjects in all arms had an initial PK assessment after their first dose of rFVIIIFc. A subset of subjects from Arm 1 were assigned to a protocol-specified sequential PK subgroup to compare the stability of PK properties of rFVIIIFc over time with that of recombinant factor VIII (rFVIII, ADVATE® [anti-hemophilic factor (recombinant) plasma/albumin-free method], octocog alfa) as follows:
Prior to treatment in Arm 1, PK was assessed after a single dose of ADVATE® 50 IU/kg. PK was then assessed in these same subjects after a single dose of rFVIIIFc 50 IU/kg.
PK of rFVIIIFc was repeated at 12 to 24 weeks.
Details on the design of the sequential PK subgroup (Arm 1) dosing and PK sampling is described in Figure 1
Key efficacy outcome measures (included in initial readout):

a. Annualized bleeding rate (ABR) in Arm 1 versus Arm 3
b. Individualized prophylaxis arm compared with episodic treatment arm.
c. Number of injections required to resolve a bleeding episode
d. Number of injections required to resolve bleeding episodes
e. Median dose required to resolve bleeding episodes
f. Treating physicians' assessments of subjects' response to surgery with rFVIIIFc using a 4-point scale

PK outcome measures included:

a. The primary PK assessment were based on FVIII activity levels determined at a central laboratory by one-stage clotting assay and by the chromogenic assay against commercially available plasma standards.
b. PK of rFVIIIFc and ADVATE®
c. PK properties of rFVIIIFc were compared with rFVIII (ADVATE®; sequential PK subgroup). Overall study duration was ≤75 weeks for all subjects. The primary efficacy endpoint was annualized bleeding rate (ABR; Arms 1 and 2 vs. Arm 3). Prophylaxis dose and interval, number of injections required for treatment of bleeding episodes, and perioperative haemostasis were evaluated.

Key safety outcome measures included:

a. Incidence of inhibitor development. The study was powered to detect the occurrence of inhibitors with a 2-sided 95% confidence interval using the Clopper-Pearson exact method if 2 cases of inhibitor formation was observed.
b. Incidence of adverse events (AEs) occurring outside of the perioperative management period

Adherence with treatment (adherence measures) was assessed using patient electronic diaries.

***A-LONG Results***

Subjects

A total of 165 subjects were enrolled in the study. Median age was 30 years (range, 12-65) and 8% were <18 years. The number of patients in each arm of the study were: Arm 1 (individualized prophylaxis), n = 118; Arm 2 (weekly prophylaxis), n=24; Arm 3 (episodic treatment), n = 23; and perioperative management subgroup, n = 9, 9 surgeries (8 subjects from Arm 1 and 1 from Arm 2). Subjects from each treatment arm were eligible to enter the surgery subgroup if they required major surgery, had ≥12 EDs to rFVIIIFc and a negative inhibitor titre following this period and within 4 weeks prior to surgery. Patients on prior episodic treatment had a higher median number of bleeding episodes in the 12 months prior to the study, and a higher proportion of patients in Arms 2 and 3 had target joints. For patients on prior prophylaxis, 87% reported injecting at least three times weekly.
92.7% of subjects completed the study. In total, 153 (approximately 93%) subjects completed the study; 112/118 (95%) in Arm 1, 19/24 (795) in Arm 2, and 22/23 (96%) in Arm 3. Reasons for premature discontinuation were: Arm 1 - subject withdrawal (n=2), physician decision (n=2), other (n=1), death (n=1); Arm 2 - subject withdrawal (n=2; adverse events (AEs) related to study drug can have contributed), AEs (n=2), other (n=1); Arm 3 - other (n=1).
Age, race, and geography of subjects were representative of the global haemophilia A population who have access to treatment, as summarized in TABLE 3.

**TABLE 3. A-LONG subjects demographics**

| Demographic | Arm 1 (N=118) | Arm 2 (N=24) | Arm 3 (N=23) | Total N=165 |
|---|---|---|---|---|
| Age (years), median (min-max) | 29.0 (12-65) | 31.5 (18-59) | 34.0 (13-62) | 30.0 (12-65) |
| Weight (kg), median (min-max) | 71.65 (42.0-127.4) | 75.85 (50.0-105.0) | 70.00 (48.0-110.4) | 71.60 (42.0-127.4) |
| BMI (kg/m$^2$) median (min-max) | 23.90 (15.3-37.1) | 24.60 (18.8-37.4) | 22.80 (17.2-35.6) | 23.90 (15.3-37.4) |
| Race, n (%) | | | | |
| White | 79 (66.9) | 12 (50.0) | 16 (69.6) | 107 (64.8) |
| Black | 7 (5.9) | 1 (4.2) | 2(8.7) | 10(6.1) |
| Asian | 27 (22.9) | 11 (45.8) | 5 (21.7) | 43(26.1) |
| Other | 5 (4.2) | 0 | 0 | 5 (3.0) |
| Geographic location - n (%) | | | | |
| Europe | 34 (28.8) | 3 (12.5) | 4 (17.4) | 41 (24.8) |
| North America | 44 (37.3) | 5 (20.8) | 7 (30.4) | 56 (33.9) |
| Other* | 40 (33.9) | 16 (66.7) | 12 (52.2) | 68 (41.2) |
| Genotype - n (%) | | | | |
| Intron 22 inversion | 41 (35.0) | 7 (33.3) | 9 (39.1) | 57 (35.4) |
| Frameshift | 24 (20.5) | 4(19.0) | 6 (26.1) | 34(21.1) |
| Missense mutation | 22 (18.8) | 4 (19.0) | 1 (4.3) | 27 (16.8) |
| Nonsense mutation | 19 (16.2) | 6 (28.6) | 1 (4.3) | 26 (16.1) |
| Splice site change | 7 (6.0) | 0 | 4 (17.4) | 11 (6.8) |
| Intron 1 inversion | 3 (2.6) | 0 | 1 (4.3) | 4 (2.5) |
| Duplication | 1 (0.9) | 0 | 0 | 1 (0.6) |

(continued)

| Demographic | Arm 1 (N=118) | Arm 2 (N=24) | Arm 3 (N=23) | Total N=165 |
|---|---|---|---|---|
| NA | 0 | 0 | 1 (4.3) | 1 (0.6) |
| von Willebrand factor antigen - median IU/dl (IQR) | 118.0 (85, 151) | 129.0 (86, 166) | 131.0 (83, 155) | 118.0 (85, 153) |
| Pre-study FVIII regimen -n (%) | | | | |
| Prophylaxis | 87 (73.7) | 0 | 0 | 87 (52.7) |
| Episodic | 31 (26.3) | 24 (100) | 23 (100) | 78 (47.3) |
| Estimated no. of bleeds prior 12 mo - median (IQR) † | | | | |
| Prior prophylaxis | 6.0 (2, 15) | -- | -- | 6 (2, 15) |
| Prior episodic | 27.0 (17, 41) | 29.5 (19, 44) | 24.0 (15, 36) | 27 (18, 40) |
| ≥1 Target joint - n (%) | | | | |
| Prior prophylaxis | 47 (39.8) | - | - | 47 (28.5) |
| Prior episodic | 26 (22.0) | 22 (91.7) | 18 (78.3) | 66 (40.0) |
| Family history of inhibitor | 4 (3.4) | 1 (4.2) | 2(8.7) | 7(4.2) |
| HIV positive | 25 (21.2) | 4 (16.7) | 7 (30.4) | 36 (21.8) |
| HCV positive | 55 (46.6) | 14 (58.3) | 13 (56.5) | 82 (49.7) |

BMI, body mass index; HIV, human immunodeficiency virus; HCV, hepatitis C virus
IU/dl denotes international units per deciliter, BMI body mass index, HIV human immunodeficiency virus, HCV hepatitis C virus, NA not applicable.
*Other included Australia, New Zealand, Brazil, Hong Kong, India, Japan, Russia, and South Africa.
† Calculation was based on available data.

Subjects received rFVIIFc for a median (min-max) of 30.5 (<1-54) weeks with a median (minimum to maximum) in arms 1, 2, and 3 of 32.1 (9, 54), 28.0 (<1, 38), and 28.9 (15, 32) weeks, respectively. In total, 111 subjects (67.7%) had >50 EDs to study drug. A total of 9356 injections were administered during the study, corresponding to 9170 EDs (100.2 patient-years of exposure). Overall, 93.6% of patients were compliant with both the prescribed dose and interval in the prophylaxis arms.

Overall, 93.6% of subjects were adherent with both the prescribed dose and the prescribed dosing interval in the prophylaxis treatment arms.

Efficacy

In total, 163 subjects were included in efficacy analyses. One subject was excluded due to only receiving ADVATE® (Arm 1); another subject withdrew before efficacy assessment (Arm 2). Median ABR with the 25th and 75th percentiles (interquartile range [IQR]) were as follows: Arm1 - individualized prophylaxis arm: 1.6 (0.0, 4.7); Arm 2 - weekly prophylaxis arm: 3.6 (1.9, 8.4); and Arm 3 - episodic treatment arm: 33.6 (21.1, 48.7). Most bleeding episodes were spontaneous. In Arms 1, 2, and 3, respectively, 45.3%, 17.4%, and 0% had no bleeding episodes and 13.7%, 34.8%, and 0% had 1 bleeding episode.

Median dosing interval:

a. In the individualized prophylaxis arm, the median dosing interval was 3.5 days, during the last 3 months on study.

b. 30 percent of patients in the individualized prophylaxis arm achieved a mean dosing interval of at least 5 days.

c. Overall, the median (IQR) dosing interval with individualised prophylaxis aimed to achieve a FVIII trough of at least 1 to 3 IU/dL (with the maximum 5-day dosing interval permitted in the protocol) was 3.5 (3.2-4.4) days (Table 4) based on the median (IQR) weekly dose of 78 (72-91) IU/kg.

d. Approximately 30% of subjects achieved a mean dosing interval of 5 days over the last 3 months on study. 100% of subjects had mean dosing intervals ≥2 days throughout the study.

The median dose per injection for Arm 3 episodic regimen was 26.5 IU/kg (n=23) and the median total dose per bleeding episode was 27.4 IU/kg (n=23).

**TABLE 4. Comparative pharmacokinetics for rFVIIIFc vs. rFVIII (n=28)[1]**

| PK parameter | Geometric mean for rFVIIIFc PK (95% CI) | Geometric mean for rFVIII PK (95% CI) | Geometric mean of intra-subject ratio (95% CI) p-value |
|---|---|---|---|
| Elimination $t_{1/2}$ (h) | 18.97 (17.03, 21.12) | 12.43 (11.14, 13.86) | 1.53 (1.36, 1.71) <0.001 |
| CL (mL/h/kg) | 1.95 (1.71, 2.22) | 3.041 (2.71, 3.41) | 0.64 (0.60, 0.69) <0.001 |
| Time to 1 IU/dL (days) | 4.92 | 3.30 | 1.49 |
| (50 IU/kg dose) | (4.43, 5.46) | (2.99, 3.65) | (1.41, 1.57) <0.001 |
| [1]Arm 1 sequential PK group, compartmental model, one-stage assay. Estimates and 95% CIs for geometric means and geometric mean ratios; statistical significance assessed at the 2-sided 0.05 level; PK, pharmacokinetics; CI, confidence interval; Cmax, maximal concentration; AUC, area-under-the-curve; t½, half-life; CL, clearance; MRT, mean residence time; Vss, volume of distribution at steady state. | | | |

Control of bleeding: approximately 98% (97.7%) of bleeding episodes were controlled by one or two injections of rFVIIIFc. 87.3% of bleeds were controlled with one injection. 1.7% required three injections.

Perioperative management: Overall, 9 major surgeries were performed in 9 subjects (8 subjects from Arm 1; 1 subject from Arm 2), including knee arthroplasty (n=5), laparoscopic inguinal hernia repair (n=2), appendectomy (n=1), and arthroscopy (n=1). Treating physicians rated the hemostatic efficacy of rFVIIIFc as excellent (8/9) or good (1/9) in 100% of surgeries. Median (min, max) estimated blood loss available for 7/9 surgeries was 15.0 (0, 600) mL during surgery and 0.0 (0, 1100) mL post-operatively; post-surgical drainage).

PK

Comparative PK data for rFVIIIFc and ADVATE® available from 28 subjects in the Arm 1 sequential PK subgroup are summarized in Table 4.

a. rFVIIIFc demonstrated an approximate 50% longer elimination half-life (Figure 2) and mean residence time compared with ADVATE® (P<0.001) due to a 35% reduction in rFVIIIFc clearance with respect to ADVATE®.
b. Geometric mean time to 1 IU/dL FVIII activity following a 50-IU/kg dose was approximately 5 days for rFVIIIFc versus approximately 3 days for ADVATE® (*P*<0.001; Table 4, Figure 2).
c. Incremental recovery for rFVIIIFc was clinically comparable with ADVATE®.
d. There was no shift in the PK properties of rFVIIIFc as evidenced by comparable PK properties between the baseline and repeat rFVIIIFc PK profiles.
e. Analogous PK results were obtained when the analysis was based on the chromogenic FVIII assay.
f. The one-stage and chromogenic clotting assays accurately and precisely measured both rFVIIIFc and ADVATE® utilising commercially available plasma FVIII standards.

The geometric mean (95% confidence interval) terminal half-life of rFVIIIFc was approximately 19.0 hours (17.0, 21.1) hours, which is 1.53-fold longer than that of ADVATE® (approximately 12.4 (11.1, 13.9) hours).

Geometric mean time (IQR) to 1% FVIII activity following 50IU/kg of rFVIIIFc was approximately 5 days (4.92 (4.43, 5.46) days). Comparable PK profiles of rFVIIIFc were observed at Week 14. As indicated in Table 5, the median dosing interval with individualized prophylaxis was 3.5 days and the median dose per week was 78 IU/kg; approximately 30% of subjects achieved a mean dosing interval of ≥ 5 days over the last 3 months on study (subjects with ≥6 months on study). 98% of bleeding episodes were controlled with 1-2 injections.

**TABLE 5. Prophylactic dosing summary**

| | n | Arm 1 Individualised prophylaxis | n | Arm 2 Weekly prophylaxis |
|---|---|---|---|---|
| Median dose, IU/kg/week (IQR) | | | | |

(continued)

| | n | Arm 1 Individualised prophylaxis | n | Arm 2 Weekly prophylaxis |
|---|---|---|---|---|
| Overall | 117 | 77.9 (72.3,91.2) | 23 | 65.6 (64.2, 68.2) |
| Last 3 months[1] | 112 | 77.7 (71.9, 106.2) | 16 | 65.5[2] (64.3, 67.3) |
| Median dosing interval, days (IQR) | | | | |
| Overall | 117 | 3.5 (3.2, 4.4) | | |
| Last 3 months[1] | 112 | 3.5[3] (3.0, 5.0) | | |

[1]Based on last 3 months on study for subjects on study ≥6 months
[2]Fixed dose of 65 IU/kg/wk.
[3]30% of subjects achieved every 5-day pharmacokinetic-driven dosing to maintain troughs 1% to 3% above baseline.
IQR, interquartile range.

Safety

No inhibitors were detected to rFVIIIFc, and no cases of anaphylaxis, allergy, or serious thrombic events were reported.

rFVIIIFc was generally well tolerated, and no serious adverse events were assessed to be related to rFVIIIFc.

The most common AEs, regardless of causality, (incidence ≥5%) occurring outside of the perioperative management period were nasopharyngitis, arthralgia (joint pain), headache, and upper respiratory tract infection, as summarized in Table 6.

12 subjects (7.3%) experienced at least one serious AE (SAE) outside of the perioperative management period.

No SAEs were assessed to be related to drug by the investigator.

Insignificant incidence of non-neutralizing antibody (NNA) has been observed under NNA assay, which is approximately 20 times more sensitive than Bethesda assay. Five subjects were found positive for NNAs at baseline, but all had at least one negative evaluation during the study. Six subjects, four in Arm 1 study and two in Arm 2 study, became positive during the study. In almost all cases, however, antibodies were transient and of low titer, and in all cases antibodies were directed against FVIII.

Overall, 14 (8.5%) subjects reported SAEs. No SAEs were determined by the investigator as related to study drug, and SAEs reported were not experienced in more than 1 subject. There was 1 death during the study, attributed to polysubstance overdose, and assessed as unrelated to rFVIIIFc by the investigator.

**TABLE 6. Summary of adverse events (AEs)**

| | Total N=164 n (%) |
|---|---|
| Any AE | 108 (65.9) |
| Most common AEs (≥5% of subjects) | |
| Nasopharyngitis | 20 (12.2) |
| Arthralgia | 13 (7.9) |
| Headache | 9 (5.5) |
| Upper respiratory infection | 9 (5.5) |

Summary

Individualized and weekly prophylactic regimens resulted in low single-digit median annualized bleeding rates

In the individualized prophylaxis arm, the median dosing interval was 3.5 days. During the last 3 months on study, 30 percent of patients in the individualized prophylaxis arm achieved a mean dosing interval of at least 5 days.

98% of bleeding episodes were controlled by one or two injections of rFVIIIFc. In total, 757 bleeding episodes (Arm 1=209; Arm 2=92; Arm 3=456) were treated in 106 of the 164 subjects. 87.3% required a single injection of rFVIIIFc for resolution, and 97.8% required ≤2 injections. A total of 85.6%, 80.4% and 89.5% of bleeding episodes were resolved with 1 injection of rFVIIIFc in Arms 1, 2 and 3, respectively.

Hemostatic efficacy of rFVIIIFc during surgery was rated by treating physicians as excellent or good in 100% of surgeries.

The half-life of rFVIIIFc was approximately 19.0 hours compared to 12.4 hours for ADVATE®.

No subject developed an inhibitor or experienced an anaphylactic reaction to rFVIIIFc.

Recombinant FVIIIFc was generally well tolerated.

A-LONG was the largest registrational global pivotal phase 3 study of long-lasting rFVIII in severe haemophilia A conducted to date. The study showed that rFVIIIFc offers the potential for a markedly reduced injection frequency, decreased treatment burden, an improvement in clinical outcomes for prevention of bleeds in patients with severe haemophilia A.

Goal of the study design: The study was designed to evaluate the efficacy and safety of rFVIIIFc in the control and prevention of bleeding episodes, routine prophylaxis, and perioperative management in subjects with severe hemophilia A. In addition, the study was designed to assess the effective dose and interval of rFVIIIFc for prophylaxis, as well as the feasibility of weekly treatment at a dose of 65 IU/Kg.

Episodic treatment is the administration of replacement factor only as needed to treat bleeding episodes after they have started. Prophylactic treatment is the regular administration of replacement factor to prevent bleeding episodes.

The starting regimen was 25 IU/kg on Day land 50 IU/kg on Day 4. The dose and frequency of treatment could be adjusted based on the subject's PK profile and the goal was to maintain trough factor levels sufficient to prevent bleeding episodes using doses between 25 IU/kg and 65 IU/kg and treatment intervals of 3 to 5 days.

More than half of severe hemophilia A patients in the US still do not follow a prophylaxis regimen, due in part to the treatment burden. We therefore included the weekly dosing regimen in the A-LONG study to evaluate whether this regimen would result in a lower ABR compared to an episodic regimen. The median ABR in the weekly regimen was 3.59 (IQR 1.9, 8.4) compared with 33.6 (IQR 21.1, 48.7) for episodic treatment.

All subjects had an initial PK evaluation to characterize the PK of rFVIIIFc in a representative population of patients with Hemophilia A.

More extensive PK sampling was conducted in a subset of subjects in the individualized prophylaxis arm (Arm 1) at baseline after a single dose of ADVATE® (50 IU/kg), and after a 4 day washout, followed by a single dose of rFVIIIFc (50 IU/kg). Blood samples were taken for ADVATE® over a period of 72 hours. Blood samples were then taken for rFVIIIFc over a period of 120 hours. PK assessment of rFVIIIFc in this subset was repeated at 12 to 24 weeks with the same PK sampling schedule.

In peri-operative management, a single injection of rFVIIIFc was sufficient to maintain haemostasis to the end date/time of all major surgeries at a median dose of 51.4 IU/kg. Median rFVIIIFc consumption (summarized over all injections during each referenced time period) was 80.6 IU/kg on the day of major surgery, 161.3 IU/kg for Days 1-3 days following surgery, and 387.1 IU/kg for Days 4-14 following surgery. Perioperative haemostasis with rFVIIIFc was rated as excellent or good for all 9 major surgeries. No subjects reported a bleeding episode during the postoperative or rehabilitation periods. Overall, 7 adverse events (AEs) were reported in 4 (44.4%) subjects in the surgery subgroup, of which 6 AEs were of mild or moderate severity, and 1 AE was considered severe. Two serious AEs (inguinal hernia and appendicitis) were reported in 2 subjects. All AEs during the perioperative period were assessed by the investigators as unrelated to rFVIIIFc treatment.

Treating physicians rated the hemostatic efficacy of rFVIIIFc as excellent or good in 100% of surgeries on a 4 point scale including excellent, good, fair, and poor/none.

rFVIIIFc resulted in low median ABRs of 3.59 in the weekly prophylaxis arm and 1.60 in the individualized interval prophylaxis arm. In contrast, the episodic treatment arm had a median ABR of 33.57.

In the individualized prophylaxis arm, the median dosing interval was 3.5 days at a median dose of 77.7 IU/kg during the last 3 months on study.

The terminal half-life was approximately 18.97 hours for rFVIIIFc and approximately 12.3 hours for ADVATE®.

97.8% of bleeding episodes were controlled by one or two injections.

Hemostatic efficacy of rFVIIIFc for perioperative management was rated by treating physicians as excellent or good in 100% of surgeries.

Arm 2 was a weekly dosing regimen. It was designed to investigate benefit for patients on prophylaxis therapy, and to compare those results with subjects using an episodic treatment regimen. The median ABR in Arm 2 was 3.59 compared with 33.57 for the episodic treatment regimen (Arm 3).

The median dose per injection required for resolution of bleeding was 27.4 IU/kg, and the median total dose required was 28.2 IU/kg.

The median number of injections required for resolution of a bleeding episode was consistently 1.0 when treatment was administered within 8 hours of bleed onset, regardless of type or location of bleed. Overall, 78.8% of rFVIIIFc injections in Arm 1, 64.8% in Arm 2, and 79.7% of injections in Arm 3, were rated by subjects as producing excellent or good response. The median dosing interval of 3.5 days from Arm 1 represents the dosing interval that can be achieved by the majority of patients: 30% of patients were able to achieve a dosing interval of at least 5 days. Some patients were able to achieve a weekly prophylaxis regimen (Arm 2) with a median ABR of 3.59 bleeding episodes.

Subjects in the individualized prophylaxis arm received an initial dose of 25 IU/kg on Day 1 and 50 IU/kg on Day 4. The study design allowed for adjustment of the dosing interval and dose to maintain targeted trough factor levels and to prevent bleeding. We believe the median dosing interval and dose based on the last 6 months on study is most representative of the individualized prophylaxis regimen.

87.3% of bleeding episodes were resolved with one injection of rFVIIIFc and 97.8% were resolved with one or two injections. For the 96 (12.7%) bleeding episodes that required more than 1 injection for resolution, the median interval between the first and second injection was 30.9 hours.

The investigators' global assessment of subject response to their assigned rFVIIIFc regimen was rated as excellent or effective for 99.4%, 100%, and 98.1% of the subject visits in Arms 1, 2, and 3, respectively.

In A-LONG, the clotting factor activity was measured using a one-stage (aPTT) clotting assay and a chromogenic assay. The reported half-lives are based on the results of the one-stage clotting assay. While the half-lives of rFVIIIFc and ADVATE® were both slightly longer with the chromogenic assay, the ratios between products were consistent between the two assays.

Example 3. Further Safety, Efficacy, and Pharmacokinetics of rFVIIIFc from the A-LONG Study

The safety, efficacy, and pharmacokinetics of rFVIIIFc was evaluated in a multinational, open-label, Phase 3 study that compared the efficacy of each of 2 prophylactic treatment regimens to episodic (on-demand) treatment; determined hemostatic efficacy in the treatment of bleeding episodes; and determined hemostatic efficacy during peri-operative management in subjects undergoing major surgical procedures.

***Subjects***

The study enrolled a total of 165 previously treated male patients (PTPs) with severe hemophilia A (<1% endogenous FVIII activity or a genetic mutation consistent with severe hemophilia A). Subjects were aged 12-65, including 13 adolescent subjects aged 12 to 17 years. Hepatitis C Virus (HCV) status was positive in 82 of 165 (49.7%) subjects on study. Of the 165 enrolled subjects, 164 received at least 1 dose of rFVIIIFc, and 163 were evaluable for efficacy. A total of 153 subjects (92.7%) completed the study. Subjects on prophylaxis regimens prior to entering the study were assigned to the individualized prophylaxis arm. Those subjects on episodic (on-demand) therapy prior to entry either entered the individualized prophylaxis arm or were randomized to the weekly prophylaxis or episodic (on-demand) arms. Subjects requiring surgery could receive perioperative management (surgical prophylaxis) with rFVIIIFc during the study. Subjects were followed for up to 54 weeks.

Following a 96-hour washout and subsequent screening period, all patients (excluding the sequential pharmacokinetic subgroup) underwent pharmacokinetic sampling (rFVIIIFc only) from preinjection with rFVIIIFc up to 96-hours post-injection, according to the following schedule: Preinjection, 30 (±3) minutes, 3 hours (± 15 minutes), 72 (±2) hours (Day 3), and 96 (±2) hours (Day 4) from start of injection.

Patients were assigned to treatment arms according to the standard of care and investigator decision, following discussion with each patient. See Figure 4. Patients receiving a prophylaxis regimen prior to study entry entered only into Arm 1 (individualized prophylaxis). Patients who were on an episodic regimen had the option to enter Arm 1 or to be randomized into either Arm 2 (weekly prophylaxis) or Arm 3 (episodic regimen). Randomization into Arms 2 or 3 was stratified based on the number of bleeding episodes reported by the patient during the 12 months prior to screening.

Patients in any treatment arm could be enrolled in the surgery subgroup according to the following eligibility criteria: required major surgery; ≥12 EDs to rFVIIIFc with a negative inhibitor titer following this period and within 4 weeks prior to surgery; and, completed, at minimum, pharmacokinetic sampling. Major surgery was defined as any surgical procedure (elective or emergent) that usually, but not always, involves general anesthesia and/or respiratory assistance in which a major body cavity is penetrated and exposed, or a substantial impairment of physical or physiological functions is produced (e.g., laparotomy, thoracotomy, craniotomy, joint replacement, or limb amputation).

***Study Visit Schedule***

Study visits occurred at screening (≤8 weeks), baseline, week 7, week 14, week 28, week 38, and week 52. Additionally, patients had a 30-day follow-up phone call, unless they enrolled in the ongoing extension study ASPIRE (NCT01454739). In the surgery subgroup, patients had assessments 4 weeks prior to surgery, the day of surgery, and 24 hours post-operatively.

***Sequential Pharmacokinetic Subgroup***

Length of sampling times for rFVIII and rFVIIIFc were based upon previously reported half-lives, allowing sufficient time for decay (normally three to five times the previously observed half-life) for accurate description of pharmacokinetics. Following a 96-hour washout period, patients in the sequential pharmacokinetic subgroup in Arm 1 received an injection of 50 IU/kg rFVIII (ADVATE®) and underwent sampling up to 72 hours as follows: pre-injection, 30 (±3) minutes, 1 hour (±15 minutes), 6 (±1) hours, 24 (±2) hours (day 1), 48 (±2) hours, and 72 (±2) hours (day 3) from the start of the injection.

After the rFVIII dose, patients underwent a ≥96-hour washout. Patients then received a dose of 50 IU/kg of rFVIIIFc and underwent sampling for rFVIIIFc pharmacokinetic profiling as follows: preinjection and 30 (±3) minutes, 1 hour (±15 minutes), 6 (±1) hours, 24 (±2) hours , 72 (±2) hours (day 3), 96 (±2) hours (day 4), and 120 (±2) hours (day 5) from the start of the injection. The rFVIIIFc pharmacokinetic assessment was repeated 12 to 24 weeks later following the same sampling schedule.

Each patient had to have completed pharmacokinetic sampling through at least the 48-hour timepoint for rFVIII and the 72-hour timepoint for rFVIIIFc to be included in the pharmacokinetic analysis set.

The sequential pharmacokinetic subgroup consisted of all patients who had evaluable pharmacokinetic profiles for both rFVIII and baseline rFVIIIFc and/or evaluable pharmacokinetic profiles for both baseline rFVIIIFc and the repeat rFVIIIFc profile.

***Prophylaxis Dose and Interval Titration, and Episodic Treatment of Bleeding Episodes***

Arm 1 (individualized prophylaxis): Of the 118 subjects enrolled in the individualized prophylaxis arm, 117 received rFVIIIFc starting with a twice weekly regimen consisting of 25 IU/kg on the first day followed by 50 IU/kg on the fourth day. Blood samples were collected before the second dose to evaluate pharmacokinetics, including trough levels. After pharmacokinetic data were reviewed by the study committee (generally within 4 weeks of data availability), the dose and interval were adjusted within the range of 25-65IU/kg every 3-5 days to maintain trough between 1 and 3% above baseline or higher as clinically indicated to prevent bleeding. The median dosing interval was 3.51 days (interquartile range, 3.17, 4.43) and the median total weekly dose was 77.90 IU/kg (interquartile range 72.35, 91.20). For 112 subjects with ≥ 6 months on study, approximately 30% achieved a mean dosing interval of ≥ 5 days during the last three months on study. Subjects were on study for a median period of 32.1 weeks (range, 9, 54). Doses could also be increased and the interval decreased if the patient experienced multiple spontaneous bleeds in an 8-week period. See Figure 7.

Arm 2 (weekly prophylaxis): Twenty-four (24) subjects in the weekly prophylaxis arm were to receive 65 IU/kg of rFVIIIFc once weekly with no dose or interval adjustment. Twenty-three (23) subjects were evaluable for efficacy due to the withdrawal of one subject prior to entering the efficacy period. Subjects were on study for a median period of 28 weeks (range, <1, 38).

Arm 3 (episodic regimen): patients received rFVIIIFc doses between 10 and 50 IU/kg, according to bleed severity using published guidelines. In arm 3 (episodic treatment), and any patients who experience bleeding episodes in arms 1 and 2, minor bleeding episodes were treated with 10-20 IU/kg, moderate to major bleeding episodes with 15-30 IU/kg, and major to life-threatening bleeding episodes with 40-50 IU/kg to target trough FVIII levels of 20-40%, 30-60%, and 80-100%, respectively, based on the patient's pharmacokinetic profile. Twenty-three (23) subjects in the episodic (on-demand) arm received rFVIIIFc as needed, for the treatment of bleeding episodes. Subjects were on study for a median period of 28.9 weeks (range, 15, 32).

A total of nine subjects received rFVIIIFc for perioperative management (surgical prophylaxis) in nine major surgical procedures and a total of 15 subjects received rFVIIIFc for perioperative management (surgical prophylaxis) in 17 minor surgical procedures.

***Statistical Analysis***

Primary analyses of efficacy and safety of rFVIIIFc were performed in patients who received ≥ 1 dose of rFVIIIFc. ABRs were calculated based on the number of bleeding episodes and duration of time patients were evaluated for bleeding. Descriptive statistics were used to provide median and interquartile range (IQR) ABR values for each arm. Comparisons between each of arms 1 and 2 (prophylaxis regimens) and arm 3 (episodic treatment) were based on ABR estimates from a negative binomial regression model.

A repeated measures analysis of variance model with variables for study treatment and patient was used to compare pharmacokinetic parameters (rFVIIIFc vs. rFVIII), and 95% confidence intervals (CIs) were provided for geometric means, and for intrasubject ratios of rFVIIIFc to rFVIII, for each parameter.

A sample size of 144 patients across arms provided sufficient power to exclude an unacceptable inhibitor incidence and to detect a 50% reduction in ABR in Arm 1 versus Arm 3. All tests were performed at the two-sided, 0.05 significance level.

The study was stopped when prespecified EDs and time on study criteria were met.

***Efficacy Period***

In Arms 1 and 2, the efficacy period started with the date and time of the first prophylactic dosage following the completed pharmacokinetic sampling period and ended with last dose administered (for prophylaxis or a bleeding episode) as recorded in the electronic case report forms or patient diaries. For Arms 1 and 2, the interval of time in between the last dose before the repeat pharmacokinetic (Arm 1 sequential pharmacokinetic subgroup) or surgical/rehabilitation period and the start of the pharmacokinetic or surgical/rehabilitation period were not attributed to any of the efficacy or pharmacokinetic periods.

In Arm 3, the efficacy period started 1 minute following the last pharmacokinetic sampling timepoints and ended with either the date of last contact or the date of the last entry into the electronic patient diaries, whichever was later.

***Treatment Compliance***

Compliance with treatment dosing was monitored and documented by site staff. For between-visit administration, patients self-administered rFVIIIFc and documented treatment in the hand-held, electronic diary (eDiary), which was reviewed during periodic calls to the patient and at each visit by study site staff and the clinical monitor.

For patients in Arms 1 and 2, compliance with the prophylactic regimen was calculated in two ways, as dose compliance and as dosing interval compliance. Analyses of compliance included percentage of nominal doses taken per patient within the 80% to 125% range, and the percentage of doses taken per patient within ±24 hours of the prescribed day. Patients were considered compliant if the calculated compliance rate was at least 80%.

***Efficacy in Routine Prophylaxis***

Annualized bleeding rates were significantly lower in the prophylaxis arms compared with episodic treatment. Using a negative binomial model to calculate the annualized bleeding rate (ABR), there was a reduction in ABR of 92% (95% CI,87%, 95%) for subjects in the individualized prophylaxis arm (2.9 [2.3-3.7])and a reduction of 76% (95% CI, 54%, 88%) for subjects in the weekly prophylaxis arm (8.9 [5.5-14.5]) compared to the episodic (on-demand) arm (37.3 [24.0-57.7]), P<0.001. Median (IQR) ABR observed in arms 1, 2, and 3 were 1.6 (0.0, 4.7), 3.6 (1.9, 8.4) and 33.6 (21.1, 48.7), respectively (Table 7). Fifty-three (53) of 117 (45.3%) subjects experienced no bleeding episodes while on individualized prophylaxis and 4 of 23 (17.4%) subjects experienced no bleeding episodes while on weekly prophylaxis.

A comparison of the median ABRs in subjects evaluable for efficacy is summarized in Table 7.

**TABLE 7: Summary of Median (IQR) 1 Annualized Bleed Rate (ABR) by Treatment Arm**

| Bleeding Episode Etiology | Individualized Prophylaxis (N=117) | Weekly Prophylaxis (N=23) | Episodic (On Demand) (N=23) |
|---|---|---|---|
| **Overall ABR** | 1.60 (0.0, 4.69) | 3.59 (1.89, 8.36) | 33.57 (21.14, 48.69) |
| **Spontaneous ABR** | 0.00 (0.0, 2.03) | 1.93 (0.0, 4.78) | 20.24 (12.21, 36.81) |
| **Traumatic ABR** | 0.00 (0.0, 1.83) | 1.69 (0.00, 3.27) | 9.25 (1.74, 11.92) |
| **Joint ABR** | 0.00 (0.00,3.11) | 1.93 (0.00, 7.62) | 22.76 (15.07, 39.02) |

Although fixed weekly therapy (Arm 2) resulted in significant reductions in ABR versus episodic treatment and a median ABR of 3.6, four patients experienced ABRs of >20 on this regimen, which influenced the estimated ABR in this arm (8.9). These patients reported between 23 and 34 bleeding episodes in the year prior to study entry. No baseline disease or demographic characteristic explained their higher ABR.

With individualized prophylaxis (Arm 1), the median dosing interval was 3.5 days and the median weekly dose was 78 IU/kg; approximately 30% of patients who were on study for ≥6 months achieved a mean dosing interval of 5 days (per-protocol maximum) over the last 3 months on study. Interestingly, the median (IQR) number of annualized injections in the weekly prophylaxis arm approached that of episodic treatment (Arm 2: 59.8 [55.8, 64.3] vs. Arm 3: 40.1 [26.3, 59.9]).

The impact of baseline disease and demographic factors was evaluated in prespecified subgroups (See Figure 5). All analyses were consistent with the primary analysis, showing that pre-study regimen, age, number of target joints, and bleeding history did not influence efficacy. Subgroup analyses results were consistent with the primary analyses. Arm 1 was the only subgroup sufficient to display graphically. The median number of target joints was two. Compared with the number of bleeding episodes in the 12 months prior to the study, the observed ABR for patients in Arm 1 was substantially lower for both those on prior prophylaxis and those on prior episodic treatment (See Figure 6).

***Efficacy in Control of Bleeding***

A total of 757 new bleeding events were observed during the study. Assessment of response to each injection was recorded by subjects at 8 to 12 hours post-treatment. A 4-point rating scale of excellent, good, moderate, and no response was used to assess response. Overall, 87.3% of bleeding episodes were resolved with one injection, and 97.8% were controlled with ≤2 injections (Table 8). The median dose per injection was 27.35 IU/kg. Hemostatic response during the perioperative period was rated as excellent or good by investigators or surgeons for each of the nine major surgeries performed in nine patients, most of which were orthopedic procedures. Efficacy was similar when examined across arms, on a per subject basis, and for subjects who treated an episode more than 8 hours from the onset of symptoms. Bleeding episodes are summarized in Table 8.

**TABLE 8: Summary of Efficacy in Control of Bleeding**

| | | |
|---|---|---|
| **New bleeding episodes** | | (n= 757) |
| **# of Injections to treat bleeding episodes** | | |
| | **1 injection** | 661 (87.3%) |
| | **2 injections** | 79 (10.4%) |
| | **3 injections** | 13 (1.7%) |
| | **≥4 injection** | 4 (0.5%) |
| | | (n=755) |
| **Median dose per injection (IU/kg) to treat a bleeding episode (IQR)** | | 27.35 (22.73, 32.71) |
| **Median total dose (IU/kg) to treat a bleeding episode (IQR)** | | 28.23 (23.26, 46.88) |
| **Response to first injection** | | (n= 745) |
| | **Excellent or good** | 78.1% |
| | **Moderate** | 21.2% |
| | **No response** | 0.7% |

***Efficacy in Perioperative Management (Surgical Prophylaxis)***

Nine (9) major surgical procedures were performed in nine subjects. Hemostasis was assessed post-operatively by the investigator using a 4-point scale of excellent, good, fair, and poor/none. The hemostatic response was rated as excellent or good in 100% of major surgeries. All subjects received a single pre-operative dose to maintain hemostasis during surgery. The median dose was 51.4 IU/kg (range 50-77). On the day of surgery, most subjects got a second injection. The total dose on the day of surgery ranged from 65.8-114.4 IU/kg. Three days following surgery, subjects received in the range of 15.3-79.1 IU/kg/day.

Hemostatic response to dosing during surgery and post-operatively is summarized in Table 9A.

**TABLE 9A: Summary of Hemostatic Response During Surgery and Post-Operatively**

| | **Number of Procedures (Number of Subjects)** | **Excellent** | **Good** | **Response Fair** | **Poor/ None** |
|---|---|---|---|---|---|
| **Major Surgery** | | | | | |
| Total Knee Replacement | 3 (3) | 3 | | | |
| Knee Joint Replacement - Bilateral | 1 (1) | 1 | | | |
| Total Knee Revision | 1 (1) | 1 | | | |
| Laproscopic Inguinal Hernia Repair | 2 (2) | 1 | 1 | | |
| Arthroscopy | 1 (1) | 1 | | | |
| Appendectomy | 1 (1) | 1 | | | |

(continued)

| | Number of Procedures (Number of Subjects) | Excellent | Good | Response Fair | Poor/ None |
|---|---|---|---|---|---|
| **Minor surgery**[1] | 14 (12)[2] | 11 | 1 | | |

[1]Including 6 tooth extractions, 1 surgical extraction of complete bony impacted tooth, 1 surgical removal of wisdom teeth, 1 dental procedure with deep injection, 3 cystoscopies, 1 gastroscopy and colonoscopy, and 1 wound closure. Assessment of response not available for 2 minor surgeries

**Table 9B. Efficacy Endpoints for Prophylaxis and Episodic Treatment Groups**

| | Arm 1: Individualized Prophylaxis N = 117 | Arm 2: Weekly Prophylaxis N = 23 | Arm 3: Episodic Treatment N = 23 |
|---|---|---|---|
| ABR - negative binomial model (95% CI) | 2.9 (2.3-3.7) | 8.9 (5.5-14.5) | 37.3 (24.0-57.7) |
| Reduction (%) vs. arm 3 (P value*) ABR by type and location of bleeds - median (IQR) | 92% (<0.001) | 76% (<0.001) | |
| Overall | 1.6 (0.0, 4.7) | 3.6 (1.9, 8.4) | 33.6 (21.1, 48.7) |
| Spontaneous | 0.0 (0.0, 2.0) | 1.9 (0.0, 4.8) | 20.2 (12.2, 36.8) |
| Traumatic | 0.0 (0.0, 1.8) | 1.7 (0.0, 3.3) | 9.3 (1.7, 11.9) |
| Joint | | | |
| Spontaneous | 0.0 (0.0, 1.7) | 0.0 (0.0, 3.8) | 18.6 (7.6, 29.6) |
| Traumatic | 0.0 (0.0, 1.2) | 0.0 (0.0, 2.0) | 3.9 (0.0, 8.6) |
| Muscle | | | |
| Spontaneous | 0.0 (0.0, 0.0) | 0.0 (0.0, 0.0) | 5.1 (1.8, 6.8) |
| Traumatic | 0.0 (0.0, 0.0) | 0.0 (0.0, 0.0) | 0.0 (0.0, 2.0) |
| Patients with no bleeding episodes - n (%) | 53 (45.3) | 4 (17.4) | 0 |
| Weekly dose, median IU/kg (min, max) | | | |
| Overall | 77.9 (54.0, 141.5) | 65.6 (59.4, 70.7) | - |
| Dosing interval during last 3 mo on study†, n (%) | | | |
| ≥3 days | 111 (99.1) | - | - |
| ≥4 days | 39 (34.8) | - | - |
| 5 days | 33 (29.5) | - | - |

Reduction in ABR compared with arm 3, calculated using negative binomial model.
† Patients on study ≥6 mo.
IQR denotes interquartile range ($25^{th}$ and $75^{th}$ percentile), SD standard deviation, CI, confidence interval.

***Impact on Quality of Life***

Quality of Life was measured using the HAEM-A-QoL, a quality of life instrument specific to hemophilia. HAEM-A-QoL was performed in adults (aged 18 and older) in the individualized prophylaxis regimen. Changes from baseline to week 28 are summarized in Table 10.

**TABLE 10: Median Change from Baseline for the Haem-A-QoL Questionnaire (Individualized Prophylaxis)**

| | Pre-Study Regimen | | | | | |
|---|---|---|---|---|---|---|
| | Prophylaxis | | | Episodic (On-demand) | | |
| | N | Change from baseline | | N | Change from baseline | |
| **Total score** | 34 | -1.03 | (-26.7, 11.5) | 12 | -4.31 | (-35.6, 13.9) |
| **Domains, during the past month** | | | | | | |
| 1. Physical health | 40 | 0.00 | (-31.3, 60.0) | 17 | -25.00 | (-65.0, 25.0) |

(continued)

| Domains, during the past month | | | | | | |
|---|---|---|---|---|---|---|
| 2. Feeling | 40 | -3.13 | (-50.0, 25.0) | 17 | -6.25 | (-50.0, 18.8) |
| 3. View of yourself | 42 | 0.00 | (-40.0, 35.0) | 17 | 0.00 | (-40.0, 20.0) |
| 4. Sports and leisure | 29 | 0.00 | (-68.8, 45.0) | 10 | -5.00 | (-50.0, 5.0) |
| 5. Work and school | 34 | 0.00 | (-50.0, 37.5) | 15 | -6.25 | (-56.3, 25.0) |
| 6. Dealing with hemophilia | 40 | 0.00 | (-33.3, 33.3) | 17 | 0.00 | (-25.0, 58.3) |
| 7. Treatment | 42 | 0.00 | (-28.1, 18.8) | 14 | -4.69 | (-43.8, 12.5) |
| **Domains, recently** | | | | | | |
| 8. Future | 40 | 0.00 | (-45.0, 35.0) | 17 | -5.00 | (-45.0, 60.0) |
| 9. Family planning | 19 | 0.00 | (-25.0, 16.7) | 10 | 0.00 | (-18.8, 33.3) |
| 10. Partnership and sexuality | 37 | 0.00 | (-25.0, 66.7) | 14 | 0.00 | (-25.0, 91.7) |
| **NOTE:** Summary statistics are median (minimum, maximum). | | | | | | |

***Analytical Methods***

FVIII activity in citrated plasma samples for rFVIII and rFVIIIFc was measured by the one-stage clotting (activated partial thromboplastin time [aPTT]) assay on a Siemens BCS XP analyzer using commercial reagents (Dade Actin FSL) with calibration against a normal reference plasma (Precision Biologics CRYOCHECK™) traceable to the World Health Organization (WHO) 5th International Standard (IS) for human plasma. The lower limit of quantification (LLOQ) for the one-stage assay was 0.5 IU/dl. The accuracy was between 92% and 116% for the one-stage assay whereas the inter-assay coefficients of variation were <10%.

In addition to the aPTT assay, FVIII activity was measured by a chromogenic substrate assay using a commercially available kit (Aniara BIOPHEN FVIII:C) that complies with European Pharmacopoeia recommendations, and was qualified for quantification of rFVIII and rFVIIIFc in human plasma. The chromogenic assay was calibrated against normal human reference plasma (Instrumentation Laboratories Catalogue no. ORKE45), which had a potency assigned against the World Health Organization 6th International Standard for human plasma. The chromogenic assay had the LLOQ of 0.8 IU/dl and the inter-assay coefficients of variation were <15%.

***Pharmacokinetic Analyses***

Since patients had lowest observed FVIII activity in the study below the LLOQ (0.5 IU/dl) in the one-stage assay, the endogenous or baseline activity was therefore set to zero for all patients.

The FVIII activity over time profiles were analyzed using a mixed one- and two-compartmental model in Phoenix WinNonLin (Version 6.2.1.51). The optimal model for each patient was selected based on Akaike Information Criterion and the precision of the parameter estimates.

***Pharmacokinetic Properties***

The pharmacokinetics (PK) of rFVIIIFc versus ADVATE® (rFVIII) was evaluated following a 10-minute IV infusion in 28 evaluable subjects (≥ 15 years) from a clinical study. One patient was not evaluable, and one patient did not have an evaluable PK profile for rFVIII. The subjects underwent a washout period of 5 days prior to receiving 50 IU/kg of ADVATE®. PK sampling was conducted pre-dose followed by assessments at 7 time points up to 72 hours post-dose. Following a washout period of 120 hours (5 days), the subjects received a single dose of 50 IU/kg of rFVIIIFc. PK samples were collected pre-dose and then subsequently at 9 time points up to 120 hours (5 days) post-dose. A repeat PK evaluation of rFVIIIFc was conducted at Week 14.

PK parameters for rFVIIIFc were estimated based on the plasma FVIII activity over the time profile (see Figure 2). For rFVIIIFc, the maximum activity (Cmax) was observed following the end of the infusion. The geometric mean increase in circulating FVIII activity from pre-infusion level was 2.24 IU/dL per IU/kg and the elimination half-life was 19 hours. The 1.5 fold prolongation of half- life for rFVIIIFc relative to ADVATE® was consistent across subjects over the range of half-lives. The half-life of rFVIIIFc is influenced by the Fc region, which in animal models was shown to be mediated by the FcRn cycling pathway. The rFVIIIFc PK profile was stable over repeated dosing as shown by comparable PK parameters at Week 14.

Incremental recovery was 2.2 IU/dl per IU/kg for rFVIIIFc and 2.4 IU/dl per IU/kg for rFVIII (P=0.025), representing a statistically significant yet clinically insignificant difference. Time to 1 IU/dl above baseline was 4.9 days for rFVIIIFc versus 3.3 days for rFVIII (P<0.001); *i.e.*, time above 1 IU/dl activity was longer with rFVIIIFc than rFVIII when administered at the same dose. The pharmacokinetic profile of rFVIIIFc was stable following repeated dosing over 14 weeks (data not shown). Baseline von Willebrand factor (VWF) antigen concentration was positively correlated with rFVIIIFc half-life (n=155; r=0.67), P<0.001.

A summary of PK parameters after a 50 IU/kg dose for rFVIIIFc and ADVATE® are presented in Table 11.

**TABLE 11: Pharmacokinetic Parameters of rFVIIIFc and ADVATE® (rFVIII)**

| **PK Parameters**[1] | **fFVIIIFc (95% CI)** | **ADVATE® (95% CI)** | **Ratio of fFVIIIFc to ADVATE®** (95% **CI)** | **P value** |
|---|---|---|---|---|
| | **N=28** | **N=28** | **N=28** | |
| **$C_{max}$ (IU/dL)** | **108** (101, 115) | **120** (111, 128) | **0.90** (0.86, 0.95) | |
| **AUC/Dose (IU*h/dL per IU/kg)** | **51.2** (45.0, 58.4) | **32.9** (29.3, 36.9) | **1.56** (1.46, 1.67) | <0.001 |
| **Terminal half-life $t_{1/2}$ (h)** | **19.0** (17.0, 21.1) | **12.4** (11.1, 13.9) | **1.53** (1.36, 1.71) | <0.001 |
| **CL (mL/h/kg)** | **1.95** (1.71, 2.22) | **3.04** (2.71, 3.41) | **0.64** (0.60, 0.69) | <0.001 |
| **MRT (h)** | **25.2** (22.7, 27.9) | **16.8** (15.2,18.6) | **1.49** (1.41, 1.58) | <0.001 |
| **$V_{ss}$ (mL/kg)** | **49.1** (46.6, 51.7) | **51.2** (47.2,55.5) | **0.96** (0.90, 1.02) | 0.197 |
| **Incremental Recovery (IU/dL per IU/kg)** | **2.24** (2.11, 2.38) | **2.35** (2.21, 2.50) | **0.95** (0.91, 0.99) | 0.025 |
| **Time to 1% (days)** | **4.918** (4.434, 5.455) | **3.298** (2.985, 3.645) | **1.49** (1.41, 1.57) | <0.001 |
| **Time to 3 IU/kd (days)**‡ | **3.7** (3.3-4.1) | **2.5** (2.2-2.7) | **1.5** (1.4-1.6) | <0.001 |
| [1]PK parameters are presented in Geometric Mean (95% CI) **Abbreviations:** CI = confidence interval; $C_{max}$= maximum activity; AUC = area under the FVIII activity time curve; $t_{1/2}$ = terminal half-life; CL = clearance; MRT = mean residence time; $V_{ss}$ = volume of distribution at steady-state | | | | |
| ‡Following 50 IU/kg injection. | | | | |

Figure 8 is a plot comparing the time to 1% in subjects who received both ADVATE® and rFVIIIFc.

A summary of the PK parameters for adolescent (age 12-17 years, N=11) and adult (age ≥18 years, N=144) subjects is given in Table 12.

**TABLE 12: Comparison of PK Parameters of fVIIIFc by Age Category**

| | **12-17 years** (N= 11) | ≥ **18 years** (N= 144) |
|---|---|---|
| **Geometric Mean (95% CI)** | | |
| **Incremental Recovery (IU/dL per IU/kg)** | 1.81 (1.56, 2.09) | 1.92 (1.85, 2.00) |
| **AUC/Dose (IU*h/dL per IU/kg)** | 39.69 (35.17, 44.80) | 45.04 (42.45, 47.79) |
| **Terminal half-life t1/2 (h)** | 16.05 (13.90, 18.54) | 17.17 (16.39, 18.00) |
| **MRT (h)** | 22.63 (19.68, 26.01) | 24.32 (23.24, 25.45) |
| **CL (mL/h/kg)** | 2.52 (2.23, 2.84) | 2.22 (2.09, 2.36) |
| **Vss (mL/kg)** | 57.0 (50.2, 64.7) | 54.0 (52.1, 56.0) |

### *Detection of Inhibitors (Neutralizing Antibodies)*

The Nijmegen-modified Bethesda assay to detect neutralizing antibodies was performed at screening, baseline, and each visit during study treatment to monitor for the development of an inhibitor. Following the first dose with rFVIIIFc, inhibitor testing in Arms 1 and 2 was conducted at trough levels at each scheduled clinic visit, with trough defined as a point after the longest interval between scheduled doses. In addition, patients in Arms 1 and 2 were required to have inhibitor testing after 10 to 15 EDs to rFVIIIFc and again after they completed 50 to 75 EDs to rFVIIIFc. If these timepoints did not coincide with scheduled visits, additional visits were scheduled for the testing. Patients in Arm 2 may not have achieved 50 EDs on this study. For Arm 3, inhibitor testing was performed at each scheduled visit, at least 48 hours after the previous injection.

Formation of an inhibitor was defined as a neutralizing antibody value ≥0.6 Bethesda units (BU)/ml, identified and confirmed by retesting of a second sample within 2 to 4 weeks. The upper bound of the confidence interval for the inhibitor incidence rate was evaluated using the Clopper-Pearson exact method for a binomial proportion. The acceptable inhibitor risk in clinical trials of previously treated FVIII patients allows no more than 2 of 104 patients to experience an inhibitor while in the study, with each patient requiring at least two valid tests for inhibitors over 50 to 75 EDs.

### *Detection of Non-neutralizing Antibodies (rFVIIIFc Binding Antibodies)*

Monitoring for non-neutralizing antibodies that bind to rFVIIIFc was performed at the same time points as the neutralizing antibody assay. A bridging assay format was employed to detect all possible classes of antibodies, with electrochemiluminescent readout on an MSD instrument. Samples were positive if the signal was above a statistically derived cut point and confirmed by inhibition with excess rFVIIIFc product in at least two of three replicate assays. Positive samples were further characterized for binding specificity to rFVIII or Fc. This assay was approximately 20-fold more sensitive than the Nijmegen-modified Bethesda assay.

### *Adverse Drug Reactions*

In total, 111 patients had ≥50 EDs. No inhibitors were detected in any patients, providing an incidence of 0% (95% CI: 0-2.2%) overall, and an incidence of 0% (95% CI: 0-3.3%) for patients ≥50 EDs. Five patients tested positive for NNAs at baseline. Six additional patients who tested negative at baseline had a positive test on study; detection persisted in two of these patients at the final study visit. All NNAs were low titer and directed against FVIII, not Fc. In one patient (Arm 1), NNA presence resulted in a transient effect on clearance at the week 14 pharmacokinetic assessment, but did not impact clinical outcome nor actual peak and trough FVIII activity levels. This patient continued on to the extension study.

Overall, 65.9% of patients reported at least one adverse event (excluding the surgical period; Table 13A and B). Most adverse events were judged by investigators as unrelated to treatment.

The most common adverse drug reactions observed in the multinational, open label, Phase 3 study (incidence ≥1 %) for rFVIIIFc were arthralgia and malaise.

No serious adverse drug reactions were reported in subjects who received rFVIIIFc.

In the multinational, open-label, Phase 3 clinical study with rFVIIIFc, 164 subjects were evaluated, with 146 subjects treated at least 26 weeks and 23 subjects treated for at least 39 weeks.

Adverse events judged to be related to treatment were reported in 10 (6.1%) of the 164 patients in the combined arms; only arthralgia and malaise were reported in >1 patient (2 patients each). Adverse drug reactions (ADRs) were reported in 9 of 164 (5.5 %) subjects treated with routine prophylaxis or episodic (on-demand) therapy. Adverse drug reactions are considered adverse events assessed as related to treatment with rFVIIIFc. Adverse drug reactions are summarized in Tables 13A and B (percentages) or Table 14 (frequencies). There was one death secondary to polysubstance overdose (suicide) in a patient with prior history of depression, which was assessed as unrelated to rFVIIIFc by the investigator. No events of anaphylaxis or severe vascular thrombosis were reported and no clinically meaningful changes were observed in total serum immunoglobulin G or the four subclasses in any treatment arm.

One (1) subject was withdrawn from study due to an adverse drug reaction of rash. In the study, no inhibitors were detected and no events of anaphylaxis were reported.

**TABLE 13A: Adverse Drug Reactions reported for rFVIIIFc**

| MedDRA* System Organ Class | MedDRA Preferred Term | N=164[1] Number of Subjects n (%) |
|---|---|---|
| **General disorders and administration site conditions** | Malaise<br>Chest pain<br>Feeling cold<br>Feeling hot | 2 (1.2)<br>1 (0.6)<br>1 (0.6)<br>1 (0.6) |
| **Nervous system disorders** | Dizziness<br>Dysgeusia<br>Headache | 1 (0.6)<br>1 (0.6)<br>1 (0.6) |
| **Musculoskeletal disorders** | Arthralgia<br>Joint swelling<br>Myalgia | 2 (1.2)<br>1 (0.6)<br>1 (0.6) |
| **Gastrointestinal disorders** | Abdominal pain, lower<br>Abdominal pain, upper | 1 (0.6)<br>1 (0.6) |
| **Vascular disorders** | Angiopathy[2]<br>Hypertension | 1 (0.6)<br>1 (0.6) |
| **Cardiac disorders** | Bradycardia | 1 (0.6) |
| **Injury, poisoning, and procedural complications** | Procedural hypotension | 1 (0.6) |
| **Respiratory, thoracic, and mediastinal disorders** | Cough | 1 (0.6) |
| **Skin and subcutaneous tissue disorders** | Rash | 1 (0.6) |

*MedDRA version 15.0
[1]164 previously treated patients (PTPs) on routine prophylaxis or episodic (on-demand) therapy
[2]Investigator term: *vascular pain after injection of study drug*

**TABLE 13B: Summary of Adverse Drug Reactions**

| | Individualized Prophylaxis (N=117) | Weekly Prophylaxis (N=24) | Episodic Treatment (N=23) |
|---|---|---|---|
| Total adverse events, no. | 219 | 46 | 23 |
| Patients with ≥ 1 adverse event, n (%) | 80 (68.4) | 18 (75.0) | 10 (43.5) |
| Most common adverse events (≥3%†), n (%) | | | |
| Nasopharyngitis | 16 (13.7) | 1 (4.2) | 3 (13.0) |
| Arthralgia | 10 (8.5) | 2 (8.3) | 1 (4.3) |
| Headache | 5 (4.3) | 6 (25.0) | 2(8.7) |
| Upper respiratory infection | 6 (5.1) | 0 | 3 (13.0) |
| Influenza | 5 (4.3) | 0 | 0 |
| Pyrexia | 3 (2.6) | 1 (4.2) | 1 (4.3) |

*Excluding the surgery subgroup.
†Based on pooled analysis of arms 1-3.

The incidence of the adverse reactions below is expressed according to the following categories:

Very common (≥1/10)

Common (≥1/100 to <1/10)

Uncommon (≥1/1,000 to <1/100)

Rare (≥1/10,000 to <1/1,000)

Very rare (<1/10,000)

**TABLE 14: Adverse Reactions reported for rFVIIIFc**

| | Frequency | |
|---|---|---|
| **MedDRA* System Organ Class** | **Common (≥1/100 to <1/10)** | **Uncommon (≥ 1/1,000 to <1/100)** |
| **General disorders and administration site conditions** | Malaise | Chest pain Feeling cold Feeling hot |
| **Nervous system disorders** | | Dizziness Dysgeusia Headache |
| **Musculoskeletal disorders** | Arthralgia | Joint swelling Myalgia |
| **Gastrointestinal disorders** | | Abdominal pain, lower Abdominal pain, upper |
| **Vascular disorders** | | Angiopathy[1] Hypertension |
| **Cardiac disorders** | | Bradycardia |
| **Injury, poisoning, and procedural complications** | | Procedural hypotension |
| **Respiratory, thoracic, and mediastinal disorders** | | Cough |
| **Skin and subcutaneous tissue disorders** | | Rash |
| *MedDRA version 15.0<br>[1]Investigator term: *vascular pain after injection of study drug* | | |

***Study Stopping Rules***

The study could conclude when all the following predefined criteria were met:

1) Thirteen patients in the sequential pharmacokinetic subgroup in Arm 1 had completed rFVIII pharmacokinetic profile, the rFVIIIFc day 0 pharmacokinetic profile, and a repeat 5-day rFVIIIFc pharmacokinetic profile 12 to 24 weeks later with adequate estimate of the terminal half-life.

2) A minimum of 104 patients from any arm had completed 50 rFVIIIFc EDs with an inhibitor test result from the central laboratory following at least 50 EDs, and at least 50 of these patients had an inhibitor test result from the central laboratory following 10 to 15 and 50 to 75 EDs.

3) Approximately 20 patients from Arm 2 and 20 patients from Arm 3 had completed at least 28 (±2) weeks on study.

4) A minimum of 10 major surgeries had been conducted in at least 5 patients.

When all of these criteria had been met, all ongoing patients were asked to return to the clinic for end-of-study assessments.

***Sample Size Determination***

The study was sufficiently powered to predict the occurrence of inhibitors, to compare ABR between Arms 1 and 3, and to detect an increase in rFVIIIFc pharmacokinetic parameters over rFVIII. Assuming an observed incidence of inhibitors of ≤1.9% in 104 patients with ≥50 EDs, then the upper bound of a Clopper-Pearson exact 95% CI would exclude a rate of ≥6.8%. Assuming a true inhibitor population incidence of ≤1%, there was ≥80% probability of ≤2 patients of 144 developing an inhibitor. To compare ABR with a greater than 90% power to detect a 60% reduction, a minimum of 1320 and 400 patient-weeks in Arms 1 and 3, respectively, were required, assuming that the true ABR for patients using episodic treatment was ≥10 bleeds per patient per year. A reduction in ABR of ≥50% had to be observed to be considered clinically important. Assuming the standard deviation of differences was ≤0.45 for primary pharmacokinetic endpoints, comparison of log-transformed endpoints using an analysis of variance model with factors for study treatment and patient would have ≥90% power to detect a 1.5-fold increase in rFVIIIFc over rFVIII.

A-LONG, and its companion study, B-LONG in hemophilia B, are the first studies to show that a technology to lengthen the terminal half-life of coagulation factors can extend dosing intervals, while retaining physiological activity in the coagulation cascade, reflecting the first major advance in the management of hemophilia since the availability of recombinant coagulation factor therapy 20 years ago. A-LONG demonstrated that molecular engineering to fuse Fc to FVIII did not impact the structure or function of the FVIII portion of the molecule. Moreover, the extended half-life of rFVIIIFc mediated by the FcRn recycling pathway translated into prolonged clinical activity, lengthening prophylactic dosing intervals to 1-2 times weekly from current regimens of 3-4 times weekly. The potential for reduced injection frequency with rFVIIIFc may improve both short- and long-term outcomes for patients with hemophilia A, which remain suboptimal with current therapies.

A-LONG confirmed data obtained in the first comparative study of rFVIIIFc and rFVIII, demonstrating significant improvements in all clearance-related pharmacokinetic parameters for rFVIIIFc versus rFVIII. All patients evaluated in A-LONG had an increased half-life and longer time above a trough FVIII level of 1 IU/dl relative to rFVIII. Additionally, these parameters demonstrated stability with continued treatment, indicating that pharmacokinetics were predictable from the first dose. The interaction between FVIII and VWF in the circulation plays a critical role; VWF binding protects FVIII from proteolytic degradation and binding with FVIII clearance receptors, possibly stabilizing FVIII structurally. However, the interaction between VWF and FVIII also likely limits the ability to extend FVIII half-life, and all strategies studied to date have failed to extend FVIII half-life beyond that of VWF (approximately 18 hours). This is supported by the correlation of VWF levels with rFVIIIFc half-life and is consistent with the approximate 19-hour half-life of rFVIIIFc observed here and in the phase 1/2a study. In contrast, rFIXFc pharmacokinetics are not dependent upon VWF, and Fc fusion to rFIX resulted in a 82-hour half-life. Importantly, FVIII activity could be monitored accurately using conventional one-stage and chromogenic assays and plasma standards.

The efficacy of rFVIIIFc for routine prophylaxis was demonstrated through clinically meaningful reductions in ABR at dosing intervals between 3 and 5 days with individualized prophylaxis (Arm 1) and weekly dosing (Arm 2) compared with episodic treatment (Arm 3). These results were obtained using median weekly prophylaxis dosages less than those recommended by current treatment guidelines. Although most patients on prior prophylaxis reported injecting FVIII at least three times weekly prior to entry into Arm 1, the on-study median dosing interval was 3.5 days (twice-weekly dosing), with a significant proportion (30%) of patients who were on study for at least 6 months achieving a maximum allowed per-protocol interval of 5 days. ABRs in this arm were consistent with those reported with other prophylactic regimens, with approximately 45% of patients experiencing no bleeding episodes. This dose, regimen, and outcome are not possible with existing treatment.

In contrast to Arm 1, the prophylactic regimen for Arm 2 was not designed to maintain FVIII activity above 1 IU/dl in all patients, but rather to provide efficacy data to inform therapeutic decision-making for patients unwilling or unable to undertake a more intensive protective regimen. For patients on prior episodic therapy randomized to Arm 2, there was a clinically meaningful and statistically significant reduction in ABR relative to episodic therapy, and most patients achieved an ABR in the range reported for existing prophylaxis regimens. The low, and observation that some patients (17.4%) had no breakthrough bleeding episodes, indicate that once-weekly prophylaxis can provide positive clinical outcomes for some patients. However, four patients had high bleeding rates in this arm, and would be switched to a more intensive dosing regimen in practice.

Overall, rFVIIIFc was well-tolerated, and adverse events were as expected for the hemophilia A population, with no evidence of increased immunogenicity. The high rates of patient retention and compliance with the prophylaxis regimens further support the tolerability of rFVIIIFc. This is the largest global pivotal study of severe hemophilia A, including a total of 9170 EDs and 100.2 patient-years of exposure. Additionally, a substantial proportion of patients (111) received ≥50 EDs to rFVIIIFc, the period during which patients typically develop inhibitors to exogenous replacement therapy. No inhibitors were detected, suggesting that inhibitor risk may be low with switching to rFVIIIFc, as observed with other rFVIII products. NNAs were observed in a total of 11 (6.7%) patients (6 at screening, 5 on study), consistent with previously reported rates in the inhibitor-negative hemophilia A population and above the ~5% observed in the normal population. The significance of NNAs in normal and hemophilic individuals has not been longitudinally studied until the present study. In A-LONG, all NNAs were of low titer and had no discernible clinical impact. There is not yet clinical evidence that manufacturing of rFVIIIFc in a human cell line and the related post-translation modifications (e.g., human glycosylation) or the Fc moiety will have any impact on immunogenicity for rFVIIIFc.

Limitations of this study must be considered. This study was partially-randomized, and patients could elect to enter individualized prophylaxis (Arm 1) or, if on episodic treatment, to be randomized to Arms 2 (weekly dosing) or 3 (episodic treatment). This study design did not appear to affect results, because subgroup analyses of ABRs by prior regimen in Arm 1 were consistent with the primary analysis, and efficacy was demonstrated when comparing ABR in the randomized arms. Interestingly, when compared with historical bleeding rates by prior regimen, treatment with rFVIIIFc resulted in reductions in ABR in patients on both prior prophylaxis and episodic treatment (Figure 6). Although the number of patients was insufficient to power a statistical comparison of the prophylaxis arms, the study was sufficiently powered to compare ABR in each prophylaxis arm to episodic treatment as well as to assess inhibitor risk, with the study designed in-line with current regulatory guidance. An additional study limitation was the per-protocol maximum 5-day dosing interval in Arm 1, which potentially restricted determination of the maximum therapeutic interval for some patients. Nevertheless, weekly dosing resulted in effective prophylaxis in most patients in Arm 2. Finally, although some of the end points used in clinical trials of hemophilia are subjective, the efficacy observed here was as expected for a long-acting prophylaxis therapy. Additionally, rFVIIIFc was effective at both maintaining hemostasis during surgery, in which absence of efficacy would have been catastrophic, and in treating bleeding episodes with efficacy comparable with or better than existing treatment. The results for this study are summarized in Table 9B.

In this phase 3 study, rFVIIIFc was well-tolerated and effective in the prevention and treatment of bleeding in patients with severe hemophilia A. The potential for dosing one to two times weekly with rFVIIIFc, with either no bleeding episodes or a low breakthrough bleeding rate, is a clinically meaningful improvement over existing rFVIII therapy, reducing the number of injections by approximately half. This potential for reduced injection frequency may increase adherence with prophylaxis and, ultimately, improve outcomes for hemophilia A patients.

Example 4. Population Pharmacokinetic Analysis of Long-Acting Recombinant Factor VIII-Fc Fusion Protein (rFVIIIFc) in Patients with Severe Haemophilia A

Introduction: In a recently completed Phase 3 clinical study (A-LONG), rFVIIIFc, a recombinant fusion protein composed of a single molecule of B domain deleted human coagulation factor VIII (FVIII) attached to the Fc domain of human immunoglobulin G1 (IgG1), was well-tolerated and effective in the treatment of bleeding, routine prophylaxis, and perioperative management. The duration of activity of rFVIIIFc was prolonged, compared to another recombinant FVIII product (ADVATE®).

Objectives: To characterize the activity-time profiles of rFVIIIFc in hemophilia A patients as measured by the one-stage clotting assay by population PK analysis; to identify intrinsic and extrinsic covariates that can affect the variability of rFVIIIFc PK; and to simulate the rFVIIIFc dosing scenarios of interest using the model developed.

Methods: The modeling dataset included activity-time profiles in a total of 180 subjects (16 from a Phase 1/2a study and 164 from A-LONG) taken over up to 52 weeks of treatment.

The Phase 1/2a study was an open-label, multicenter, dose-escalation study that included 2 dose levels. A total of 16 subjects received either 25 IU/kg (n=6) or 65 IU/kg (n=10) of ADVATE® followed 3 or 4 days later, respectively, by an equal dose of rFVIIIFc with an extensive PK sampling scheme following each dose administration.

A-LONG was an open-label, multinational, multicenter study that included 3 treatment arms (described in Example 2).

Subjects were 12 to 65 years old and weighing between 41 kg and 132 kg. The analysis was done with NONMEM 7 software, and included model building, covariate search, and model qualification steps. The data were analyzed using mixed-effects modeling with maximal likelihood parameter estimation methods. Covariate-parameter relationships were explored for parameters with inter-individual variability (IIV) terms, and model diagnostics were performed to guide model selection. rFVIIIFc dosing regimens of therapeutic interest were simulated using the current model.

Results: A 2-compartmental model was found to describe adequately the activity of rFVIIIFc, described by the equation below:

$$CL = TVCL \cdot \left[\frac{VWF}{118}\right]^{\Theta_{10}}$$

$$V_1 = TVV_1 \cdot \left[\frac{WT}{73}\right]^{\Theta_8} \cdot \left[\frac{HCT}{45}\right]^{\Theta_9}$$

$$\varepsilon_{add} = STUD \cdot \Theta_5 + (1 - STUD) \cdot \Theta_6 \qquad STUD = \begin{cases} 1 & for\ Phase\ 1 \\ 0 & for\ Phase\ 3 \end{cases}$$

The tight confidence intervals (Cis) indicate that the parameters are estimated with very good precision (Table 17).

**Table 17: rFVIIIFc Population PK parameters and bootstrap-derived 95% Confidence Intervals**

| | | | |
|---|---|---|---|
| Clearance, CL, [dL/h] | $\Theta 1$ | 1.65 | 1.57 - 1.74 |
| Exponent on VWF | $\Theta 10$ | -0.343 | -0.439 - -0.247 |
| Central Volume, V1, [dL] | $\Theta 2$ | 37.5 | 36.5 - 38.4 |
| Allometric exponent on VI | $\Theta 8$ | 0.382 | 0.271 -0.499 |
| Exponent on HCT | $\Theta 9$ | -0.419 | -0.656 - -0.208 |
| Intercompartmental Clearance, Q, [dL/h] | $\Theta 3$ | 0.0746 | 0.0594 - 0.184 |
| Peripheral Volume, V2, [dL] | $\Theta 4$ | 6.92 | 3.80 - 13.8 |
| IIV on CL, [%] | $\eta 1$ | 24.3 | 20.5 - 27.7 |
| IIV on V1, [%] | $\eta 2$ | 13.4 | 11.0 - 15.5 |
| Correlation between IIV on CL and V1 | $\eta 12$ | 0.548 | Not calculated |
| IOV on CL, [%] | n3 | 20.6 | 16.7 - 25.1 |
| IOV on V1, [%] | $\eta 4$ | 12.0 | 7.46 - 16.3 |
| Correlation between IOV on CL and V1 | $\eta 34$ | 0.639 | Not calculated |
| Additive error, Phase 1/2A study, [IU/dL] | $\Theta 5$ | 0.421 | 0.172 - 0.612 |
| Additive error, Phase 3 study, [IU/dL] | $\Theta 6$ | 0.208 | 0.126 - 0.275 |
| Proportional error, [%] | $\Theta 7$ | 13.6 | 12.0 - 15.3 |

The IIV and the residual errors are very low. The goodness of fit diagnostics (Figure 9) indicate that the model adequately describes the data.

The population estimate for the clearance (CL) is 1.65 dL/h, and Vss is 44.4 dL. The inter-individual variability (IIV) of CL is moderate (CV=24.3%) and of central volume of distribution (VI) is low (CV=13.4%). The inter-occasional variability (IOV) of both CL and VI is low (20.6 and 12.0% respectively). The additive residual error is very low (0.208 IU/dL), and so is the proportional residual error (13.6%), approximating the precision of the one-stage clotting assay for FVIII activity. Von Willebrand Factor (VWF) level was identified as the major covariate for CL. Consistent with mechanistic knowledge (Lillicrap 2008), higher levels of VWF yielded lower clearance values, reflecting the protective role that VWF has on FVIII activity. This is reflected in the negative exponent on VWF. Body Weight (BW) and Hematocrit (HCT) were identified as weak covariates on VI. This is the first population PK analysis that systematically describes and characterizes the prolonged activity profile of the long-acting rFVIIIFc.

Conclusion: The population PK model of rFVIII activity adequately describes the observed activity-time profiles after long term administration. The clearance of rFVIIIFc activity is lower than the clearance reported for ADVATE®, resulting in longer duration of activity. The low IIV underlines the consistency and homogeneity of the activity profiles. The low IOV indicates that rFVIIIFc maintains stable and predictable activity with long term administration over time. The set of covariates identified is physiologically relevant. Therefore, the population model developed can be used to simulate various dosing scenarios in support of dosing regimen selection and other decision making related to rFVIIIFc therapy.

The model was used to predict the activity time profile following a single dose of rFVIIIFc in patients with severe hemophilia A. In addition the model was used to predict trough activity for three different prophylaxis regimens.

Table 18 presents the model-predicted steady state peaks and troughs of the rFVIIIFc activity-time profiles with 50 IU/kg dose administered every 3, 4, or 5 days. The simulated steady-state activity profiles are presented in Figure 10.

**Table 18: Predicted Steady-State Peaks and Troughs of rFVIIIFc Activity-Time Profiles with 50 IU/kg Dose Administered Every 3, 4, or 5 Days**

| Percentile of Subjects | Dosing Frequency | | | | | |
|---|---|---|---|---|---|---|
| | Every 3 Days | | Every 4 Days | | Every 5 Days | |
| | Peak | Trough | Peak | Trough | Peak | Trough |
| Median (5th, 95th prediction interval) | 102 (70.4, 149) | 5.27 (0.774, 20.4) | 100(69.9, 141) | 2.32 (<0.5a, 11.4) | 98.5 (69.1, 139) | 1.10 (<0.5, 6.17) |

A dosing regimen of 50 IU/kg every 5 days is predicted to yield troughs above 1 IU/dL in 53.4% of individuals and a dosing regimen of 65 IU/kg administered weekly is predicted to yield troughs above 1 IU/dL in 26.6 % of the individuals treated.

The population PK models for rFVIIIFc and ADVATE® adequately described the combined activity data from the two clinical studies. The major covariate for rFVIIIFc activity identified by the population PK analysis was VWF level on CL. Weight and hematocrit were identified as minor covariates on the central volume of distribution. The results from the simulations support the dosing recommendations derived from the Phase 3 study results and can be used to aid dosing regimen selection and adjustment.

**Simulation of Regimens for Perioperative Management**

According to the WFH Guidelines, minor surgical procedures may require the attainment of target factor levels of 50 to 80 IU/dL, which can be achieved with a single rFVIIIFc infusion of 25 to 40 IU/kg. If a finer adjustment of the target is desirable, the required rFVIIIFc dose can be determined based on the predicted activity profiles in Table 19, below:

**Table 19: Predicted rFVIIIFc Activity-Time Profiles (in IU/dL) After a Single Administration**

| Dose (IU/kg) | Time (h) | | | | | | |
|---|---|---|---|---|---|---|---|
| | EOI | 12 | 24 | 36 | 48 | 72 | 96 |
| | Median (5 th, 95th Prediction Interval) | | | | | | |
| 20 | 38.7 (27.3, 54.5) | 22.7 (13.5, 35.0) | 13.4 (5.79, 23.8) | 7.92 (2.44, 16.7) | 4.72 (1.06, 12.0) | 1.79 (<0.5*-6.52) | 0.763 (<0.5*-3.63) |
| 25 | 48.4 (34.2, 68.1) | 28.3 (16.9, 43.7) | 16.8 (7.24, 29.8) | 9.90 (3.05, 20.8) | 5.90 (1.32, 15.0) | 2.24 (<0.5*-8.15) | 0.953 (<0.5*-4.54) |
| 30 | 58.1 (41.0, 81.7) | 34.0 (20.2, 52.5) | 20.2 (8.69, 35.8) | 11.9 (3.66. 25.0) | 7.07 (1.59, 18.0) | 2.69 (<0.5*-9.78) | 1.14 (<0.5*-5.44) |
| 40 | 77.5 (54.7, 109) | 45.3 (27.0, 70.0) | 26.9 (11.6, 47.7) | 15.8 (4.88, 33.3) | 9.43 (2.11, 24.0) | 3.58 (<0.5*-13.0) | 1.53 (<0.5*-7.26) |
| 50 | 96.8 (68.3, 136) | 56.6 (33.7, 87.5) | 33.6 (14.5, 59.6) | 19.8 (6.10, 41.7) | 11.8 (2.64, 30.0) | 4.48 (0.615-16.3) | 1.91 (<0.5*-9.07) |
| 65 | 126 (88.9, 177) | 73.6 (43.8, 114) | 43.7 (18.8, 77.5) | 25.7 (7.94, 54.2) | 15.3 (3.44, 38.9) | 5.82 (0.800-21.2) | 2.48 (<0.5*-11.8) |
| EOI = end of infusion.<br>* BLQ values were set at <0.5. | | | | | | | |

For major surgery, WFH recommends a stricter control of Factor VIII dosing, with high activity levels attained in the pre-operative period and during surgery, followed by stepwise decrease of the activity levels during the post-operative period, e.g., within 1 to 3 days, 4 to 6 days, and as long as needed. The population PK model developed can be used as a tool to design such complicated activity profiles.

An evaluation of the population PK model, similar to external model validation, indicated that the population PK model is in good concordance with the activity observed during the surgical period. The correlation coefficient between the observed and individual predicted activity during surgery is high (R = 0.742). The minimal discrepancies between observed and model-predicted activities during surgery are not more than 10% and point to a modest over-prediction associated with peak activity. This may be due to minimal loss of rFVIIIFc during surgery, associated with blood loss. For the surgeries performed in this study, the average blood loss was less than 200 mL.

Table 20 presents the dosing times, doses, and predicted FVIII activity for a hypothetical perioperative dosing regimen, to achieve levels recommended by WFH Guidelines. The doses, times, and administration as well as the predicted activities are listed for each subsequent dose. All doses after the second one are administered at 24-hour intervals. The resulting activity time profile is presented in Figure 11.

**Table 20: Dosing Times, Doses and Predicted FVIII Activity for a Hypothetical Perioperative Dosing Regimen for rFVIIIFc**

| Dosing Day | Dosing Time (h) | Dose (IU/kg) | Trough (IU/dL) Median ($5^{th}$, $95^{th}$ Prediction Interval) |
|---|---|---|---|
| **0** | 0 | 60 | |
| **0** | 12 | 50 | 67.3 (39.6, 105) |
| **1** | 24 | 50 | 95.4 (50.7, 159) |
| **2** | 48 | 50 | 67.0 (23.6, 140) |
| **3** | 72 | 40 | 57.9 (19.0, 132) |
| **4** | 96 | 40 | 48.1 (15.4, 119) |
| **5** | 120 | 40 | 45.0 (14.8, 112) |
| **6** | 144 | 40 | 44.0 (14.7, 109) |
| **7** | 168 | 30 | 43.6 (14.7, 108) |
| **8** | 192 | 30 | 36.9 (11.9, 95.6) |
| **9** | 216 | 30 | 34.5 (11.3, 88.9) |
| **10** | 240 | 30 | 33.6 (11.2, 85.6) |
| **11** | 264 | 30 | 33.2 (11.2, 83.6) |
| **12** | 288 | 30 | 33.0 (11.2, 82.4) |
| **13** | 312 | 30 | 32.9 (11.1, 81.7) |

Table 21 presents the dosing times, doses and predicted FVIII activity for a simpler hypothetical perioperative dosing regimen. All doses after the fourth dose are administered at 48 h intervals. Figure 12 shows the predicted FVIII activity for the hypothetical perioperative dosing regimen in Table 21.

**Table 21: Dosing times, Doses and Predicted FVIII Activity for a Sample Perioperative Dosing Regimen II**

| Dosing Day | Dosing Time (hr) | Dose (IU/kg) | Trough (IU/dL) Median [$5^{th}$, $95^{th}$ PI] |
|---|---|---|---|
| 0 | 0 | 60 | |
| 0 | 12 | 50 | 68.2 [39.8, 105] |
| 1 | 24 | 50 | 97.7 [50.8, 160] |
| 2 | 48 | 50 | 68.6 [23.7, 144] |
| 4 | 96 | 50 | 22.0 [3.96, 75.1] |
| 6 | 144 | 50 | 16.4 [3.32, 57.6] |
| 8 | 192 | 50 | 15.6 [3.25, 52.7] |
| 10 | 240 | 50 | 15.4 [3.23, 51.0] |

(continued)

| Dosing Day | Dosing Time (hr) | Dose (IU/kg) | Trough (IU/dL) Median [5th, 95th PI] |
|---|---|---|---|
| 12 | 288 | 50 | 15.3 [3.22, 50.4] |

The model was used to predict FVIII activity in patients during the post-operative period based on subjects' presurgery baseline PK. In the Phase 3 study, there were 13 subjects who underwent major and minor surgeries and also had their rFVIIIFc activity measured during the perioperative period. These subjects had variable dosing regimens and time intervals in perioperative management. To check whether the observed FVIII activity during this period (coded as OCC=0 in the database) are in agreement with the population PK model, an evaluation, similar to external model validation, was performed.

Predicted FVIII activity was largely consistent with observed FVIII activity. Figure 13 is a representative plot comparing the predicted and observed FVIII activities within the first 21 days after the first rFVIIIFc surgical dose (n=13; 9 major surgeries, 4 minor surgeries). There was good correlation between the observed FVIII activity data and that predicted by the PK model (relative prediction error 95% CI). The resulting Goodness of Fit (observed DV, versus individual predicted, IPRED) diagnostics, shown in Figure 13, indicates that the population PK model is in concordance with the activity observed during the surgical period. The correlation coefficient between the observed and individual predicted activity is high ($R = 0.742$).

The nonparametric fit of the data, represented by the lower line in Figure 13, diverges only slightly from the identity line (upper line). This divergence is not more than 10% and points to a modest over-prediction in the region of high activity. This may be due to minimal loss of rFVIIIFc during surgery, associated with blood loss. For the surgeries performed in this study, the average blood loss was less than 200 mL. These results indicate that the surgical procedures that have been studied in the Phase 3 study had no significant impact on the PK properties of rFVIIIFc.

In conclusion, it is feasible to develop a general dosing guidance to achieve target FVIII levels recommended for perioperative management in patients with haemophilia A using a population PK model.

Example 5. Paediatric Study

*Study Design*

A phase 3, open-label, multicenter, paediatric study to evaluate the safety, PK, and efficacy of rFVIIIFc for the control and prevention of bleeding in previously treated children (<12 years in age) with severe haemophilia A (endogenous FVIII activity <1 IU/dL [1%]) is described.

The enrolment is approximately 50 children with severe haemophilia A. Eligibility criteria for this pediatric previously treated patient (PTP) study requires the children to have had ≥50 documented prior exposure days (EDs) to FVIII, weigh ≥13 kg at the time of consent, and have no current or history of inhibitors to FVIII.

The treatment regimen is for prophylaxis (see study design shown in Figure 3). PK analysis of FVIII and rFVIIIFc will be performed in a subgroup of the study participants prior to initiation of prophylactic treatment for all subjects.

Primary and secondary outcome measures will include the following:

- primary outcome measures: frequency of inhibitor development
- secondary outcome measures: number of annualised bleeding episodes; assessments of response to treatment with rFVIIIFc for bleeding episodes

The safety and efficacy of rFVIIIFc in previously untreated patients (PUPs), defined by the European Medicines Agency as patients with no prior exposure to any factor products will also be assessed.

The results of the paediatric PTP and PUP studies will provide further insight into the clinical safety and haemostatic parameters of rFVIIIFc for treating haemophilia in children.

*Interim Analysis*

Prophylactic factor VIII (FVIII) administration is the standard of care for patients with severe hemophilia A; however, frequent injections are required to maintain protective factor levels. To reduce injection frequency, we developed a long-lasting recombinant FVIII Fc fusion protein (rFVIIIFc) consisting of one rFVIII molecule covalently linked to the Fc domain of immunoglobulin G1 (IgG1). rFVIIIFc had a 1.53-fold higher half-life and a 36% reduction in clearance (CL) versus FVIII (ADVATE®) in a phase 3 study of adults and adolescents. The Kids A-LONG study (NCT01458106) was designed to investigate the pharmacokinetics (PK), safety, and efficacy of rFVIIIFc in pediatric subjects with hemophilia A who were previously treated with FVIII products. The objective of this planned interim analysis was to determine the PK parameters of rFVIIIFc in pediatric subjects and compare these parameters to those of the subjects' prescribed FVIII products.

Methods: This multi-center, open-label, phase 3 study is currently enrolling previously-treated subjects aged <12 years with severe hemophilia A (was U/dL endogenous FVIII), at least 50 exposure days (EDs) to FVIII products, and no history of or current inhibitors to FVIII. Subjects are stratified into two age cohorts (<6 years and 6 to <12 years). All subjects are started on a twice-weekly rFVIIIFc prophylactic regimen 25 IU/kg on day 1 and 50 IU/kg on day 4 with subsequent dosing adjustment based on PK data and bleeding frequency. The primary endpoint is the incidence of inhibitor development. A sequential PK analysis is performed to compare the PK parameters of rFVIIIFc with that of the prescribed FVIII product. Subjects undergo a washout period of at least 72 hours before receiving the first dose of either FVIII or rFVIIIFc. For FVIII PK analysis, subjects receive 50 IU/kg of their currently prescribed FVIII product with sampling at baseline and at 4 additional time points after for up to 48 hours. For rFVIIIFc PK assessment, subjects receive 50 IU/kg rFVIIIFc, with sampling prior to rFVIIIFc administration and at 5 additional time points after for up to 72 hours. PK parameters were derived from FVIII activity-over-time profiles estimated by the non-compartmental analysis using the PK data analysis software PHOENIX™ WinNonlin 6.2.1.51. FVIII activity was measured by the one-stage clotting assay calibrated against a commercially available FVIII plasma standard. A data cut-off date of 8 February 2013 was used to report PK data in this interim analysis.

Results: At the time of this analysis, 52 subjects were enrolled and received at least one dose of FVIII and/or rFVIIIFc. Of 37 subjects with evaluable PK profiles, 30 received both FVIII and rFVIIIFc. For PK assessment of FVIII, 7 different FVIII products were used, of which ADVATE®, HAEMOSOLVATE®, and KOGENATE FS® were the most common. A comparison of PK parameters for rFVIIIFc versus FVIII for both age cohorts demonstrated that rFVIIIFc had a longer half-life (~1.5 fold increase) and a lower CL (30% to 50% reduction) than FVIII (Table 22).

**Table 22: PK of FVIII and rFVIIIFc in pediatric subjects (Geometric mean [95% CI])**

| | | Half-life (hr) | CL (mL/dL/kg) | IR (IU/dL per IU/kg) | Vss (mL/kg) |
|---|---|---|---|---|---|
| < 6 yrs | FVIII (pre-study*) n=15 | 7.82 (6.9 - 8.9) | 5.69 (4.7 - 6.8) | 1.84 (1.6 - 2.1) | 63.25 (54.0 - 74.1) |
| | rFVIIIFc (on-study) n=10 | 11.54 (9.4 - 14.2) | 3.70 (2.9 - 4.7) | 1.88 (1.8 - 2.0) | 58.42 (54.7 - 62.4) |
| | rFVIIIF c/FVIII Ratio n=8 | 1.45 (1.2 - 1.8) | 0.71 (0.6 - 0.9) | 1.02 (0.8 - 1.3) | 0.98 (0.8 - 1.2) |
| 6 to <12 yrs | FVIII (pre-study*) n=29 | 9.91 (9.1 - 10.8) | 4.47 (3.8 - 5.2) | 1.98 (1.7 - 2.3) | 63.21 (55.3 - 72.2) |
| | rFVIIIFc (on-study) n=27 | 13.22 (11.1 - 15.7) | 2.50 (2.1 - 3.0) | 2.28 (2.0 - 2.6) | 48.13 (43.2 - 53.7) |
| | rFVIIIF c/FVIII Ratio n=22 | 1.44 (1.3 - 1.7) | 0.52 (0.5 - 0.6) | 1.14 (1.0 - 1.4) | 0.77 (0.7 - 0.9) |
| * Pre-study FVIII includes both plasma-derived and recombinant FVIII products | | | | | |

Conclusion: In comparison to currently available FVIII products, rFVIIIFc had an extended half-life and reduced CL in children. These results are in agreement with those previously observed in adults and adolescents. The final analysis of the Kids A-LONG study will provide additional PK information and evaluate the safety and efficacy of rFVIIIFc in children.

Example 6. Use of the Population Pharmacokinetic Model of rFVIIFc to Simulate or Estimate Individualized and Median Patient Treatment

As is discussed in Example 4, the rFVIIIFc population model that has now been developed can be used to simulate (estimate) various dosing scenarios in support of dosing regimen selection and other decision making related to rFVIIIFc therapy.

For example, individualized patient treatment, e.g., pharmacokinetics (PK) and dosing regimens, can be selected using Bayesian estimation (or similar machine learning algorithm) based on the population pharmacokinetic (popPK) model described in Example 4, above (e.g., Table 17). In this way, one can determine alternative prophylactic dosing regimens and optimized dosing regimens for peri-operative management that have not previously been studied in the A-LONG trials. Alternatively, the selected dosing regimen is based on population PK (median PK) rather than making an individualized selection.

In some embodiments, the rFVIIIFc popPK model of Example 4 (e.g., Table 17) is used without the Bayesian or similar machine learning algorithm.

In some embodiments of this aspect of the invention, the method is carried out on a computer-based system, e.g., a server, a desk top computer, a lap top computer, a tablet, a hand held device, or a smart phone. In some embodiments, the computer-based system is a computer application. The computer-based system includes a storage medium for the rFVIIIFc popPK model discussed in Example 4, e.g., the parameters of Table 17. In some embodiments, the storage medium can also contain a Bayesian estimating program, e.g., NONMEM or Phoenix NLME. *E.g.,* Example 4; Kiang et al., Clin. Pharmacokinet 51:515-525 (2012).

In some embodiments, the system comprises two or more computer-based systems. In some embodiments, the user can input information into a first computer-based system that communicates with a second computer-based system, and the second computer-based system carries out calculations and communicates output information to the first computer-based system. This output information can include recommendations about individualized or non-individualized dosing regimens.

In some embodiments, the user inputs information into the system and the system calculates and outputs one or more PK or dosing regimens. In some embodiments, the system uses the received information to calculate and output individualized or median PK information. In some embodiments, the system calculates individualized dosing or interval information.

Information that can be input by a user and received by the system includes patient information and desired treatment outcome information. Based on the type and value of the received information, the computer-based system calculates output information based on the rFVIIIFc popPK model and optional machine learning algorithm on the storage medium.

Patient information includes, e.g., age, Von Willebrand Factor (VWF) level, hematocrit (HCT), body weight (BW), diagnostic (baseline) FVIII level, PK determinations, time of PK sampling, dosing history if PK samples were taken from multiple doses, actual dose, FVIII activity level, etc.

Desired treatment outcome information includes desired PK or desired regimen outcome, e.g., desired rise in plasma FVIII activity level following dose, desired dosing interval, and desired dose.

Based on the information that was input and received by the system, the system can output various information, e.g., PK curve, PK parameter such as incremental recovery (Cmax/dose), mean residence time, terminal t1/2, clearance, Vss, AUC/dose, doses and associated troughs, and intervals and associated troughs.

For example, for assessing individualized patient PK, the system can recommend that the user input 2-3 optimized PK sampling time points. In this case, system output can include PK curve and one or more selected PK parameters, e.g., incremental recovery (Cmax/Dose), mean residence time, terminal t1/2, clearance, Vss, AUC, and time to 1 or X%, etc. E.g., Figure 14.

As additional examples, to select an individualized dosing regimen using the output individual PK parameters discussed in the preceding paragraph, (i) the dose selected for acute treatment can be based on user input of the desired rise in plasma FVIII activity level following the dose, (ii) the dose selected for prophylaxis can be based on user input of the desired dosing interval, or (iii) the selected interval for prophylaxis can be based on user input for the desired dose. In the first case, the system can output the dose (IU) based in the patient's incremental recovery. E.g., Figure 15. In the second case, system output can be a table of doses and associated troughs, e.g., x IU/kg, 1% trough, y IU/kg, 2% trough, etc. E.g., Figure 16, top. In the third case, system output can be a table of intervals and associated troughs, e.g., x days, 1% trough, y IU/kg, 2% trough, etc., E.g., Figure 16, bottom.

The user may wish to use the system without inputting any individualized PK data. In this case, the dosing output would be based on the population median rather than being individualized for the particular patient. E.g., Figure 17. In this way, the user inputs, e.g., body weight and age, and (i) the desired rise in plasma FVIII activity level following the dose, (ii) the desired dose interval for prophylaxis, or (iii) the desired dose for prophylaxis. In the first case, the system can output the dose. In the second case, the system can output the dose and associated trough. E.g., Table 19 In the third case, the system can output the interval and associated trough. E.g., Table 18.

Age can be input to determine if the system is suitable for the patient because the current version of the popPK model was built for patients 12 years and older.

In some embodiments, the system is compliant with patient privacy laws. In some embodiments, the system is encrypted, e.g., with SSL. In some embodiments, input patient information is made anonymous.

In some embodiments, the system includes a user help function.

The user can be, e.g., a physician, a nurse, or another healthcare practitioner.

In some embodiments, the method further includes selecting a dosing regimen based on the system's output information and administering rFVIIIFc to the patient according to the selected regimen.

Example 7. The Bleeding Tendency in Relation to Predicted FVIII Activity Levels in Severe Hemophilia A Patients Treated with rFVIIIFc

Prophylactic regimens with coagulation factor in patients with hemophilia A have been designed to maintain FVIII activity above 1 IU/dL, based on previous studies demonstrating that joints can be preserved and bleeding is minimized when circulating factor levels remain above this level. This observation was confirmed that increasing time spent under 1 IU/dL FVIII activity in patients on prophylaxis with recombinant FVIII (rFVIII) was associated with an increase in bleeding episodes and hemarthroses. The pharmacokinetics (PK), safety, and efficacy of rFVIII Fc fusion protein (rFVIIIFc) have been evaluated in the A-LONG study and recently reported. Briefly, previously treated male patients, ≥ 12 years old with severe hemophilia A, were enrolled in A-LONG and allocated to 1 of 3 treatment arms: Arm 1) individualized prophylaxis with dose and dosing interval adjustments (25-65 IU/kg every 3-5 days) based on PK and clinical indications, Arm 2, fixed dose (65 IU/kg) weekly prophylaxis, and Arm 3, episodic treatment (10-50 IU/kg) for bleeding episodes. The median annualized bleeding rates for individualized prophylaxis and weekly prophylaxis were 1.6 and 3.6, respectively, and 33.6 for episodic treatment. In this analysis, we evaluated the bleeding tendency in relation to predicted FVIII activity levels in 163 subjects in the A-LONG study.

Methods: A 2-compartment population PK model of rFVIIIFc was developed based on activity-time profiles in 180 severe Hemophilia A subjects aged 12 - 65 years old (15 from a Phase 1/2a study and 165 from A-LONG collected over ≤ 52 weeks of treatment). Individual post-hoc PK parameters were derived to construct continuous FVIII activity-time profiles for each dose administered over the course of the study for 163 subjects in A-LONG. The cumulative time under 1IU/dL FVIII levels for each individual on study was calculated and normalized to obtain annualized time under 1IU/dL. Negative binomial regression models were used to evaluate associations between the number of bleeding episodes (overall, spontaneous, traumatic, and joint) and annualized time (days) under 1IU/dL FVIII activity. Models were adjusted for age, body mass index, baseline HIV and HCV status, blood type, number of bleeding episodes in the 12 months prior to study entry, and each subject's time on study.

Results: The predicted median annualized time under 1IU/dL FVIII activity for subjects on episodic treatment was 225 days, which was shortened to 51.50 days in subjects on a fixed prophylactic dose of 65 IU/kg of rFVIIIFc once weekly, and further reduced to 2.17 days in subjects on tailored prophylactic regimes (25 - 65 IU/kg, every 3 - 5 days). Multivariable negative binomial regression analysis showed that the number of overall bleeding episodes increased with increased time spent under 1IU/dL of FVIII activity level ($p<0.001$). A significant association was also observed for the time under 1% and the individual bleed types (spontaneous, traumatic, and joint) when analyzed separately. In addition, across all subtypes of bleeding, there was a significant decrease in the odds of being bleed-free for each one day increase in the time spent under 1IU/dL of FVIII activity.

Conclusions: In subjects treated with rFVIIIFc in the A-LONG study, there was a significant association between increased time spent under 1IU/dL of FVIII activity and increased bleeding tendency, as well as decreased probability of being bleed-free, in both adolescent and adult subjects with severe hemophilia A. The findings reinforce the importance of a therapeutic threshold of 1 IU/dL of FVIII activity as reported previously, and contribute to building a stronger foundation for designing effective prophylactic dosing regimens based on population simulations of FVIII activity PK.

Example 8. Dosing Long-Lasting Recombinant Factor VIII Fc Fusion Protein: Experience in the A-LONG Clinical Study

Prophylaxis with factor VIII (FVIII) in patients with severe hemophilia A requires frequent intravenous injections (3-4 per week), impacting compliance and outcomes. A long-lasting recombinant FVIII Fc fusion protein (rFVIIIFc) was developed to reduce the frequency of injections. The pharmacokinetics (PK), safety, and efficacy of rFVIIIFc were evaluated in the phase 3 A-LONG study (NCT01181128). To illustrate differences in dosing regimens and clinical outcomes with rFVIIIFc and currently available FVIII products, we compared the prestudy and on-study dose, dose interval, and bleeding rates for patients in the A-LONG study who reported receiving a prophylactic regimen with any FVIII product prior to study entry. We also used population PK models to estimate trough FVIII levels on various dosing regimens of rFVIIIFc and rFVIII (ADVATE®).

Methods: Previously treated male patients who were on various dosing regimens of rdy who reporenrolled in A-LONG and assigned to 1 of 3 treatment arms: Arm 1, individualized prophylaxis with PK-driven dose and dose interval adjustments (25-65 IU/kg every 3-5 days); Arm 2, once weekly prophylaxis (65 IU/kg); and Arm 3, episodic treatment (10-50 IU/kg) for bleeding episodes. A 2-compartmental population PK model of rFVIIIFc was developed based on activity-time profiles in 180 severe hemophilia A subjects aged 12-65 years old (15 from a phase 1/2a study and 165 from A-LONG collected over ≤ 52 weeks of treatment). A 2-compartment population PK model of ADVATE® was developed based on the single-dose PK profiles from 16 subjects in the phase 1/2a study and 30 subjects in the sequential PK subgroup in A-LONG. The population PK estimates for ADVATE® and rFVIIIFc from A-LONG were used in the dosing simulations. We identified Arm 1 subjects who reported use of a prophylactic regimen at least 2 times per week with any FVIII product prior to study entry and compared their dosing regimens and bleeding rate in the 12 months prior to study with their rFVIIIFc dosing regimens and annualized bleed rate (ABR) on study. Only subjects on study for ≥ 6 months were included. The median ABR, dose, and dose interval during the last 3 months on study were analyzed.

Results: Of 165 total patients, 118 were in Arm 1, of whom 80 received a prophylactic regimen at least 2 times per week prestudy and were in the study for udy for 8 were in Arm 1, of whom 80 rprestudy dosing interval. Table 23, below, provides prestudy and on study dose, dose interval, and bleeding rates for these groups.

**Table 23**

| **Prestudy FVIII reported dosing interval** | **5x/week** | **4x/week** | **3x/week** | **2x/week** |
|---|---|---|---|---|
| Number of patients in each dosing interval group | 1 | 5 | 65 | 9 |
| **Dosing Interval by days** | | | | |
| Prestudy[a] FVIII | 1.4 days | 1.75 days | 2.33 days | 3.5 days |
| On-study[b] rFVIIIFc | 5 days | 3 days | 3.5 days | 5 days |
| **Median Weekly Dose (IU/kg/week)** | | | | |
| Prestudy FVIII[c] | 50 | 140 | 84 | 60 |
| On-study[b] rFVIIIFc | 68.5 | 122.8 | 80.2 | 70.8 |
| **Median (range) Bleeding Events and ABR** | | | | |
| FVIII bleeding events 12 months prior to study entry | 24 (Not applicable) | 10 (0 - 52) | 5.5 (0 - 105) | 12.0 (0 - 103) |
| On study[b] rFVIIIFc ABR | 0 (Not applicable) | 8.0 (0 - 20) | 0.0 (0 - 16) | 0.0 (0 - 12) |

[a]Weekly dose interval obtained by dividing 7 days by the reported "times per week"
[b]On study refers to the last 3 months on study
[c]4 patients reported a range of FVIII doses used prior to study entry. The mean of the minimum and maximum dose was used as their prestudy FVIII dose

The majority of patients (65/80) reported receiving FVIII ~25IU/kg 3 times a week prior to study entry with a median of 5.5 bleeding events 12 months prior to study entry. At the end of the study, these same patients were receiving rFVIIIFc ~40 IU/kg twice a week (every 3.5 days) with a median ABR of 0. Population PK simulation indicated that 76.1% of patients treated with 40 IU/kg of rFVIIIFc twice a week would maintain FVIII levels above 1% at all times. In contrast, population PK simulation indicated that 42.3% of patients treated with 25 IU/kg of ADVATE® 3 times a week would maintain FVIII levels above 1% at all times. Overall, rFVIIIFc was well tolerated and no inhibitor development was detected during the A-LONG study.

Conclusion: The results from this descriptive analysis of dose, dose interval, and bleeding rates for subjects with severe hemophilia A who are currently on prophylaxis suggest that switching from current FVIII products to rFVIIIFc may require less frequent dosing intervals to maintain FVIII levels >1%.

Example 9. Repeated Time To Event Modeling of the Relationship Between rFVIIIFc Activity and Spontaneous Bleeding in Hemophilia A

In a pivotal Phase III study, A-LONG, individualized and weekly prophylactic treatments with rFVIIIFc reduced the bleeding rate by 92% and 76% compared to an episodic regimen. The objective in this example is to apply Repeated-Time-to-Event (RTTE) methodology to model the relationship between rFVIIIFc activity and spontaneous bleeding in subjects with episodic regimen.

23 severe hemophilia A subjects in the episodic treatment arm of the A-LONG study and 16 patients from a Phase 1/2a study were included in the analysis. A population PK model was developed to predict rFVIIIFc activity for each individual. In the RTTE model, baseline hazard rate is assumed to be constant over time within an individual and follows a log-normal distribution. The hazard at any time is reduced by the instantaneous rFVIIIFc activity level, described by an Emax model. NONMEM with Laplacean and EM algorithms were used for estimation.

The Laplacean estimated baseline hazard was 0.00745 (SE: 0.002) $hr^{-1}$, yielding mean time to bleeding of 5.6 days. The estimated inter-subject variability (CV%) of baseline hazard is 110% (SE: 16.9%). The estimated IC50 was 0.283 (SE: 0.083) IU/dL, indicating rFVIIIFc is an effective treatment to decrease bleeding risk. Various EM algorithm based estimation methods yielded similar results.

In conclusion, the proposed model provides a quantitative description of the effect of rFVIIIFc activity on reducing the risk of spontaneous bleeding. While low frequency of events could lead to difficulty in identifying baseline hazard and IC50 in an RTTE model, simulations suggested that data collected from on-demand regimen could provide adequate information on both parameters.

Example 10. Population Pharmacokinetics of recombinant factor VIII:Fc

The main objective of this example is to present the development of a population PK model based on rFVIIIFc activity data in hemophilia A patients. The PK parameters of the model were estimated and intrinsic and extrinsic factors (covariates) that may be significant determinants of variability in rFVIIIFc PK in hemophilia A patients were identified. The population PK model was then used to simulate rFVIIIFc dosing regimens of interest. A population PK model for ADVATE®, a rFVIII product used as comparator in the clinical studies performed, was also developed.

Methods: Clinical Studies

The clinical data that served as a basis for the population PK model development originated from two studies - a Phase 1/2a and a phase 3 study.

The Phase 1/2a study was an open-label, crossover, dose-escalation, multicenter study to determine the safety, tolerability, and PK of a single intravenous (IV) injection of rFVIIIFc in previously treated patients with severe hemophilia A. (Powell J S, Josephson N.c, Quon D, et al, Safety and prolonged activity of recombinant factor VIII Fc fusion protein in hemophilia A patients. Blood, 2012, 119 (13), 3031-7). The study included two cohorts dosed at the 25 IU/kg (Cohort A) and 65 IU/kg (Cohort B) level. After screening and a minimum of 4 days initial washout period, subjects from both cohorts received a single 25 IU/kg (Cohort A) or 65 IU/kg (Cohort B) dose of ADVATE® followed by a 3-day (Cohort A) or 4-day (Cohort B) blood sampling regimen for PK assessment (see upper panel of Table 24 for the Phase 1/2a sampling schedule). These patients then received a 25 IU/kg (Cohort A) or 65 IU/kg (Cohort B) single dose of rFVIIIFc followed by a 7-day (Cohort A) or 10-day (Cohort B) blood sampling regimen for PK assessment.

**Table 24: Intensive sampling schedules in the Phase 1/2a and Phase 3 studies**

| Study | Arm/Cohort | Agent | Dose | Sampling Schedule |
|---|---|---|---|---|
| | Cohort A | ADVATE® | 25 IU/kg | 0, 0.167, 0.5, 1,3,6, 9, 24, 48, 72 h |
| Phase 1/2a | | rFVIIIFc | 25 IU/kg | 0,0.167,0.5, 1, 3, 6, 9, 24, 48, 72, 96, 120, 168 h |
| | Cohort B | ADVATE® | 65 IU/kg | 0, 0.167, 0.5, 1, 3, 6, 9, 24, 48, 72, 96 h |
| | | rFVIIIFc | 65 IU/kg | 0,0.167,0.5, 1, 3, 6, 9, 24, 48, 72, 96, 120, 168, 192, 216, 240 h |
| Phase 3 | Arm 1 PK | ADVATE® | 50 IU/kg | 0, 0.5, 1, 6, 24, 48, 72 h |
| | Arm 1 PK | rFVIIIFc | 50 IU/kg | 0, 0.5, 1, 6, 24, 72, 96 and 120 h |
| | Arm 1 Non-PK | rFVIIIFc | 50 IU/kg | 0, 0.5, 3, 72, 96 h |
| | Arm 2 | rFVIIIFc | 65 IU/kg | 0, 0.5, 3, 72, 96 h |
| | Arm 3 | rFVIIIFc | 50 IU/kg | 0, 0.5, 3, 72, 96 h |

The Phase 3 study was an open-label, multinational, multicenter study to evaluate the safety, PK, and efficacy of rFVIIIFc administered as an IV injection to subjects with severe hemophilia A, at least 12 years of age (Mahlangu J. et al. Phase 3 study of recombinant factor VIII Fc fusion protein in haemophilia A, Lancet, submitted 2013). At study start, all subjects underwent a washout of FVIII products for 72-96 hours followed by an initial PK sampling. The study comprises 3 treatment arms:

Arm 1: Individualized Prophylaxis Regimen, consisting of two subgroups - a Sequential PK Subgroup and a Non-sequential PK Subgroup. Subjects assigned to the Arm 1 Sequential PK subgroup received a single dose of ADVATE® 50 IU/kg on Day 0 followed by 3-day blood sampling for PK assessment (7 timepoints) according to the schedules shown in Table 24 (lower panel). They subsequently received a single dose of rFVIIIFc 50 IU/kg followed by a 4-day PK sampling at 8 timepoints. A second, repeat PK profiling of rFVIIIFc 50 IU/kg occurred between Week 12 and Week 24. Subjects in Arm 1 Non-sequential PK subgroup received a single rFVIIIFc dose of 50 IU/kgof rFVIIIFc, on Day 0 followed by a 96-hour PK blood sampling performed at 5 timepoints. Upon completion of the rFVIIIFc PK assessment, an individualized prophylaxis regimen was established for each individual in Arm 1 (both subgroups) based on their PK, in which the dose (25 - 65 IU/kg) and dosing interval (3-5 days) were determined to maintain a trough level of 1% to 3% rFVIIIFc activity.

Arm 2: Weekly Prophylaxis Regimen. Subjects in Arm 2 received a single rFVIIIFc dose of 65 IU/kg of rFVIIIFc on Day 0 followed by a 96-hour PK blood sampling performed at 5 timepoints. After the PK assessment, subjects in Arm 2 were administered a fixed weekly prophylaxis rFVIIIFc dose of 65 IU/kg.

Arm 3: Episodic Dosing Regimen. Subjects in Arm 3 received a single rFVIIIFc dose of 50 IU/kg of rFVIIIFc on Day 0 followed by a 96-hour PK blood sampling performed at 5 timepoints. During the study, subjects in Arm 3 were treated episodically at rFVIIIFc doses between 10 and 50 IU/kg depending on the severity of the bleed.

In addition to the PK profiling, peak and trough measurements were carried out periodically, at nominal times spread over the whole course of study participation (such as at week 7, 14, 28, 38, 52, etc.) for subjects in Arms 1 and 2.

Assay Methodology

For the Phase 1/2a study, the FVIII activity was measured in citrated plasma samples on an MDA coagulation analyzer using the aPTT reagent obtained from Trinity Biotech ("Auto aPTT" composed of a silica-based activator and a phospholipid mixture), calcium chloride solution, and imidazole buffer against the Normal Reference Plasma from Precision Biologic with an activity assignment against the World Health Organization (WHO) fifth plasma international standard for FVIII. The assay was validated for accuracy and precision over a reportable range from 0.5 to 1000 IU/dL. Accuracy of this assay for FVIII measurements was shown to be within 15% of nominal activity with 3 validator samples ranging from 1.9 to 90 IU/dL FVIII. Intra- and inter-assay variabilities were below 15% coefficient of variation (CV) over this assay range.

In the Phase 3 study, the FVIII activity in citrated plasma samples for ADVATE® and rFVIIIFc was measured by the one-stage clotting (aPTT) assay on a Siemens BCS XP analyzer using commercial reagents (Dade Actin FSL) with calibration against a normal reference plasma (Precision Biologics CRYOcheck™) traceable to the World Health Organization (WHO) 5th International Standard (IS) for human plasma. The lower limit of quantification (LLOQ) for the one-stage assay was 0.5 IU/dL. The reportable range for this assay was from 0.7 to 600 IU/dL for rFVIIIFc and 0.5 to 600 IU/dL for ADVATE® and plasma FVIII. For all 3 products, the accuracy ranged from 92% to 116% for 5 validators. Intra-assay precision was typically below 10% CV (range 3.0% to 11.6%) and inter-assay variability was, on average, 4.8% CV.

Data Assembly and Manipulation

Datasets generated from both studies were merged together to form the population PK analysis dataset. An occasion (OCC) variable was added to the rFVIIIFc data set to enable the inclusion of inter-occasional variability (IOV) in the models. Occasions are defined within each individual, as clusters of observations, separated from previous observations by at least 1 week of dosing. Occasions 1 and 2 are reserved for the Baseline (first) and Repeat (second) intensive PK sampling period. The rest of the occasions are allocated to trough and peak measurements, and as such, represent sparse observation periods.

Some missing covariate values were imputed by substituting with a typical (median) value. Where missing covariate values could not be imputed, these were replaced by negative values and ignored. Where only a fraction of the covariate values for a given individual were missing, they were imputed from the remaining values by a LOCF (Last Observation Carried Forward) technique - this was the case for von Willebrand Factor (VWF) antigen levels, IgG1 levels (IGG1), and hematocrit (HCT).

Baseline Correction and Residual Decay

Activity levels observed before administration of a rFVIII product represent a combination of endogenous activity (baseline) and residual activity from pre-study drug. To account for that, baseline and residual drug corrections were performed on the observed FVIII activity results for both rFVIIIFc and ADVATE®. Similar corrections are typical for PK analyses of other FVIII and factor IX products (Björkman S, C.M., Berntop E, Pharmacokinetics of Factor IX in patients with hemophilia B. Eur J Clin Pharmacol, 1994. 46: p. 325-332; Carlsson M, B.S., Berntop E, Multidose pharmacokinetics of factor IX: Implications for dosing in prophylaxis. Hemophilia, 1998. 4: p. 83-88; Björkman S, S.A., Berntop E, Pharmacokinetics of recombinant factor IX in relation to age of the patients: Implications for dosing in prophylaxis. Hemophilia, 2001. 7: p. 133-139; Björkman S, O.M., Spotts G, et al., Population pharmacokinetics of recombinant factor VIII- the relationships of pharmacokinetics to age and body weight. Blood, 2012. 119: p. 612-618 ("Bjorkman 2012 A"); Björkman S, A.V., Population pharmacokinetics of plasma-derived factpr IX in adult patients with hemophilia B: Implications for dosing in prophylaxis. Eur J Clin Pharmacol, 2012. 68(6): p. 969-77). These corrected activity-time profiles were included in the population PK datasets. It should be noted that only the first (baseline) ADVATE® and rFVIIIFc activity-time profiles were corrected for residual decay.

Residual decay was performed using the terminal half-life (HL) obtained from noncompartmental analysis (NCA) of the raw, observed activity data. For the Arm 1 PK subgroup, ADVATE® and Baseline rFVIIIFc PK profiles were decayed using the respective subject's ADVATE® HL. For the Arm 1 non PK subgroup, Arm 2, and Arm 3, rFVIIIFc PK profiles were decayed using the average ADVATE® HL obtained from the Arm 1 PK subgroup.

Population Analysis Methodology

Two population PK models were developed independently of each other - one for rFVIIIFc, based on the rFVIIIFc data, and one for ADVATE®, based on the ADVATE® data alone.

Mixed-effects modeling with maximum likelihood parameter estimation methods were used to evaluate the population characteristics of rFVIIIFc and ADVATE® in hemophilia A patients. For the description of random inter-individual variability (IIV), a log-normal distribution of the random effects, with a block covariance matrix was used. Various random residual error configurations were tested by combinations of additive and proportional variance terms. Inclusion of IOV to account for the change in the system properties with time was evaluated. First order conditional estimation with interaction (FOCEI) method was implemented for parameter fitting.

Diagnostic plots, minimum value of the objective function (OFV), and the evaluation of shrinkage was used to guide model building and assess goodness-of-fit. The Likelihood Ratio Test was used to compare hierarchical models.

The base pharmacokinetic model was developed by exploring typical structural models, including one- and two-compartment linear models. Models were compared by goodness-of-fit diagnostics including the Likelihood Ratio Test, diagnostic plots, and estimates and standard errors of model parameters. The Likelihood Ratio Test was used to discriminate hierarchical models with a significance level of $p<0.01$ (change in objective function value=6.64 for 1 degree of freedom). The statistical model was built by testing and discriminating between various IIV and IOV structures and between different combinations of additive and proportional residual error terms. Candidate models were run from a number (usually 25-50) of different randomly generated initial estimates to assess the model stability and convergence.

Weight (WT) and Study (STUD), although technically assumed covariates, are sometimes considered as intrinsic to the basic model as their inclusion can be postulated a priori. For this reason the base models developed included these covariates.

The following factors were considered as potential covariates: height, age, race, blood type, hematocrit, von Willebrand factor antigen levels, IgG1 levels, albumin levels, non-neutralizing antibody (ADA) presence, HCV status, HIV status.

Once an appropriate base model was defined, covariate-parameter relationships were explored for the PK parameters that had IIV terms attached. The effect of covariates was evaluated using a forward inclusion, backward elimination process. In all covariate model scripts, the covariate was centered around a standard value - usually the median of the study population. For the forward covariate inclusion procedure, the Likelihood Ratio Test was used to compare hierarchical models with a significance level of $p<0.01$. Decrease in the respective IIV term and other Goodness of Fit measures were also considered for inclusion. Once the full population covariate model was constructed a stepwise backwards deletion method with a significance level of $p<0.001$ (change in objective function value=10.8 for 1 degree of freedom) was used to determine the final model. In all cases, physiological relevance of the covariates was considered before accepting a covariate.

A bootstrap procedure (nominally 1000 bootstraps), implemented by the bootstrap function of the Perl speaks Nonmem software package (PsN) was used to characterize the uncertainty in the model parameter estimates. Nonparametric 95% confidence intervals (CI) of all parameters were constructed based on the bootstrap results.

The predictive ability of the final model(s) was evaluated using simulation in a simplified visual predictive check. A series of Monte-Carlo simulations of the original trials were conducted (without parameter uncertainty) and summaries of resulting simulations were compared to the original data using qualitative methods. For this predictive check, the vpc function of PsN was used.

Further model evaluation, including internal and external validation, and inspection of the IIV term distributions, etc. was conducted as appropriate.

In order to evaluate the impact of values below the level of quantitation (BLQ), the analysis was performed using various approaches, either (i) excluding the BLQ values or (ii) using method(s) outlined in the literature to handle BLQ values (Beal, S., Ways to fit a PK model with some data below the quantification limit. J Pharmacokin Pharmacodyn, 2001. 28: p. 481-504; Jae Eun Ahn, M.O.K., Adrian Dunne and Thomas M. Ludden, Likelihood based approaches to handling data below the quantification limit using NONMEM VI J Pharmacokin Pharmacodyn, 2008. 35(4): p. 401-21).

Data points with |CWRES (Conditional Weighted RESiduals)| > 5 were considered potential outliers and were evaluated for impact on the qualified models by sensitivity analysis.

Atypical drug activity data (such as very low or very high activity levels, or data not compliant with the trough - dose - peak sequence) were excluded from the analysis if no apparent explanation for these observations was provided.

Activity data were excluded from the analysis if corresponding dosing or sampling times were missing or could not be reconstructed.

Simulations

A number of rFVIIIFc dosing regimens were simulated. During the simulations, the activity - time profiles at Steady State (SS) for 2000 individuals with each dosing scenario were generated. Weight characteristics of the population simulated were derived from the Phase 3 study data. Where IOV was part of the model, the SS dosing interval or period simulated was set up as a single occasion, i.e. IOV was sampled once. Uncertainty of parameter estimates was not included in the simulations. All dosing regimens were simulated using the same value of the random seed(s) to ensure comparability of the results. All simulations represent the activity time profiles with their central tendency (median) and variability, represented by the 5th, 25th, 75th, and 95th percentiles of the response (activity).

Software and Hardware

NONMEM (ICON plc, Dublin, Ireland, versions 7.1.2, and 7.2) was used for population PK analysis with Intel Fortran compiler (Intel Corporation, Santa Clara, California, version 11.1.048 and version 12.1).

Most of the model development was done on a workstation with a Quad Xeon Intel Processor and 8 GB of RAM. The computer intensive procedures such as bootstraps, visual predictive checks, etc. were run with up to 24 parallel cores on an HP 20-node cluster, each node with 2 quad-core Intel Xeon E5630 (160 cores in total) at 2.53 GHz and 24 to 60 GB of RAM.

Results: Base Model for rFVIIIFc

The base model for rFVIIIFc is a two compartment model with covariate WT on the central volume of distribution (VI); IOV, and BLOCK(2) IIV on clearance (CL) and V1; common proportional error, and separate additive residual error for the Phase 1/2a and Phase 3 data:

$$V_1 = TVV_1 \cdot \left[\frac{WT}{73}\right]^{\Theta_8}$$

$$\varepsilon_{add} = STUD \cdot \Theta_5 + (1 - STUD) \cdot \Theta_6 \text{, where } STUD = \begin{cases} 1 & for\,Phase\,1 \\ 0 & for\,Phase\,3 \end{cases} \quad \text{(Equation 1)}$$

The population parameters and the bootstrap-derived nonparametric 95% CIs are given in Table 25:

**Table 25: Population parameters of the base model for rFVIIIFc**

| Method | BLQ values commented out | | | | M3 Method |
|---|---|---|---|---|---|
| Parameter | Symbol | Population Estimate | Nonparametric 95% CI | Population Estimate |
| Clearance, CL, [dL/h] | $\Theta_1$ | 1.63 | 1.54-1.73 | 1.72 |
| Central Volume, V1, [dL] | $\Theta_2$ | 37.9 | 36.9 - 38.8 | 36.4 |
| Allometric exponent on VI | $\Theta_8$ | 0.448 | 0.341 - 0.552 | 0.498 |
| Intercompartmental Clearance, Q, [dL/h] | $\Theta_3$ | 0.0742 | 0.0581 - 0.187 | 1.15 |
| Peripheral Volume, V2, [dL] | $\Theta_4$ | 6.77 | 3.83 - 12.7 | 5.79 |
| IIV on CL, [%] | $\eta_1$ | 29.3 | 24.9 - 33.5 | 31.1 |
| IIV on V1, [%] | $\eta_2$ | 13.5 | 11.3-15.5 | 13.8 |
| Correlation between IIV on CL and VI | $\eta_{12}$ | 0.464 | N.C.[a] | 0.461 |

(continued)

| Method | | BLQ values commented out | | M3 Method |
|---|---|---|---|---|
| **Parameter** | **Symbol** | **Population Estimate** | **Nonparametric 95% CI** | **Population Estimate** |
| IOV on CL, [%] | $\eta_3$ | 20.7 | 16.5 - 25.1 | 21.9 |
| IOV on V1, [%] | $\eta_4$ | 12.2 | 7.81 - 16.2 | 10.5 |
| Correlation between IOV on CL and VI | $\eta_{34}$ | 0.643 | N.C. | 0.558 |
| Additive error, Phase 1/2a study, [IU/dL] | $\Theta_5$ | 0.419 | 0.150 - 0.634 | 0.469 |
| Additive error, Phase 3 study, [IU/dL] | $\Theta_6$ | 0.207 | 0.112 - 0.270 | 0.264 |
| Proportional error, [%] | $\Theta_7$ | 13.7 | 12.1 - 15.5 | 14.6 |

[a] Nonparametric 95% CI of 0.00972 - 0.0281 for a population mean of the covariance ω 12 of 0.0184
[b] Nonparametric 95% CI of 0.00637 - 0.0310 for a population mean of the covariance ω34 of 0.0163

The observation records containing BLQ activity values were excluded while developing the base model. In order to assess the influence of the BLQ values on the population estimates, these data records were re-introduced and the estimation was re-run using the M3 and M4 methods. Those runs experienced some instability in both the estimation and covariance steps; M4 was more unstable than M3. When an Importance Sampling estimation step (METHOD=IMP) was implemented following the FOCEI estimation, both the minimization and covariance step converged with the M3 method. Despite the inherent estimation instability with the inclusion of the BLQ values, whenever the estimation converged, the parameter estimates were very close to the estimates obtained in the same model that excluded BLQ values (see Table 25).

Covariate Model Building and Final Model for rFVIIIFc

As a result of the forward covariate inclusion, the full covariate model was identified, with VWF, AGE, and Hepatitis C virus (HCV, binary) as covariates on CL, and hematocrit (HCT), in addition to the base model WT and STUD as covariates on the central volume of distribution.

According to the accepted mechanistic hypothesis about the influence of antibodies on protein kinetics, the presence of antibodies usually increases the clearance of the target proteins by speeding up their rate of elimination. The incidence of antibodies in the dataset was low - 5 individuals had antibodies at baseline, while 6 individuals acquired antibodies during the course of the clinical trial. It should be noted also that the antibody detection was transient within the same individual.

Testing the antibody presence (ADA) as a binary covariate on VI unexpectedly led to a statistically significant drop in the OFV, while the influence on ADA on clearance was negligible. A closer inspection of the model revealed that although the drop in the OFV is statistically significant, the IIV term on VI decreased only marginally (from 0.018 in to 0.0173) following the covariate inclusion. The central volume of distribution in the presence of antibodies increased by 18%, which is unlikely to be clinically meaningful. A possible explanation for the assignment of the ADA covariate to the central volume, rather than to the clearance, is due to the dominant influence of those individuals that are antibody-positive at baseline over the individuals that acquire antibodies during therapy.

Due to the small sample size of those individuals who are antibody positive, as well as the transient character of the antibody response, and the related lack of physiological relevance of the ADA covariate, the antibody covariate (ADA) on V1 was rejected. Thus, no definitive effect of the presence of non-neutralizing antibodies on the kinetics of rFVIIIFc activity could be detected by this analysis.

At the second stage of the covariate model development process, a backward elimination of each covariate was implemented one at a time. As a result of this stepwise procedure, STUD covariate on V1, as well as HCV and AGE covariates on clearance were removed from the full covariate model without a significant impact on the OFV. The removal of the HCT on the central volume of distribution, however, led to a significant increase in the OFV, therefore HCT was retained.

The final population PK model for rFVIIIFc was a two compartment model with covariates VWF on CL, WT and HCT on V1, IOV (7 occasions), BLOCK(2) variance-covariance matrix on CL and V1, and separate additive residual error for the Phase 1/2a and Phase 3 with a common proportional error term, as follows:

$$CL = TVCL \cdot \left[\frac{VWF}{118}\right]^{\Theta_{10}}$$

$$V_1 = TVV_1 \cdot \left[\frac{WT}{73}\right]^{\Theta_8} \cdot \left[\frac{HCT}{45}\right]^{\Theta_9}$$

$$\varepsilon_{add} = STUD \cdot \Theta_5 + (1 - STUD) \cdot \Theta_6, \text{ where } STUD = \begin{cases} 1 & for\,Phase\,1 \\ 0 & for\,Phase\,3 \end{cases} \quad \text{(Equation 2)}$$

The population parameters of the final model and the bootstrap-derived nonparametric 95% CIs are given in Table 26:

Table 26: Population parameters of the final model for rFVIIIFc

| Method | BLQ values commented out | | | M3 Method |
|---|---|---|---|---|
| **Parameter** | **Symbol** | **Population Estimate** | **Nonparametric 95% CI** | **Population Estimate** |
| Clearance, CL, [dL/h] | $\Theta_1$ | 1.65 | 1.57-1.74 | 1.73 |
| Exponent on VWF | $\Theta_{10}$ | -0.343 | -0.439 - -0.247 | -0.391 |
| Central Volume, V1, [dL] | $\Theta_2$ | 37.5 | 36.5-38.4 | 36.8 |
| Allometric exponent on VI | $\Theta_8$ | 0.382 | 0.271 - 0.499 | 0.423 |
| Exponent on HCT | $\Theta_9$ | -0.419 | -0.656 - -0.208 | -0.412 |
| Intercompartmental Clearance, Q, | $\Theta_3$ | 0.0746 | 0.0594 - 0.184 | 0.279 |
| Peripheral Volume, V2, [dL] | $\Theta_4$ | 6.92 | 3.80 - 13.8 | 4.09 |
| IIV on CL, [%] | $\eta_1$ | 24.3 | 20.5 - 27.7 | 25.1 |
| IIV on V1, [%] | $\eta_2$ | 13.4 | 11.0 - 15.5 | 13.6 |
| Correlation between IIV on CL and V1 | $\eta_{12}$ | 0.548 | N.C.[a] | 0.563 |
| IOV on CL, [%] | $\eta_3$ | 20.6 | 16.7 - 25.1 | 22.0 |
| IOV on V1, [%] | $\eta_4$ | 12.0 | 7.46 - 16.3 | 9.27 |
| Correlation between IOV on CL and V1 | $\eta_{34}$ | 0.639 | N.C.[b] | 0.526 |
| Additive error, Phase 1/2a study, [IU/dL] | $\Theta_5$ | 0.421 | 0.172 - 0.612 | 0.416 |
| Additive error, Phase 3 study, [IU/dL] | $\Theta_6$ | 0.208 | 0.126 - 0.275 | 0.240 |
| Proportional error, [%] | $\Theta_7$ | 13.6 | 12.0 - 15.3 | 15.4 |
| [b] Nonparametric 95% CI of 0.00956 - 0.0264 for a population mean of the covariance $\omega$12 of 0.0179<br>[c] Nonparametric 95% CI of 0.00579 - 0.0312 for a population mean of the covariance $\omega$ 34 of 0.0158 | | | | |

As with the base model case, the influence of the BLQ values on the population estimates was assessed by running the M3 method on the final model with the BLQ values re-included in the dataset. The run completed with a successful minimization, but the covariance step was aborted, confirming the inherent instability introduced by the inclusion of BLQ values. There are minimal differences between the two sets of parameter estimates (Table 26); all M3 method parameters, except the intercompartmental clearance and the proportional error terms, lie within the nonparametric 95% CI identified for the final model.

The Goodness of Fit (GoF) diagnostics of the final model are shown in Figure 19A-D; the results of a Visual Predictive Check are presented in Figure 20.

External Validation of the rFVIIIFc Model

In order to further qualify the predictive performance of the PK model, an external validation procedure was performed. A model development (training) dataset was constructed, containing the intensive sampled profiles from the two studies (a total of 1162 observation records in the training set). In addition, a model validation set included all of the remaining observed data (predominantly peak and trough observations, a total of 888 observation records in the validation set). As the first step of the external validation, a model development procedure, similar to the base model development, was carried out with the training dataset. The parameters estimated from the whole dataset and from the training dataset only were very similar (results not shown).

At the validation step, the validation data (peak and trough data for occasions 3 through 7) were re-introduced. The model was run with parameter estimates set to the population means from the training set model with the NONMEM option MAXEVAL=0. The Goodness of Fit diagnostics for the validation set only (troughs and peaks) are shown in Figure 21. There was a very good agreement between the observed data in the validation dataset and the predicted data by the model developed on the training set.

Base Model for ADVATE®

The base model for ADVATE® (model Adv0D) is a two compartment model with covariates WT on VI and STUDY on V2, BLOCK(3) IIV on CL, V1, and V2, common additive error and separated proportional residual error by study:

$$V_1 = TVV_1 \cdot \left[\frac{WT}{73}\right]^{\Theta_8}$$

$$V_2 = TVV_2 \cdot [\Theta_9]^{STUD}$$

$$\varepsilon_{prop} = STUD \cdot \Theta_6 + (1 - STUD) \cdot \Theta_7 \text{, where } STUD = \begin{cases} 1 & for\,Phase\,1 \\ 0 & for\,Phase\,3 \end{cases} \quad \text{(Equation 4)}$$

The population parameters of model Adv0D and the bootstrap-derived nonparametric 95% CIs are given in Table 27. No IOV was included in the ADVATE® model as only single dose data were available for this agent. No covariate model was built in this case as developing a full ADVATE® model was outside the scope of this modeling exercise.

The GoF diagnostics of model Adv0D are shown in Figure 22.

Table 27: Population parameters and bootstrap-derived nonparametric 95% CI's for the base ADVATE® model

| Parameter | Symbol | Population Estimate | Nonparametric 95% CI[a] |
|---|---|---|---|
| Clearance, CL, [dL/h] | $\Theta_1$ | 2.53 | 2.32 - 2.78 |
| Central Volume, V1, [dL] | $\Theta_2$ | 34.6 | 32.5 - 36.2 |
| Allometric exponent on V1 | $\Theta_8$ | 0.508 | 0.277 - 0.762 |
| Intercompartmental Clearance, Q, [dL/h] | $\Theta_3$ | 0.548 | 0.401 - 0.929 |
| Peripheral Volume for Phase 3, V2, | $\Theta_4$ | 4.94 | 3.82 - 6.51 |
| V2 Correcting Coefficient for Phase 1/2a | $\Theta_9$ | 2.17 | 1.64 - 3.00 |
| IIV on CL, [%] | $\eta_1$ | 30.4 | 24.4 - 35.8 |
| IIV on V1, [%] | $\eta_2$ | 16.2 | 11.4 - 19.3 |
| Correlation between IIV on CL and V1 | $\eta_{12}$ | 0.532 | N.C.[b] |

(continued)

| Parameter | Symbol | Population Estimate | Nonparametric 95% CI[a] |
|---|---|---|---|
| IIV on V2, [%] | $\eta_4$ | 30.6 | 0.31 - 42.4 |
| Additive error, [IU/dL] | $\Theta_5$ | 0.110 | 0.00110 - 0.256 |
| Proportional error, Phase 1/2a study, | $\Theta_6$ | 10.9 | 8.42 - 12.6 |
| Proportional error, Phase 3 study [%] | $\Theta_7$ | 16.8 | 10.0 - 22.4 |

[a] Out of 1000 bootstraps, 23 runs with minimization terminated were skipped when calculating the bootstrap results.
[b] Nonparametric 95% CI of 0.00773 - 0.0442 for a population mean of the covariance $\omega_{12}$ of 0.0263

Model Implementation: Simulations of Prospective Dosing Regimens

The population PK models adequately describe the time course of both ADVATE® and rFVIIIFc activity. Therefore, they can be used to simulate various dosing scenarios in the target population with the aim to explore and compare the resulting activity profiles. Such model based simulations have been widely used, especially in recent years, and have become a valuable tool in support of operational, regulatory, and therapeutic decision making.

In hemophilia, it is widely accepted that 1 IU/dL (or 1% activity) is a threshold value under which the risk of bleeding increases (Collins PW, B.V., Fischer K, et al., Break-through bleeding in relation to predicted factor VIII levels in patients receiving prophylactic treatment for severe hemophilia A. J Thromb Haemost., 2009. 7(3): p. 413-20; MASAC, Recommendation #190 Concerning Products Licensed for the Treatment of Hemophilia and Other Bleeding Disorders. March 2009 (Replaced by Recommendation #215, November 2012). New York, NY: National Hemophilia Foundation). It should be noted, however, that the threshold cannot be considered an "absolute criterion" by itself; other factors, such as duration of time with low activity levels, patient characteristics, etc. are likely also involved.

For the simulation of rFVIIIFc activity, the base model (Eq. 1) was used. The base model was preferred to the final model as, at this moment, there is no relationship describing the course of the time-variant covariates, VWF and HCT, which renders the generation of credible values for these covariates for simulations with the final model problematic. On the other hand, the parameter values of the base and final models from Table 25 and Table 26 indicate that the base model is adequate and can be used for simulations.

Several simulations have been performed for FVIII prophylaxis dosing regimens that are of therapeutic interest. These simulations can be used to support labeling considerations, and can serve as an aid to physicians in the adjustment of rFVIIIFc therapy.

Table 29 presents the predicted steady state (SS) peaks and troughs of the rFVIIIFc activity-time profiles (5th, 25th, 50th [median], 75th, and 95th percentiles) at various dose levels administered every 3, 4, or 5 days (E3D, E4D, and E5D administration, respectively). The simulated Steady State activity profiles of selected rFVIIIFc prophylaxis dosing regimens are given in Figure 24A-D for illustrative purposes. These results can be considered when determining long-term prophylaxis dosing regimens. It should be noted also that a dosing regimen of 65 IU/kg administered weekly (QW) is predicted to yield troughs above 1 IU/dL in 26.6 % of the individuals treated (also shown in Figure 24A-D), i.e. such an infrequent dosing regimen may be appropriate for approximately a quarter of the population.

**Table 28: Comparison of ADVATE® population PK parameters from the current analysis and the analysis published in Björkman 2012 A**

| Parameter | This Analysis Estimates | Estimates from [Björkman 2012a] |
|---|---|---|
| Clearance, CL, [dL/h] | 2.53 | 2.30* |
| Allometric exponent on CL | Not estimated | 0.8 |
| Central Volume, V1, [dL] | 34.6 | 28.6* |
| Allometric exponent on V1 | 0.508 | 0.95 |
| Intercompartmental Clearance, Q, [dL/h] | 0.548 | 1.47 |
| Peripheral Volume, V2, [dL] | 4.94 | 8.92* |
| Allometric exponent on V2 | Not estimated | 0.76 |

(continued)

| Parameter | This Analysis Estimates | Estimates from [Björkman 2012a] |
|---|---|---|
| V2 Correcting Coefficient for Phase 1/2a | 2.17 | Not estimated |
| IIV on CL, [%] | 30.4 | 30.0 |
| IIV on V1, [%] | 16.2 | 21.0 |
| Correlation between IIV on CL and V1 | 0.532 | 0.45 |
| IIV on V2, [%] | 30.6 | Not estimated |
| Additive error, [IU/dL] | 0.110 | 8.90 |
| Proportional error, Phase 1/2a study, [%] | 10.9 | Not estimated |
| Proportional error, Phase 3 study [%] | 16.8 | Not estimated |
| * CL, V1 and V2 calculated for an average 73 kg, 30 yr old subject from equations 4-7 (Björkman 2012 A) | | |

The dosing regimens simulated include dose levels (20-65 IU/kg) and dosing intervals that have been tested in the Phase 1/2a and Phase 3 studies. Therefore, the simulations can be considered to interpolate within the existing clinical experience, increasing the credibility of the predictions.

Other Implementations

Although the rFVIIIFc and ADVATE® population PK models were developed separately, a comparative evaluation of the individual Bayesian PK parameter estimates of the subjects (from the Phase 1 study and the Arm 1 PK Subgroup of the Phase 3 study) who received both ADVATE® and rFVIIIFc indicates that they are highly correlated. Figure 25A-C presents the individual Bayesian CL, V1, and Time to 1 IU/dL (Time 1%) estimates for ADVATE® and rFVIIIFc, where each point represents one individual. The correlation coefficients are uniformly high: R=0.839 (R2=0.7043) for CL, R=0.862 (R2=0.7437) for V1, and R=0.865 (R2=0.7481) for Time 1%.

Discussion

The parameter values of both the base and final models for rFVIIIFc and their tight confidence intervals indicate that the population PK parameters are estimated with good precision. The PK behavior of the activity profiles as assessed by population analysis is typical for a FVIII product (Björkman *et al.* 2012a) with a peripheral compartment much smaller than the central volume (V2 is more than 5 times lower than VI). The central volume of distribution approximates the plasma volume.

The IIV of the activity is low to moderate. Typically, as in other FVIII products (Björkman *et al.* 2012a) the clearance has higher IIV while the IIV of the volume, characterized by a CV of 13.4 %, is very low.

Similarly, the IOV is low, which is in line with observations from other FVIII products (Björkman *et al.* 2012a). Again, as with the IIV, the IOV of the clearance is higher than the IOV of the volume of distribution.

The GoF plots indicate that the model fits the experimental data very well, including at the lower end of the activities (which is the range of interest with respect to therapeutic effects). There are no systematic deviations or shapes detectable in the residual plots and no notable deviations from the symmetry distribution assumption for most of the ETA terms.

The residual error values for both the base and the final models are very low. The additive error is less than the LLOQ. The proportional error is of the same magnitude as the precision of the aPTT assay (CV < 10%). This shows that the final model has almost completely extracted the information contained in the data; the unexplained variance is almost exclusively due to the inherent activity assay variability.

The ETA shrinkage of the IIV terms is moderate while the ETA shrinkage on the IOV terms is higher (above 25%) and is especially high for those occasions containing sparse data.

Unlike other FVIII products (Björkman *et al.* 2012a), the results from both the base and the final models indicate that the activity of rFVIIIFc does not display strong dependence on weight. Clearance does not accept a WT covariate, while the retained WT covariate on the central volume of distribution has a low value exponent (0.382 with 95% CI of 0.271 - 0.499). This opens the opportunity of exploring flat (weight-independent) dosing regimens for rFVIIIFc if such are of benefit to the patients.

AGE was included in the full covariate model as a very weak covariate, which was cancelled during the backward elimination of covariates. The dataset included individuals from the Phase 3 study, who were all greater than 12 years of age. Therefore, this indicates that the activity of rFVIIIFc is not impacted by age for individuals greater than 12 years.

By far the strongest covariate for rFVIII identified in this analysis was VWF on CL. As a result of the inclusion of the VWF covariate on clearance, the CV of the IIV on this parameter decreased by approximately 20%. These results are expected, as the vast majority of circulating FVIII is in complex with VWF, and is protected from proteolytic degradation, premature binding to its ligands, and rapid clearance from the blood (Lillicrap D., Extending half-life in coagulation factors: where do we stand? Thromb Res., 2008. 122 (Suppl 4): p. 2-8). The negative exponent on VWF indicates that the higher the measured level of VWF, the lower the rFVIII clearance, which is in line with the above mechanistic hypothesis.

The (across and within) individual variation of VWF in our study was in the range of 10 - 380%. This would result in a variation of the rFVIIIFc clearance from 3.85 to 1.10 dL/h, the population mean being 1.65 dL/h. The identification of VWF as a significant covariate on clearance implies that during prophylaxis, individuals with VWF levels close to or above the median may require less frequent administration of rFVIIIFc than subjects with low VWF levels. It should be noted that VWF is a time-varying parameter within the same individual. The exact mechanisms and relationships governing this within-individual variation are not well elucidated or quantitatively characterized. A quantitative framework for the intra-individual VWF level variations needs to be combined with the current population PK model to permit further investigation of the possible dosing implications of VWF covariate.

Another time-varying covariate that was identified was HCT on the central volume of distribution; however, the influence of HCT was rather weak. Such a relationship has not been reported for FVIII products before and a mechanistic hypothesis linking HCT with FVIII activity has not been proposed.

Based on these results, it was concluded that inclusion of the BLQ values in the modeling process does not provide additional value, but could potentially destabilize the runs. For this reason, it was decided to proceed with model development without including the BLQ activity records (Table 25). Based on these results, the conclusion that inclusion of the BLQ values in the modeling process does not bring additional value was confirmed. (Table 26)

The results from the external validation exercise underline the strong predictive potential of the current population PK model and indicate that the peak and troughs in the long term can be predicted by a model developed on (an) intensive sampling schedule. This represents one possible treatment scenario, when the physician adjusts the dosing regimen (initially or during the course of therapy) based on PK information derived from more or less intensive sampling scheme. Due to the low IOV, this adjustment is likely to be valid for extended periods of time in the absence of abrupt changes in the system state (such as illness, trauma, etc.).

The parameter values for the ADVATE® model and their tight confidence intervals indicate that the population PK parameters are estimated with very good precision. The central volume of distribution approximates the plasma volume. The IIV of the activity is low to moderate. The residual errors of the ADVATE® model are very low. The additive error is less than a quarter of the LLOQ. The proportional errors for both studies are of the same magnitude as the precision of the aPTT assay (CV < 10%). This shows that the ADVATE® model has almost completely captured the information contained in the data; the unexplained variance is almost exclusively due to the measurement errors. The ETA shrinkage of the IIV terms is low for the clearance and for the central volume, but is high for the peripheral volume. This indicates that the data do not contain a lot of information for this parameter.

The model diagnostics indicate that the model describes the data adequately. There are no systematic deviations or shapes detectable in the residual plots. The lower end of the activities (which is the range of interest with respect to therapeutic effects) is approximated very well.

Table 28 presents a comparison of ADVATE® population PK parameters from the current analysis and the analysis published by Björkman *et al.* a. The results presented indicate that the population estimates derived from the ADVATE® analysis are similar to the ones published in the literature (Björkman *et al.* 2012a) although there are differences in the analysis methodology. Bjorkman's model identified a weight dependence of CL and V2, which was not substantiated by our data. Age was not identified by us as a covariate of clearance as well. Finally, the different sampling schemes and population characteristics of the datasets are likely to have some impact on the parameter values as well. It is worth mentioning that the data used by Björkman *et al.* a include PK profiles from subjects as young as 3 years of age, hence the age and weight interval in this dataset may be considerably wider. The latter would facilitate the identification of weight / age covariate from such data.

A comparison between the population mean profiles (for a standard 73 kg, 22 year old individual) derived from our base ADVATE® model (50 IU/kg in the Phase 3 study) and a reconstructed version of Bjorkman's model (simulated for 50 IU/kg dose) are presented in Figure 23 along with the observed FVIII activity data following administration of 50 IU/kg of ADVATE® from the Phase 3 study. It can be seen that the two models perform similarly and are in good agreement with the data.

It is worth noting that Bjorkman et al. a reported a very high additive residual error of 8.9 IU/dL. Given that the region of therapeutic interest for FVIII is around 1 IU/dL that could be a serious problem when predicting low activities.

An analysis of the predictive properties of this model in the low band of activities is not included in the manuscript. In contrast, the additive error of our ADVATE® model is more than 80 times lower and less than a quarter of the LLOQ of the aPTT assay used, which makes it more suited for prediction purposes.

The high positive correlations between the PK parameters of ADVATE® and rFVIIIFc, illustrated in Figure 25A-C, mean that individuals who had high CL values for ADVATE® are very likely to have had high CL values for rFVIIIFc as well, and vice versa. The population models predicted that the average population CL for rFVIIIFc (1.65 dL/h) is about 35% lower than that of ADVATE® (2.53 dL/h). This is consistent with the geometric mean [95% CI] for the intra-subject clearance ratio of rFVIII to ADVATE® (0.64 [0.60, 0.69]) derived from the conventional two-stage compartmental analysis (Mahlangu J *et al,* submitted). At the same time, the V1 panel of Figure 25A-C indicates that individuals had similar VI values for ADVATE® and for rFVIIIFc. The above two relationships raise the hypothesis that individuals who had high time related characteristics (such as half-life, MRT, Time to 1% and Time to 3%) for ADVATE® are very likely to have had high characteristics for rFVIIIFc, and vice versa. This hypothesis is confirmed in the Time 1% panel of Figure 25A-C, where the individual Times to 1%, calculated based on the Bayesian estimates from the ADVATE® and rFVIIIFc model, are plotted.

Time to 1% activity is a PK outcome parameter that is directly related to frequency of dosing under the paradigm of maintaining target activity above this threshold. Therefore, based on the relationship identified, a conclusion can be made, that individuals who were on a less frequent ADVATE® dosing would require less frequent rFVIIIFc dosing, and vice versa. Such a relationship could be useful when transition between the two products is considered.

Conclusions

The population PK analysis and simulations presented above provide a comprehensive quantitative characterization of the activity-time profiles for rFVIIIFc and ADVATE®. The population PK model for rFVIIIFc adequately describes the combined activity data from the Phase 1/2a and Phase 3 studies. The residual errors are small and in the same range as the precision of the activity assay; the precision of the parameter estimates is very good, while the diagnostics and the model qualification results confirm the appropriate behavior of the model.

The kinetics of rFVIIIFc activity displays a two-compartmental behavior. The major covariate for activity identified was von Willebrand Factor level on clearance; Weight and Hematocrit were identified as minor covariates on the central volume of distribution. The covariates identified are physiologically relevant. The IIV of the rFVIIIFc activity was low reflecting the consistent and well behaved activity profiles observed. The IOV of the rFVIIIFc activity was very low indicating that the activity profiles are stable with long term administration.

The two-compartment population PK model for ADVATE® adequately describes the combined activity data from the Phase 1 and Phase 3 studies. Both the structure and the parameter values of the ADVATE® model are in good agreement with published population PK results in the literature.

The results of the population PK analyses for rFVIIIFc and ADVATE® confirm that the clearance of rFVIIIFc is much lower than the clearance of ADVATE®, while the volumes of distribution at steady state are very similar. These results were first observed following noncompartmental analysis and conventional two-stage analysis of the same data and explain the extended duration of activity achieved by rFVIIIFc, compared to ADVATE® (Mahlangu et al, submitted).

The population PK model for rFVIIIFc was used for simulation of various dosing scenarios to aid dosing regimen selection and adjustment. Based on the population PK analyses, it was concluded that individuals on a less frequent ADVATE® dosing regimen would require less frequent rFVIIIFc dosing, and vice versa. Such a relationship could be useful when transitioning from one agent to the other.

The simulations were performed using models that did not account for the uncertainty in the model parameter estimates and in the modeling process. Therefore, the resulting predictions are expressed as single predicted values and deterministic single profiles. Possible extensions of the current work would be to include the parameter uncertainty in the simulations as well as to simulate alternative dosing regimens for treatment of bleeding and for surgery, where the control of the activity needs to be more stringent.

The population PK models are useful tools for predicting peak, trough and average activity of various dosing scenarios. Although such tools have been used by others, there is scope for further improvement. Population PK modeling can be further extended to develop aids and methods for individualizing treatment. Alternative dosing paradigms, such as flat (not per weight) dosing also can be explored.

Many efforts have been made to correlate activity levels with bleeding risk, although a definitive quantitative relationship to calculate bleeding probability based on activity has not yet been identified. These population PK models can serve as a basis for the development of a population PK/PD model to better understand the relationship between FVIII activity and bleeding.

Table 29: Predicted steady state (SS) peaks and troughs of the rFVIIIFc activity-time profiles with various dose levels administered every 3, 4, or 5 days

| Unit Dose Level | Percentile of Subjects | Dosing Frequency | | | | | |
|---|---|---|---|---|---|---|---|
| | | Every 3 Days | | Every 4 Days | | Every 5 Days | |
| | | Peak | Trough | Peak | Trough | Peak | Trough |
| 25 IU/kg | 5% | 35.2 | <0.5[a] | 34.9 | <0.5[a] | 34.6 | <0.5[a] |
| | 25% | 43.7 | 1.28 | 43.0 | 0.540 | 42.3 | <0.5[a] |
| | 50% | 51.2 | 2.64 | 50.0 | 1.16 | 49.2 | 0.549 |
| | 75% | 59.7 | 4.88 | 58.0 | 2.32 | 56.6 | 1.17 |
| | 95% | 74.5 | 10.2 | 70.7 | 5.69 | 69.6 | 3.08 |
| 40 IU/kg | 5% | 56.3 | 0.619 | 55.9 | <0.5[a] | 55.3 | <0.5[a] |
| | 25% | 70.0 | 2.04 | 68.7 | 0.864 | 67.6 | <0.5[a] |
| | 50% | 82.0 | 4.22 | 80.0 | 1.85 | 78.8 | 0.878 |
| | 75% | 95.6 | 7.81 | 92.8 | 3.70 | 90.5 | 1.88 |
| | 95% | 119 | 16.3 | 113 | 9.11 | 111 | 4.94 |
| 50 IU/kg | 5% | 70.4 | 0.774 | 69.9 | <0.5[a] | 69.1 | <0.5[a] |
| | 25% | 87.5 | 2.55 | 85.9 | 1.08 | 84.5 | 0.516 |
| | 50% | 102 | 5.27 | 100 | 2.32 | 98.5 | 1.10 |
| | 75% | 119 | 9.76 | 116 | 4.63 | 113 | 2.34 |
| | 95% | 149 | 20.4 | 141 | 11.4 | 139 | 6.17 |

Embodiments

E1. A method of estimating a rFVIIIFc dosing information individualized for a patient, the method comprising:

(a) receiving, by a computer-based system containing the rFVIIIFc population pharmacokinetic (popPK) model of Example 4 and a Bayesian estimation program, at least one of patient information and desired treatment outcome information,
(b) calculating, by the computer-based system, individualized rFVIIIFc dosing information using the popPK model, the Bayesian estimation program, and the received information, and
(c) outputting, by the computer-based system, the individualized dosing information.

E2. The method of embodiment E1, further comprising selecting a dosing regimen based on the output individualized dosing information of (c) and administering rFVIIIFc to the patient according to the selected dosing regimen.
E3. A computer readable storage medium having instructions stored thereon that, when executed by a processor, cause the processor to perform the method of embodiment E1.
E4. A system comprising a processor and a memory, the memory having instructions stored thereon that, when executed by the processor, cause the processor to perform the method of embodiment E1.
E5. A method of estimating a rFVIIIFc dosing regimen based on median popPK, the method comprising:

(a) receiving, by a computer-based system containing the rFVIIIFc popPK model of Example 4 and a Bayesian estimation program, at least one of patient information and desired treatment outcome information,
(b) calculating, by the computer-based system, median rFVIIIFc PK information using the popPK model, the Bayesian estimation program, and the received information, and
(c) outputting, by the computer-based system, the median PK information.

E6. The method of embodiment E5, further comprising selecting a dosing regimen based on the output median PK information of (c), and administering rFVIIIFc to a patient according to the selected dosing regimen.
E7. A computer readable storage medium having instructions stored thereon that, when executed by a processor, cause the processor to perform the method of embodiment E5.
E8. A system comprising a processor and a memory, the memory having instructions stored thereon that, when executed by the processor, cause the processor to perform the method of embodiment E5.
E9. A method of estimating individual patient rFVIIIFc PK, the method comprising:

(a) receiving, by a computer-based system containing the rFVIIIFc population pharmacokinetic (popPK) model of Example 4 and a Bayesian estimation program, individual rFVIIIFc PK information;
(b) estimating, by the computer-based system, individualized patient rFVIIIFc PK information using the popPK model, the Bayesian estimation program, and the received information, and
(c) outputting, by the computer-based system, the individualized patient PK information.

E10. The method of embodiment E9, further comprising selecting a dosing regimen based on the output individualized patient PK information of (c), and administering rFVIIIFc to the patient according to the selected regimen.
E11. A computer readable storage medium having instructions stored thereon that, when executed by a processor, cause the processor to perform the method of embodiment E10.
E12. A system comprising a processor and a memory, the memory having instructions stored thereon that, when executed by the processor, cause the processor to perform the method of embodiment E10.
E13. The method of embodiment E1, wherein the desired treatment outcome information is desired rise in plasma FVIII activity level following dosing and the output information is dose for acute treatment.
E14. The method of embodiment E1, wherein the desired treatment outcome information is desired dosing interval and the output information is dose for prophylaxis.
E15. The method embodiment E1, wherein the desired treatment outcome information is desired dose and the output information is interval for prophylaxis.
E16. The method of embodiment E10, wherein (a) further comprises receiving, by the computer-based system, additional patient information.
E17. The method of any of embodiments E1, E5, or E10, wherein the patient information is age, Von Willebrand Factor (VWF) level, body weight (BW), or hematocrit (HCT).

**TABLE 15: Polynucleotide Sequences**

**A. B-Domain Deleted FVIIIFc**

(i) B-Domain Deleted FVIIIFc Chain DNA Sequence (FVIII signal peptide underlined. Fc region in bold) (SEQ ID NO:1. which encodes SEQ ID NO:2)

(continued)

**A. B-Domain Deleted FVIIIFc**

```
 661                                                        A TGCAAATAGA GCTCTCCACC TGCTTCTTTC
 721  TGTGCCTTTT GCGATTCTGC TTTAGTGCCA CCAGAAGATA CTACCTGGGT GCAGTGGAAC
 781  TGTCATGGGA CTATATGCAA AGTGATCTCG GTGAGCTGCC TGTGGACGCA AGATTTCCTC
 841  CTAGAGTGCC AAAATCTTTT CCATTCAACA CCTCAGTCGT GTACAAAAAG ACTCTGTTTG
 901  TAGAATTCAC GGATCACCTT TTCAACATCG CTAAGCCAAG GCCACCCTGG ATGGGTCTGC
 961  TAGGTCCTAC CATCCAGGCT GAGGTTTATG ATACAGTGGT CATTACACTT AAGAACATGG
1021  CTTCCCATCC TGTCAGTCTT CATGCTGTTG GTGTATCCTA CTGGAAAGCT TCTGAGGGAG
1081  CTGAATATGA TGATCAGACC AGTCAAAGGG AGAAAGAAGA TGATAAAGTC TTCCCTGGTG
1141  GAAGCCATAC ATATGTCTGG CAGGTCCTGA AAGAGAATGG TCCAATGGCC TCTGACCCAC
1201  TGTGCCTTAC CTACTCATAT CTTTCTCATG TGGACCTGGT AAAAGACTTG AATTCAGGCC
1261  TCATTGGAGC CCTACTAGTA TGTAGAGAAG GGAGTCTGGC CAAGGAAAAG ACACAGACCT
1321  TGCACAAATT TATACTACTT TTTGCTGTAT TTGATGAAGG GAAAAGTTGG CACTCAGAAA
1381  CAAAGAACTC CTTGATGCAG GATAGGGATG CTGCATCTGC TCGGGCCTGG CCTAAAATGC
1441  ACACAGTCAA TGGTTATGTA AACAGGTCTC TGCCAGGTCT GATTGGATGC CACAGGAAAT
1501  CAGTCTATTG GCATGTGATT GGAATGGGCA CCACTCCTGA AGTGCACTCA ATATTCCTCG
1561  AAGGTCACAC ATTTCTTGTG AGGAACCATC GCCAGGCGTC CTTGGAAATC TCGCCAATAA
1621  CTTTCCTTAC TGCTCAAACA CTCTTGATGG ACCTTGGACA GTTTCTACTG TTTTGTCATA
1681  TCTCTTCCCA CCAACATGAT GGCATGGAAG CTTATGTCAA AGTAGACAGC TGTCCAGAGG
1741  AACCCCAACT ACGAATGAAA AATAATGAAG AAGCGGAAGA CTATGATGAT GATCTTACTG
1801  ATTCTGAAAT GGATGTGGTC AGGTTTGATG ATGACAACTC TCCTTCCTTT ATCCAAATTC
1861  GCTCAGTTGC CAAGAAGCAT CCTAAAACTT GGGTACATTA CATTGCTGCT GAAGAGGAGG
1921  ACTGGGACTA TGCTCCCTTA GTCCTCGCCC CCGATGACAG AAGTTATAAA AGTCAATATT
1981  TGAACAATGG CCCTCAGCGG ATTGGTAGGA AGTACAAAAA AGTCCGATTT ATGGCATACA
2041  CAGATGAAAC CTTTAAGACT CGTGAAGCTA TTCAGCATGA ATCAGGAATC TTGGGACCTT
2101  TACTTTATGG GGAAGTTGGA GACACACTGT TGATTATATT TAAGAATCAA GCAAGCAGAC
2161  CATATAACAT CTACCCTCAC GGAATCACTG ATGTCCGTCC TTTGTATTCA AGGAGATTAC
2221  CAAAAGGTGT AAAACATTTG AAGGATTTTC CAATTCTGCC AGGAGAAATA TTCAAATATA
2281  AATGGACAGT GACTGTAGAA GATGGGCCAA CTAAATCAGA TCCTCGGTGC CTGACCCGCT
2341  ATTACTCTAG TTTCGTTAAT ATGGAGAGAG ATCTAGCTTC AGGACTCATT GGCCCTCTCC
2401  TCATCTGCTA CAAAGAATCT GTAGATCAAA GAGGAAACCA GATAATGTCA GACAAGAGGA
2461  ATGTCATCCT GTTTTCTGTA TTTGATGAGA ACCGAAGCTG GTACCTCACA GAGAATATAC
2521  AACGCTTTCT CCCCAATCCA GCTGGAGTGC AGCTTGAGGA TCCAGAGTTC CAAGCCTCCA
2581  ACATCATGCA CAGCATCAAT GGCTATGTTT TTGATAGTTT GCAGTTGTCA GTTTGTTTGC
2641  ATGAGGTGGC ATACTGGTAC ATTCTAAGCA TTGGAGCACA GACTGACTTC CTTTCTGTCT
2701  TCTTCTCTGG ATATACCTTC AAACACAAAA TGGTCTATGA AGACACACTC ACCCTATTCC
2761  CATTCTCAGG AGAAACTGTC TTCATGTCGA TGGAAAACCC AGGTCTATGG ATTCTGGGGT
2821  GCCACAACTC AGACTTTCGG AACAGAGGCA TGACCGCCTT ACTGAAGGTT TCTAGTTGTG
2881  ACAAGAACAC TGGTGATTAT TACGAGGACA GTTATGAAGA TATTTCAGCA TACTTGCTGA
2941  GTAAAAACAA TGCCATTGAA CCAAGAAGCT TCTCTCAAAA CCCACCAGTC TTGAAACGCC
3001  ATCAACGGGA AATAACTCGT ACTACTCTTC AGTCAGATCA AGAGGAAATT GACTATGATG
3061  ATACCATATC AGTTGAAATG AAGAAGGAAG ATTTTGACAT TTATGATGAG GATGAAAATC
3121  AGAGCCCCCG CAGCTTTCAA AAGAAAACAC GACACTATTT TATTGCTGCA GTGGAGAGGC
3181  TCTGGGATTA TGGGATGAGT AGCTCCCCAC ATGTTCTAAG AAACAGGGCT CAGAGTGGCA
3241  GTGTCCCTCA GTTCAAGAAA GTTGTTTTCC AGGAATTTAC TGATGGCTCC TTTACTCAGC
3301  CCTTATACCG TGGAGAACTA AATGAACATT TGGGACTCCT GGGGCCATAT ATAAGAGCAG
3361  AAGTTGAAGA TAATATCATG GTAACTTTCA GAAATCAGGC CTCTCGTCCC TATTCCTTCT
3421  ATTCTAGCCT TATTTCTTAT GAGGAAGATC AGAGGCAAGG AGCAGAACCT AGAAAAAACT
3481  TTGTCAAGCC TAATGAAACC AAAACTTACT TTTGGAAAGT GCAACATCAT ATGGCACCCA
3541  CTAAAGATGA GTTTGACTGC AAAGCCTGGG CTTATTTCTC TGATGTTGAC CTGGAAAAAG
3601  ATGTGCACTC AGGCCTGATT GGACCCCTTC TGGTCTGCCA CACTAACACA CTGAACCCTG
```

(continued)

**A. B-Domain Deleted FVIIIFc**

3661 CTCATGGGAG ACAAGTGACA GTACAGGAAT TTGCTCTGTT TTTCACCATC TTTGATGAGA
3721 CCAAAAGCTG GTACTTCACT GAAAATATGG AAAGAAACTG CAGGGCTCCC TGCAATATCC
3781 AGATGGAAGA TCCCACTTTT AAAGAGAATT ATCGCTTCCA TGCAATCAAT GGCTACATAA
3841 TGGATACACT ACCTGGCTTA GTAATGGCTC AGGATCAAAG GATTCGATGG TATCTGCTCA
3901 GCATGGGCAG CAATGAAAAC ATCCATTCTA TTCATTTCAG TGGACATGTG TTCACTGTAC
3961 GAAAAAAAGA GGAGTATAAA ATGGCACTGT ACAATCTCTA TCCAGGTGTT TTTGAGACAG
4021 TGGAAATGTT ACCATCCAAA GCTGGAATTT GGCGGGTGGA ATGCCTTATT GGCGAGCATC
4081 TACATGCTGG GATGAGCACA CTTTTTCTGG TGTACAGCAA TAAGTGTCAG ACTCCCCTGG
4141 GAATGGCTTC TGGACACATT AGAGATTTTC AGATTACAGC TTCAGGACAA TATGGACAGT
4201 GGGCCCCAAA GCTGGCCAGA CTTCATTATT CCGGATCAAT CAATGCCTGG AGCACCAAGG
4261 AGCCCTTTTC TTGGATCAAG GTGGATCTGT TGGCACCAAT GATTATTCAC GGCATCAAGA
4321 CCCAGGGTGC CCGTCAGAAG TTCTCCAGCC TCTACATCTC TCAGTTTATC ATCATGTATA
4381 GTCTTGATGG GAAGAAGTGG CAGACTTATC GAGGAAATTC CACTGGAACC TTAATGGTCT
4441 TCTTTGGCAA TGTGGATTCA TCTGGGATAA AACACAATAT TTTTAACCCT CCAATTATTG
4501 CTCGATACAT CCGTTTGCAC CCAACTCATT ATAGCATTCG CAGCACTCTT CGCATGGAGT
4561 TGATGGGCTG TGATTTAAAT AGTTGCAGCA TGCCATTGGG AATGGAGAGT AAAGCAATAT
4621 CAGATGCACA GATTACTGCT TCATCCTACT TTACCAATAT GTTTGCCACC TGGTCTCCTT
4681 CAAAAGCTCG ACTTCACCTC CAAGGGAGGA GTAATGCCTG GAGACCTCAG GTGAATAATC
4741 CAAAAGAGTG GCTGCAAGTG GACTTCCAGA AGACAATGAA AGTCACAGGA GTAACTACTC
4801 AGGGAGTAAA ATCTCTGCTT ACCAGCATGT ATGTGAAGGA GTTCCTCATC TCCAGCAGTC
4861 AAGATGGCCA TCAGTGGACT CTCTTTTTTC AGAATGGCAA AGTAAAGGTT TTTCAGGGAA
4921 ATCAAGACTC CTTCACACCT GTGGTGAACT CTCTAGACCC ACCGTTACTG ACTCGCTACC
4981 TTCGAATTCA CCCCCAGAGT TGGGTGCACC AGATTGCCCT GAGGATGGAG GTTCTGGGCT
5041 GCGAGGCACA GGACCTCTAC **GACAAAACTC ACACATGCCC ACCGTGCCCA GCTCCAGAAC**
**5101 TCCTGGGCGG ACCGTCAGTC TTCCTCTTCC CCCCAAAACC CAAGGACACC CTCATGATCT**
**5161 CCCGGACCCC TGAGGTCACA TGCGTGGTGG TGGACGTGAG CCACGAAGAC CCTGAGGTCA**
**5221 AGTTCAACTG GTACGTGGAC GGCGTGGAGG TGCATAATGC CAAGACAAAG CCGCGGGAGG**
**5281 AGCAGTACAA CAGCACGTAC CGTGTGGTCA GCGTCCTCAC CGTCCTGCAC CAGGACTGGC**
**5341 TGAATGGCAA GGAGTACAAG TGCAAGGTCT CCAACAAAGC CCTCCCAGCC CCCATCGAGA**
**5401 AAACCATCTC CAAAGCCAAA GGGCAGCCCC GAGAACCACA GGTGTACACC CTGCCCCCAT**
**5461 CCCGGGATGA GCTGACCAAG AACCAGGTCA GCCTGACCTG CCTGGTCAAA GGCTTCTATC**
**5521 CCAGCGACAT CGCCGTGGAG TGGGAGAGCA ATGGGCAGCC GGAGAACAAC TACAAGACCA**
**5581 CGCCTCCCGT GTTGGACTCC GACGGCTCCT TCTTCCTCTA CAGCAAGCTC ACCGTGGACA**
**5641 AGAGCAGGTG GCAGCAGGGG AACGTCTTCT CATGCTCCGT GATGCATGAG GCTCTGCACA**
**5701 ACCACTACAC GCAGAAGAGC CTCTCCCTGT CTCCGGGTAA A**

(ii) Fc DNA sequence (mouse Igκ signal peptide underlined) (SEQ ID NO:3. which encodes SEQ ID NO:4)

7981 ATGGA GACAGACACA
8041 CTCCTGCTAT GGGTACTGCT GCTCTGGGTT CCAGGTTCCA CTGGTGACAA AACTCACACA
8101 TGCCCACCGT GCCCAGCACC TGAACTCCTG GGAGGACCGT CAGTCTTCCT CTTCCCCCCA
8161 AAACCCAAGG ACACCCTCAT GATCTCCCGG ACCCCTGAGG TCACATGCGT GGTGGTGGAC
8221 GTGAGCCACG AAGACCCTGA GGTCAAGTTC AACTGGTACG TGGACGGCGT GGAGGTGCAT
8281 AATGCCAAGA CAAAGCCGCG GGAGGAGCAG TACAACAGCA CGTACCGTGT GGTCAGCGTC
8341 CTCACCGTCC TGCACCAGGA CTGGCTGAAT GGCAAGGAGT ACAAGTGCAA GGTCTCCAAC
8401 AAAGCCCTCC CAGCCCCCAT CGAGAAAACC ATCTCCAAAG CCAAAGGGCA GCCCCGAGAA
8461 CCACAGGTGT ACACCCTGCC CCCATCCCGC GATGAGCTGA CCAAGAACCA GGTCAGCCTG
8521 ACCTGCCTGG TCAAAGGCTT CTATCCCAGC GACATCGCCG TGGAGTGGGA GAGCAATGGG
8581 CAGCCGGAGA ACAACTACAA GACCACGCCT CCCGTGTTGG ACTCCGACGG CTCCTTCTTC
8641 CTCTACAGCA AGCTCACCGT GGACAAGAGC AGGTGGCAGC AGGGGAACGT CTTCTCATGC
8701 TCCGTGATGC ATGAGGCTCT GCACAACCAC TACACGCAGA AGAGCCTCTC CCTGTCTCCG
8761 GGTAAA

**B. Full Length FVIIIFc**

(i) Full Length FVIIIFc DNA Sequence (FVIII signal peptide underlined, Fc region in bold) (SEQ ID NO:5, which encodes SEQ ID NO:6)

(continued)

**B. Full Length FVIIIFc**

661 ATG CAAATAGAGC TCTCCACCTG
721 CTTCTTTCTG TGCCTTTTGC GATTCTGCTT TAGTGCCACC AGAAGATACT ACCTGGGTGC
781 AGTGGAACTG TCATGGGACT ATATGCAAAG TGATCTCGGT GAGCTGCCTG TGGACGCAAG
841 ATTTCCTCCT AGAGTGCCAA AATCTTTTCC ATTCAACACC TCAGTCGTGT ACAAAAAGAC
901 TCTGTTTGTA GAATTCACGG ATCACCTTTT CAACATCGCT AAGCCAAGGC CACCCTGGAT
961 GGGTCTGCTA GGTCCTACCA TCCAGGCTGA GGTTTATGAT ACAGTGGTCA TTACACTTAA
1021 GAACATGGCT TCCCATCCTG TCAGTCTTCA TGCTGTTGGT GTATCCTACT GGAAAGCTTC
1081 TGAGGGAGCT GAATATGATG ATCAGACCAG TCAAAGGGAG AAAGAAGATG ATAAAGTCTT
1141 CCCTGGTGGA AGCCATACAT ATGTCTGGCA GGTCCTGAAA GAGAATGGTC CAATGGCCTC
1201 TGACCCACTG TGCCTTACCT ACTCATATCT TTCTCATGTG GACCTGGTAA AAGACTTGAA
1261 TTCAGGCCTC ATTGGAGCCC TACTAGTATG TAGAGAAGGG AGTCTGGCCA AGGAAAAGAC
1321 ACAGACCTTG CACAAATTTA TACTACTTTT TGCTGTATTT GATGAAGGGA AAAGTTGGCA
1381 CTCAGAAACA AAGAACTCCT TGATGCAGGA TAGGGATGCT GCATCTGCTC GGGCCTGGCC
1441 TAAAATGCAC ACAGTCAATG GTTATGTAAA CAGGTCTCTG CCAGGTCTGA TTGGATGCCA
1501 CAGGAAATCA GTCTATTGGC ATGTGATTGG AATGGGCACC ACTCCTGAAG TGCACTCAAT
1561 ATTCCTCGAA GGTCACACAT TTCTTGTGAG GAACCATCGC CAGGCGTCCT TGGAAATCTC-
1621 GCCAATAACT TTCCTTACTG CTCAAACACT CTTGATGGAC CTTGGACAGT TTCTACTGTT
1681 TTGTCATATC TCTTCCCACC AACATGATGG CATGGAAGCT TATGTCAAAG TAGACAGCTG
1741 TCCAGAGGAA CCCCAACTAC GAATGAAAAA TAATGAAGAA GCGGAAGACT ATGATGATGA
1801 TCTTACTGAT TCTGAAATGG ATGTGGTCAG GTTTGATGAT GACAACTCTC CTTCCTTTAT
1861 CCAAATTCGC TCAGTTGCCA AGAAGCATCC TAAAACTTGG GTACATTACA TTGCTGCTGA
1921 AGAGGAGGAC TGGGACTATG CTCCCTTAGT CCTCGCCCCC GATGACAGAA GTTATAAAAG
1981 TCAATATTTG AACAATGGCC CTCAGCGGAT TGGTAGGAAG TACAAAAAAG TCCGATTTAT
2041 GGCATACACA GATGAAACCT TTAAGACTCG TGAAGCTATT CAGCATGAAT CAGGAATCTT
2101 GGGACCTTTA CTTTATGGGG AAGTTGGAGA CACACTGTTG ATTATATTTA AGAATCAAGC
2161 AAGCAGACCA TATAACATCT ACCCTCACGG AATCACTGAT GTCCGTCCTT TGTATTCAAG
2221 GAGATTACCA AAAGGTGTAA AACATTTGAA GGATTTTCCA ATTCTGCCAG GAGAAATATT
2281 CAAATATAAA TGGACAGTGA CTGTAGAAGA TGGGCCAACT AAATCAGATC CTCGGTGCCT
2341 GACCCGCTAT TACTCTAGTT TCGTTAATAT GGAGAGAGAT CTAGCTTCAG GACTCATTGG
2401 CCCTCTCCTC ATCTGCTACA AAGAATCTGT AGATCAAAGA GGAAACCAGA TAATGTCAGA
2461 CAAGAGGAAT GTCATCCTGT TTTCTGTATT TGATGAGAAC CGAAGCTGGT ACCTCACAGA
2521 GAATATACAA CGCTTTCTCC CCAATCCAGC TGGAGTGCAG CTTGAGGATC CAGAGTTCCA
2581 AGCCTCCAAC ATCATGCACA GCATCAATGG CTATGTTTTT GATAGTTTGC AGTTGTCAGT
2641 TTGTTTGCAT GAGGTGGCAT ACTGGTACAT TCTAAGCATT GGAGCACAGA CTGACTTCCT
2701 TTCTGTCTTC TTCTCTGGAT ATACCTTCAA ACACAAAATG GTCTATGAAG ACACACTCAC
2761 CCTATTCCCA TTCTCAGGAG AAACTGTCTT CATGTCGATG GAAAACCCAG GTCTATGGAT
2821 TCTGGGGTGC CACAACTCAG ACTTTCGGAA CAGAGGCATG ACCGCCTTAC TGAAGGTTTC
2881 TAGTTGTGAC AAGAACACTG GTGATTATTA CGAGGACAGT TATGAAGATA TTTCAGCATA
2941 CTTGCTGAGT AAAAACAATG CCATTGAACC AAGAAGCTTC TCCCAGAATT CAAGACACCC
3001 TAGCACTAGG CAAAAGCAAT TTAATGCCAC CACAATTCCA GAAAATGACA TAGAGAAGAC
3061 TGACCCTTGG TTTGCACACA GAACACCTAT GCCTAAAATA CAAAATGTCT CCTCTAGTGA
3121 TTTGTTGATG CTCTTGCGAC AGAGTCCTAC TCCACATGGG CTATCCTTAT CTGATCTCCA
3181 AGAAGCCAAA TATGAGACTT TTTCTGATGA TCCATCACCT GGAGCAATAG ACAGTAATAA
3241 CAGCCTGTCT GAAATGACAC ACTTCAGGCC ACAGCTCCAT CACAGTGGGG ACATGGTATT
3301 TACCCCTGAG TCAGGCCTCC AATTAAGATT AAATGAGAAA CTGGGGACAA CTGCAGCAAC
3361 AGAGTTGAAG AAACTTGATT TCAAAGTTTC TAGTACATCA AATAATCTGA TTTCAACAAT
3421 TCCATCAGAC AATTTGGCAG CAGGTACTGA TAATACAAGT TCCTTAGGAC CCCCAAGTAT
3481 GCCAGTTCAT TATGATAGTC AATTAGATAC CACTCTATTT GGCAAAAAGT CATCTCCCCT
3541 TACTGAGTCT GGTGGACCTC TGAGCTTGAG TGAAGAAAAT AATGATTCAA AGTTGTTAGA
3601 ATCAGGTTTA ATGAATAGCC AAGAAAGTTC ATGGGGAAAA AATGTATCGT CAACAGAGAG
3661 TGGTAGGTTA TTTAAAGGGA AAAGAGCTCA TGGACCTGCT TTGTTGACTA AAGATAATGC
3721 CTTATTCAAA GTTAGCATCT CTTTGTTAAA GACAAACAAA ACTTCCAATA ATTCAGCAAC

(continued)

**B. Full Length FVIIIFc**

3781 TAATAGAAAG ACTCACATTG ATGGCCCATC ATTATTAATT GAGAATAGTC CATCAGTCTG
3841 GCAAAATATA TTAGAAAGTG ACACTGAGTT TAAAAAAGTG ACACCTTTGA TTCATGACAG
3901 AATGCTTATG GACAAAAATG CTACAGCTTT GAGGCTAAAT CATATGTCAA ATAAAACTAC
3961 TTCATCAAAA AACATGGAAA TGGTCCAACA GAAAAAAGAG GGCCCCATTC CACCAGATGC
4021 ACAAAATCCA GATATGTCGT TCTTTAAGAT GCTATTCTTG CCAGAATCAG CAAGGTGGAT
4081 ACAAAGGACT CATGGAAAGA ACTCTCTGAA CTCTGGGCAA GGCCCCAGTC CAAAGCAATT
4141 AGTATCCTTA GGACCAGAAA AATCTGTGGA AGGTCAGAAT TTCTTGTCTG AGAAAAACAA
4201 AGTGGTAGTA GGAAAGGGTG AATTTACAAA GGACGTAGGA CTCAAAGAGA TGGTTTTTCC
4261 AAGCAGCAGA AACCTATTTC TTACTAACTT GGATAATTTA CATGAAAATA ATACACACAA
4321 TCAAGAAAAA AAAATTCAGG AAGAAATAGA AAAGAAGGAA ACATTAATCC AAGAGAATGT
4381 AGTTTTGCCT CAGATACATA CAGTGACTGG CACTAAGAAT TTCATGAAGA ACCTTTTCTT
4441 ACTGAGCACT AGGCAAAATG TAGAAGGTTC ATATGACGGG GCATATGCTC CAGTACTTCA
4501 AGATTTTAGG TCATTAAATG ATTCAACAAA TAGAACAAAG AAACACACAG CTCATTTCTC
4561 AAAAAAAGGG GAGGAAGAAA ACTTGGAAGG CTTGGGAAAT CAAACCAAGC AAATTGTAGA
4621 GAAATATGCA TGCACCACAA GGATATCTCC TAATACAAGC CAGCAGAATT TTGTCACGCA
4681 ACGTAGTAAG AGAGCTTTGA AACAATTCAG ACTCCCACTA GAAGAAACAG AACTTGAAAA
4741 AAGGATAATT GTGGATGACA CCTCAACCCA GTGGTCCAAA AACATGAAAC ATTTGACCCC
4801 GAGCACCCTC ACACAGATAG ACTACAATGA GAAGGAGAAA GGGGCCATTA CTCAGTCTCC
4861 CTTATCAGAT TGCCTTACGA GGAGTCATAG CATCCCTCAA GCAAATAGAT CTCCATTACC
4921 CATTGCAAAG GTATCATCAT TTCCATCTAT TAGACCTATA TATCTGACCA GGGTCCTATT
4981 CCAAGACAAC TCTTCTCATC TTCCAGCAGC ATCTTATAGA AAGAAAGATT CTGGGGTCCA
5041 AGAAAGCAGT CATTTCTTAC AAGGAGCCAA AAAAAATAAC CTTTCTTTAG CCATTCTAAC
5101 CTTGGAGATG ACTGGTGATC AAAGAGAGGT TGGCTCCCTG GGGACAAGTG CCACAAATTC
5161 AGTCACATAC AAGAAAGTTG AGAACACTGT TCTCCCGAAA CCAGACTTGC CCAAAACATC
5221 TGGCAAAGTT GAATTGCTTC CAAAAGTTCA CATTTATCAG AAGGACCTAT TCCCTACGGA
5281 AACTAGCAAT GGGTCTCCTG GCCATCTGGA TCTCGTGGAA GGGAGCCTTC TTCAGGGAAC
5341 AGAGGGAGCG ATTAAGTGGA ATGAAGCAAA CAGACCTGGA AAAGTTCCCT TTCTGAGAGT
5401 AGCAACAGAA AGCTCTGCAA AGACTCCCTC CAAGCTATTG GATCCTCTTG CTTGGGATAA
5461 CCACTATGGT ACTCAGATAC CAAAAGAAGA GTGGAAATCC CAAGAGAAGT CACCAGAAAA
5521 AACAGCTTTT AAGAAAAAGG ATACCATTTT GTCCCTGAAC GCTTGTGAAA GCAATCATGC
5581 AATAGCAGCA ATAAATGAGG GACAAAATAA GCCCGAAATA GAAGTCACCT GGGCAAAGCA
5641 AGGTAGGACT GAAAGGCTGT GCTCTCAAAA CCCACCAGTC TTGAAACGCC ATCAACGGGA
5701 AATAACTCGT ACTACTCTTC AGTCAGATCA AGAGGAAATT GACTATGATG ATACCATATC
5761 AGTTGAAATG AAGAAGGAAG ATTTTGACAT TTATGATGAG GATGAAAATC AGAGCCCCCG
5821 CAGCTTTCAA AAGAAAACAC GACACTATTT TATTGCTGCA GTGGAGAGGC TCTGGGATTA
5881 TGGGATGAGT AGCTCCCCAC ATGTTCTAAG AAACAGGGCT CAGAGTGGCA GTGTCCCTCA
5941 GTTCAAGAAA GTTGTTTTCC AGGAATTTAC TGATGGCTCC TTTACTCAGC CCTTATACCG
6001 TGGAGAACTA AATGAACATT TGGGACTCCT GGGGCCATAT ATAAGAGCAG AAGTTGAAGA
6061 TAATATCATG GTAACTTTCA GAAATCAGGC CTCTCGTCCC TATTCCTTCT ATTCTAGCCT
6121 TATTTCTTAT GAGGAAGATC AGAGGCAAGG AGCAGAACCT AGAAAAAACT TTGTCAAGCC
6181 TAATGAAACC AAAACTTACT TTTGGAAAGT GCAACATCAT ATGGCACCCA CTAAAGATGA
6241 GTTTGACTGC AAAGCCTGGG CTTATTTCTC TGATGTTGAC CTGGAAAAAG ATGTGCACTC
6301 AGGCCTGATT GGACCCCTTC TGGTCTGCCA CACTAACACA CTGAACCCTG CTCATGGGAG
6361 ACAAGTGACA GTACAGGAAT TTGCTCTGTT TTTCACCATC TTTGATGAGA CCAAAAGCTG
6421 GTACTTCACT GAAAATATGG AAAGAAACTG CAGGGCTCCC TGCAATATCC AGATGGAAGA
6481 TCCCACTTTT AAAGAGAATT ATCGCTTCCA TGCAATCAAT GGCTACATAA TGGATACACT
6541 ACCTGGCTTA GTAATGGCTC AGGATCAAAG GATTCGATGG TATCTGCTCA GCATGGGCAG
6601 CAATGAAAAC ATCCATTCTA TTCATTTCAG TGGACATGTG TTCACTGTAC GAAAAAAAGA
6661 GGAGTATAAA ATGGCACTGT ACAATCTCTA TCCAGGTGTT TTTGAGACAG TGGAAATGTT
6721 ACCATCCAAA GCTGGAATTT GGCGGGTGGA ATGCCTTATT GGCGAGCATC TACATGCTGG
6781 GATGAGCACA CTTTTTCTGG TGTACAGCAA TAAGTGTCAG ACTCCCCTGG GAATGGCTTC
6841 TGGACACATT AGAGATTTTC AGATTACAGC TTCAGGACAA TATGGACAGT GGGCCCCAAA
6901 GCTGGCCAGA CTTCATTATT CCGGATCAAT CAATGCCTGG AGCACCAAGG AGCCCTTTTC
6961 TTGGATCAAG GTGGATCTGT TGGCACCAAT GATTATTCAC GGCATCAAGA CCCAGGGTGC
7021 CCGTCAGAAG TTCTCCAGCC TCTACATCTC TCAGTTTATC ATCATGTATA GTCTTGATGG
7081 GAAGAAGTGG CAGACTTATC GAGGAAATTC CACTGGAACC TTAATGGTCT TCTTTGGCAA
7141 TGTGGATTCA TCTGGGATAA AACACAATAT TTTTAACCCT CCAATTATTG CTCGATACAT
7201 CCGTTTGCAC CCAACTCATT ATAGCATTCG CAGCACTCTT CGCATGGAGT TGATGGGCTG
7261 TGATTTAAAT AGTTGCAGCA TGCCATTGGG AATGGAGAGT AAAGCAATAT CAGATGCACA
7321 GATTACTGCT TCATCCTACT TTACCAATAT GTTTGCCACC TGGTCTCCTT CAAAAGCTCG

(continued)

**B. Full Length FVIIIFc**

```
7381    ACTTCACCTC CAAGGGAGGA GTAATGCCTG GAGACCTCAG GTGAATAATC CAAAAGAGTG
7441    GCTGCAAGTG GACTTCCAGA AGACAATGAA AGTCACAGGA GTAACTACTC AGGGAGTAAA
7501    ATCTCTGCTT ACCAGCATGT ATGTGAAGGA GTTCCTCATC TCCAGCAGTC AAGATGGCCA
7561    TCAGTGGACT CTCTTTTTTC AGAATGGCAA AGTAAAGGTT TTTCAGGGAA ATCAAGACTC
7621    CTTCACACCT GTGGTGAACT CTCTAGACCC ACCGTTACTG ACTCGCTACC TTCGAATTCA
7681    CCCCCAGAGT TGGGTGCACC AGATTGCCCT GAGGATGGAG GTTCTGGGCT GCGAGGCACA
7741    GGACCTCTAC GACAAAACTC ACACATGCCC ACCGTGCCCA GCTCCAGAAC TCCTGGGCGG
7801    ACCGTCAGTC TTCCTCTTCC CCCCAAAACC CAAGGACACC CTCATGATCT CCCGGACCCC
7861    TGAGGTCACA TGCGTGGTGG TGGACGTGAG CCACGAAGAC CCTGAGGTCA AGTTCAACTG
7921    GTACGTGGAC GGCGTGGAGG TGCATAATGC CAAGACAAAG CCGCGGGAGG AGCAGTACAA
7981    CAGCACGTAC CGTGTGGTCA GCGTCCTCAC CGTCCTGCAC CAGGACTGGC TGAATGGCAA
8041    GGAGTACAAG TGCAAGGTCT CCAACAAAGC CCTCCCAGCC CCCATCGAGA AAACCATCTC
8101    CAAAGCCAAA GGGCAGCCCC GAGAACCACA GGTGTACACC CTGCCCCCAT CCCGGGATGA
8161    GCTGACCAAG AACCAGGTCA GCCTGACCTG CCTGGTCAAA GGCTTCTATC CCAGCGACAT
8221    CGCCGTGGAG TGGGAGAGCA ATGGGCAGCC GGAGAACAAC TACAAGACCA CGCCTCCCGT
8281    GTTGGACTCC GACGGCTCCT TCTTCCTCTA CAGCAAGCTC ACCGTGGACA AGAGCAGGTG
8341    GCAGCAGGGG AACGTCTTCT CATGCTCCGT GATGCATGAG GCTCTGCACA ACCACTACAC
8401    GCAGAAGAGC CTCTCCCTGT CTCCGGGTAA A
```

(ii) Fc (same sequence as A (ii) (SEQ ID NO:3))]

**TABLE 16: Polypeptide Sequences**

**A. B-Domain Deleted FVIII-Fc Monomer Hybrid (BDD FVIIIFc monomer dimer): created by coexpressing BDD FVIIIFc and Fc chains.**

Construct = HC-LC-Fc fusion. An Fc expression cassette is cotransfected with BDDFVIII-Fc to generate the BDD FVIIIFc monomer-. For the BDD FVIIIFc chain, the Fc sequence is shown in bold; HC sequence is shown in double underline; remaining B domain sequence is shown in italics. Signal peptides are underlined.

i) B domain deleted FVIII-Fc chain (19 amino acid signal sequence underlined) (SEQ ID NO:2)

MQIELSTCFFLCLLRFCFS
ATRRYYLGAVELSWDYMQSDLGELPVDARFPPRVPKSFPFNTSVVYKKTLFVEFTDHLF
NIAKPRPPWMGLLGPTIQAEVYDTVVITLKNMASHPVSLHAVGVSYWKASEGAEYDDQ
TSQREKEDDKVFPGGSHTYVWQVLKENGPMASDPLCLTYSYLSHVDLVKDLNSGLIGA
LLVCREGSLAKEKTQTLHKFILLFAVFDEGKSWHSETKNSLMQDRDAASARAWPKMHT
VNGYVNRSLPGLIGCHRKSVYWHVIGMGTTPEVHSIFLEGHTFLVRNHRQASLEISPITFL
TAQTLLMDLGQFLLFCHISSHQHDGMEAYVKVDSCPEEPQLRMKNNEEAEDYDDDLTD
SEMDVVRFDDDNSPSFIQIRSVAKKHPKTWVHYIAAEEEDWDYAPLVLAPDDRSYKSQY
LNNGPQRIGRKYKKVRFMAYTDETFKTREAIQHESGILGPLLYGEVGDTLLIIFKNQASRP
YNIYPHGITDVRPLYSRRLPKGVKHLKDFPILPGEIFKYKWTVTVEDGPTKSDPRCLTRY
YSSFVNMERDLASGLIGPLLICYKESVDQRGNQIMSDKRNVILFSVFDENRSWYLTENIQ
RFLPNPAGVQLEDPEFQASNIMHSINGYVFDSLQLSVCLHEVAYWYILSIGAQTDFLSVFF
SGYTFKHKMVYEDTLTLFPFSGETVFMSMENPGLWILGCHNSDFRNRGMTALLKVSSC
DKNTGDYYEDSYEDISAYLLSKNNAIEPR*SFSQNPPVLKRHQR*EITRTTLQSDQEEIDYDD

(continued)

**A. B-Domain Deleted FVIII-Fc Monomer Hybrid (BDD FVIIIFc monomer dimer): created by coexpressing BDD FVIIIFc and Fc chains.**

TISVEMKKEDFDIYDEDENQSPRSFQKKTRHYFIAAVERLWDYGMSSSPHVLRNRAQSG
SVPQFKKVVFQEFTDGSFTQPLYRGELNEHLGLLGPYIRAEVEDNIMVTFRNQASRPYSF
YSSLISYEEDQRQGAEPRKNFVKPNETKTYFWKVQHHMAPTKDEFDCKAWAYFSDVDL
EKDVHSGLIGPLLVCHTNTLNPAHGRQVTVQEFALFFTIFDETKSWYFTENMERNCRAP
CNIQMEDPTFKENYRFHAINGYIMDTLPGLVMAQDQRIRWYLLSMGSNENIHSIHFSGH
VFTVRKKEEYKMALYNLYPGVFETVEMLPSKAGIWRVECLIGEHLHAGMSTLFLVYSN
KCQTPLGMASGHIRDFQITASGQYGQWAPKLARLHYSGSINAWSTKEPFSWIKVDLLAP
MIIHGIKTQGARQKFSSLYISQFIIMYSLDGKKWQTYRGNSTGTLMVFFGNVDSSGIKHNI
FNPPIIARYIRLHPTHYSIRSTLRMELMGCDLNSCSMPLGMESKAISDAQITASSYFTNMF
ATWSPSKARLHLQGRSNAWRPQVNNPKEWLQVDFQKTMKVTGVTTQGVKSLLTSMY
VKEFLISSSQDGHQWTLFFQNGKVKVFQGNQDSFTPVVNSLDPPLLTRYLRIHPQSWVH
QIALRMEVLGCEAQDLY**DKTHTCPPCPAPELLGGPSVFLFPPKPKDTLMISRTPEVTC**
**VVVDVSHEDPEVKFNWYVDGVEVHNAKTKPREEQYNSTYRVVSVLTVLHQDWLN**
**GKEYKCKVSNKALPAPIEKTISKAKGQPREPQVYTLPPSRDELTKNQVSLTCLVKG**
**FYPSDIAVEWESNGQPENNYKTTPPVLDSDGSFFLYSKLTVDKSRWQQGNVFSCSV**
**MHEALHNHYTQKSLSLSPGK**

ii) Fc chain (20 amino acid heterologous signal peptide from mouse Igκ chain underlined) (SEQ ID NO:4)

METDTLLLWVLLLWVPGSTG
DKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK

**B. Full length FVIIIFc monomer hybrid (Full length FVIIIFc monomer dimer): created by coexpressing FVIIIFc and Fc chains.**

Construct = HC-B-LC-Fc fusion. An Fc expression cassette is cotransfected with full length FVIII-Fc to generate the full length FVIIIFc monomer. For the FVIIIFc chain, the Fc sequence is shown in bold; HC sequence is shown in double underline; B domain sequence is shown in italics. Signal peptides are underlined.

i) Full length FVIIIFc chain (FVIII signal peptide underlined (SEQ ID NO:6)

MQIELSTCFFLCLLRFCFS
ATRRYYLGAVELSWDYMQSDLGELPVDARFPPRVPKSFPFNTSVVYKKTLFVEFTDHLF
NIAKPRPPWMGLLGPTIQAEVYDTVVITLKNMASHPVSLHAVGVSYWKASEGAEYDDQ
TSQREKEDDKVFPGGSHTYVWQVLKENGPMASDPLCLTYSYLSHVDLVKDLNSGLIGA
LLVCREGSLAKEKTQTLHKFILLFAVFDEGKSWHSETKNSLMQDRDAASARAWPKMHT
VNGYVNRSLPGLIGCHRKSVYWHVIGMGTTPEVHSIFLEGHTFLVRNHRQASLEISPITFL
TAQTLLMDLGQFLLFCHISSHQHDGMEAYVKVDSCPEEPQLRMKNNEEAEDYDDDLTD
SEMDVVRFDDDNSPSFIQIRSVAKKHPKTWVHYIAAEEEDWDYAPLVLAPDDRSYKSQY
LNNGPQRIGRKYKKVRFMAYTDETFKTREAIQHESGILGPLLYGEVGDTLLIIFKNQASRP
YNIYPHGITDVRPLYSRRLPKGVKHLKDFPILPGEIFKYKWTVTVEDGPTKSDPRCLTRY
YSSFVNMERDLASGLIGPLLICYKESVDQRGNQIMSDKRNVILFSVFDENRSWYLTENIQ

(continued)

**B. Full length FVIIIFc monomer hybrid (Full length FVIIIFc monomer dimer): created by coexpressing FVIIIFc and Fc chains.**

RFLPNPAGVQLEDPEFQASNIMHSINGYVFDSLQLSVCLHEVAYWYILSIGAQTDFLSVFF
SGYTFKHKMVYEDTLTLFPFSGETVFMSMENPGLWILGCHNSDFRNRGMTALLKVSSC
DKNTGDYYEDSYEDISAYLLSKNNAIEPR*SFSQNSRHPSTRQKQFNATTIPENDIEKTDPWF*
*AHRTPMPKIQNVSSSDLLMLLRQSPTPHGLSLSDLQEAKYETFSDDPSPGAIDSNNSLSEMTHF*
*RPQLHHSGDMVFTPESGLQLRLNEKLGTTAATELKKLDFKVSSTSNNLISTIPSDNLAAGTDNT*
*SSLGPPSMPVHYDSQLDTTLFGKKSSPLTESGGPLSLSEENNDSKLLESGLMNSQESSWGKNV*
*SSTESGRLFKGKRAHGPALLTKDNALFKVSISLLKTNKTSNNSATNRKTHIDGPSLLIENSPSVW*
*QNILESDTEFKKVTPLIHDRMLMDKNATALRLNHMSNKTTSSKNMEMVQQKKEGPIPPDAQN*
*PDMSFFKMLFLPESARWIQRTHGKNSLNSGQGPSPKQLVSLGPEKSVEGQNFLSEKNKVVVG*
*KGEFTKDVGLKEMVFPSSRNLFLTNLDNLHENNTHNQEKKIQEEIEKKETLIQENVVLPQIHT*
*VTGTKNFMKNLFLLSTRQNVEGSYDGAYAPVLQDFRSLNDSTNRTKKHTAHFSKKGEEENLE*
*GLGNQTKQIVEKYACTTRISPNTSQQNFVTQRSKRALKQFRLPLEETELEKRIIVDDTSTQWSK*
*NMKHLTPSTLTQIDYNEKEKGAITQSPLSDCLTRSHSIPQANRSPLPIAKVSSFPSIRPIYLTRVLF*
*QDNSSHLPAASYRKKDSGVQESSHFLQGAKKNNLSLAILTLEMTGDQREVGSLGTSATNSVTY*
*KKVENTVLPKPDLPKTSGKVELLPKVHIYQKDLFPTETSNGSPGHLDLVEGSLLQGTEGAIKW*
*NEANRPGKVPFLRVATESSAKTPSKLLDPLAWDNHYGTQIPKEEWKSQEKSPEKTAFKKKDTI*
*LSLNACESNHAIAAINEGQNKPEIEVTWAKQGRTERLCSQNPPVLKRHQR*EITRTTLQSDQEEI
DYDDTISVEMKKEDFDIYDEDENQSPRSFQKKTRHYFIAAVERLWDYGMSSSPHVLRNR
AQSGSVPQFKKVVFQEFTDGSFTQPLYRGELNEHLGLLGPYIRAEVEDNIMVTFRNQASR
PYSFYSSLISYEEDQRQGAEPRKNFVKPNETKTYFWKVQHHMAPTKDEFDCKAWAYFS
DVDLEKDVHSGLIGPLLVCHTNTLNPAHGRQVTVQEFALFFTIFDETKSWYFTENMERN
CRAPCNIQMEDPTFKENYRFHAINGYIMDTLPGLVMAQDQRIRWYLLSMGSNENIHSIHF
SGHVFTVRKKEEYKMALYNLYPGVFETVEMLPSKAGIWRVECLIGEHLHAGMSTLFLV
YSNKCQTPLGMASGHIRDFQITASGQYGQWAPKLARLHYSGSINAWSTKEPFSWIKVDL
LAPMIIHGIKTQGARQKFSSLYISQFIIMYSLDGKKWQTYRGNSTGTLMVFFGNVDSSGIK
HNIFNPPIIARYIRLHPTHYSIRSTLRMELMGCDLNSCSMPLGMESKAISDAQITASSYFTN
MFATWSPSKARLHLQGRSNAWRPQVNNPKEWLQVDFQKTMKVTGVTTQGVKSLLTS
MYVKEFLISSSQDGHQWTLFFQNGKVKVFQGNQDSFTPVVNSLDPPLLTRYLRIHPQSW
VHQIALRMEVLGCEAQDLY**DKTHTCPPCPAPELLGGPSVFLFPPKPKDTLMISRTPEV**
**TCVVVDVSHEDPEVKFNWYVDGVEVHNAKTKPREEQYNSTYRVVSVLTVLHQDW**
**LNGKEYKCKVSNKALPAPIEKTISKAKGQPREPQVYTLPPSRDELTKNQVSLTCLV**
**KGFYPSDIAVEWESNGQPENNYKTTPPVLDSDGSFFLYSKLTVDKSRWQQGNVFSC**
**SVMHEALHNHYTQKSLSLSPGK**

ii) Fc chain (20 amino acid heterologous signal peptide from mouse Igκ chain underlined) (SEQ ID NO:4)

METDTLLLWVLLLWVPGSTG
DKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

All patents and publications cited herein are incorporated by reference herein in their entirety.

Preferred embodiments of the present invention are described below and are referred to as embodiments E1 to E52.

E1. A method of reducing the annualized bleeding rate in a subject having hemophilia comprising administering to the subject an effective dose of a long-acting FVIII polypeptide at a dosing interval of about three days or longer.

E2. The method of E 1, wherein the administration is prophylactic and individualized for the subject resulting in an annualized bleeding rate less than about 5.0, less than about 4.9, less than about 4.8, less than about 4.7, less than about 4.6, or less than about 4.5.

E3. The method of E 2, wherein the median annualized bleeding rate is about 1.6.

E4. The method of E 1, wherein the administration is prophylactic and weekly resulting in an annualized bleeding rate less than about 9.0, less than about 8.9, less than about 8.8, less than about 8.7, less than about 8.6, less than about 8.5, or less than about 8.4.

E5. The method of E 4, wherein the median annualized bleeding rate is about 3.6.

E6. The method of E 1, wherein the administration is on-demand or episodic resulting in an annualized bleeding rate less than about 55, less than about 54, less than about 53, less than about 52, less than about 51, less than about 50, less than about 49, less than about 48, or less than about 47.

E7. The method of E 6, wherein the median annualized bleeding rate is about 33.6.

E8. The method of any one of E 1 to 7, wherein the effective dose is between about 20/ IU/kg to about 90IU/kg.

E9. The method of any one of E 1 to 8, wherein the effective dose is 20-30 IU/kg, 30-40 IU/ kg, 40-50 IU/kg, 50-60 IU/kg, 60-70 IU/kg, 70-80 IU/kg, or 80-90 IU/kg.

E10. The method of any one of E 1 to 9, wherein the effective dose is 20 IU/kg, 25 IU/kg, 30 IU/kg, 35 IU/kg, 40 IU/kg, 45 IU/kg, 50 IU/kg, 55 IU/kg, 60 IU/kg, 65 IU/kg, 70 IU/kg, 75 IU/kg, 80 IU/kg, 85 IU/kg, or 90 IU/kg.

E11. The method of any of E 8 to 10, wherein the administration is prophylactic and individualized at an effective dose of about 25IU/kg to about 65 IU/kg twice weekly or every three days or about 50 IU/kg to about 65 IU/kg every 4 or 5 days.

E12. The method of E 11, wherein a first dose of the long-acting FVIII polypeptide is administered is about 25 IU/kg and a second dose of the long-acting FVIII polypeptide is about 50 IU/kg.

E13. The method of any of E 8 to 10, wherein the administration is prophylactic and weekly at an effective dose of 65 IU/kg weekly.

E14. The method of any of E 8 to 10, wherein the administration is on-demand or episodic at an effective dose of 10 IU/kg to 75 IU/kg every 12 to 24 hours.

E15. The method of any one of E 1 to 7, wherein the effective dose is a fixed dose, which is standard across all body weights.

E16. The method of E 15, wherein the fixed dose is about 2,000 IU, about 2,500 IU, about 3,000 IU, about 3,500 IU, or about 4,000 IU per dose.

E17. The method of any one of E 1 to 7, wherein the effective dose is a stratified dose.

E18. The method of any one of E 1 to 17, wherein a trough level of the long-acting FVIII polypeptide is above 1%, above 2%, or above 3% of normal.

E19. The method of any one of E 1 to 18, wherein the long-acting FVIII polypeptide has a $T_{1/2beta}$ (activity) of about 7 hours to about 48 hours, about 6 hours to about 49 hours, about 5 hours to about 50 hours.

E20. The method of any one of E 19, wherein the long-acting FVIII has a $T_{1/2beta}$ (activity) mean of at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, at least about 25 hours.

E21. The method of E 20, wherein the $T_{1/2beta}$ (activity) mean is about 19 hours.

E22. The method of E 19 or 20, wherein the $T_{1/2beta}$ (activity) mean is at least about 1.5 fold higher than a polypeptide consisting of amino acids 20 to 1457 of SEQ ID NO:2, 20 to 2351 of SEQ ID NO: 6, or ADVATE®.

E23. The method of any one of E 1 to 22, wherein the plasma trough level of the long-acting FVIII polypeptide is maintained between about 1% and about 5%, between about 1% and about 6%, between about 1% and about 7%, between about 1% and about 8%, between about 1% and about 9%, between about 1% and about 10%, between about 1% and about 11%, between about 1% and about 12%, between about 1% and about 13%, between about 1% and about 14%, between about 1% and about 15% above the baseline in the subject.

E24. The method of any one of E 1 to 23, wherein the long-acting FVIII polypeptide is a long-acting FVIII polypeptide comprising a FVIII polypeptide and a heterologous moiety.

E25. The method of E 24, wherein the heterologous moiety is an FcRn binding partner.

E26. The method of E 25, wherein the FcRn binding partner comprises an Fc region.

E27. The method of E 25 or 26, wherein the long-acting FVIII polypeptide further comprises a second FcRn binding partner.

E28. The method of E 27, wherein the second FcRn binding partner comprises a second Fc region.

E29. The method of E 27 or 28, wherein the FcRn binding partner and the second FcRn binding partner are associated.

E30. The method of E 29, wherein the association is a covalent bond.

E31. The method of E 30, wherein the covalent bond is a disulfide bond.

E32. The method of any one of E 27 to 31, wherein the second FcRn binding partner is not linked to an amino acid sequence by a peptide bond.

E33. The method of any one of E 1 to 32, wherein the long-acting FVIII polypeptide is FVIII monomer dimer hybrid.

E34. The method of any one of E 24 to 33, wherein the FVIII polypeptide in the long-acting polypeptide is a human FVIII.

E35. The method of any one of E 24 to 34, wherein the FVIII polypeptide in the long-acting polypeptide is a full-length FVIII or a B-domain deleted FVIII.

E36. The method of any one of E 24 to 34, wherein the FVIII polypeptide is single chain.

E37. The method of any one of E 24 to 34, wherein the FVIII polypeptide is unprocessed.

E38. The method of any one of E 24 to 34, wherein the FVIII polypeptide is in two chains, a first chain comprising a heavy chain of the FVIII polypeptide and a second chain comprising a light chain of the FVIII polypeptide.

E39. The method of any one of E 25 to 38, wherein the FcRn binding partner in the chimeric polypeptide is a human Fc.

E40. The method of any one of E 24 to 39, wherein the FVIII polypeptide is at least 60%, 70%, 80%, 90%, 95%, 95%, 97%, 98%, 99%, or 100% identical to a FVIII amino acid sequence shown in Table 16A or 16B without a signal sequence (amino acids 20 to 1457 of SEQ ID NO: 2 or amino acids 20 to 2351 of SEQ ID NO: 6).

E41. The method of any one of E 25 to 40, wherein the FcRn binding partner is at least 60%, 70%, 80%, 90%, 95%, 96%, 97%, 98%, 99%, or 100% identical to a Fc amino acid sequence shown in Table 16B without a signal sequence (amino acids 21 to 247 SEQ ID NO:4).

E42. The method of any one of E 27 to 41, wherein the second FcRn binding partner in the chimeric polypeptide is a human Fc.

E43. The method of E 42, wherein the second FcRn binding partner is at least 60%, 70%, 80%, 90%, 95%, 96%, 97%, 98%, 99%, or 100% identical to a Fc amino acid sequence shown in Table 16B without a signal sequence (amino acids 21 to 247 SEQ ID NO:4)

E44. The method of any one of E 1 to 43, wherein the fixed dose of the clotting factor is for perioperative management of a bleeding episode.

E45. The method of any one of E 1 to 44, wherein the subject is in need of controlling or preventing bleeding or bleeding episodes.

E46. The method of E 45, wherein the subject is in need of controlling or preventing bleeding in minor hemorrhage, hemarthroses, superficial muscle hemorrhage, soft tissue hemorrhage, moderate hemorrhage, intramuscle or soft tissue hemorrhage with dissection, mucous membrane hemorrhage, hematuria, major hemorrhage, hemorrhage of the pharynx, hemorrhage of the retropharynx, hemorrhage of the retroperitonium, hemorrhage of the central nervous system, bruises, cuts, scrapes, joint hemorrhage, nose bleed, mouth bleed, gum bleed, intracranial bleeding, intraperitoneal bleeding, minor spontaneous hemorrhage, bleeding after major trauma, moderate skin bruising, or spontaneous hemorrhage into joints, muscles, internal organs or the brain.

E47. The method of E 46, wherein the subject is in need of management of bleeding associated with surgery or dental extraction.

E48. The method of E 47, wherein the subject will undergo, is undergoing, or has undergone major surgery.

E49. The method of E 48, wherein the major surgery is orthopedic surgery, extensive oral surgery, urologic surgery, or hernia surgery.

E50. The method of E 49, wherein the orthopedic surgery is replacement of knee, hip, or other major joint.

E51. The method of any one of E 1 to 50, wherein the subject is in need of long-term treatment.

E52. The method of any one of E 1 to 51, wherein the long-acting FVIII polypeptide is administered intravenously or subcutaneously.

## Claims

1. A chimeric Factor VIII (FVIII) polypeptide comprising FVIII and a FcRn binding partner (FcRn BP) for use in prophylactically treating hemophilia A in a pediatric subject in need thereof, wherein the pediatric subject is less than 12 years, wherein the chimeric FVIII polypeptide is to be administered to the pediatric subject at multiple doses of 20 IU/kg to 90 IU/kg at a dosing interval of once every one or more days between doses.

2. The chimeric FVIII polypeptide for use of claim 1, wherein the pediatric subject is birth to 11 years, birth to 6 years, birth to 5 years, birth to 2 years, or 2 to 11 years of age.

3. The chimeric FVIII polypeptide for use of claim 1 or claim 2, wherein each of the multiple doses is 20-30 IU/kg, 30-40 IU/kg, 40-50 IU/kg, 50-60 IU/kg, 60-70 IU/kg, 70-80 IU/kg, or 80-90 IU/kg.

**4.** The chimeric FVIII polypeptide for use of any one of claims 1 to 3, wherein each of the multiple doses is 20 IU/kg, 25 IU/kg, 30 IU/kg, 35 IU/kg, 40 IU/kg, 45 IU/kg, 50 IU/kg, 55 IU/kg, 60 IU/kg, 65 IU/kg, 70 IU/kg, 75 IU/kg, 80 IU/kg, 85 IU/kg, or 90 IU/kg.

**5.** The chimeric FVIII polypeptide for use of any one of claims 1 to 4, wherein the dosing interval is once every 2 days, every 3 days, every 4 days, or every 5 days.

**6.** The chimeric FVIII polypeptide for use of any one of claims 1 to 5, wherein a first dose is 25 IU/kg and a second dose is 50 IU/kg.

**7.** The chimeric FVIII polypeptide for use of claim 6, wherein the dosing interval between the first dose and the second dose is 3 days.

**8.** The chimeric FVIII polypeptide for use of any one of claims 1 to 7, wherein the FVIII comprises a full-length FVIII, mature FVIII, or FVIII with a full or partial deletion of the B domain.

**9.** The chimeric FVIII polypeptide for use of any one of claims 1 to 8, wherein the chimeric FVIII polypeptide is a FVIII monomer dimer hybrid.

**10.** The chimeric polypeptide for use according to any one of claims 1 to 9, wherein the FVIII comprises an amino acid sequence that is at least 90% or 95% identical to amino acids 20 to 1457 of SEQ ID NO: 2 or amino acids 20 to 2351 of SEQ ID NO: 6.

**11.** The chimeric polypeptide for use according to any one of claims 1 to 9, wherein the FVIII comprises amino acids 20 to 1457 of SEQ ID NO: 2 or amino acids 20 to 2351 of SEQ ID NO: 6.

**12.** The chimeric polypeptide for use according to any one of claims 1 to 11, wherein the FcRn BP is Fc.

**13.** The chimeric polypeptide for use according to claim 12, wherein the Fc comprises an amino acid sequence that is at least 90% or 95% identical to amino acids 1458 to 1684 of SEQ ID NO: 2 or amino acids 2352 to 2578 of SEQ ID NO: 6.

**14.** The chimeric polypeptide for use according to claim 12, wherein the Fc comprises amino acids 1458 to 1684 of SEQ ID NO: 2 or amino acids 2352 to 2578 of SEQ ID NO: 6.

**15.** The chimeric polypeptide for use according to any one of claims 1 to 11, wherein the FcRn BP is albumin.

**Figure 1.** Sequential pharmacokinetics subgroup (Arm 1) dosing and pharmacokinetics sampling.

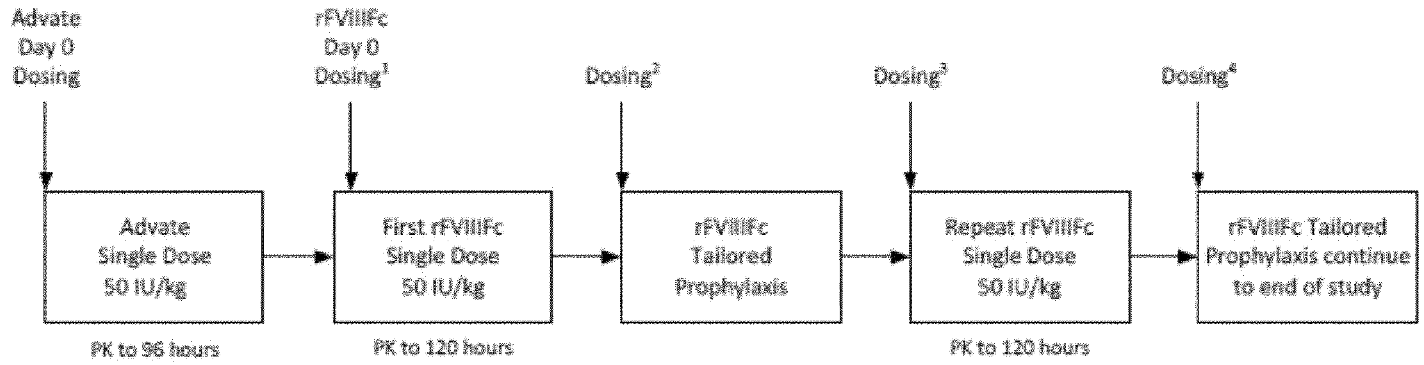


1. Minimum interval of 96 hours, maximum interval of 8 weeks from rFVIII injection to first injection of 50 IU/kg rFVIIIFc for sequential PK profiling.
2. Starting regimen for twice weekly individualized (tailored) prophylaxis of 25 IU/kg on first day of week followed by 50 IU/kg on fourth day, with dose and interval adjusted to achieve target trough level of approximately 1% to 3% (or higher, as clinically indicated). FVIII level assessed at peak and trough.
3. Repeat dose of 50 IU/kg rFVIIIFc for sequential PK profiling administered at Week 14, or at 12 to 24 weeks after first injection of rFVIIIFc.
4. Continued individualized prophylaxis regimen until 28 (±2) weeks up to 52 (±2) weeks.

PK, pharmacokinetic

**Figure 2.** Mean observed FVIII activity with rFVIIIFc and octocog alfa over time: one-stage clotting assay (logarithmic scale) in the sequential pharmacokinetic subgroup

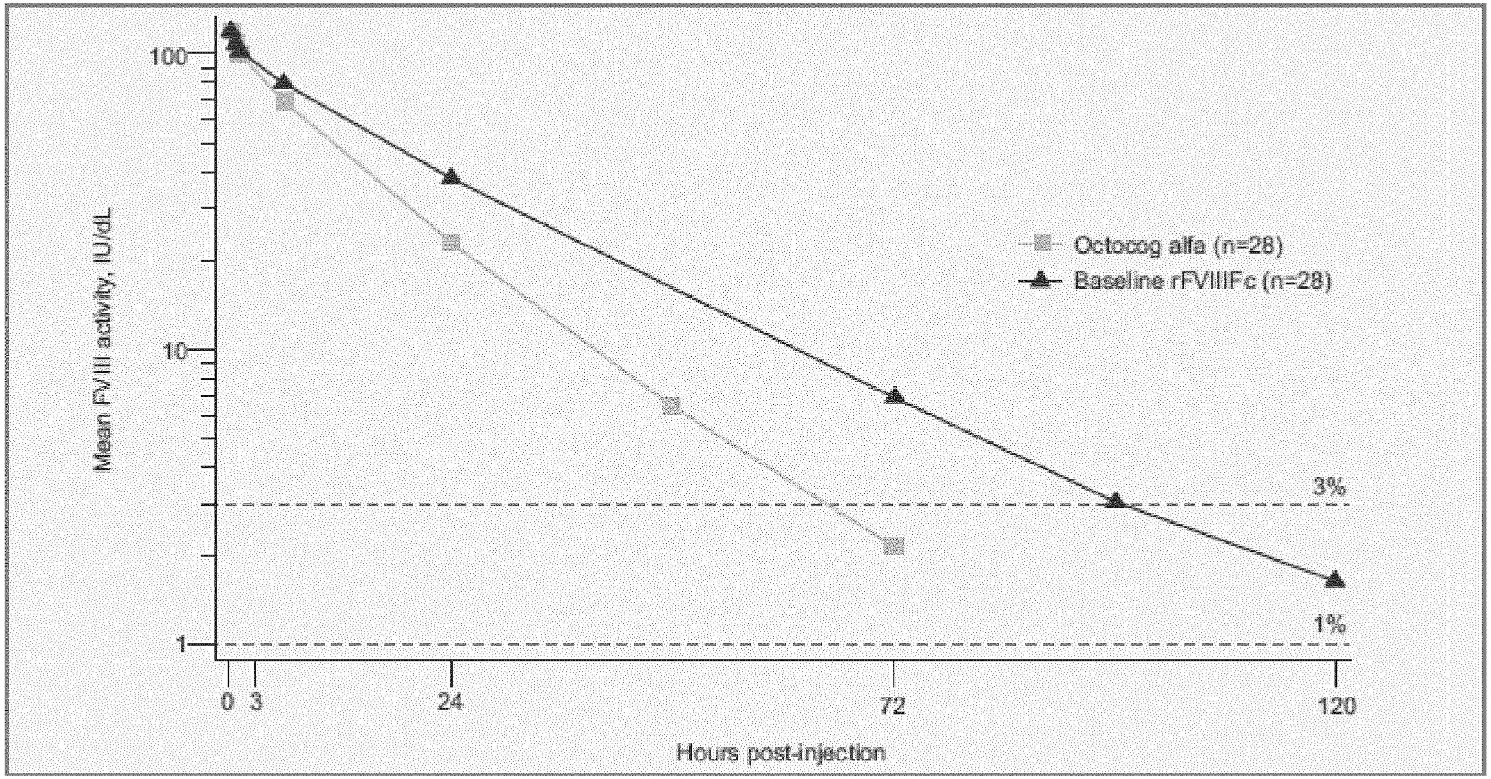


Data presented are observed FVIII activity without subtraction of endogenous baseline FVIII level and FVIII level from the previous treatment prior to the pharmacokinetic dose.

**Figure 3.** Study design for Kids-A-LONG

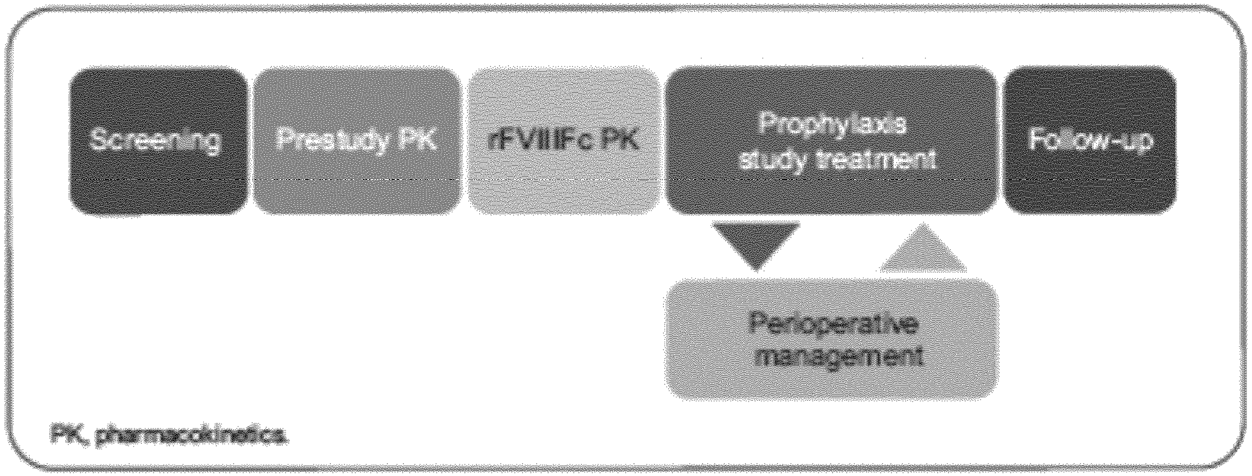

**Figure 4.** Patient Disposition

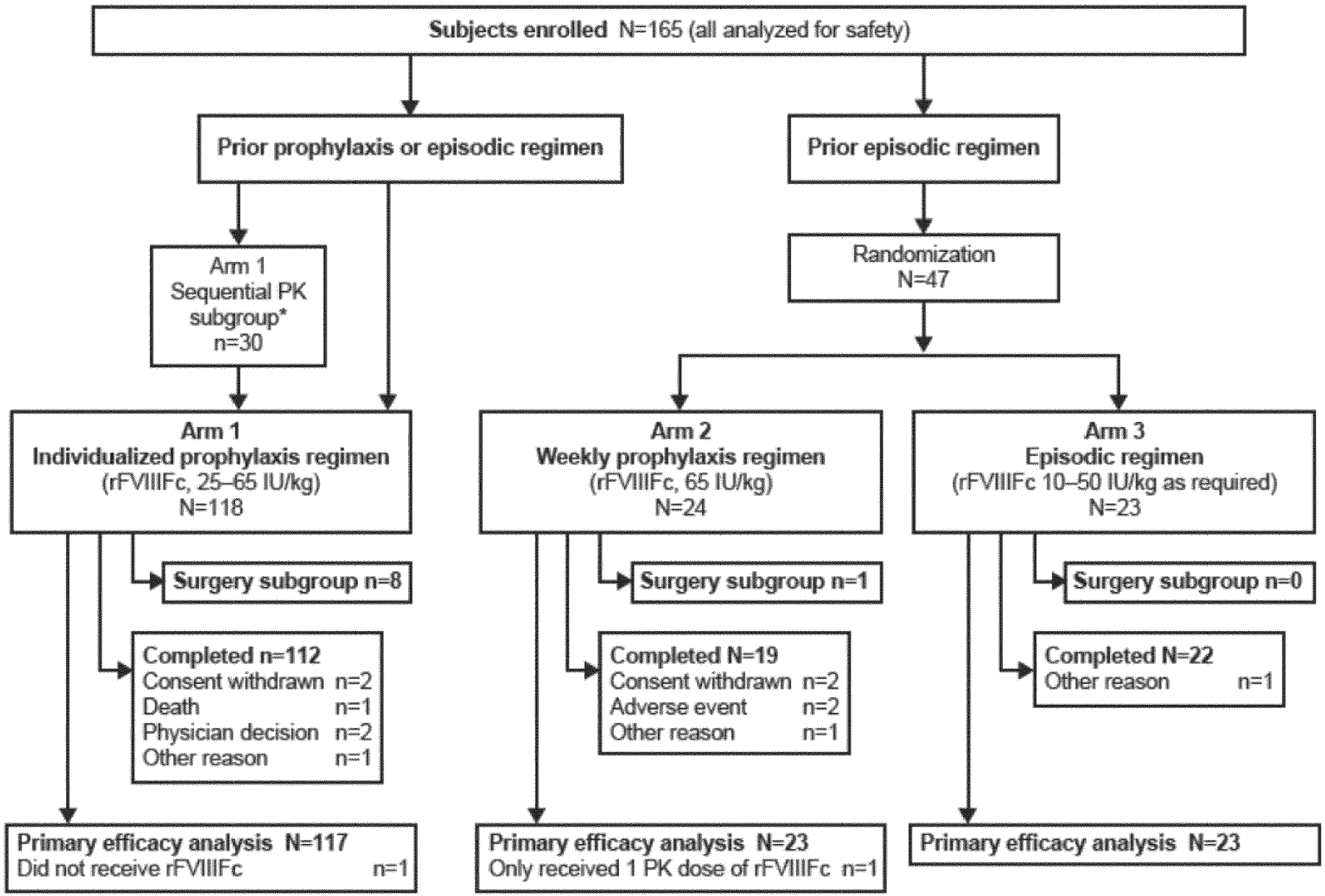

**Figure 5.** Subgroup Analyses of Annualized Bleeding Rates

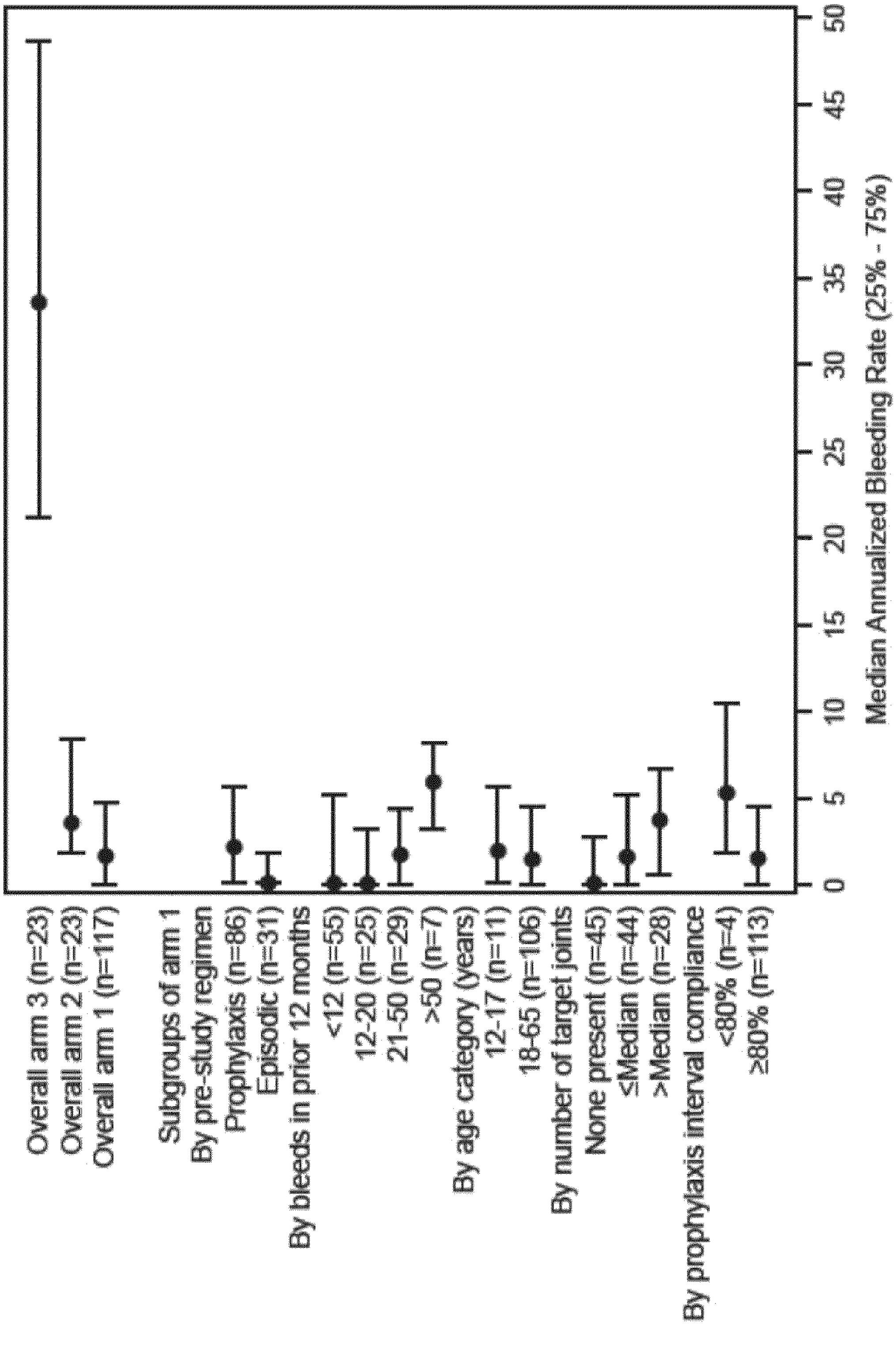

**Figure 6.** Number of Bleeding Episodes in the Prior 12 Months Compared with the On-study ABR by Prior FVIII Regimen

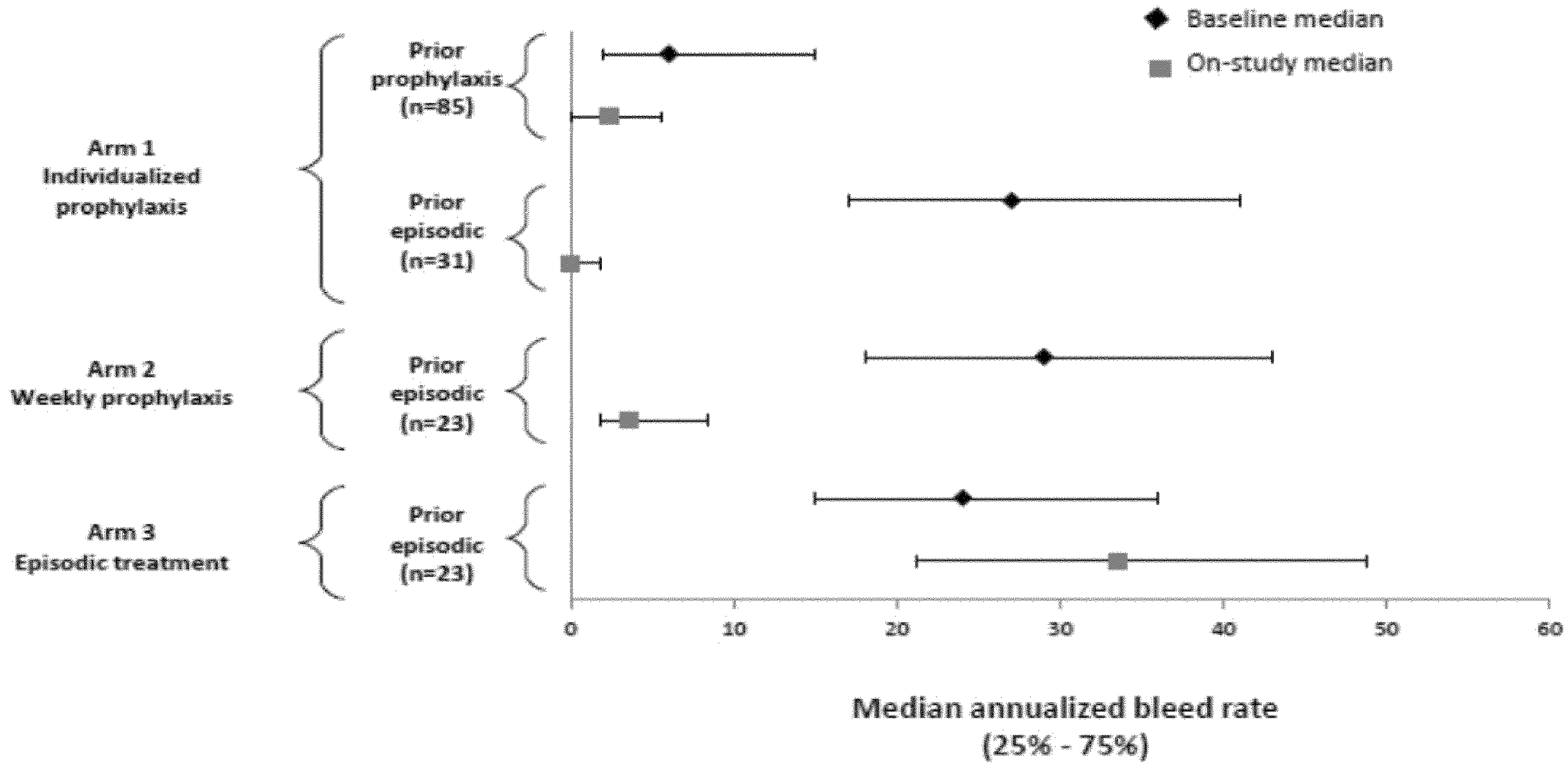

**Figure 7.** Dose Modification in Arm 1 (Individualized Prophylaxis)

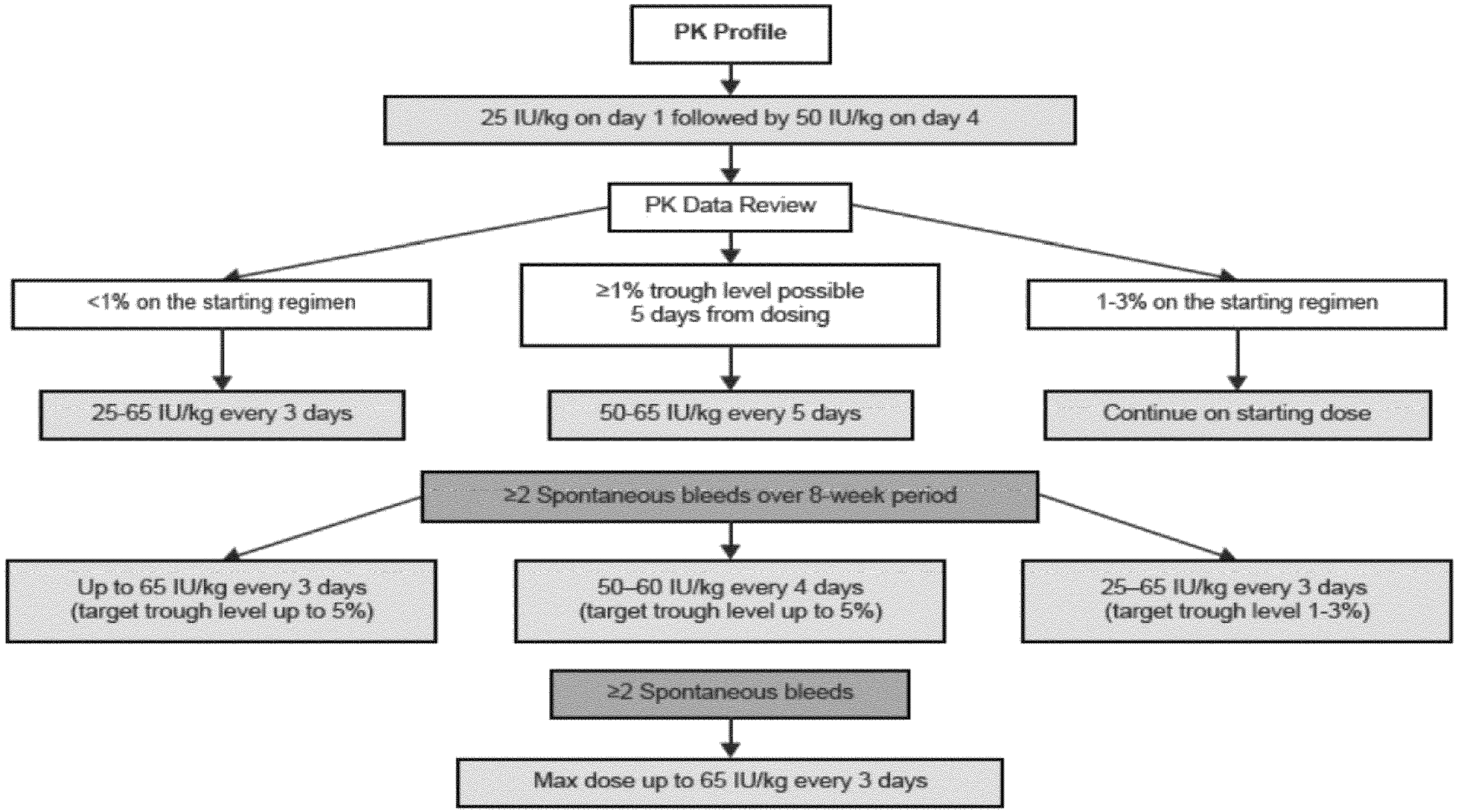

**Figure 8.** Individual Time to 1% Estimates for Advate and rFVIIIFc

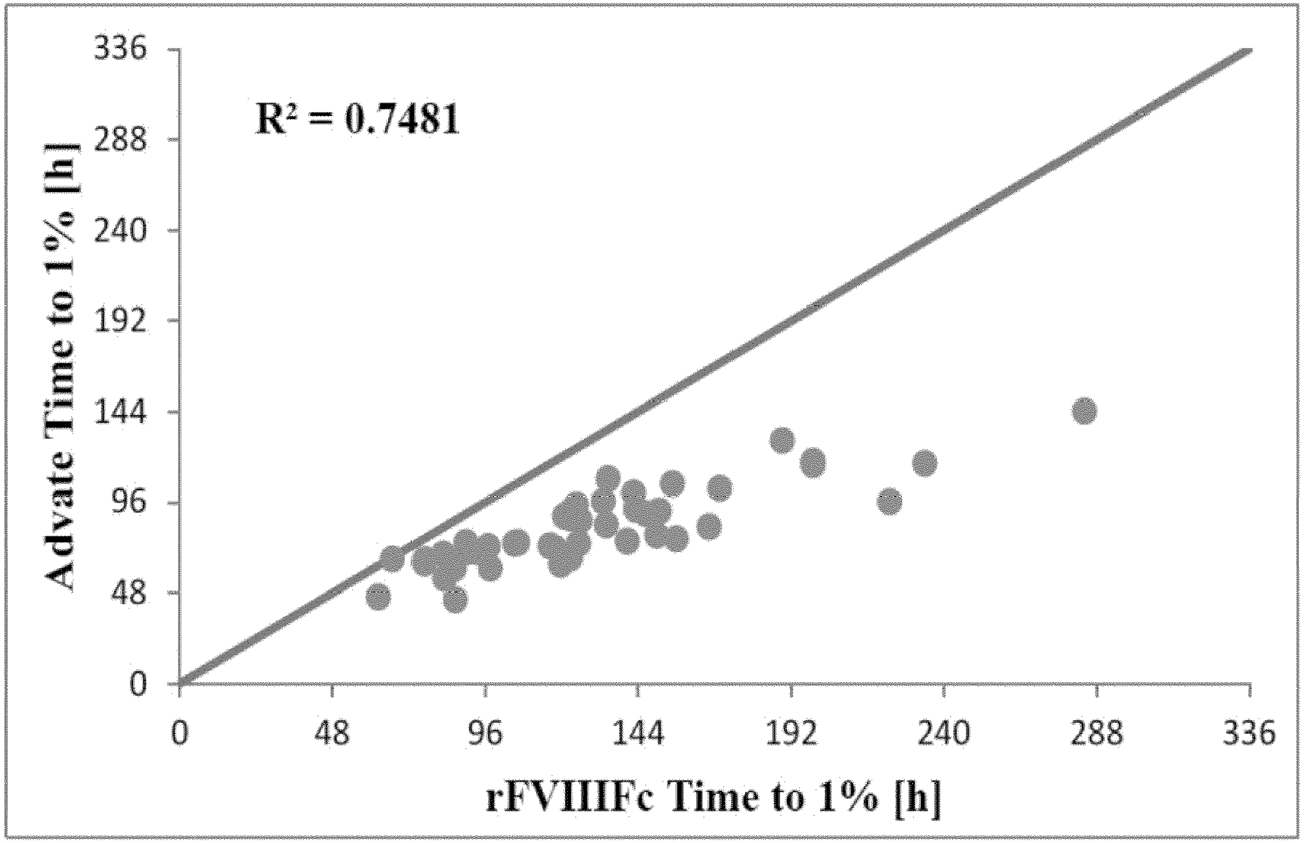


Each point represents 1 subject who received both Advate and rFVIIIFc. Doses were 25, 50, or 65 IU/kg. The diagonal blue line is the identity line.

**Figure 9.** Goodness of Fit Diagnostics

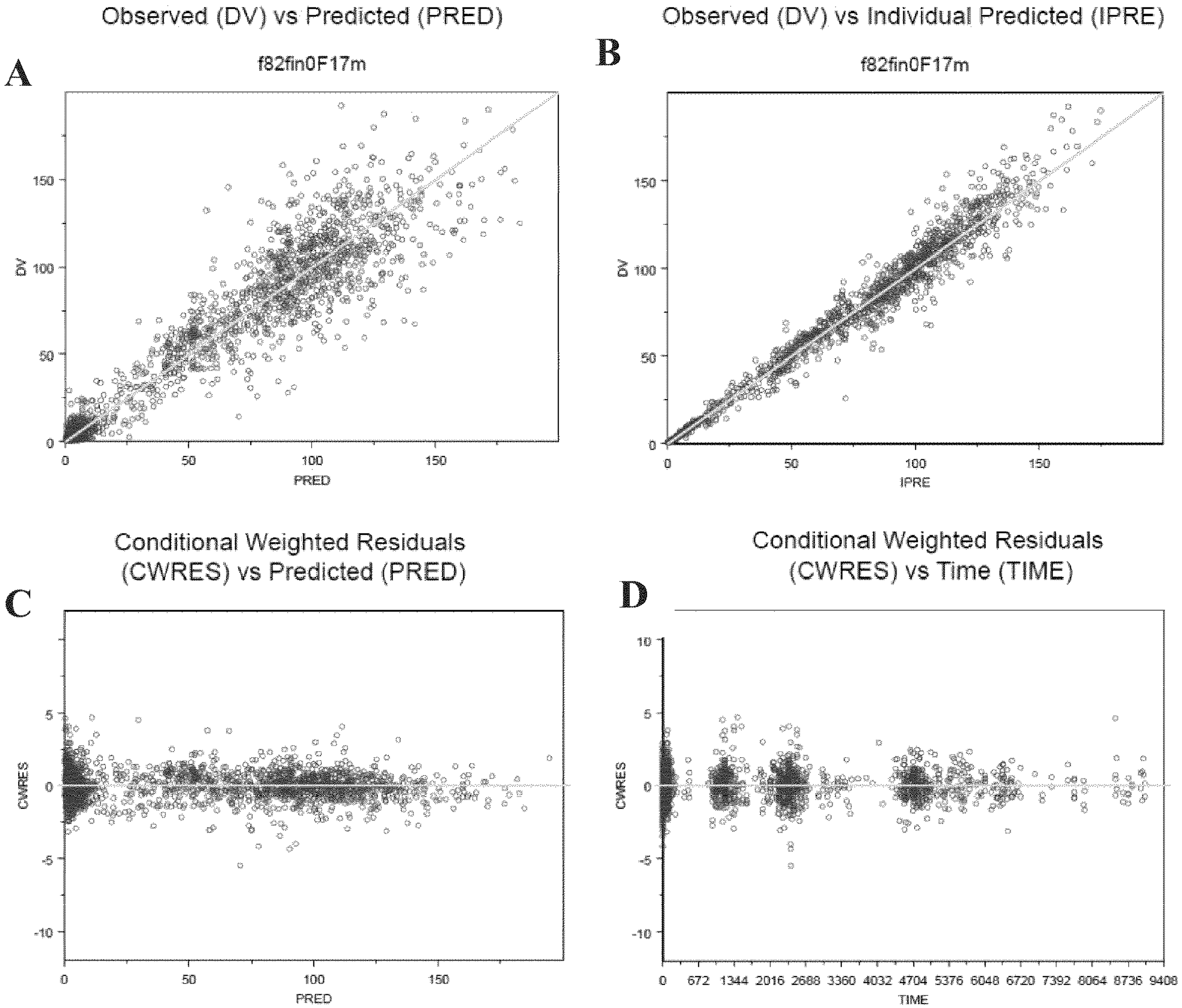

**Figure 10.** Predicted Steady-State Activity Profiles of Selected rFVIIIFc Prophylaxis Dosing Regimens

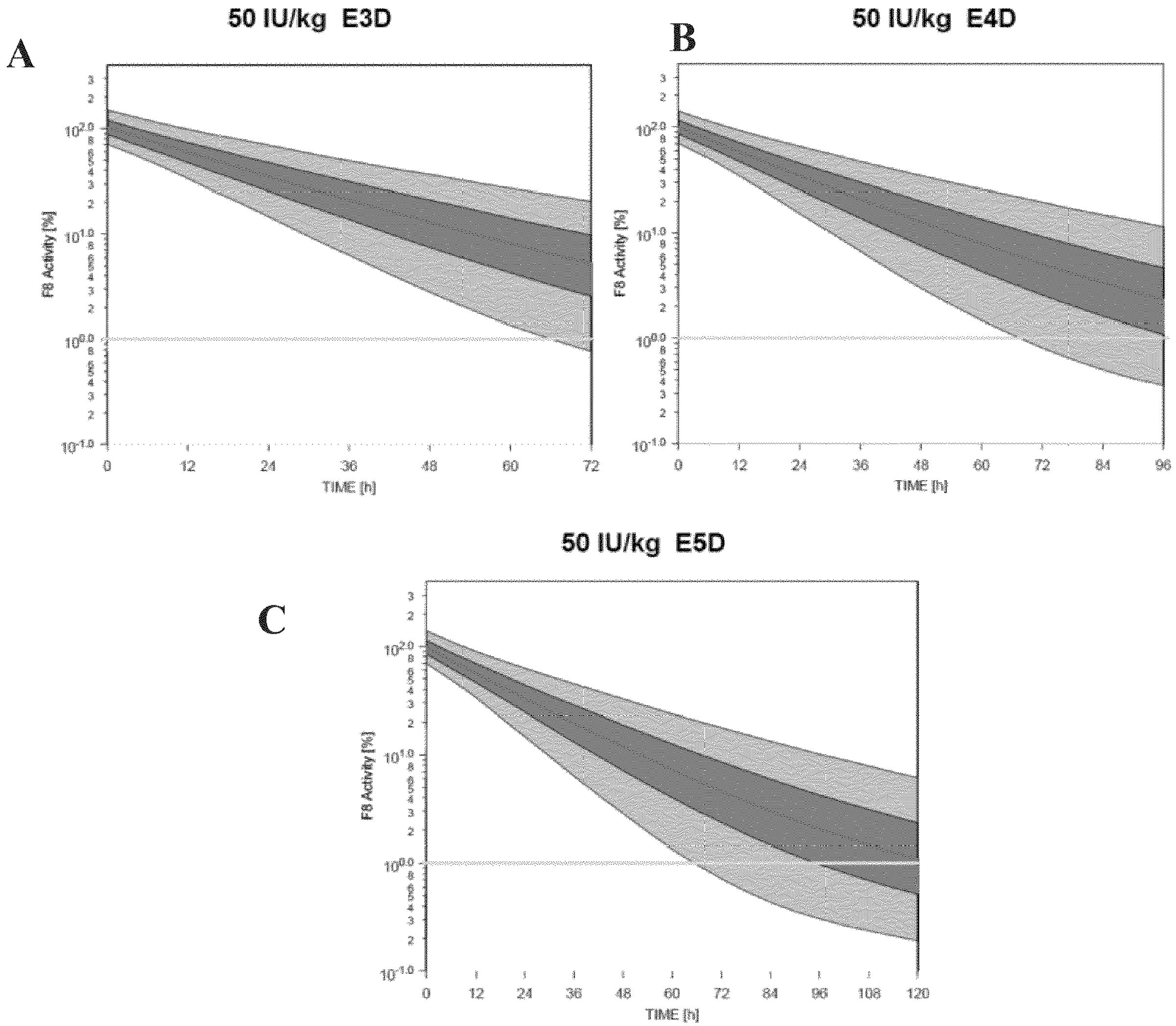


The red line represents the median; the dark grey shaded area coveres the 25th to 75th percentiles; the light grey shaded area covers the 5th to 95th percentiles.

**Figure 11.** Predicted FVIII Activity for the Hypothetical Perioperative Dosing Regimen in Table 20

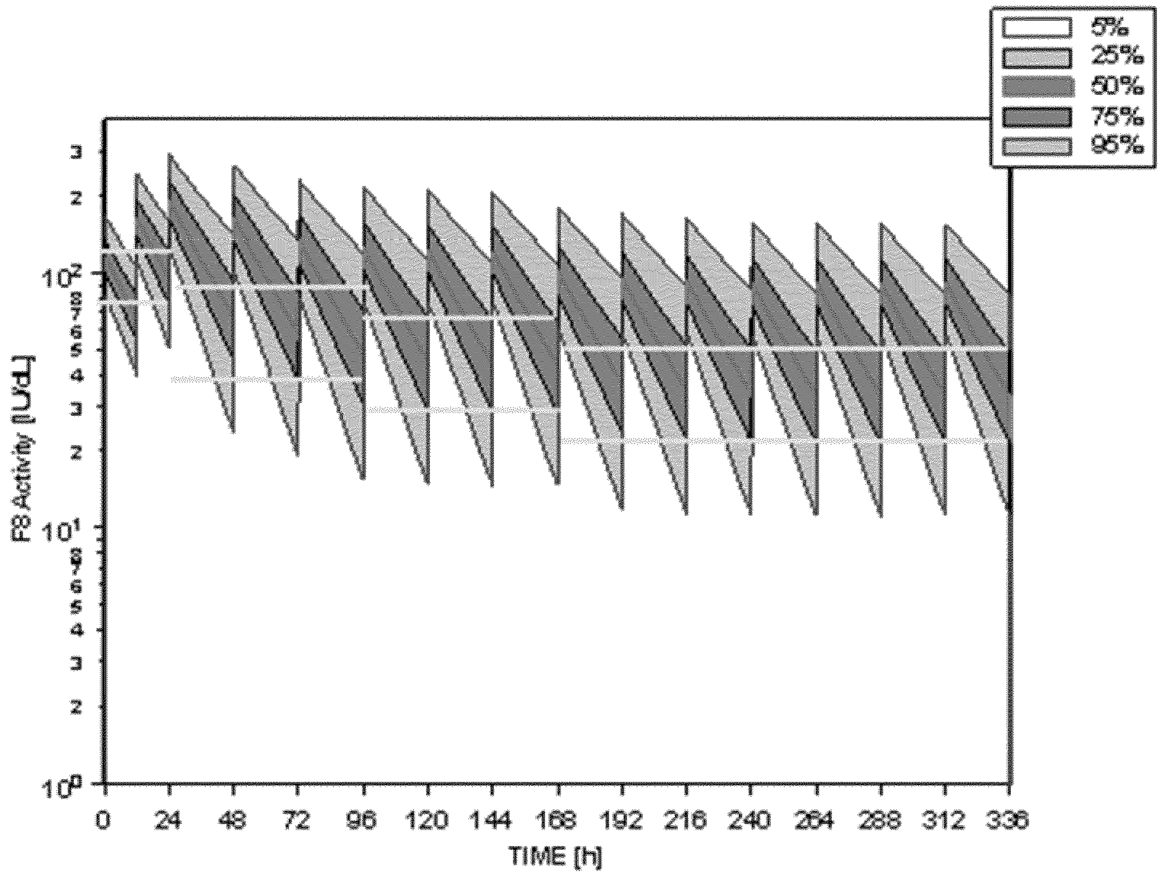

**Figure 12.** Predicted FVIII Activity for the Hypothetical Perioperative Dosing Regimen in Table 21

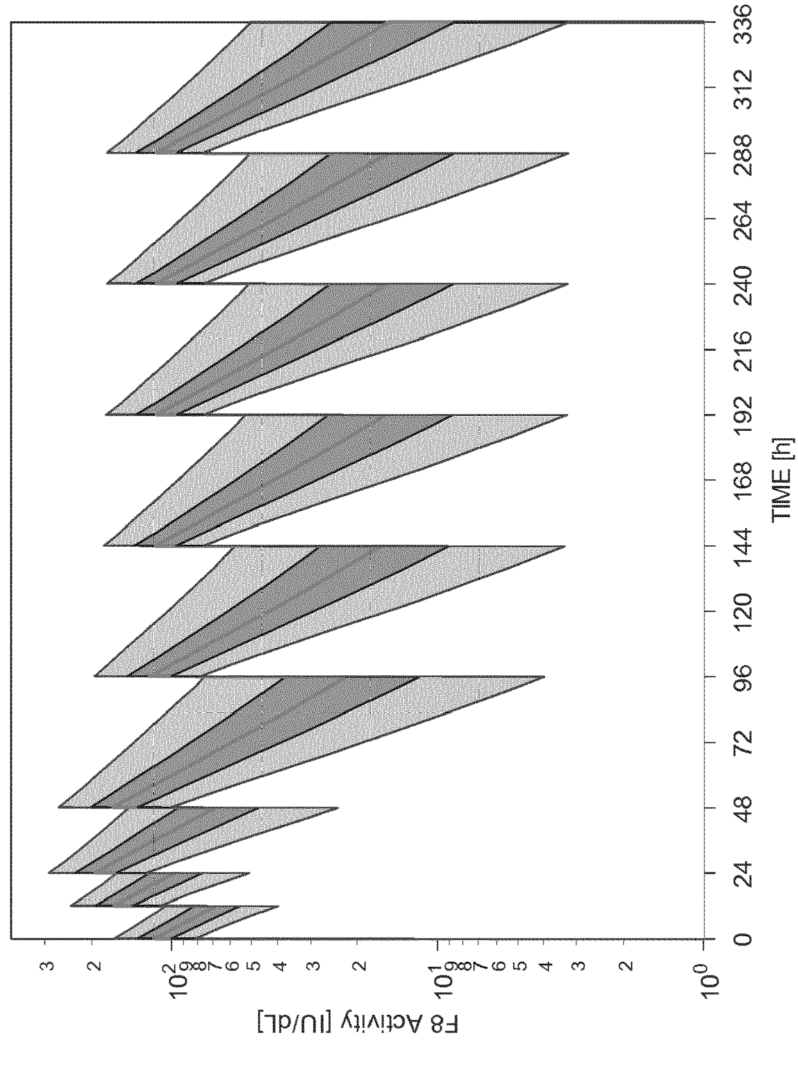

**Figure 13.** Surgical Pharmacokinetic Profiles

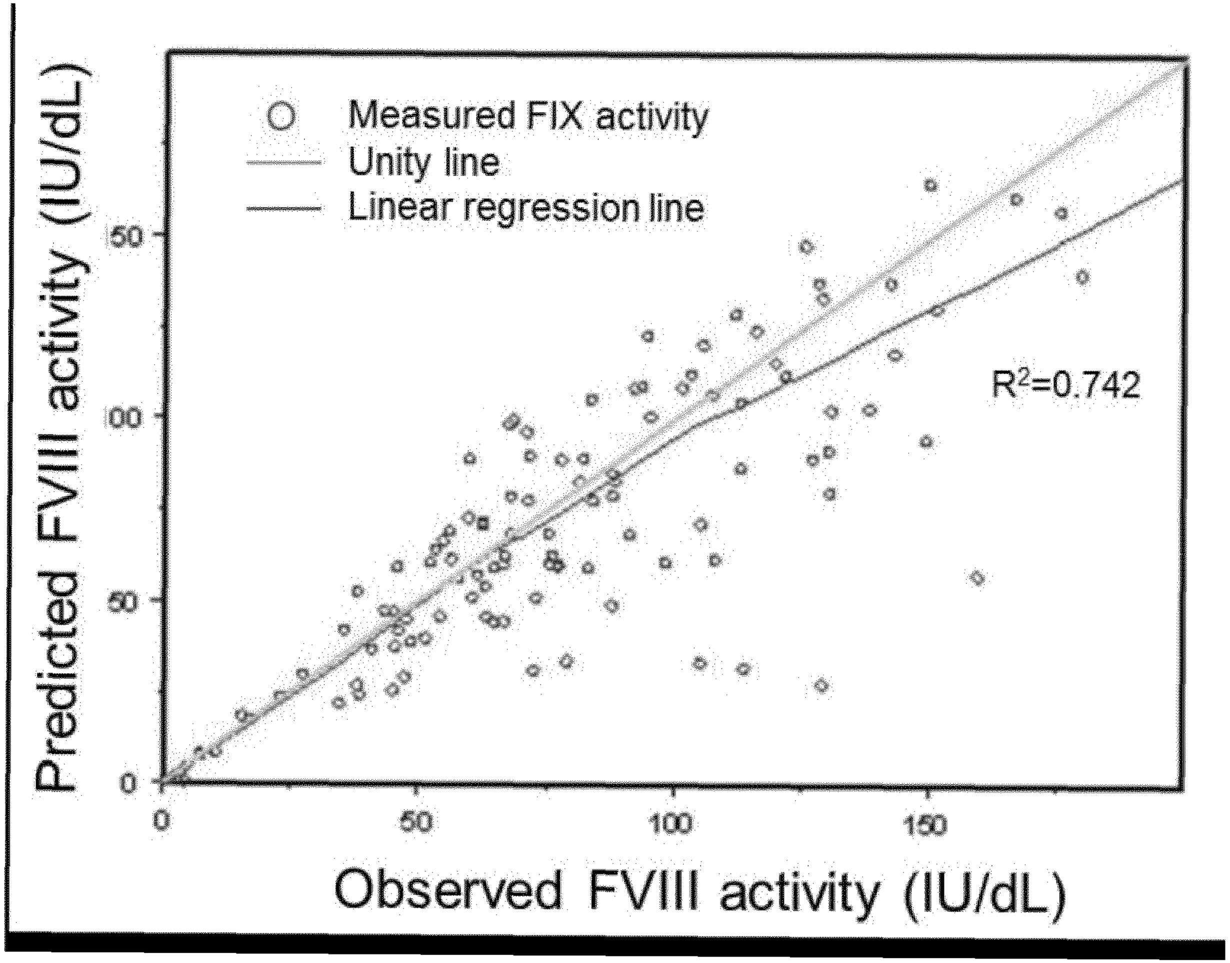

**Figure 14.** Proposed Output for Individual PK Assessment

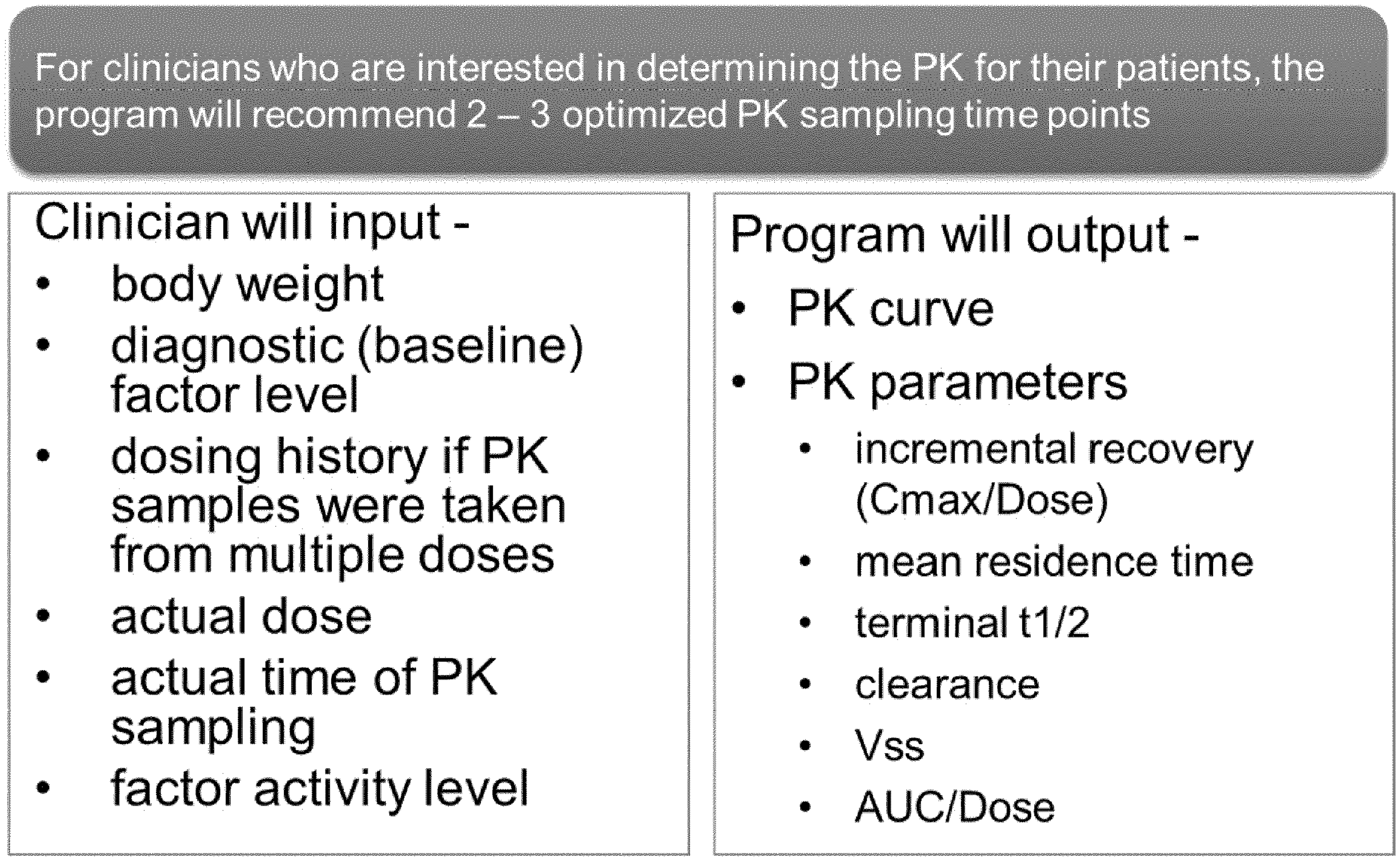

**Figure 15.** Proposed Output for Individualized Dosing Regimen Selection – Episodic Treatment

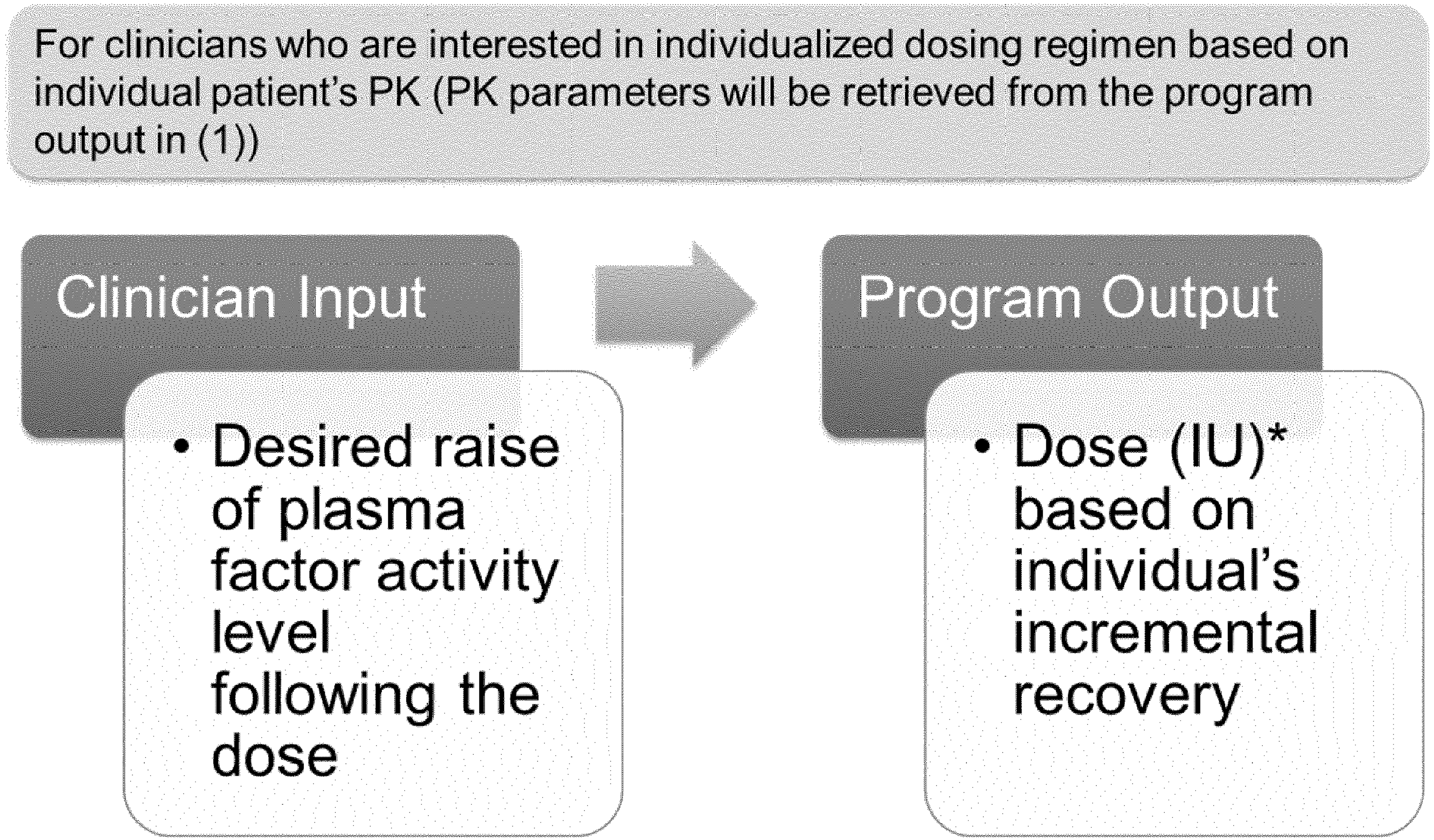

**Figure 16.** Proposed Output for Dosing Regimen Selections without Individualized PK Assessment

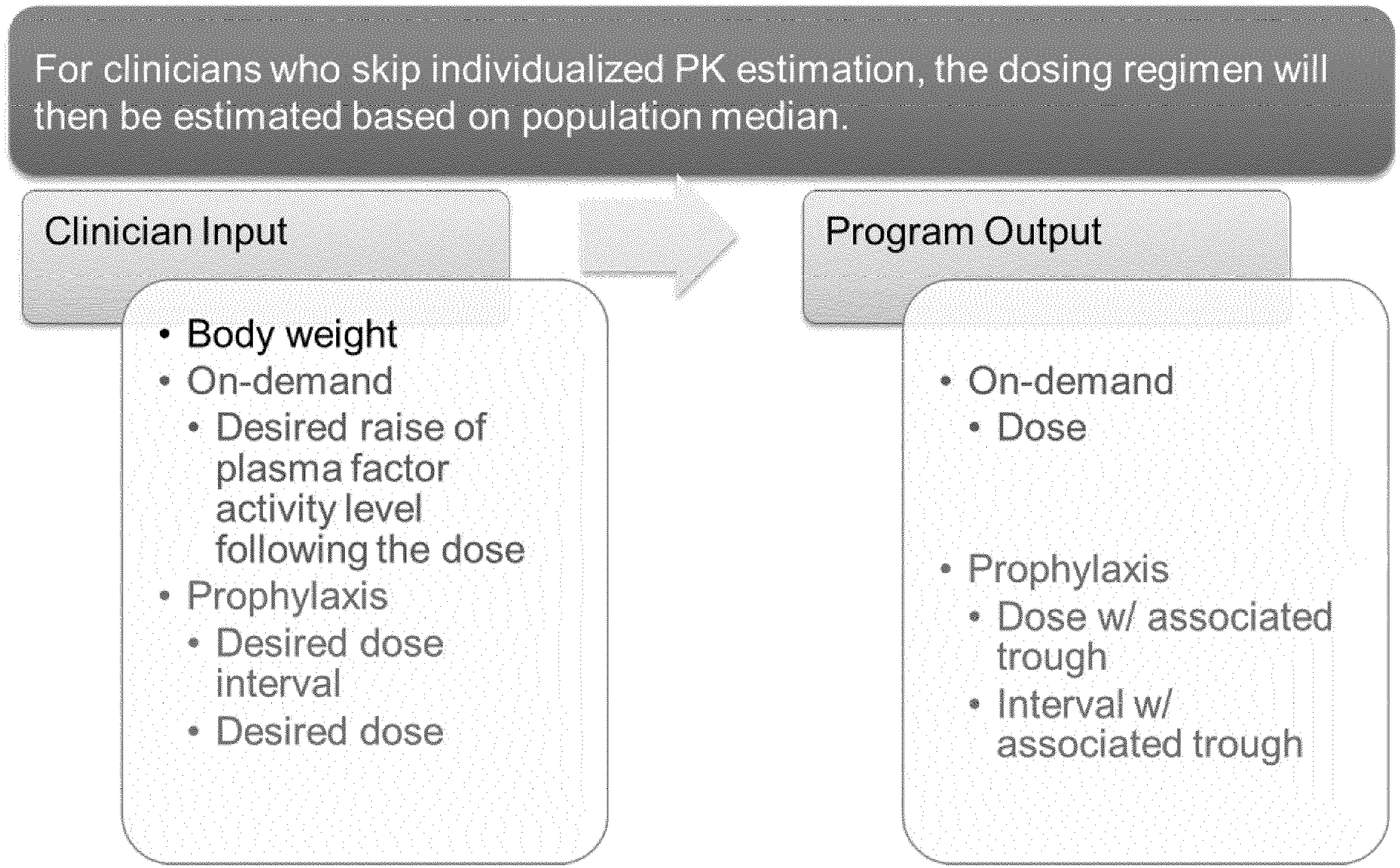

**Figure 17.** Proposed Output for Dosing Regimen Selections without Individualized PK Assessment (2)

Alternatively, for clinicians who skip individualized PK estimation, post product label (see below) without providing the option to estimate other potential doses, intervals, or troughs

| Dose, IU/kg | EOI median [5th, 95th] | Day 1 median [5th, 95th] | Day 3 median [5th, 95th] | Day 5 median [5th, 95th] | Day 7 median [5th, 95th] | Day 10 median [5th, 95th] | Day 14 median [5th, 95th] |
|---|---|---|---|---|---|---|---|
| 50 | 52.6 [32.1, 89.3] | 16.9 [11.2, 26.1] | 7.17 [3.85, 12.3] | 4.16 [1.93, 7.83] | 2.67 [1.02, 5.49] | NA | NA |
| 100 | 102 [60.0, 166] | 30.0 [19.6, 46.7] | 12.0 [6.62, 19.9] | 6.78 [3.24, 12.2] | 4.28 [1.82, 8.06] | 2.29 [0.688, 5.33] | 1.07 [0.0758, 3.23] |

EOI, end of infusion; NA, not applicable.

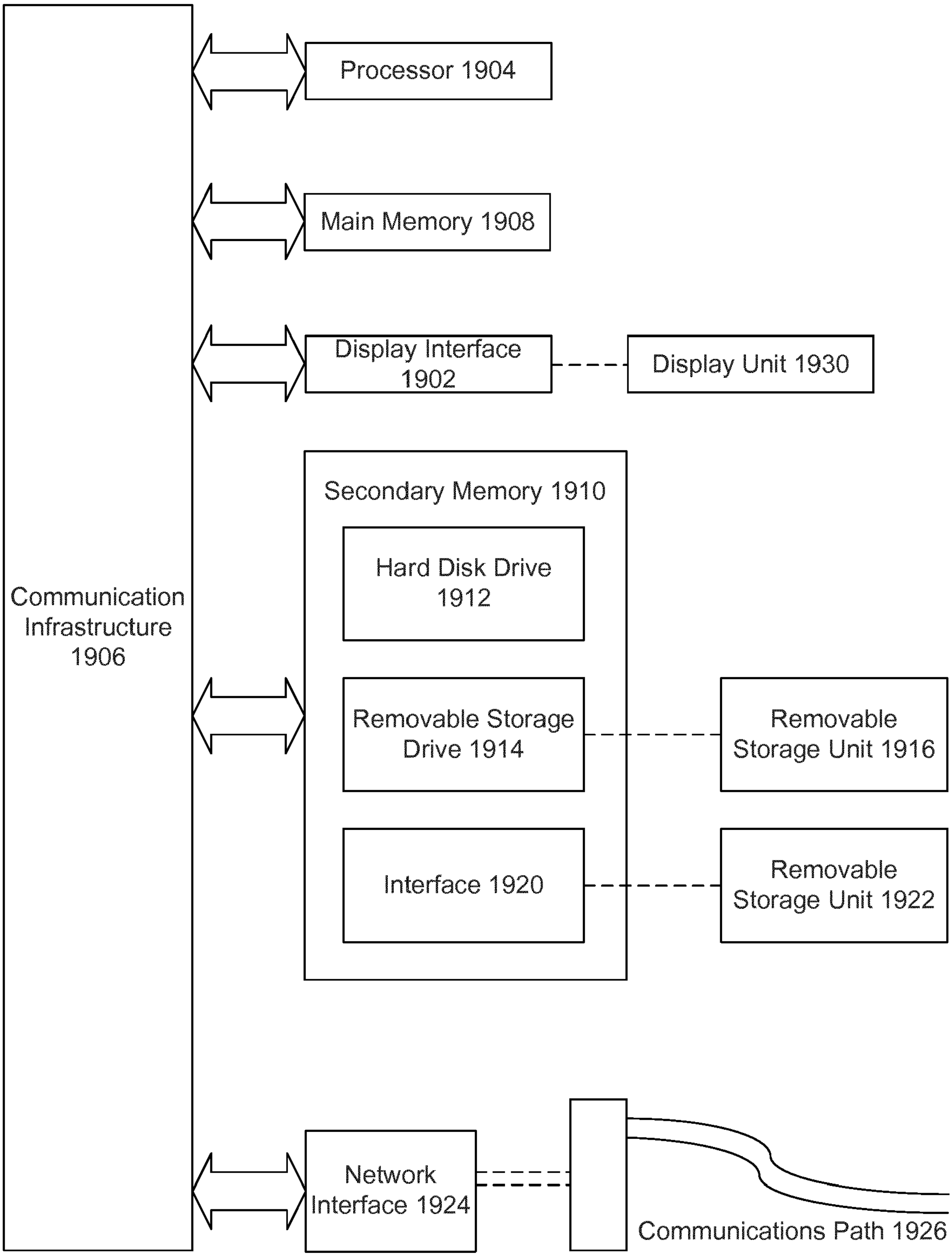
Processor 1904
Main Memory 1908
Display Interface 1902
Display Unit 1930
Secondary Memory 1910
Hard Disk Drive 1912
Communication Infrastructure 1906
Removable Storage Drive 1914
Removable Storage Unit 1916
Interface 1920
Removable Storage Unit 1922
Network Interface 1924
Communications Path 1926

FIG. 18

**Figure 19A-B.** GoF diagnostics of the final model for rFVIIIFc

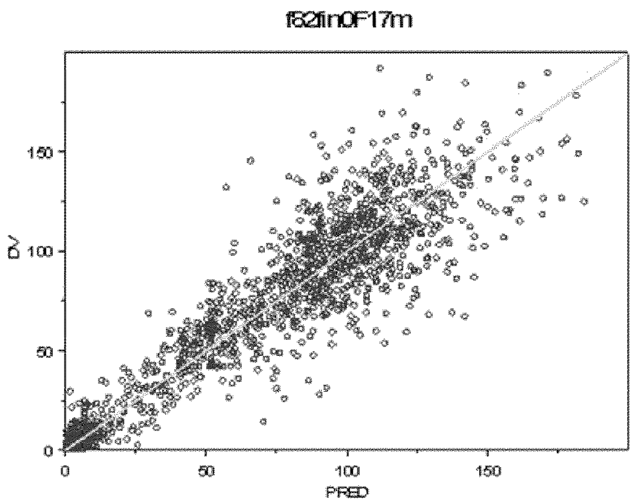


A

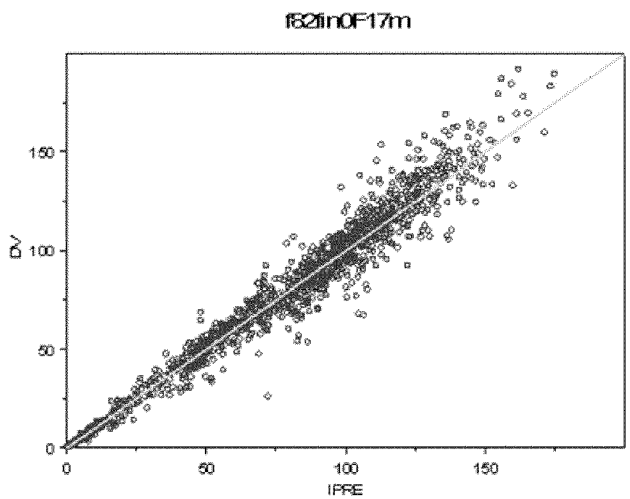


B

**Figure 19C-D.** GoF diagnostics of the final model for rFVIIIFc

Upper panel – Conditional Weighted Residuals (CWRES) vs Predicted (PRED);
Lower panel – Conditional Weighted Residuals (CWRES) vs Time (TIME).

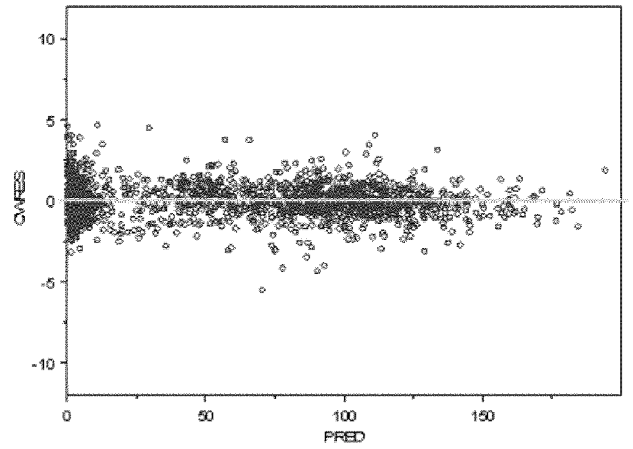


C

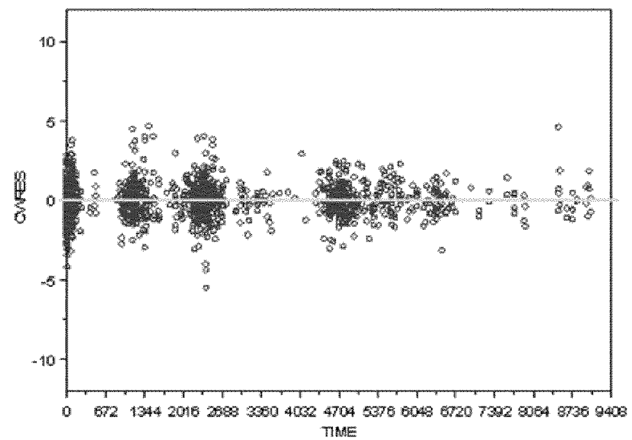


D

**Figure 20:** VPC results (Baseline PK profiles) of the final model for rFVIIIFc

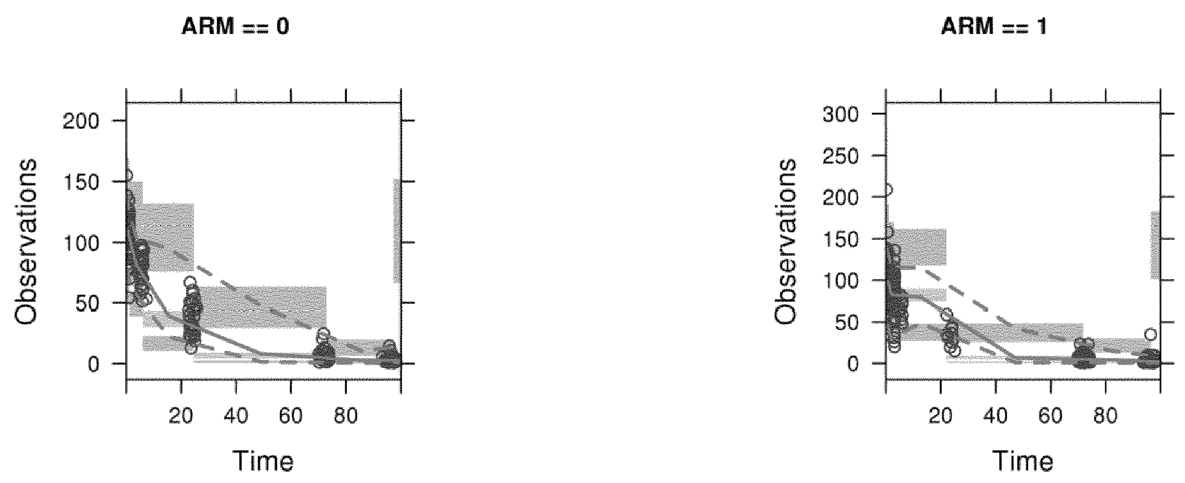

**Figure 21:** External validation of the rFVIIIFc model – Observed (DV) vs Individual Predicted (IPRE) values from the validation set only.

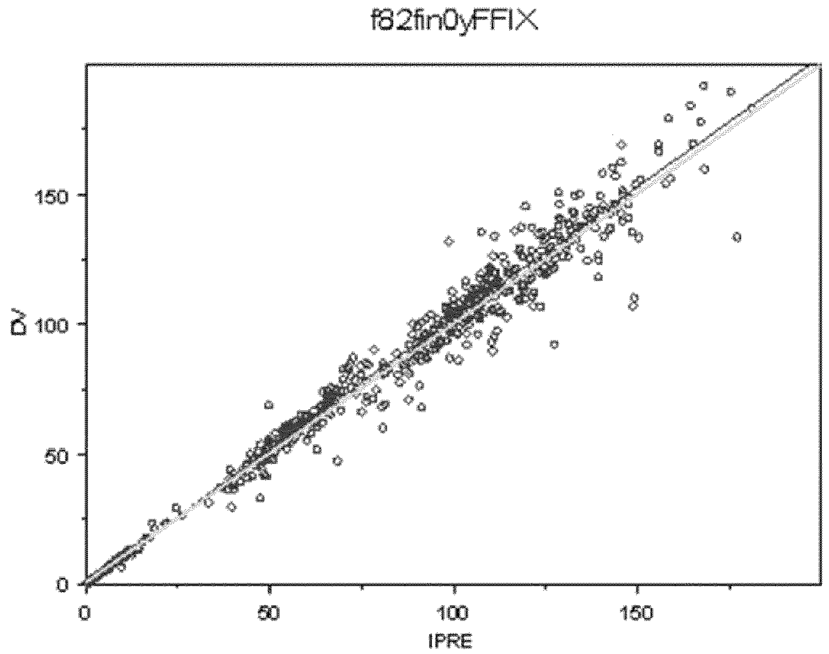

**Figure 22:** GoF diagnostics of the base model for Advate

Upper panel – Observed (DV) vs Individual Predicted (IPRE); Lower panel – Conditional Weighted Residuals (CWRES) vs Predicted (PRED)

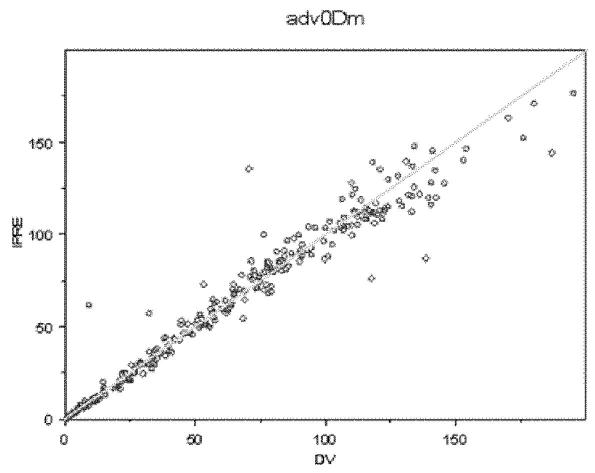


A

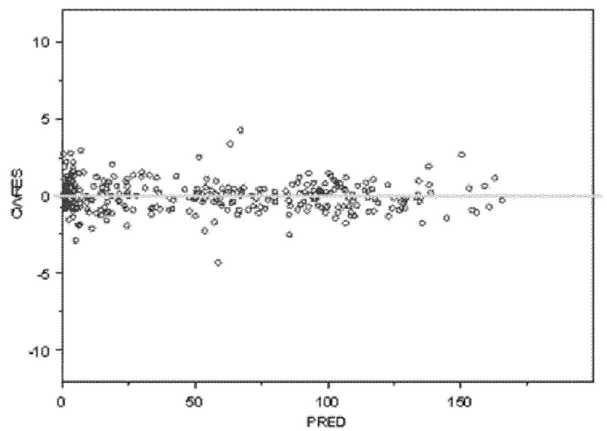


B

**Figure 23:** Advate models comparison

Lower line – current model; upper line – model, reported by Bjorkman *et al*.
Superimposed are the Advate activity data from the Phase 3 study dataset.

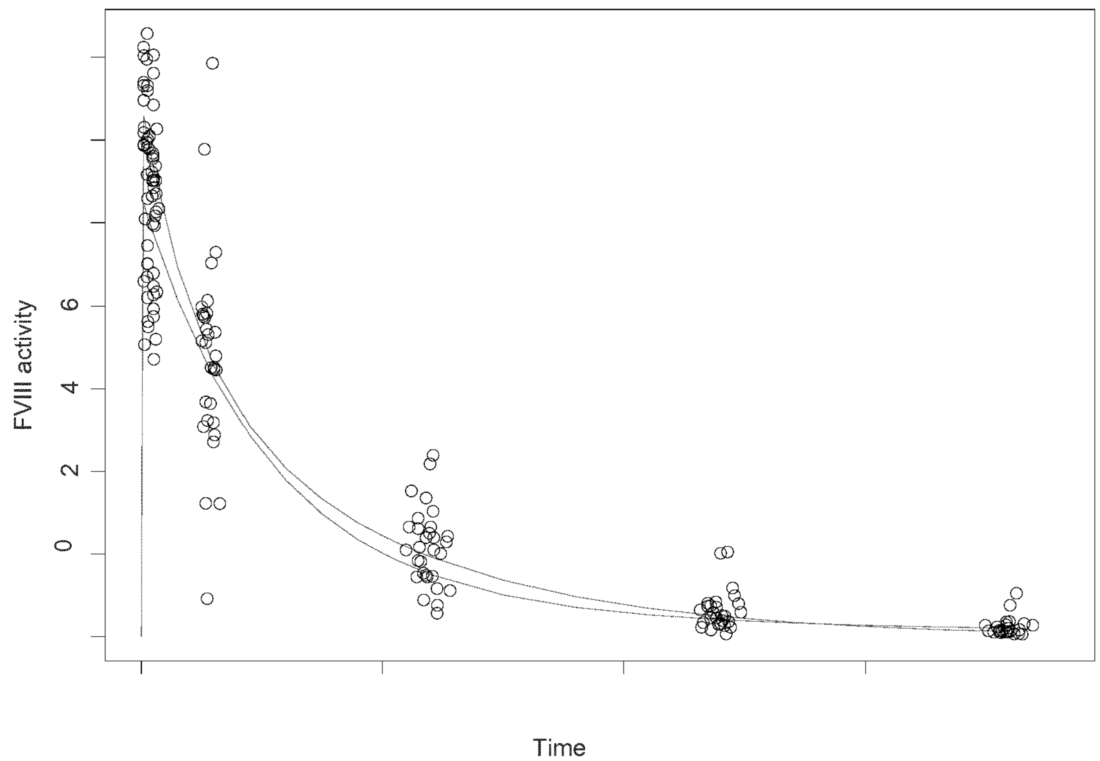

**Figure 24A-B.** Steady State activity profiles of selected rFVIII prophylaxis dosing regimens.
Upper panel – 50 IU/dL E3D; Lower panel - 50 IU/dL E4D

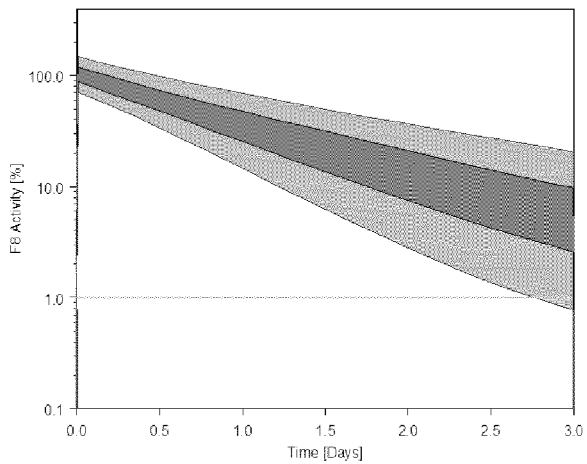


A

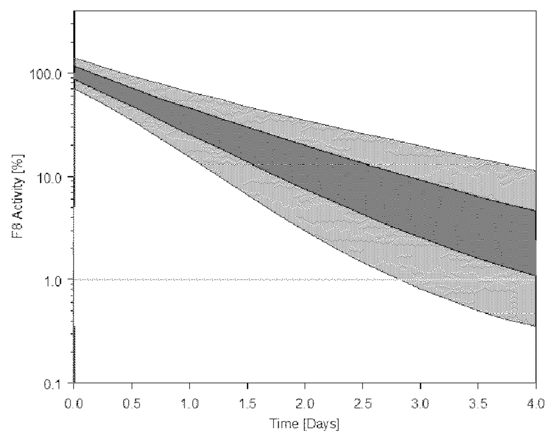


B

**Figure 24C-D:** Steady State activity profiles of selected rFVIII prophylaxis dosing regimens.

Upper panel – 50 IU/dL E5D; Lower panel - 65 IU/dL QW

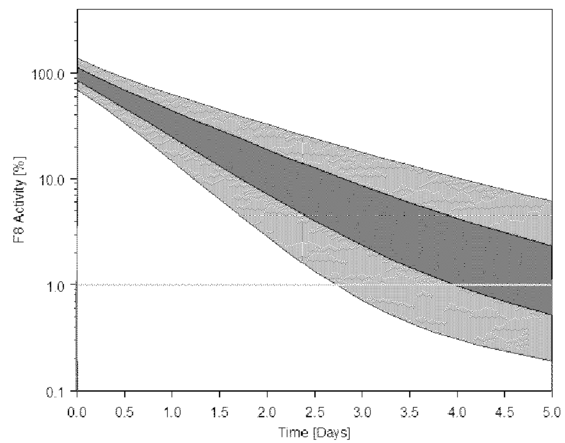


C

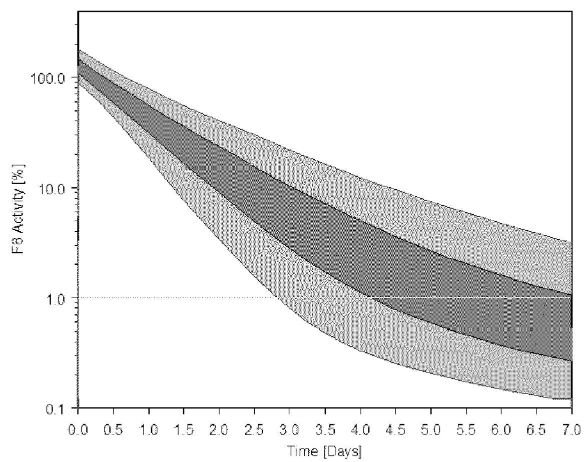


D

**Figure 25A:** Comparison of Individual Bayesian parameter estimates for Advate and rFVIIIFc.

Clearance - Each point represents one individual. Both agents were administered at a nominal dose of 50 IU/kg.

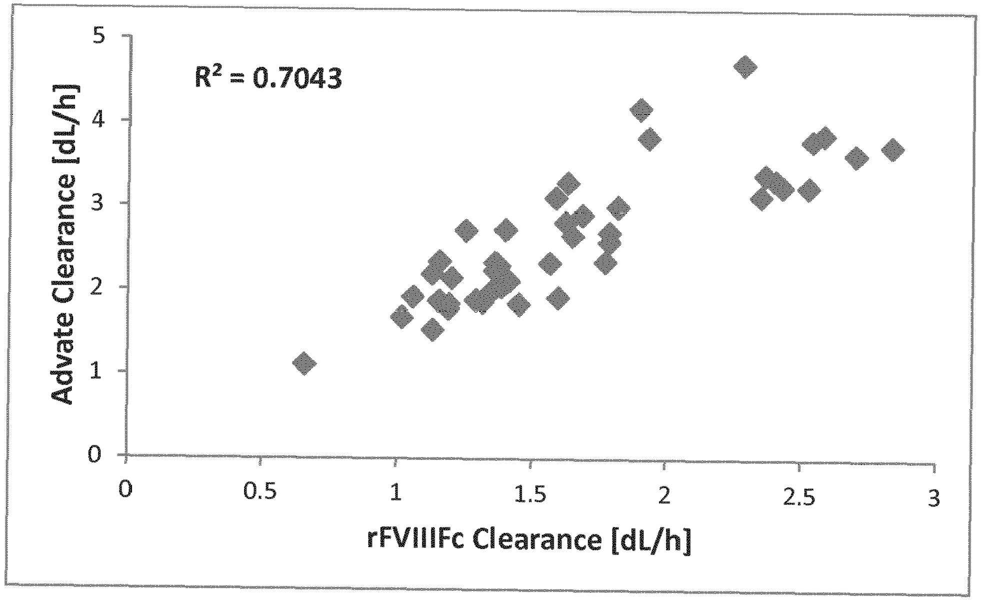

**Figure 25B:** Comparison of Individual Bayesian parameter estimates for Advate and rFVIIIFc.

Central Volume, each point represents one individual.

The center line is the identity line. Both agents were administered at a nominal dose of 50 IU/kg.

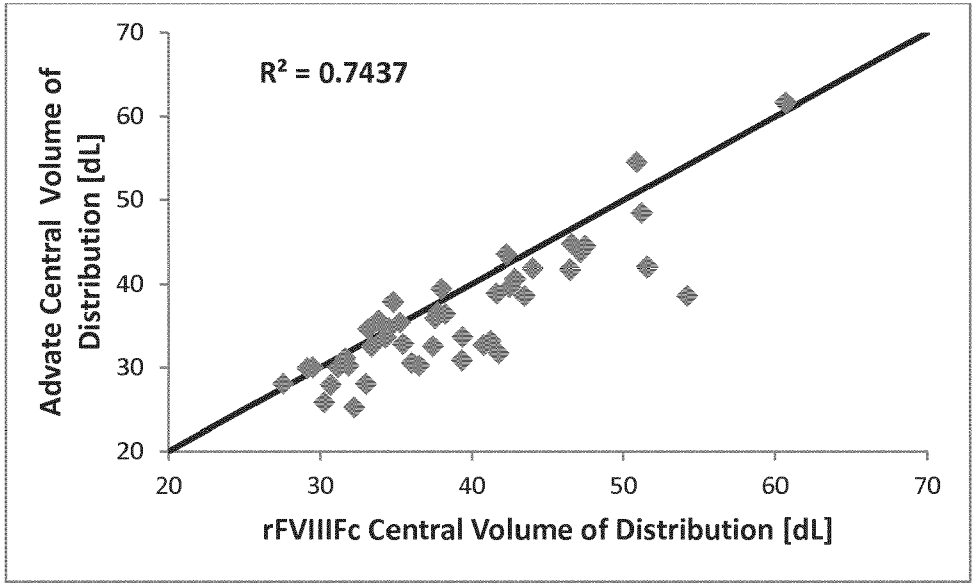

**Figure 25C:** Comparison of Individual Bayesian parameter estimates for Advate and rFVIIIFc

Time to 1 IU/dL (Time 1%), each point represents one individual.

The center line is the identity line. Both agents were administered at a nominal dose of 50 IU/kg.

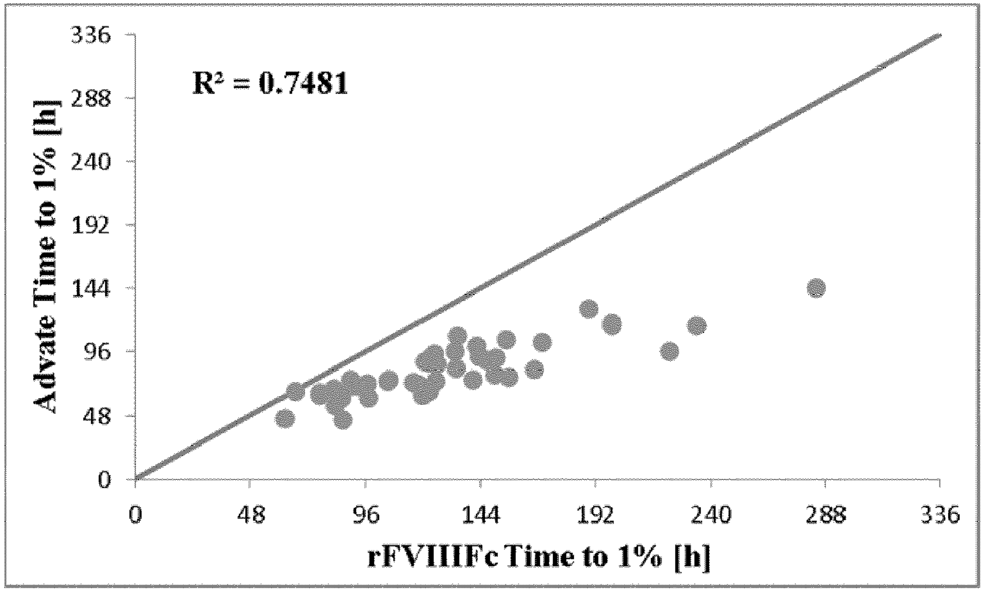

Europäisches Patentamt
European Patent Office
Office européen des brevets

# EUROPEAN SEARCH REPORT

Application Number
EP 18 17 4446

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/069164 A2 (BIOGEN IDEC INC [US]; DUMONT JENNIFER A [US]; LOW SUSAN [US]; BITONTI) 9 June 2011 (2011-06-09)<br>* paragraphs [0070], [0090], [0091] *<br>* examples 1, 6, 8 *<br>----- | 1-15 | INV.<br>A61K38/37 |
| X | POWELL JERRY S ET AL: "Safety and prolonged activity of recombinant factor VIII Fc fusion protein in hemophilia A patients.",<br>29 March 2012 (2012-03-29), BLOOD 29 MAR 2012, VOL. 119, NR. 13, PAGE(S) 3031 - 3037, XP002727554,<br>ISSN: 1528-0020<br>* page 3032, column 2, paragraph 2 *<br>----- | 1-15 | |
| X | J. A. DUMONT ET AL: "Prolonged activity of a recombinant factor VIII-Fc fusion protein in hemophilia A mice and dogs",<br>BLOOD,<br>vol. 119, no. 13,<br>29 March 2012 (2012-03-29), pages 3024-3030, XP55065095,<br>ISSN: 0006-4971, DOI: 10.1182/blood-2011-08-367813<br>* the whole document *<br>----- | 1-15 | TECHNICAL FIELDS SEARCHED (IPC)<br>A61K<br>A61P |
| X,P | WO 2013/106789 A1 (BIOGEN IDEC INC [US]; PUGET SOUND BLOOD CT [US])<br>18 July 2013 (2013-07-18)<br>* example 6 *<br>-----<br>-/-- | 1-15 | |

The present search report has been drawn up for all claims

2

| Place of search | Date of completion of the search | Examiner |
|---|---|---|
| Munich | 15 January 2019 | Schnack, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt / European Patent Office / Office européen des brevets

# EUROPEAN SEARCH REPORT

Application Number
EP 18 17 4446

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | MAHLANGU JOHNNY ET AL: "Phase 3 study of recombinant factor VIII Fc fusion protein in severe hemophilia A", BLOOD, AMERICAN SOCIETY OF HEMATOLOGY, US, vol. 123, no. 3, 13 November 2013 (2013-11-13), pages 317-325, XP009179251, ISSN: 0006-4971, DOI: 10.1182/BLOOD-2013-10-529974 * the whole document * | | |
| X,P | WO 2013/009627 A2 (BIOGEN IDEC HEMOPHILIA INC [US]; DUMONT JENNIFER A [US]; LOW SUSAN [US) 17 January 2013 (2013-01-17) * example 10 * | 1-15 | |
| T | C. CHAI-ADISAKSOPHA ET AL: "A systematic review of definitions and reporting of bleeding outcome measures in haemophilia", HAEMOPHILIA, vol. 21, no. 6, 14 November 2015 (2015-11-14), pages 731-735, XP055342338, GB ISSN: 1351-8216, DOI: 10.1111/hae.12750 * page 733 * | | TECHNICAL FIELDS SEARCHED (IPC) |
| X | U.S. National Library Of Medicine: "Archive History for NCT01458106", , 25 October 2012 (2012-10-25), XP055541953, Retrieved from the Internet: URL:https://clinicaltrials.gov/ct2/history/NCT01458106?V_7=View#StudyPageTop [retrieved on 2019-01-15] * the whole document * | 1-15 | |

-/--

The present search report has been drawn up for all claims

2

| Place of search | Date of completion of the search | Examiner |
|---|---|---|
| Munich | 15 January 2019 | Schnack, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 17 4446

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | G. YOUNG ET AL: "Recombinant factor VIII Fc fusion protein for the prevention and treatment of bleeding in children with severe hemophilia A", JOURNAL OF THROMBOSIS AND HAEMOSTASIS, vol. 13, no. 6, 23 April 2015 (2015-04-23), pages 967-977, XP55460215, GB ISSN: 1538-7933, DOI: 10.1111/jth.12911 * the whole document * ----- | | |

TECHNICAL FIELDS SEARCHED (IPC)

The present search report has been drawn up for all claims

2

| Place of search | Date of completion of the search | Examiner |
|---|---|---|
| Munich | 15 January 2019 | Schnack, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 18 17 4446

This annex lists the patent family members relating to the patent documents cited in the above-mentioned European search report. The members are as contained in the European Patent Office EDP file on
The European Patent Office is in no way liable for these particulars which are merely given for the purpose of information.

15-01-2019

| Patent document cited in search report | Publication date | Patent family member(s) | Publication date |
|---|---|---|---|
| WO 2011069164 A2 | 09-06-2011 | AU 2010325787 A8 | 19-07-2012 |
| | | AU 2016213822 A1 | 01-09-2016 |
| | | AU 2017268648 A1 | 21-12-2017 |
| | | BR 112012013502 A2 | 10-01-2017 |
| | | CA 2782424 A1 | 09-06-2011 |
| | | CN 102791285 A | 21-11-2012 |
| | | CO 6561782 A2 | 15-11-2012 |
| | | CY 1119919 T1 | 27-06-2018 |
| | | DK 2506868 T3 | 29-01-2018 |
| | | EA 201290443 A1 | 28-06-2013 |
| | | EA 201691109 A1 | 30-01-2017 |
| | | EP 2506868 A2 | 10-10-2012 |
| | | EP 3326643 A1 | 30-05-2018 |
| | | ES 2659888 T3 | 19-03-2018 |
| | | HR P20180135 T1 | 06-04-2018 |
| | | HU E036233 T2 | 28-06-2018 |
| | | IL 220204 A | 30-08-2018 |
| | | JP 5903048 B2 | 13-04-2016 |
| | | JP 6062459 B2 | 18-01-2017 |
| | | JP 6385410 B2 | 05-09-2018 |
| | | JP 2013512678 A | 18-04-2013 |
| | | JP 2015083608 A | 30-04-2015 |
| | | JP 2017025098 A | 02-02-2017 |
| | | JP 2018024706 A | 15-02-2018 |
| | | KR 20120129882 A | 28-11-2012 |
| | | LT 2506868 T | 26-02-2018 |
| | | ME 02964 B | 20-07-2018 |
| | | MX 336830 B | 03-02-2016 |
| | | MX 353233 B | 08-01-2018 |
| | | MY 159135 A | 15-12-2016 |
| | | NZ 600709 A | 30-01-2015 |
| | | NZ 703153 A | 27-02-2015 |
| | | PL 2506868 T3 | 29-06-2018 |
| | | PT 2506868 T | 23-02-2018 |
| | | SG 181130 A1 | 30-07-2012 |
| | | SG 10201408049S A | 27-02-2015 |
| | | SI 2506868 T1 | 30-04-2018 |
| | | US 2013108629 A1 | 02-05-2013 |
| | | US 2013274194 A1 | 17-10-2013 |
| | | US 2016199455 A1 | 14-07-2016 |
| | | WO 2011069164 A2 | 09-06-2011 |
| | | ZA 201204874 B | 29-06-2016 |
| WO 2013106789 A1 | 18-07-2013 | AU 2013204237 A1 | 01-08-2013 |
| | | AU 2016210768 A1 | 25-08-2016 |
| | | AU 2018267631 A1 | 13-12-2018 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 18 17 4446

This annex lists the patent family members relating to the patent documents cited in the above-mentioned European search report.
The members are as contained in the European Patent Office EDP file on
The European Patent Office is in no way liable for these particulars which are merely given for the purpose of information.

15-01-2019

| Patent document cited in search report | Publication date | Patent family member(s) | Publication date |
|---|---|---|---|
| | | BR 112014017111 A2 | 13-06-2017 |
| | | CA 2863329 A1 | 18-07-2013 |
| | | CN 104254614 A | 31-12-2014 |
| | | CN 108465106 A | 31-08-2018 |
| | | DK 2802668 T3 | 19-11-2018 |
| | | EA 201491211 A1 | 29-05-2015 |
| | | EA 201790221 A1 | 31-10-2017 |
| | | EP 2802668 A1 | 19-11-2014 |
| | | HR P20181714 T1 | 28-12-2018 |
| | | JP 6234378 B2 | 22-11-2017 |
| | | JP 2015504898 A | 16-02-2015 |
| | | JP 2017149784 A | 31-08-2017 |
| | | LT 2802668 T | 26-11-2018 |
| | | NZ 627078 A | 29-07-2016 |
| | | US 2014370035 A1 | 18-12-2014 |
| | | WO 2013106789 A1 | 18-07-2013 |
| WO 2013009627 A2 | 17-01-2013 | AR 087091 A1 | 12-02-2014 |
| | | AU 2012282875 A1 | 02-05-2013 |
| | | AU 2016204986 A1 | 04-08-2016 |
| | | AU 2018202936 A1 | 17-05-2018 |
| | | BR 112014000466 A2 | 21-02-2017 |
| | | CA 2841066 A1 | 17-01-2013 |
| | | CL 2014000024 A1 | 21-11-2014 |
| | | CN 103796670 A | 14-05-2014 |
| | | CN 107261122 A | 20-10-2017 |
| | | CO 6940381 A2 | 09-05-2014 |
| | | EA 201490039 A1 | 30-06-2014 |
| | | EP 2729161 A2 | 14-05-2014 |
| | | JP 2014522838 A | 08-09-2014 |
| | | JP 2017088623 A | 25-05-2017 |
| | | KR 20140066157 A | 30-05-2014 |
| | | KR 20180020305 A | 27-02-2018 |
| | | MX 350581 B | 11-09-2017 |
| | | NZ 619438 A | 24-06-2016 |
| | | TW 201315480 A | 16-04-2013 |
| | | TW 201822788 A | 01-07-2018 |
| | | US 2014294821 A1 | 02-10-2014 |
| | | US 2018360982 A1 | 20-12-2018 |
| | | WO 2013009627 A2 | 17-01-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004101740 A
- WO 2006074199 A
- US 7404956 B
- US 7348004 B
- US 6316226 B
- US 6346513 B
- US 7041635 B
- US 5789203 A
- US 6060447 A
- US 5595886 A
- US 6228620 B
- US 5972885 A
- US 6048720 A
- US 5543502 A
- US 5610278 A
- US 5171844 A
- US 5112950 A
- US 4868112 A
- US 6458563 B
- WO 9109122 A
- WO 8704187 A, Kaufman
- WO 8800831 A, Pasek
- EP 295597 A
- US 20100286067 A
- US 20120093840 A
- US 7592010 B
- US 6686179 B
- US 5712122 A
- US 20090087411 A1
- US 20100292130 A1
- WO 2008155134A1 A
- EP 2173890 A
- EP 0401384 A
- EP 0154316 A
- WO 9216221 A
- WO 9534326 A
- US 7632921 B
- US 6251632 B
- WO 8808035 A
- WO 8803558 A
- US 4757006 A
- US 4965199 A, Capon
- US 4994371 A
- US 5004803 A
- US 20050100990 A
- US 5859204 A, Lollar, J. S.
- US 6376463 B, Lollar, J. S.
- US 5364771 A, Lollar and Runge
- WO 9320093 A
- WO 9411503 A
- US 20090264627 A1
- US 20090163699 A1


### Non-patent literature cited in the description


- **PEYVANDI, F. et al.** *Haemophilia,* 2006, vol. 12, 82-89
- **RODRIGUEZ-MERCHAN, E.C.** *Semin. Thromb. Hemost.,* 2003, vol. 29, 87-96
- **LOLLAR, P. ; E. T. PARKER.** *J. Biol. Chem.,* 1991, vol. 266, 12481-12486
- **LOLLAR, P. et al.** *J. Biol. Chem.,* 1992, vol. 267, 23652-23657
- **MANNUCCI, P.M. et al.** *N. Engl. J. Med.,* 2001, vol. 344, 1773-1779
- Concise Dictionary of Biomedicine and Molecular Biology, Juo, Pei-Show. CRC Press, 2002
- The Dictionary of Cell and Molecular Biology. Academic Press, 1999
- Oxford Dictionary Of Biochemistry And Molecular Biology. Oxford University Press, 2000
- **MANNUCCI, P.M. et al.** *N. Engl. J. Med.,* 2001, vol. 344, 1773-9
- **HOEBEN R.C. et al.** *J. Biol. Chem.,* 1990, vol. 265 (13), 7318-7323
- **MEULIEN P. et al.** *Protein Eng.,* 1988, vol. 2 (4), 301-6
- **TOOLE et al.** *Proc. Natl. Acad. Sci. U.S.A.,* 1986, vol. 83, 5939-5942
- **EATON et al.** *Biochemistry,* 1986, vol. 25, 8343-8347
- **SARVER et al.** *DNA,* 1987, vol. 6, 553-564
- **LAGNER.** *Behring Inst. Mitt.,* 1988, vol. 82, 16-25
- **FATOUROS et al.** *Intern. J. Pharm.,* 1997, vol. 155 (1), 121-131
- **WAKABAYASHI et al.** *JBC,* 2004, vol. 279 (13), 12677-12684
- **SCHULTE.** *Thrombosis Res.,* 2009, vol. 124 (2), S6-S8
- **KRAULIS et al.** *FEBS Lett.,* 1996, vol. 378, 190-194

- **LINHULT et al.** *Protein Sci.,* 2002, vol. 11, 206-213
- **DENNIS et al.** *J. Biol. Chem. 2002,* 2002, vol. 277, 35035-35043
- **MULLER ; KONTERMANN.** *Curr. Opin. Mol. Ther.,* 2007, vol. 9, 319-326
- **ROOVERSET et al.** *Cancer Immunol. Immunother.,* 2007, vol. 56, 303-317
- **HOLT et al.** *Prot. Eng. Design Sci.,* 2008, vol. 21, 283-288
- **TRUSSELET et al.** *Bioconjugate Chem.,* 2009, vol. 20, 2286-2292
- **LI et al.** *Trends Pharmacol. Sci.,* 2002, vol. 23, 206-209
- **BRANDSMA et al.** *Biotechnol. Adv.,* 2011, vol. 29, 230-238
- **BAI et al.** *Proc. Natl. Acad. Sci. USA,* 2005, vol. 102, 7292-7296
- **KIM et al.** *J. Pharmacol. Exp. Ther.,* 2010, vol. 334, 682-692
- **WANG et al.** *J. Controlled Release,* 2011, vol. 155, 386-392
- **MALIK F et al.** *Exp. Hematol.,* 1992, vol. 20, 1028-35
- **FRANCIS.** *Focus on Growth Factors,* 1992, vol. 3 (2), 4-10
- **MEI et al.** *Blood,* 2010, vol. 116, 270-279
- **SOMMERMEYER et al.** *Krankenhauspharmazie,* 1987, vol. 8, 271-278
- **WEIDLER et al.** *Arzneim.-Forschung/Drug Res.,* 1991, vol. 41, 494-498
- **CUTLER et al.** *Hum. Mutat.,* 2002, vol. 19, 274-8
- **CAMERON et al.** *Thromb. Haemost.,* 1998, vol. 79, 317-22
- **TOOLE, J. J. et al.** *Nature,* 1984, vol. 312, 342-347
- **GITSCHIER, J. et al.** *Nature,* 1984, vol. 312, 326-330
- **WOOD, W. I. et al.** *Nature,* 1984, vol. 312, 330-337
- **VEHAR, G. A. et al.** *Nature,* 1984, vol. 312, 337-342
- **TOOLE, J. J. et al.** *Proc. Natl. Acad. Sci. USA,* 1986, vol. 83, 5939-5942
- **HEALEY, J. F. et al.** *Blood,* 1996, vol. 88, 4209-4214
- **BURMEISTER et al.** *Nature,* 1994, vol. 372, 379
- **KABAT et al.** Sequences of Proteins of Immunological Interest. U. S. Department of Public Health, 1991
- **STORY et al.** *J. Exp. Med.,* 1994, vol. 180, 2377
- **OGANESYAN et al.** *Mol. Immunol.,* 2009, vol. 46, 1750
- **VACCARO et al.** *Nat. Biotechnol.,* 2005, vol. 23, 1283
- **ROUTLEDGE et al.** *Transplantation,* 1995, vol. 60, 847
- **FRIEND et al.** *Transplantation,* 1999, vol. 68, 1632
- **SHIELDS et al.** *J. Biol. Chem.,* 1995, vol. 276, 6591
- **WARD ; GHETIE.** *Therapeutic Immunology,* 1995, vol. 2, 77
- **ARMOUR et al.** *Eur. J. Immunol.,* 1999, vol. 29, 2613
- **SHIELDS et al.** *J. Biol. Chem.,* 2001, vol. 276, 6591
- **BRUTLAG et al.** *Comp. App. Biosci.,* 1990, vol. 6, 237-245
- Genes II. John Wiley & Sons, 1985
- **RON et al.** *J. Biol. Chem.,* 1993, vol. 268, 2984-2988
- **DOBELI et al.** *J. Biotechnology,* 1988, vol. 7, 199-216
- **GAYLE.** *J. Biol. Chem,* 1993, vol. 268, 22105-22111
- **MEI et al.** *Blood,* 2010, vol. 116, 270-79
- **SAMBROOK et al.** Molecular Cloning: A Laboratory Manual. Cold Spring Harbor Laboratory Press, 1989
- **WIGLER et al.** *Cell,* 1978, vol. 14, 725
- **NEUMANN et al.** *EMBO J,* 1982, vol. 1, 841
- **MEI et al.** *Mol. Biotechnol.,* 2006, vol. 34 (2), 165-78
- **KIANG et al.** *Clin. Pharmacokinet,* 2012, vol. 51, 515-525
- **POWELL J S ; JOSEPHSON N.C ; QUON D et al.** Safety and prolonged activity of recombinant factor VIII Fc fusion protein in hemophilia A patients. *Blood,* 2012, vol. 119 (13), 3031-7
- **MAHLANGU J. et al.** Phase 3 study of recombinant factor VIII Fc fusion protein in haemophilia A. *Lancet,* 2013
- **BJÖRKMAN S, C.M. ; BERNTOP E.** Pharmacokinetics of Factor IX in patients with hemophilia B. *Eur J Clin Pharmacol,* 1994, vol. 46, 325-332
- **CARLSSON M, B.S. ; BERNTOP E.** Multidose pharmacokinetics of factor IX: Implications for dosing in prophylaxis. *Hemophilia,* 1998, vol. 4, 83-88
- **BJÖRKMAN S, S.A. ; BERNTOP E.** Pharmacokinetics of recombinant factor IX in relation to age of the patients: Implications for dosing in prophylaxis. *Hemophilia,* 2001, vol. 7, 133-139
- **BJÖRKMAN S, O.M. ; SPOTTS G et al.** Population pharmacokinetics of recombinant factor VIII- the relationships of pharmacokinetics to age and body weight. *Blood,* 2012, vol. 119, 612-618
- **BJÖRKMAN S, A.V.** Population pharmacokinetics of plasma-derived factpr IX in adult patients with hemophilia B: Implications for dosing in prophylaxis. *Eur J Clin Pharmacol,* 2012, vol. 68 (6), 969-77

- **BEAL, S.** Ways to fit a PK model with some data below the quantification limit. *J Pharmacokin Pharmacodyn,* 2001, vol. 28, 481-504
- **JAE EUN AHN, M.O.K. ; ADRIAN DUNNE ; THOMAS M. LUDDEN.** Likelihood based approaches to handling data below the quantification limit using NONMEM VI. *J Pharmacokin Pharmacodyn,* 2008, vol. 35 (4), 401-21
- **COLLINS PW, B.V. ; FISCHER K et al.** Break-through bleeding in relation to predicted factor VIII levels in patients receiving prophylactic treatment for severe hemophilia A. *J Thromb Haemost.,* 2009, vol. 7 (3), 413-20
- Recommendation #190 Concerning Products Licensed for the Treatment of Hemophilia and Other Bleeding Disorders. MASAC. National Hemophilia Foundation, March 2009
- **LILLICRAP D.** Extending half-life in coagulation factors: where do we stand?. *Thromb Res.,* 2008, vol. 122 (4), 2-8